# EUROPEAN PATENT SPECIFICATION

(11) **EP 3 274 453 B1**
(45) Date of publication and mention of the grant of the patent: **27.01.2021**
(21) Application number: 16766104.0
(22) Date of filing: 25.03.2016
(51) Int. Cl.: C12N 15/10, C12N 15/55, C12N 9/22, A61K 38/46, A61K 48/00

(54) **CRISPR/CAS-MEDIATED GENE CONVERSION**
CRISPR/CAS-VERMITTELTE GENUMWANDLUNG
CONVERSION GÉNIQUE VIA CRISPR/CAS

(30) Priority: 26.03.2015 US 201562138948 P; 19.06.2015 US 201562182416 P; 17.07.2015 US 201562194078 P; 18.09.2015 US 201562220660 P; 25.09.2015 US 201562232683 P; 25.09.2015 US 201562232675 P; 09.11.2015 WO PCT/US2015/059782
(43) Date of publication of application: 31.01.2018
(73) Proprietor: Editas Medicine, Inc., Cambridge, MA 02142 (US)
(72) Inventor: COTTA-RAMUSINO, Cecilia, Cambridge, MA 02139 (US); GORI, Jennifer, Leah, Jamaica Plain, MA 02130 (US)
(74) Representative: Lock, Graham James
(86) International application number: PCT/US2016/024307
(87) International publication number: WO 2016/154579

(56) References cited:
- WO-A1-2014/065596
- WO-A1-2014/191518
- WO-A2-2015/006498
- WO-A2-2015/048577
- WO-A2-2015/148863
- Jennifer Hollywood: "Cystic fibrosis gene repair: correction of [Delta]F508 using ZFN and CRISPR/Cas9 guide RNA gene editing tools", , 1 January 2013 (2013-01-01), pages 1-193, XP055209264, Retrieved from the Internet: URL:https://cora.ucc.ie/bitstream/handle/1 0468/1407/JH thesis.pdf?sequence=3 [retrieved on 2015-08-24]
- RAN F ANN ET AL: "Double Nicking by RNA-Guided CRISPR Cas9 for Enhanced Genome Editing Specificity", CELL, vol. 154, no. 6, 12 September 2013 (2013-09-12), pages 1380-1389, XP055299681, ISSN: 0092-8674, DOI: 10.1016/J.CELL.2013.08.021
- TREVINO ALEXANDRO E ET AL: "Genome editing using Cas9 nickases", METHODS IN ENZYMOLOGY, ACADEM. PRESS, USA, vol. 546, 12 November 2014 (2014-11-12), pages 161-174, XP009187160, ISSN: 1557-7988, DOI: 10.1016/B978-0-12-801185-0.00008-8
- RAN F ANN ET AL: "Genome engineering using the CRISPR-Cas9 system", NATURE PROTOCOLS, NATURE PUBLISHING GROUP, GB, vol. 8, no. 11, 1 November 2013 (2013-11-01), pages 2281-2308, XP009174668, ISSN: 1750-2799
- S. W. CHO ET AL: "Analysis of off-target effects of CRISPR/Cas-derived RNA-guided endonucleases and nickases", GENOME RESEARCH, vol. 24, no. 1, 5 November 2015 (2015-11-05), pages 132-141, XP055227885, ISSN: 1088-9051, DOI: 10.1101/gr.162339.113
- L. CONG ET AL: "Multiplex Genome Engineering Using CRISPR/Cas Systems", SCIENCE, vol. 339, no. 6121, 15 February 2013 (2013-02-15), pages 819-823, XP055067741, ISSN: 0036-8075, DOI: 10.1126/science.1231143 cited in the application -& L. CONG ET AL: "Supplementary Material to: Multiplex Genome Engineering Using CRISPR/Cas Systems", SCIENCE, vol. 339, no. 6121, 3 January 2013 (2013-01-03), pages 819-823, XP055153963, ISSN: 0036-8075, DOI: 10.1126/science.1231143
- P. MALI ET AL: "RNA-Guided Human Genome Engineering via Cas9", SCIENCE, vol. 339, no. 6121, 15 February 2013 (2013-02-15), pages 823-826, XP055300091, ISSN: 0036-8075, DOI: 10.1126/science.1232033 cited in the application
- PRASHANT MALI ET AL: "CAS9 transcriptional activators for target specificity screening and paired nickases for cooperative genome engineering", NATURE BIOTECHNOLOGY, vol. 31, no. 9, 1 August 2013 (2013-08-01) , pages 833-838, XP055299678, ISSN: 1087-0156, DOI: 10.1038/nbt.2675 -& PRASHANT MALI ET AL: "Supplementary Information: CAS9 transcriptional activators for target specificity screening and paired nickases for cooperative genome engineering (Nature Biotechnology Vol. 31:833-838,2013)", NATURE BIOTECHNOLOGY, vol. 31, no. 9, 1 August 2013 (2013-08-01) , pages 1-36, XP055294730, ISSN: 1087-0156, DOI: 10.1038/nbt.2675

## Description

### FIELD OF THE INVENTION

The invention relates to CRISPR/CAS-related methods and components for increasing editing of a target nucleic acid sequence by gene conversion using an endogenous homologous region, and applications thereof.

### BACKGROUND

The CRISPR (Clustered Regularly Interspaced Short Palindromic Repeats)/Cas (CRISPR-associated) system evolved in bacteria and archaea as an adaptive immune system to defend against viral attack. Upon exposure to a virus, short segments of viral DNA are integrated into the CRISPR locus. RNA is transcribed from a portion of the CRISPR locus that includes the viral sequence. That RNA, which contains sequence complimentary to the viral genome, mediates targeting of a Cas9 protein to the sequence in the viral genome. The Cas9 protein cleaves and thereby silences the viral target.

Recently, the CRISPR/Cas system has been adapted for genome editing in eukaryotic cells. The introduction of site-specific double strand breaks (DSBs) enables target nucleic acid alteration. After the formation of a DNA double-stranded break (DSB), the major decision point affecting DNA repair pathway choice is whether or not the DNA ends are endo- and exonucleolytically processed in a process referred to as end resection. When no end resection takes places, the repair pathway engaged to repair the DSB is referred to as classical non-homologous end joining (C-NHEJ). The C-NHEJ repair pathway leads to either perfect repair of the DSBs, in which case the locus is restored without sequence alterations, or to the formation of small insertions and deletions.

In contrast, if the end resection machinery processes the DSB, a 3' overhang is exposed, which engages in homology search. A not yet completely characterized class of pathways that can engage the repair of DSBs after resection is initiated is referred to as alternative non-homologous end joining (ALT-NHEJ). Examples of pathways that are categorized as ALT-NHEJ include blunt end-joining (blunt EJ) and microhomology mediated end joining (MMEJ) leading to deletions, as well as synthesis dependent micro homology mediated end joining (SD-MMEJ), leading to the formation of insertions.

When the end resection is extensive, the exposed 3' overhang can undergo strand invasion of highly homologous sequences, followed by repair of the DSB by a homology-dependent recombination (HDR) pathway, which encompasses gene correction and gene conversion. Gene correction refers to the process of repairing DNA damage by HDR using an exogenous nucleic acid, *e.g.,* an exogenous donor template nucleic acid. Gene conversion is a process whereby a DNA break leads to repair of the sequence in the vicinity of the break by an endogenous homologous sequence, referred to as an endogenous donor template sequence. An endogenous donor template sequence can be derived from a non-sister chromatid sequence or from a heterologous sequence that has a high degree of homology with the sequence undergoing repair. The DNA sequence that is converted can comprise a small portion of a gene *(e.g.,* fewer than 100 bp) or an entire gene sequence *(e.g.,* greater than 5 kb). Gene conversion is a common form of DNA double-strand break repair in both non-mammalian and mammalian, *e.g.,* human, cells.

Jennifer Hollywood: "Cystic fibrosis gene repair: correction of [Delta]F508 using ZFN and CRISPR/Cas9 guide RNA gene editing tools", 1 January 2013, pages 1-193, discloses the use of the CRISPR/Cas9/gRNA system to repair the ΔF508 mutation in a cystic fibrosis cell line by a homology-dependent recombination (HDR) pathway using an exogenous nucleic acid.

Ran F Ann et al: "Double Nicking by RNA-Guided CRISPR Cas9 for Enhanced Genome Editing Specificity", Cell, vol. 154, no. 6, 12 September 2013, pages 1380-1389, and Trevino Alexandro E et al: Genome editing using Cas9 nickases", Methods in Enzymology, vol. 546, 12 November 2014, pages 161-174, disclose methods of modifying a gene using Cas9 nickases with paired gRNAs to introduce double stranded breaks and then contacting the cell with an exogenously introduced single-stranded oligodeoxynucleotide for use as the repair template.

Ran F Ann et al: "Genome engineering using the CRISPR-Cas9 system", Nature Protocols, vol. 8, no. 11, 1 November 2013, pages 2281-2308 discloses the use of the CRISPR/Cas9/gRNA system for genome editing where double-strand breaks at the target locus are repaired via non-homologous end joining and result in deletion formation.

WO 2014/191518 A1 discloses a method for precise nucleic acid cleavage in a genetic sequence in a cell using a Cas9-tracrRNA:crRNA complex to induce a nick event in double-stranded nucleic acid targets and introducing into the cell an exogenous nucleic acid comprising a sequence homologous to a portion of the target nucleic acid sequence.

WO 2014/065596 A1 discloses a method for preparing a eukaryotic cell or organism comprising the CRISPR/Cas9/gRNA system wherein the gRNA is a dual RNA.

Prashant Mali et al: "Cas9 transcriptional activators for target specificity screening and paired nickases for cooperative genome engineering", Nature Biotechnology, vol. 31, no. 9, 1 August 2013, pages 833-838, & Prashant Mali et al: "Supplementary Information: Cas9 transcriptional activators for target specificity screening and paired nickases for cooperative genome engineering", Nature Biotechnology, vol. 31, no. 9, 1 August 2013, pages 1-36, discloses that prokaryotic type II CRISPR-Cas systems can be engineered to enable targeted RNA-guided genome modification in human cells.

WO 2015/006498 A2 discloses a method for altering target severe combined immunodeficiency (SCID)-, sickle cell disease (SCD)- or β-thalassemia-associated polynucleotide sequences in a cell, where target polynucleotide sequences are cleaved resulting in single or double-strand breaks followed by HDR using an exogenously introduced DNA repair template.

While a cell could, in theory, repair DNA breaks via any of a number of DNA damage repair pathways, in certain circumstances it is particularly useful to provide an environment more favorable for repair of a break by gene conversion using an endogenous homologous region as a donor template. However, there remains a need to improve the efficiency of gene conversion-mediated modification in order to broaden the applicability and efficiency of genome editing by CRISPR/Cas systems.

### SUMMARY

The methods and compositions described herein surprisingly increase DNA repair via gene conversion pathways to alter or modify, *e.g.,* repair, sequences, *e.g.,* genes, such as disease causing genes, using an endogenous homologous region which functions as a donor template, following a Cas9 molecule-mediated cleavage event.

In one aspect, the invention relates to an *in vitro* or *ex vivo* method of modifying an endogenous target gene in a cell the method comprising contacting the cell with a first gRNA molecule, a first enzymatically active Cas9 (eaCas9) molecule, a second gRNA molecule, and a second eaCas9 molecule; wherein the first gRNA molecule and the first eaCas9 molecule associate with the target gene and generate a first single strand break on a first strand of the target gene; wherein the second gRNA molecule and the second eaCas9 molecule associate with the target gene and generate a second single strand break on a second strand of the target gene, thereby forming a double strand break having a first overhang and a second overhang; and wherein the first overhang and the second overhang in the target gene are repaired using an endogenous homologous region, wherein the cell is not contacted with an exogenous nucleic acid homologous to the target gene, thereby modifying the endogenous target gene in the cell, wherein the target gene is an *HBB* gene; and wherein the cell is a population of cells, and wherein the first overhang and the second overhang in the target gene are repaired by (a) gene conversion in about 12% to about 45% of the cell in the population of cells; or (b) non-homologous end joining (NHEJ) in less than 40% of the cells in the population of cells.

In one embodiment of the method, after repair of the first overhang and the second overhang, the target gene comprises the sequence of the endogenous homologous region.

In one embodiment of the method, the first overhang and the second overhang are repaired by homology directed repair (HDR); optionally wherein the HDR is gene conversion.

In one embodiment of the method, the first overhang is a 5' overhang, and the second overhang is a 5' overhang; optionally wherein the first overhang and the second overhang undergo processing, exposing a first 3' overhang and a second 3' overhang.

In one embodiment of the method, the method is used to correct a mutation in the endogenous target gene, and wherein the mutation is located (a) between the first single strand break and the second single strand break; and/or (b) within fewer than 50 nucleotides of the first single strand break, or within fewer than 50 nucleotides of the second single strand break.

In one embodiment of the method, the endogenous target gene has at least 80% sequence homology with the endogenous homologous region.

In one embodiment of the method, the method is used to correct a mutation in the endogenous target gene, and wherein the endogenous homologous region does not comprise the mutation.

In one embodiment of the method, the endogenous homologous region comprises at least one of: a control region which is homologous to a control region of the target gene; a coding region which is homologous to a coding region of the target gene; a non-coding region which is homologous to a non-coding region of the target gene; an intron which is homologous to an intron of the target gene; or an exon which is homologous to an exon of the target gene.

In one embodiment of the method, the first eaCas9 molecule is a first nickase molecule and the second eaCas9 molecule is a second nickase molecule; and/or wherein the first eaCas9 molecule and the second eaCas9 molecule are the same species of eaCas9 molecule; optionally wherein (i) the eaCas9 molecule (a) comprises HNH-like domain cleavage activity but has no N-terminal RuvC-like domain cleavage activity; or (b) is an HNH-like domain nickase; and/or (ii) the eaCas9 molecule comprises a mutation at an amino acid position corresponding to amino acid position D10 of *Streptococcus pyogenes* Cas9.

In one embodiment of the method, (a) the first gRNA molecule is a gRNA molecule comprising SEQ ID NO: 387, and wherein the second gRNA molecule is a gRNA molecule comprising SEQ ID NO: 16318; or (b) the first gRNA molecule is a gRNA molecule comprising any one of SEQ ID NOs:387-485, 6803-6871, or 16010-16256, and wherein the second gRNA molecule is a gRNA molecule comprising any one of SEQ ID NOs: 387-485, 6803-6871, or 16010-16256; and/or (c) the cell is taken from a patient having sickle cell disease or beta thalassemia; and/or (d) the endogenous homologous region comprises a region of an endogenous *HDB* gene.

In one embodiment of the method, the NHEJ results in a deletion in the target gene or wherein the NHEJ results in an insertion in the target gene.

In one embodiment of the method, the cell is selected from the group consisting of a blood cell, a neuronal cell, an immune cell, a muscle cell, a stem cell, a progenitor cell, and a diseased cell; and/or wherein the cell is a mammalian cell; and/or a human cell.

In another aspect, the invention is a composition comprising: a first non-naturally occurring gRNA molecule, a first non-naturally occurring enzymatically active Cas9 (eaCas9) molecule, a second non-naturally occurring gRNA molecule, and a second non-naturally occurring eaCas9 molecule; wherein the first gRNA molecule and the first eaCas9 molecule are designed to associate with a target gene and produce a first single strand break on a first strand of the target gene; wherein the second gRNA molecule and the second eaCas9 molecule are designed to associate with the target gene and produce a second single strand break on a second strand of the target gene, thereby forming a double strand break having a first overhang and a second overhang; and wherein the first overhang and the second overhang in the target gene can be repaired by gene conversion using an endogenous homologous region, wherein the target gene is the *HBB* gene; optionally wherein the first non-naturally occurring eaCas9 molecule is an HNH-like domain nickase, and wherein the second non-naturally occurring eaCas9 molecule is an HNH-like domain nickase.

In one embodiment, the composition is for use as a medicament; or for use in the treatment of sickle cell disease or beta thalassemia.

In another aspect, the invention is an *in vitro* or *ex vivo* method of modifying a target region of a target gene in a mammalian cell, the method comprising generating, within the cell, a first single strand break on a first strand of the target gene and second single strand break on a second strand of the target gene, thereby forming a double strand break in the target gene having a first 5' overhang and a second 5' overhang; wherein the target region of the target gene is located (a) between the first single strand break and the second single strand break, (b) within fewer than 50 nucleotides of the first single strand break, and/or (c) within fewer than 50 nucleotides of the second single strand break; wherein the double strand break is repaired by gene conversion, thereby modifying the target region of the target gene in the mammalian cell, wherein the target gene is an *HBB* gene; optionally wherein (a) the step of generating the first single strand break and the second single strand break comprises contacting the cell with at least one eaCas9 molecule, a first gRNA molecule, and a second gRNA molecule, wherein the first gRNA molecule and the at least one eaCas9 molecule associate with the target gene and generate the first single strand break, and wherein the second gRNA molecule and the at least one eaCas9 molecule associate with the target gene and generate the second single strand break; optionally wherein the at least one eaCas9 molecule is at least one eaCas9 nickase molecule; optionally wherein the at least one eaCas9 nickase molecule is at least one HNH-type nickase molecule.

In one embodiment of the method, the double strand break is repaired by gene conversion using an endogenous homologous region, and wherein the endogenous homologous region is a region of an *HBD* gene.

In this embodiment, (a) the first gRNA molecule comprises a targeting sequence comprising SEQ ID NO: 387 and the second gRNA molecule comprises a targeting sequence comprising SEQ ID NO: 16318, or (b) the first gRNA molecule is a gRNA molecule comprising any one of SEQ ID NOs: 387-485, 6803-6871, or 16010-16256, and wherein the second gRNA molecule is a gRNA molecule comprising any one of SEQ ID NOs: 387-485, 6803-6871, or 16010-16256.

Headings, including numeric and alphabetical headings and subheadings, are for organization and presentation and are not intended to be limiting.

Other features and advantages of the invention will be apparent from the detailed description, drawings, and from the claims.

### BRIEF DESCRIPTION OF THE DRAWINGS

**Fig. 1** depicts a scheme of the pair 8/15 of gRNAs surrounding the sickle mutation in combination with a Cas9 nickase (D10A or N863A). The nickases are shown as the grey ovals.
**Fig. 2** depicts the percentages of total editing event after a wild type Cas9 or a Cas9 nickase (D10A or N863A). A repetition of at least three independent experiments for each condition is shown.
**Fig. 3A** depicts the frequency of deletions a wild type Cas9 or a Cas9 nickase (D10A or N863A). A representation of at least 3 independent experiments for each condition is shown.
**Fig. 3B** depicts the frequency distribution of the length of deletions using a wild type Cas9 and gRNA 8 (similar results have been obtained with gRNA 15).
**Fig. 3C** depicts the frequency distribution of the length of deletions using a Cas9 nickase (D10A) with gRNAs 8/15 (similar results have been obtained using Cas9 N863A).
**Fig. 4A** depicts the frequency of gene conversion using a wild type Cas9 or a Cas9 nickase (D10A or N863A).
**Fig. 4B** shows a scheme representing the region of similarity between the *HBB* and *HBD* loci.
**Fig. 5** depicts the frequency of different lengths of *HBD* sequences that were incorporated into the *HBB* locus.
**Fig. 6A** depicts the frequency of insertions using a wild type Cas9 or a Cas9 nickase (D10A or N863A). A representation of at least three independent experiments for each condition is shown.
**Fig. 6B** depicts examples of common reads observed in U2OS cells electroporated with plasmid encoding Cas9 N863 and gRNA pair 8/15. The *HBB* reference is shown on the top.
**Fig. 7A** depicts the overall modification frequency at the *HBB* locus, separated into deletions, insertions, and gene conversion events. The different repair outcomes after WT (wild type), Cas9 D10 nickase or Cas9 N863A nickase activity in U2OS cells were measured by PCT amplification of individual amplification products. Error bars were derived from 5 independent experiments. Total number of sequences analyzed: n=569 for WT Cas9, n=718 for Cas9 D10A nickase, n=556 for Cas9 N863A nickase
**Fig. 7B** depicts the deletion size distribution for WT, Cas9 D10A nickase, or Cas9 N863A nickase. Total number of sequences analyzed: n=258 for WT Cas9, n=241 for Cas9 D10A nickase, n=103 for Cas9 N863A nickase. Individual deletion size data from each Cas9 variant was scored from Sanger sequencing.

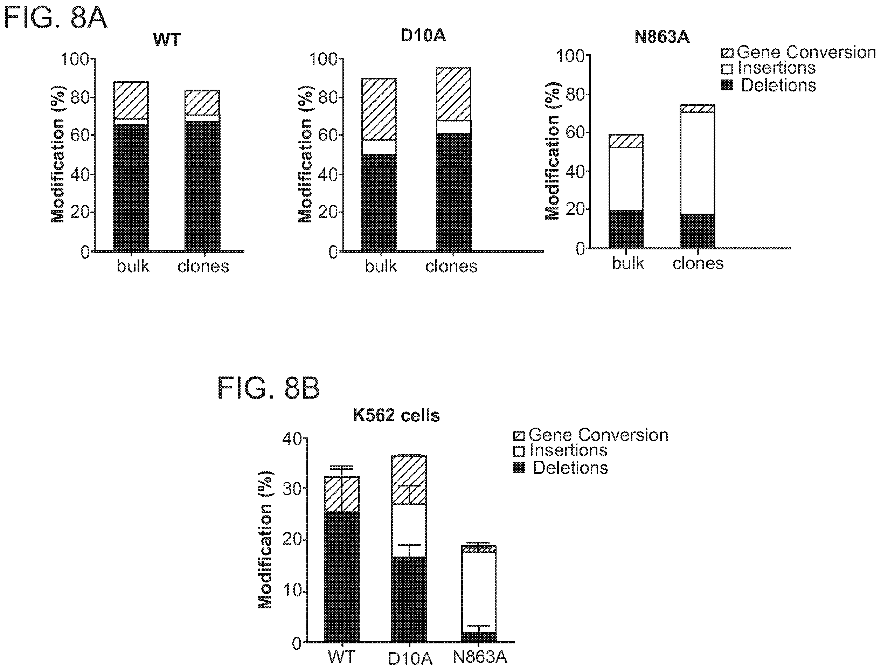

**Fig. 8A** depicts the overall modification frequency separated into deletions, insertions and gene conversion events for either WT Cas9- (WT), Cas9 D10A nickase- (D10A), or Cas9 N863A nickase-nucleofected (N863A) population of U2OS cells (bulk) or individual single U2OS cell clones. For WT Cas9, 58 sequences were analyzed for populations of cells (bulk) and 92 individual clones were analyzed. For Cas9 D10A nickase, 62 sequences were analyzed for populations of cells (bulk) and 81 individual clones were analyzed. For Cas9 N863A nickase, 73 sequences were analyzed for populations of cells (bulk) and 96 individual clones were analyzed.
**Fig. 8B** depicts the overall modification frequency separated into deletions, insertions, and gene conversion events for either WT Cas9- (WT), Cas9 D10A nickase- (D10A), or Cas9 N863A nickase-nucleofected (N863A) K562 cells. Number of sequences analyzed: n=91 for WT Cas9, n=85 for Cas9 D10A nickase, n=131 for Cas9 N863A nickase.
**Fig. 9** graphically depicts the positions of nucleotide mismatches (thin lines) and deletions (thick line) between the *HBD* and *HBB* genes and a histogram of the relative gene conversion frequency plotted as a function of the position on the *Hbb* locus.
**Fig. 10A** depicts western blot analyses of the knockdown efficiency of nucleofection of U2OS cells with siRNAs directed against firefly luciferase (FF, control), BRCA2, or RAD51 genes, as well as gel-loading controls. For BRCA2 knockdown, the gel loading control was β-actin. For RAD51 knockdown, the gel loading control was vinculin.

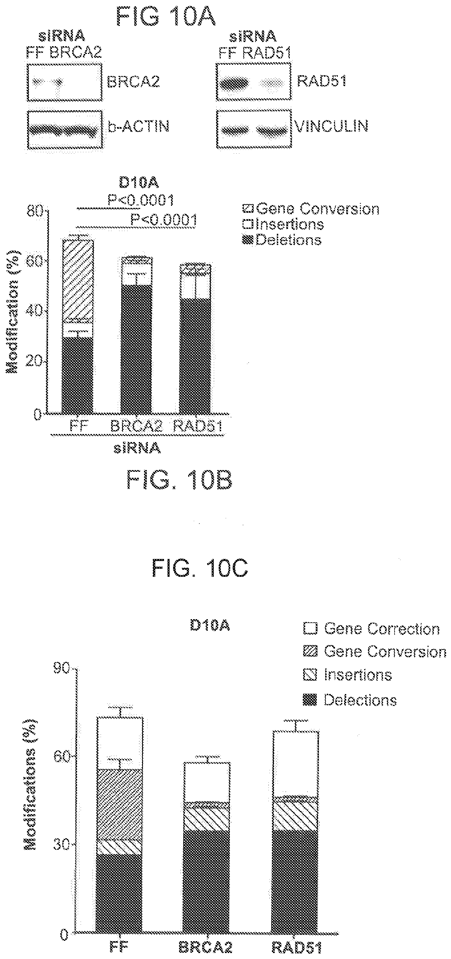

**Fig. 10B** depicts the overall modification frequency at the *HBB* locus separated into deletions, insertions, and gene conversion as determined by Sanger sequencing. U2OS cells nucleofected with siRNA against firefly luciferase (FF, control), BRCA2, or RAD51 genes, in addition to the Cas9 D10A nickase and gRNA pair 8/15. p-values for the differences in gene conversion frequency were calculated using the two-tailed Student's t-test. Four independent experiments were performed. Total number of sequences analyzed: n=476 for FF control siRNA treated cells, n=407 for BRCA2 siRNA treated cells, n=389 for RAD51 siRNA treated cells.
**Fig. 10C** depicts the overall modification frequency at the *HBB* locus resolved for deletions, insertions, and gene conversion as determined by Sanger sequencing. U2OS cells nucleofected with siRNA against firefly luciferase (FF, control), BRCA2, or RAD51 genes, in addition to the Cas9 D10A nickase and gRNA pair 8/15, in the presence of 50 pM of donor template. p-values for the differences in gene conversion frequency were calculated using the two-tailed Student's t-test. Total number of sequence from at least four independent experiments. Total number of sequences analyzed: n=770 for FF control siRNA treated cells, n=652 for BRCA2 siRNA treated cells, n=389 for RAD51 siRNA treated cells.
**Fig. 11A** depicts the raw number and percentage of insertions detected in individual cells nucleofected with the Cas9 D10A nickase or Cas9 N863A nickase and gRNA pair 8/15 that were either derived from the overhang, or that contain the full overhang repetition. Overhangs were defined as sequences that had at least a consecutive stretch of 9 nucleotides of the predicted overhang sequence present. P=value was calculated using Fisher's exact test.
**Fig. 11B** depicts histogram plots of insertion length for either the Cas9 D10A nickase- or Cas9 N863A nickase-induced lesions. Individual insertion size data from each Cas9 variant was scored from Sanger sequencing data of U2OS cells treated with either Cas9 10A nickase or Cas9 N863A nickase and gRNA pair 8/15. Difference in length between Cas9 D10A nickase- and Cas9 N863A nickase-induced lesions was significant (p=1.274x10⁻¹²; permutation test). Number of insertions plotted: n=75 for Cas9 D10A nickase, and n=325 for Cas9 N863A nickase.
**Fig. 12A** depicts histogram plots showing how frequently and the position in the overhang of 4-mers found within insertion sequences aligned to different positions along the overhang sequence in U2OS cells treated with either Cas9 10A nickase or Cas9 N863A nickase and gRNA pair 8/15. Parts of insertion sequences containing the 4-mer sequence could not be uniquely mapped to a single position within the overhang.
**Fig. 12B** depicts exemplary insertion sequences of the full overhang resulting from Cas9 D10A nickase or Cas9 N863A nickase-induced lesions. Cas9 D10 nickase-induced insertions did not show overlaps, while Cas9 N863A nickase-induced insertions showed overlaps, indicating microhomology usage. Positioning of each gRNA (gRNA8 or gRNA15) shown as indicated. Position of the PAM indicated in black.
**Fig. 13A** is a schematic representation of the donor template.
**Fig. 13B** depicts the frequency of HDR using a wild type Cas9 or a Cas9 nickase (D10A or N863A).
**Fig. 13C** depicts different forms of donors and their contribution to HDR.

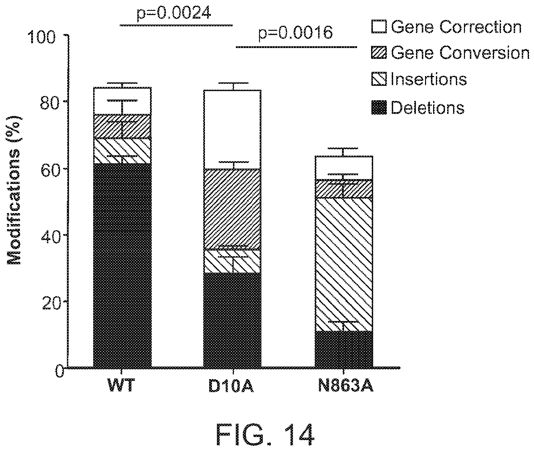

**Fig. 14** depicts the overall modification frequency at the *HBB* locus separated into repair outcomes (*i.e.,* deletions, insertions, gene conversion, and gene correction) for either WT Cas9- (WT), Cas9 D10A nickase- (D10A), or Cas9 N863A nickase-nucleofected (N863A) U2OS cells in the presence of 50 pM ssODN donor template. Repair outcomes were determined by PCR amplification of the *HBB* locus, followed by Sanger sequencing of individual amplification products. p-value for the difference in gene correction frequency was calculated using the two-tailed Student's t-test. Total number of sequences plotted from at least three independent experiments: n=200 for WT Cas9, n=448 for Cas9 D10A nickase, and n=422 for Cas9 N863A nickase.
**Fig. 15** depicts the distribution of the editing events on the presence of wild type Cas9 with gRNA 8, gRNA 15 and with the Cas9 nickase (D10A) with gRNA 8/15 pair.
**Fig. 16A** depicts the overall modification frequency of U2OS cells nucleofected with either WT Cas9, Cas9 D10 nickase, or Cas9 N863A nickase and gRNA8. Sequencing was performed using Illumina MiSeq and modifications were quantified computationally using CRISPResso.
**Fig. 16B** depicts a 15% urea polyacrylamide gel electrophoresis displaying the cutting efficiency of various amounts of Cas9 D10A nickase and gRNA8 or Cas9 N863A nickase and gRNA8 complexes with double stranded DNA. Cleavage products are indicated.
**Fig. 17A** depicts the frequency of deletions, insertions, gene conversion, and gene correction observed in U2OS cells nucleofected with either WT Cas9, Cas9 D10A nickase, or Cas9 N863A nickase, gRNA8, donor template and siRNAs against firefly luciferase (FF, control), BRCA2, or RAD51. Sequencing was performed using MiSeq and modifications were scored by computationally using CRISPResso.
**Fig. 18A** depicts a bar graph of fold change in the rates of total editing events and gene correction for U2OS cells nucleofected with either WT Cas9, Cas9 D10A nickase, or Cas9 N863A nickase, gRNA8, donor template and siRNAs against RAD51.
**Fig. 18B** depicts histograms of the length of deletions induced in U2OS cells nucleofected with either WT Cas9, or Cas9 D10A nickase, gRNA8, donor template and siRNAs against firefly luciferase (FF, control) or RAD51.
**Fig. 18C** depicts a model of the possible role of HR in the processing of nicks that escaped single strand break repair in the presence or absence of RAD51.
**Fig. 19** depicts genome editing of the *HBB* locus in bone marrow leukemia K562 hematopoietic cells after electroporation of Cas9 protein complexed to HBB gRNAs 8 and 15 (RNP) or Cas9 mRNA co-delivered with HBB gRNAs 8 and 15 (RNA). "gRNA 8" has the targeting domain sequence GUAACGGCAGACUUCUCCUC (SEQ ID NO: 388) and "gRNA 15" has the targeting domain sequence AAGGUGAACGUGGAUGAAGU (SEQ ID NO: 387).
**Fig. 20** is a schematic of the HBB locus indicating the position of the different gRNA molecules used in the study.
**Fig. 21** depicts the overall editing frequencies for the Cas9 D10A and Cas9 N863A nickases in terms of overhang length and composition.
**Fig. 22** depicts the insertion frequencies for the Cas9 D10A and Cas9 N863A nickases in terms of overhang length and composition.
**Fig. 23** depicts the percentages of insertions derived from the overhang sequence for the Cas9 D10A (left bars) and Cas9 N863 (right bars) nickases.
**Fig. 24** depicts the lengths of insertions for the Cas9 D10A (left bars) and Cas9 N863 (right bars) nickases.
**Fig. 25** depicts the effect of overhang length and composition on gene conversion induced by the Cas9 D10A and Cas9 N863 nickases.
**Fig. 26** depicts the effect of overhang length and composition on gene correction induced by the Cas9 D10A and Cas9 N863A nickases.
**Figs. 27A** and **27B** depict an analysis of stability of D10A RNP *in vitro* and *ex vivo* in adult CD34+ HSCs. **Fig. 27A** depicts Differential Scanning Fluorimetry Shift Assay after complexing D10A protein with the indicated *HBB* gRNAs added at 1:1 molar ratio gRNA:RNP. **Fig. 27B** depicts detection of Cas9 protein in cell lysates 72 hours after HSCs were electroporated with D10A RNP or D10A mRNA with gRNAs HBB-8 and HBB-15. The electroporation program (P2 or P3) used is indicated at the top of the image. 2X gRNA: 10µg each gRNA was co-delivered with D10A mRNA (vs. 5µg of each gRNA).

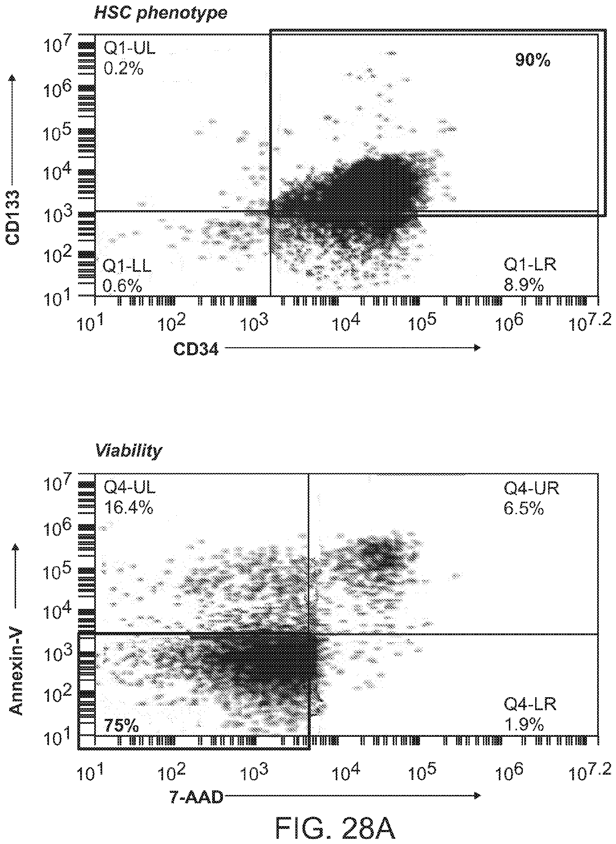

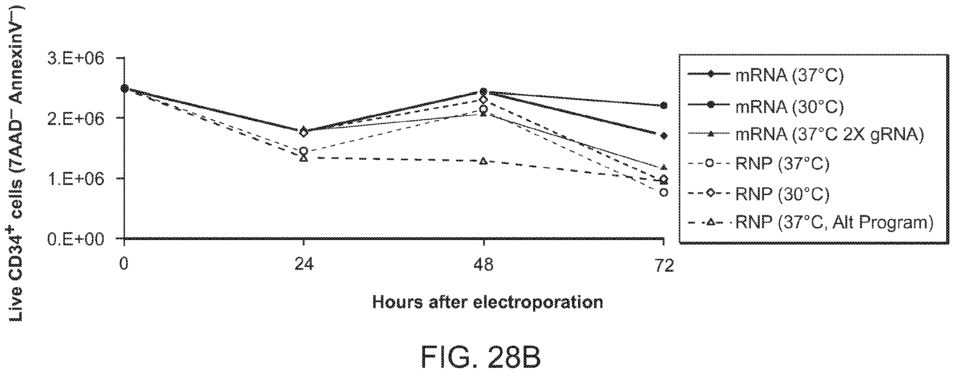

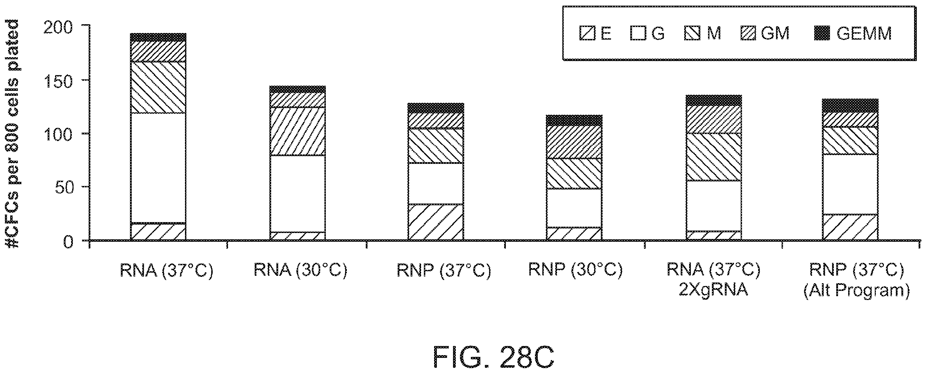

**Figs. 28A****,** **28B****,** and **28C** depict adult CD34+ HSCs maintain stem cell phenotype after electroporation with D10A nickase RNP and HBB targeting gRNA pair. **Fig 28A** depicts gene edited adult CD34+ cells maintain expression of stem cell markers CD34 and CD133 at 72 hours after electroporation. **Fig 28B** depicts absolute live (7-AAD-AnnexinV⁻) CD34+ cell number at indicated time points relative to electroporation of D10A RNP HBB gRNA pair. **Fig 28C** depicts gene edited adult CD34+ cells maintain hematopoietic colony forming cell (CFC) activity and multipotency. E: erythroid, G: granulocyte, M: macrophage, GM: granulocyte-macrophage, GEMM: granulocyte-erythrocyte-macrophage-monocyte CFCs.

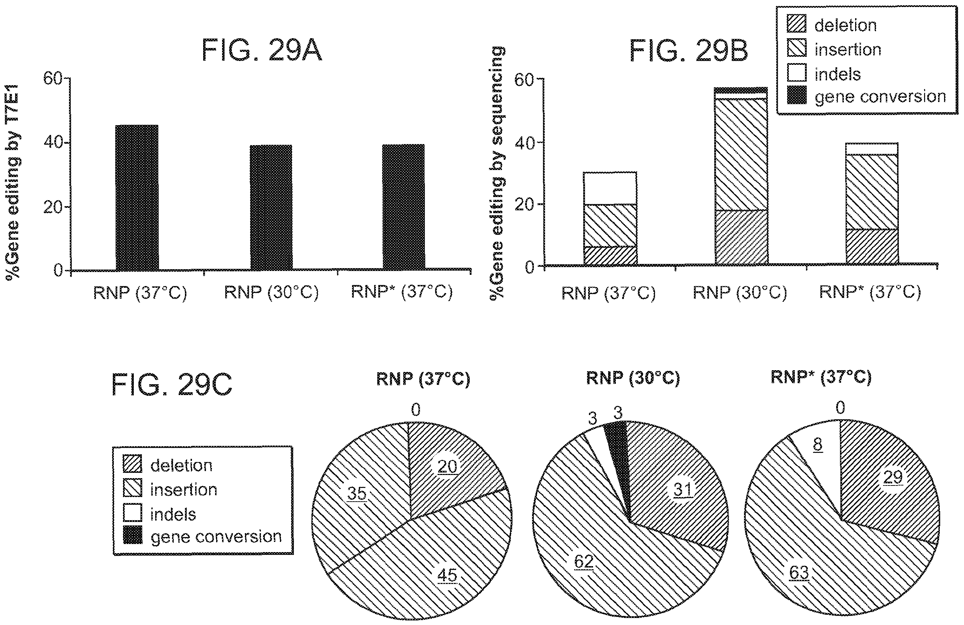

**Figs. 29A, 29B,** and **29C** depict D10A nickase RNP co-delivered with HBB targeted gRNA pair supports gene editing and HDR in adult CD34⁺HSCs. **Fig. 29A** depicts percentage of gene editing events as detected by T7E1 endonuclease assay analysis. **Fig. 29B** depicts DNA sequence analysis of the *HBB* locus. The subtypes of gene editing events (insertions, deletions, indels, and gene conversion events) are indicated. RNP* refers to use of electroporation program 3. **Fig. 29C** depicts percentages of types of editing events detected in the gDNA from the cells electroporated with the conditions shown in **Fig. 29B****.** Data are shown as a percentage of all gene editing events.

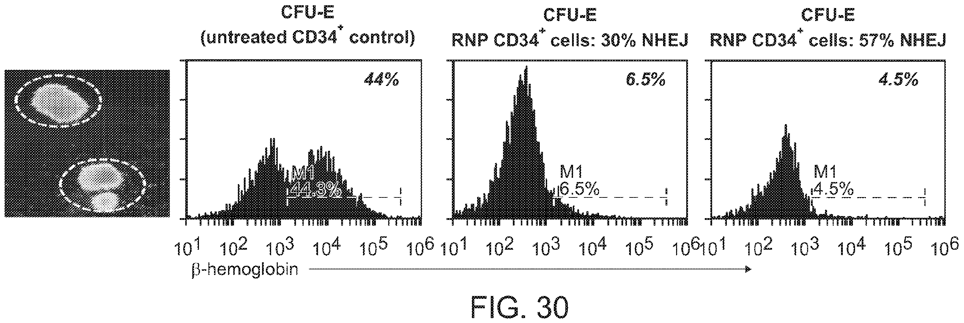

**Fig. 30** depicts flow cytometry analysis of β-hemoglobin expression in the erythroid progeny differentiated from D10A nickase RNP gene-edited adult CD34⁺ HSCs. CFU-E colonies (far left) differentiated from D10A RNP HBB gRNA electroporated CD34⁺ cells were dissociated, fixed, permeabilized, and stained for β-hemoglobin expression. The gene editing frequencies detect in the parental CD34+ cell population are indicated above the histograms for the indicated samples. The percentage of β-hemoglobin expression in each colony was determined by flow cytometry and is indicated at the top right of each histogram.

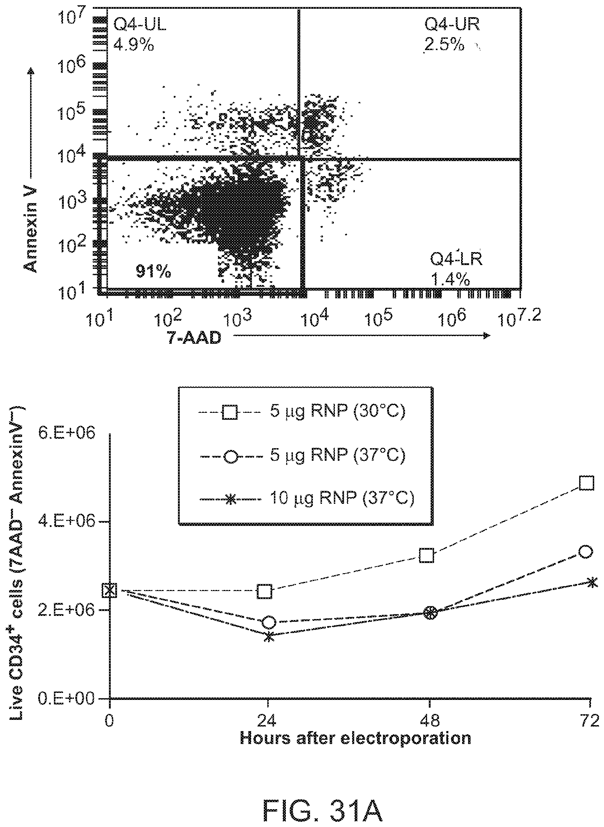

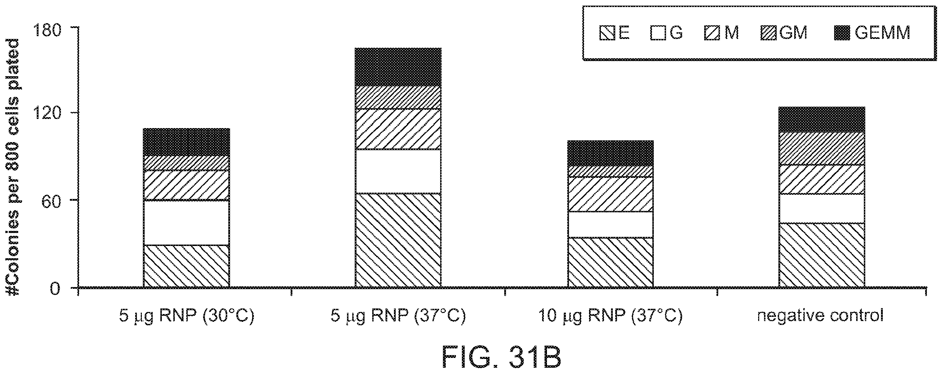

**Figs. 31A** and **31B** depict that cord blood CD34⁺HSCs maintained stem cell phenotype after electroporation with D10A nickase RNP and HBB targeting gRNA pair. **Fig. 31A** depicts gene edited CB CD34⁺ cells maintain viability after electroporation. *Right:* Absolute live (7-AAD⁻AnnexinV⁻) CD34⁺ cell number at indicated time points relative to electroporation of D10A RNP *HBB* gRNA pair. **Fig. 31B** depicts that gene edited CB CD34⁺ cells maintained hematopoietic colony forming cell (CFC) activity and multipotency. E: erythroid, G: granulocyte, M: macrophage, GM: granulocyte-macrophage, GEMM: granulocyte-erythrocyte-macrophage-monocyte CFCs. The amounts of D10A RNP delivered per million cells (5 or 10µg) and the 2-hour recovery temperature (parentheses) after electroporation of the parental CB CD34⁺ cells are indicated.

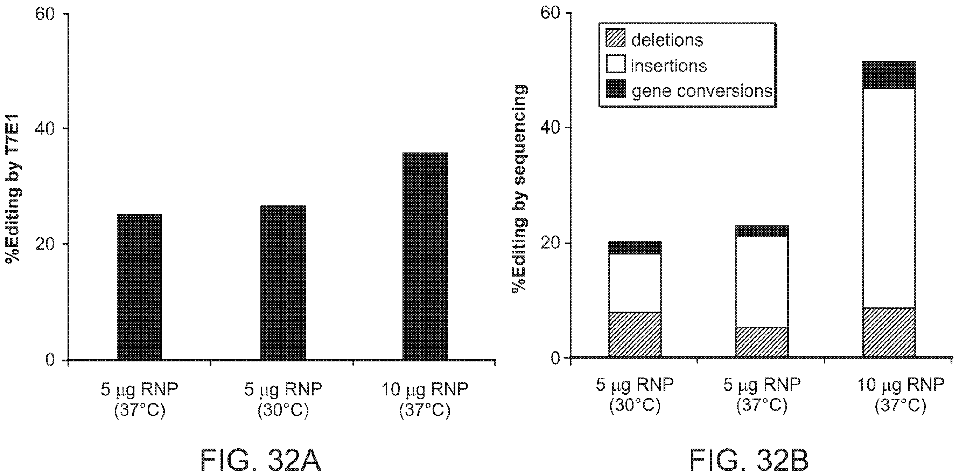

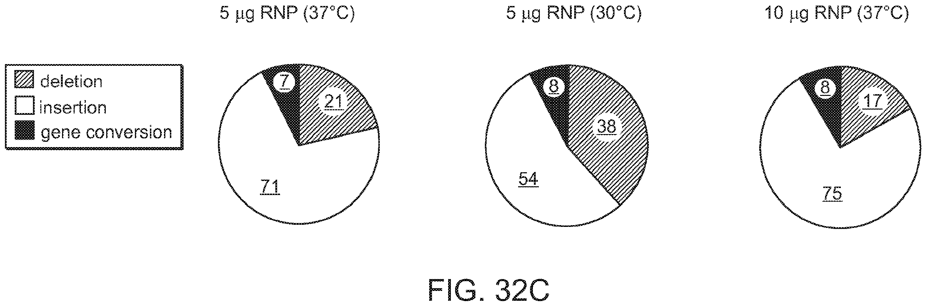

**Figs. 32A, 32B****,** and **32C** depict D10A RNP co-delivered with HBB targeted gRNA pair supported gene editing and HDR in CB CD34⁺ HSCs. **Fig. 32A** depicts percentage of gene editing events as detected by T7E1 endonuclease assay analysis. **Fig. 32B** depicts DNA sequence analysis of the *HBB* locus. The subtypes of gene editing events (insertions, deletions, indels, and gene conversion events) are indicated as a fraction of the total sequencing reads. **Fig. 32C** depicts subtypes of gene editing events expressed as relative percentage to the total number gene editing events detected. The amounts of D10A RNP delivered per million cells (5 or 10µg) and the 2-hour recovery temperature (parentheses) after electroporation of the parental CB CD34⁺ cells are indicated.

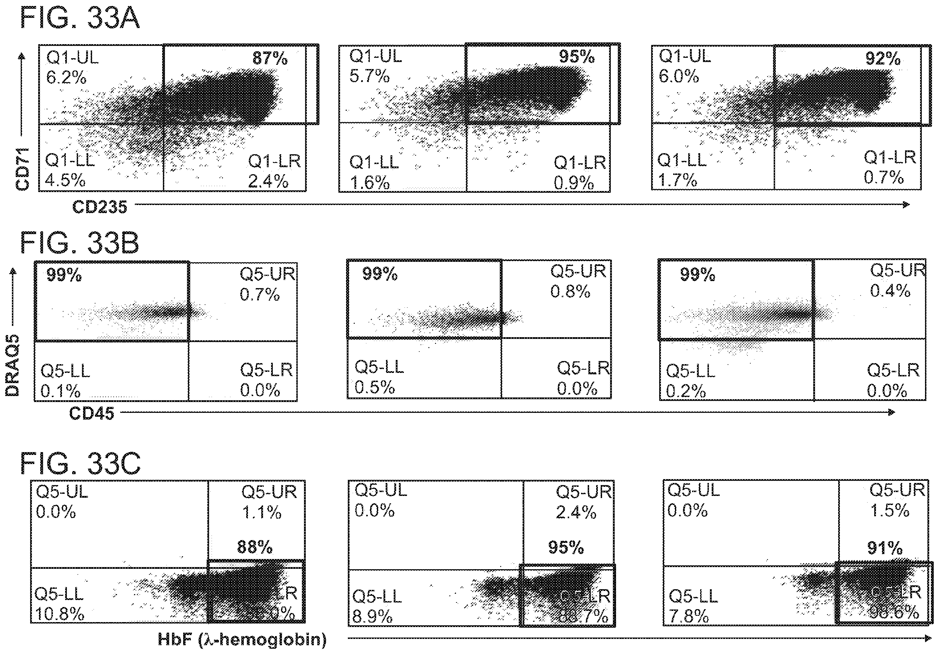

**Figs 33A, 33B,** and **33C** depict directed differentiation of gene-edited CB CD34⁺ HSCs into erythroblasts. Flow cytometry analysis of day 18 erythroblasts differentiated from gene edited CB CD34⁺ cells. **Fig. 33A** depicts CD71 (transferrin receptor and CD235 (Glycophorin A). **Fig. 33B** depicts fetal hemoglobin (g-hemoglobin). **Fig. 33C** depicts loss of CD45 and dsDNA through enucleation as indicated by the absence of dsDNA (negative for dsDNA binding dye DRAQ5). Note that, unlike adult CD34⁺ cells, CB CD34⁺ cells differentiated into fetal-like erythroblasts that express fetal g-hemoglobin (not adult b-hemoglobin).

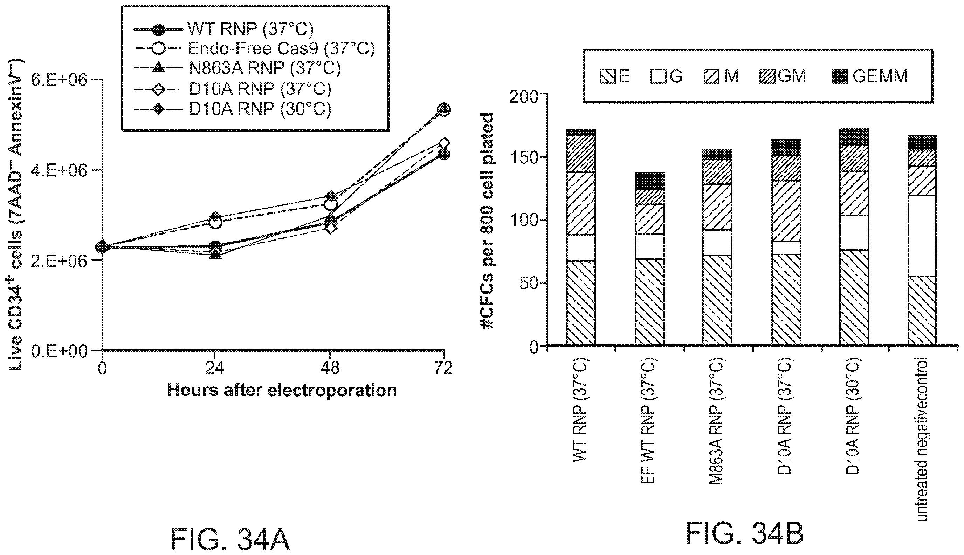

**Figs. 34A** and **34B** depict cord blood CD34⁺ HSCs maintained stem cell phenotype after electroporation with Cas9 variant RNPs and HBB targeting gRNA pair. **Fig. 34A** depicts gene edited CB CD34⁺ cells maintain viability after electroporation with WT Cas9 endotoxin-free (EF WT) Cas9, N863A nickase, or D10A nickase co-delivered with HBB gRNA pair. Absolute live (7-AAD⁻AnnexinV-⁻) CD34⁺ cell number at indicated time points relative to electroporation. **Fig. 34B** depicts gene edited CB CD34⁺ cells maintained hematopoietic colony forming cell (CFC) activity and multipotency. E: erythroid, G: granulocyte, M: macrophage, GM: granulocyte-macrophage, GEMM: granulocyte-erythrocyte-macrophage-monocyte CFCs. The amounts of RNP delivered per million cells (10µg) and the 2-hour recovery temperature (parentheses) after electroporation of the parental CB CD34⁺ cells are indicated.

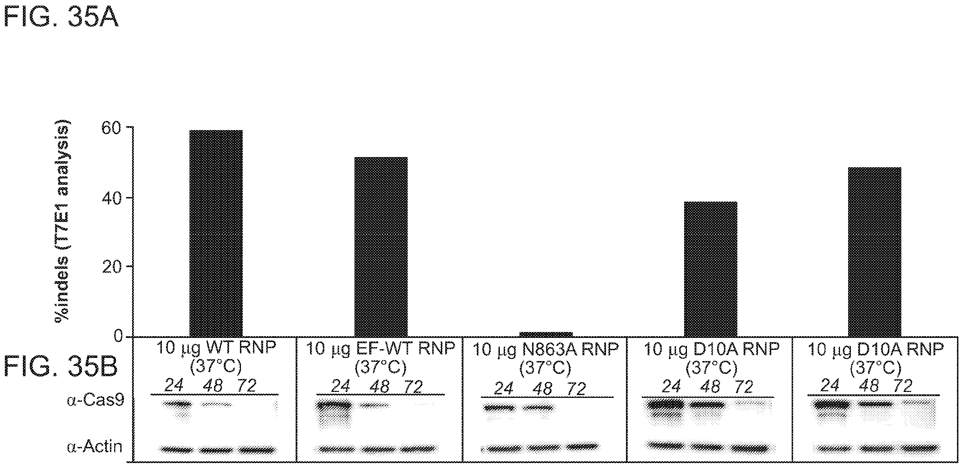

**Figs. 35A** and **35B** depict comparison of gene editing at the *HBB* locus in CB CD34⁺ cells mediated by WT and nickase Cas9 variant RNPs. **Fig. 35A** depicts T7E1 analysis of the percentage of indels detected 72 hours after electroporation at the targeted site in the *HBB* locus after electroporation of WT Cas9, Endotoxin-free WT Cas9 (EF-WT), N863A nickase, and D10A nickase RNPs, each co-delivered with HBB-8 and HBB-15 gRNA pair. **Fig. 35B** depicts Western blot analysis showing detection of Cas9 variants in cell lysates of CB CD34⁺ cells at the indicated time points after electroporation. The amounts of RNP delivered per million cells (10µg) and the 2-hour recovery temperature (parentheses) after electroporation of the parental CB CD34⁺ cells are indicated.

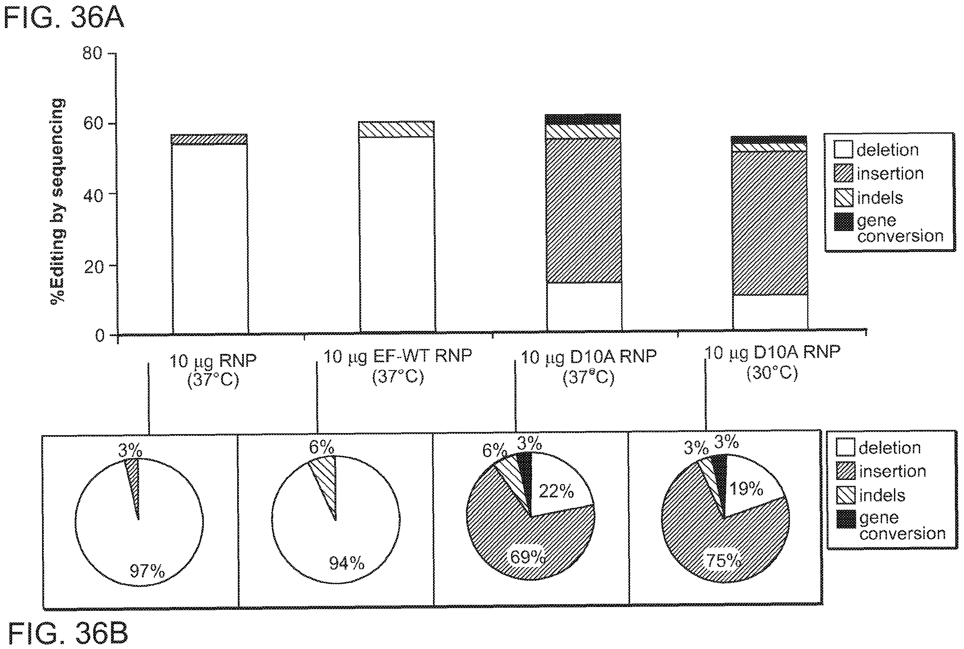

**Figs. 36A** and **36B** depict comparison of HDR and NHEJ events detected at the *HBB* locus after gene editing with WT Cas9 and D10A nickase in CB CD34⁺ HSCs. **Fig. 36A** depicts percentage of gene editing events (72 hours after electroporation) detected by DNA sequencing analysis and shown as a percentage of the total sequence reads. Cells received RNP (WT Cas9, Endotoxin-free WT Cas9 [EF-WT], N863A and D10A nickases) with HBB-8 and HBB-15 gRNA pair. **Fig. 36B** depicts percentages of types of editing events detected in the gDNA from the cells electroporated with the conditions shown in **Fig. 36A****.** Data are shown as a percentage of all gene editing events.

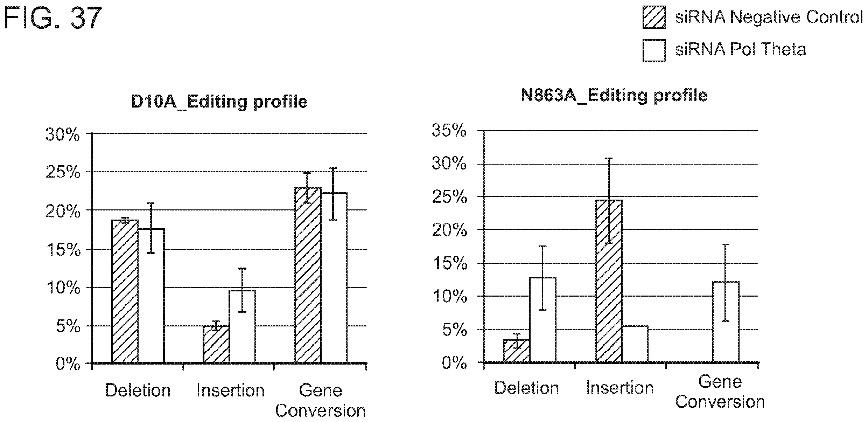

**Fig. 37** depicts that down-regulation of Pol Theta in the context of the D10A Cas9 does not affect the gene editing profile. In contrast, Pol Theta down-regulation in the context of a mutant Cas9 (such as the N863A Cas9 nuclease) leads to a strong reduction of the insertion frequency and an increase in the gene conversion rate. Cells were treated with either a control siRNA or an siRNA directed against Pol Theta. The N863A enzyme is directed to make two nicks at the target site resulting in a 3' overhang. In the absence of Pol Theta, the rates of insertion are reduced and gene conversion is increased because the Alt-NHEJ pathway (Pol Theta Dependant) has been downregulated.
**Fig. 38** depicts that the gene conversion frequency is highly impaired in the absence of BRCA2 and RAD51. D10A induced gene conversion is dependent on BRCA2 and RAD51. Cells were treated with siRNA against a negative control, BRCA2, and RAD51 in conjunction with D10A and gRNA 8 and 15.
**Fig. 39** depicts a model of the genetic requirements of the gene conversion pathway in the context of different Cas9 nucleases (wild type, D10A or N863A) using BRCA2 and RAD51.

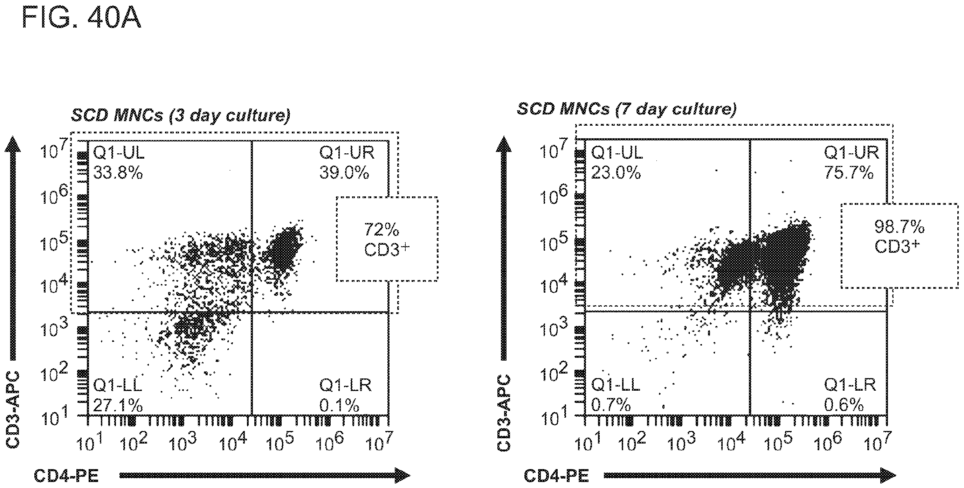

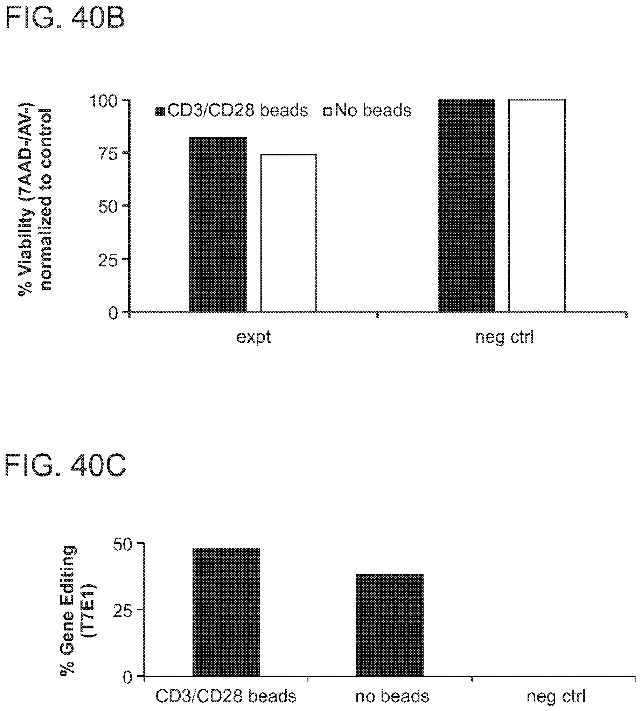

**Fig. 40A****,** **40B** and **40C** depict that Cas9 D10A nickase RNP co-delivered with a HBB targeted gRNA pair supported gene editing in T lymphocytes expanded from peripheral blood mononuclear cells (MNCs) from a sickle cell disease (SCD) patient. **Fig. 40A** depicts the expansion/activation of the T lymphocyte fraction from bulk MNCs from a SCD patient using CD3/CD28 immunomagnetic beads at day 3 (left panel) and day 7 (right panel) after activation. **Fig. 40B** depicts the total viability of expanded T lymphocytes electroporated with Cas9 D10A nickase complexed with a HBB targeted gRNA pair (HBB-8-sickle and HBB-15), and co-electroporated with a single stranded oligonucleotide donor, following reactivation CD3/CD28 immunomagnetic beads, no beads control (*i.e.,* no exposure CD3/CD28 immunomagnetic beads) or negative control. **Fig. 40C** depicts gene editing frequency at the *HBB* locus of expanded T lymphocytes electroporated with Cas9 D10A nickase complexed with a HBB targeted gRNA pair (HBB-8-sickle and HBB-15), and co-electroporated with a single stranded oligonucleotide donor, following reactivation CD3/CD28 immunomagnetic beads, no beads control (*i.e.,* no exposure CD3/CD28 immunomagnetic beads) or negative control.

### DETAILED DESCRIPTION

In order that the invention is understood, certain terms are herein defined.

### Definitions

"Alt-HDR" or "alternative HDR," or alternative homology-directed repair, as used herein, refers to the process of repairing DNA damage using a homologous nucleic acid *(e.g.,* an endogenous homologous sequence, *e.g.,* a sister chromatid, or an exogenous nucleic acid, *e.g.,* a template nucleic acid). Alt-HDR is distinct from canonical HDR in that the process utilizes different pathways from canonical HDR, and can be inhibited by the canonical HDR mediators, RAD51 and BRCA2. Also, alt-HDR uses a single-stranded or nicked homologous nucleic acid for repair of the break.

"ALT-NHEJ" or "alternative NHEJ", or alternative non-homologous end joining, as used herein, is a type of alternative end joining repair process, and utilizes a different pathway than that of canonical NHEJ. In alternative NHEJ, a small degree of resection occurs at the break ends on both sides of the break to reveal single-stranded overhangs. Ligation or annealing of the overhangs results in the deletion of sequence. ALT-NHEJ is a category that includes microhomology-mediated end joining (MMEJ), blunt end joining (EJ), and synthesis-dependent microhomology-mediated end joining (SD-MMEJ). In MMEJ, microhomologies, or short spans of homologous sequences, *e.g.,* 5 nucleotides or more, on the single-strand are aligned to guide repair, and leads to the deletion of sequence between the microhomologies.

"Amino acids" as used herein encompasses the canonical amino acids as well as analogs thereof. "Canonical HDR," or canonical homology-directed repair, as used herein, refers to the process of repairing DNA damage using a homologous nucleic acid *(e.g.,* an endogenous homologous sequence, *e.g.,* a sister chromatid, or an exogenous nucleic acid, *e.g.,* a template nucleic acid). Canonical HDR typically acts when there has been significant resection at the double-strand break, forming at least one single stranded portion of DNA. In a normal cell, HDR typically involves a series of steps such as recognition of the break, stabilization of the break, resection, stabilization of single stranded DNA, formation of a DNA crossover intermediate, resolution of the crossover intermediate, and ligation. The process requires RAD51 and BRCA2, and the homologous nucleic acid is typically double-stranded.

"Canonical NHEJ", or canonical non-homologous end joining, as used herein, refers to the process of repairing double-strand breaks in which the break ends are directly ligated. This process does not require a homologous nucleic acid to guide the repair, and can result in deletion or insertion of one or more nucleotides. This process requires the Ku heterodimer (Ku70/Ku80), the catalytic subunit of DNA-PK (DN-PKcs), and/or DNA ligase XRCC4/LIG4. Unless indicated otherwise, the term "HDR" as used herein encompasses canonical HDR and alt-HDR.

A "Cas9 molecule," as used herein, refers to a Cas9 polypeptide or a nucleic acid encoding a Cas9 polypeptide. A "Cas9 polypeptide" is a polypeptide that can interact with a gRNA molecule and, in concert with the gRNA molecule, localize to a site comprising a target domain and, in certain embodiments, a PAM sequence. Cas9 molecules include both naturally occurring Cas9 molecules and Cas9 molecules and engineered, altered, or modified Cas9 molecules or Cas9 polypeptides that differ, *e.g.,* by at least one amino acid residue, from a reference sequence, *e.g.,* the most similar naturally occurring Cas9 molecule. (The terms altered, engineered or modified, as used in this context, refer merely to a difference from a reference or naturally occurring sequence, and impose no specific process or origin limitations.) A Cas9 molecule may be a Cas9 polypeptide or a nucleic acid encoding a Cas9 polypeptide. A Cas9 molecule may be a nuclease (an enzyme that cleaves both strands of a double-stranded nucleic acid), a nickase (an enzyme that cleaves one strand of a double-stranded nucleic acid), or an enzymatically inactive (or dead) Cas9 molecule. A Cas9 molecule having nuclease or nickase activity is referred to as an "enzymatically active Cas9 molecule" (an "eaCas9" molecule). A Cas9 molecule lacking the ability to cleave target nucleic acid is referred to as an "enzymatically inactive Cas9 molecule" (an "eiCas9" molecule).

A Cas9 molecule may meet one or both of the following criteria:
it has at least 20, 30, 40, 50, 55, 60, 65, 70, 75, 80, 81, 82, 83, 84, 85, 86, 87, 88, 89, 90, 91, 92, 93, 94, 95, 96, 97, 98, 99, or 100% homology with, or it differs by no more than 1, 2, 3, 4, 5, 6, 7, 8, 9, 10, 11, 12, 13, 14, 15, 16, 17, 18, 19, 20, 25, 30, 35, 40, 35, 50, 55, 60, 65, 70, 75, 80, 85, 90, 95, 100, 150, 200, 250, 300, 350 or 400, amino acid residues from, the amino acid sequence of a reference sequences, e.g., naturally occurring Cas9 molecule.

A Cas9 molecule may meet one or both of the following criteria:
it has at least 20, 30, 40, 50, 55, 60, 65, 70, 75, 80, 81, 82, 83, 84, 85, 86, 87, 88, 89, 90, 91, 92, 93, 94, 95, 96, 97, 98, 99, or 100% homology with, or it differs by no more than 1, 2, 3, 4, 5, 6, 7, 8, 9, 10, 11, 12, 13, 14, 15, 16, 17, 18, 19, 20, 25, 30, 35, 40, 35, 50, 55, 60, 65, 70, 75, 80, 85, 90, 95, 100, 150, 200, 250, 300, 350 or 400, amino acid residues from, the amino acid sequence of a reference sequences, *e.g.,* naturally-occurring Cas9 molecule.

Each domain of the Cas9 molecule (*e.g.,* the domains named herein) may, independently have: at least 20, 30, 40, 50, 55, 60, 65, 70, 75, 80, 81, 82, 83, 84, 85, 86, 87, 88, 89, 90, 91, 92, 93, 94, 95, 96, 97, 98, 99, or 100% homology with such a domain described herein. At least 1, 2, 3, 4, 5, of 6 domains may have, independently, at least 50, 60, 70, 80, 81, 82, 83, 84, 85, 86, 87, 88, 89, 90, 91, 92, 93, 94, 95, 96, 97, 98, 99, or 100% homology with a corresponding domain, while any remaining domains will be absent, or have less homology to their corresponding naturally occurring domains.

The Cas9 molecule may be a *S. pyogenes* Cas9 variant. The Cas9 variant may be the EQR variant. The Cas9 variant may be the VRER variant. The eiCas9 molecule may be a *S. pyogenes* Cas9 variant. The Cas9 variant may be the EQR variant. The Cas9 variant may be the VRER variant. A Cas9 system may comprise a Cas9 molecule, *e.g.,* a Cas9 molecule described herein, *e.g.,* the Cas9 EQR variant or the Cas9 VRER variant.

The Cas9 molecule may be a *S. aureus* Cas9 variant. The Cas9 variant may be the KKH (E782K/N968K/R1015H) variant (see, *e.g.,* Kleinstiver 2015). The Cas9 variant may be the E782K/K929R/R1015H variant (see, *e.g.,* Kleinstiver 2015). The Cas9 variant may be the E782K/K929R/N968K/R1015H variant (see, *e.g.,* Kleinstiver 2015). The Cas9 variant may comprise one or more mutations in one of the following residues: E782, K929, N968, R1015. The Cas9 variant may comprise one or more of the following mutations: E782K, K929R, N968K, R1015H and R1015Q (see, *e.g.,* Kleinstiver 2015). A Cas9 system may comprises a Cas9 molecule, *e.g.,* a Cas9 molecule described herein, *e.g.,* the Cas9 KKH variant.

As used herein, the term "Cas9 system" or "CRISPR/Cas system" refers to a system capable of altering a target nucleic acid by one of many DNA repair pathways. The Cas9 system described herein may promote repair of a target nucleic acid via an HDR pathway. A Cas9 system may comprise a gRNA and a Cas9 molecule. A Cas9 system may further comprise a second gRNA. A Cas9 system may comprise a gRNA, a Cas9 molecule, and a second gRNA. A Cas9 system may comprise a gRNA, two Cas9 molecules, and a second gRNA. A Cas9 system may comprise a first gRNA, a second gRNA, a first Cas9 molecule, and a second Cas9 molecule. A Cas9 system may further comprise a template nucleic acid.

As used herein, the term "cleavage event" refers to a break in a nucleic acid molecule. A cleavage event may be a single-strand cleavage event, or a double-strand cleavage event. A single-strand cleavage event may result in a 5' overhang or a 3' overhang. A double-stranded cleavage event may result in blunt ends, two 5' overhangs, or two 3' overhangs.

A disorder "caused by" a mutation, as used herein, refers to a disorder that is made more likely or severe by the presence of the mutation, compared to a subject that does not have the mutation. The mutation need not be the only cause of a disorder, *i.e.,* the disorder can still be caused by the mutation even if other causes, such as environmental factors or lifestyle factors, contribute causally to the disorder. The disorder may be caused by the mutation if the mutation is a medically recognized risk factor for developing the disorder, and/or if a study has found that the mutation contributes causally to development of the disorder.

"Derived from", as used herein, refers to the source or origin of a molecular entity, *e.g.,* a nucleic acid or protein. The source of a molecular entity may be naturally-occurring, recombinant, unpurified, or a purified molecular entity. For example, a polypeptide that is derived from a second polypeptide comprises an amino acid sequence that is identical or substantially similar, *e.g.,* is more than 50% homologous to, the amino acid sequence of the second protein. The derived molecular entity, *e.g.,* a nucleic acid or protein, can comprise one or more modifications, *e.g.,* one or more amino acid or nucleotide changes.

"Domain," as used herein, is used to describe a segment of, or a portion of a protein or nucleic acid. Unless otherwise indicated, a domain is not required to have any specific functional property.

Calculations of homology or sequence identity between two sequences (the terms are used interchangeably herein) are performed as follows. The sequences are aligned for optimal comparison purposes (*e.g.,* gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frame shift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences.

As used herein, the term "endogenous" gene, "endogenous" nucleic acid, or "endogenous" homologous region refers to a native gene, nucleic acid, or region of a gene, which is in its natural location in the genome, *e.g.,* chromosome or plasmid, of a cell. In contrast, the term "exogenous" gene or "exogenous" nucleic acid refers to a gene, nucleic acid, or region of a gene which is not native within a cell, but which is introduced into the cell during the methods of the invention. An exogenous gene or exogenous nucleic acid may be homologous to, or identical to, an endogenous gene or an endogenous nucleic acid.

As used herein, the term "endogenous homologous region" refers to an endogenous template nucleic acid sequence which is homologous to at least a portion of a target gene, and which can be used in conjunction with a Cas9 molecule and a gRNA molecule to modify, *e.g.,* correct, a sequence of the target gene. The endogenous homologous region may be DNA. The endogenous homologous region may be double stranded DNA. The endogenous homologous region may be single stranded DNA. The endogenous homologous region may be at least 70%, 75%, 80%, 81%, 82%, 83%, 84%, 85%, 86%, 875, 885, 89%, 90%, 91%, 92%, 93%, 94%, 95%, 96%, 9%, 98%, or 99% homologous to at least a portion of the target gene.

As used herein, "error-prone" repair refers to a DNA repair process that has a higher tendency to introduce mutations into the site being repaired. For instance, alt-NHEJ and SSA are error-prone pathways; C-NHEJ is also error prone because it sometimes leads to the creation of a small degree of alteration of the site (even though in some instances C-NHEJ results in error-free repair); and HR, alt-HR, and SSA in the case of a single-strand oligo donor are not error-prone.

As used herein, the term "gRNA molecule" or "gRNA" refers to a guide RNA which is capable of targeting a Cas9 molecule to a target nucleic acid. The term "gRNA molecule" may refer to a guide ribonucleic acid. The term "gRNA molecule" may refer to a nucleic acid encoding a gRNA. A gRNA molecule may be non-naturally occurring. A gRNA molecule may be a synthetic gRNA molecule.

"Governing gRNA molecule," as used herein, refers to a gRNA molecule that comprises a targeting domain that is complementary to a target domain on a nucleic acid that comprises a sequence that encodes a component of the CRISPR/Cas system that is introduced into a cell or subject. A governing gRNA does not target an endogenous cell or subject sequence. A governing gRNA molecule may comprise a targeting domain that is complementary with a target sequence on: (a) a nucleic acid that encodes a Cas9 molecule; (b) a nucleic acid that encodes a gRNA molecule which comprises a targeting domain that targets the *HBB* gene (a target gene gRNA); or on more than one nucleic acid that encodes a CRISPR/Cas component, *e.g.,* both (a) and (b). A nucleic acid molecule that encodes a CRISPR/Cas component, *e.g.,* that encodes a Cas9 molecule or a target gene gRNA molecule may comprise more than one target domain that is complementary with a governing gRNA targeting domain. While not wishing to be bound by theory, it is believed that a governing gRNA molecule complexes with a Cas9 molecule and results in Cas9 mediated inactivation of the targeted nucleic acid, *e.g.,* by cleavage or by binding to the nucleic acid, and results in cessation or reduction of the production of a CRISPR/Cas system component. The Cas9 molecule may form two complexes: a complex comprising a Cas9 molecule with a target gene gRNA molecule, which complex will alter the *HBB* gene; and a complex comprising a Cas9 molecule with a governing gRNA molecule, which complex will act to prevent further production of a CRISPR/Cas system component, *e.g.,* a Cas9 molecule or a target gene gRNA molecule. A governing gRNA molecule/Cas9 molecule complex may bind to or promote cleavage of a control region sequence, *e.g.,* a promoter, operably linked to a sequence that encodes a Cas9 molecule, a sequence that encodes a transcribed region, an exon, or an intron, for the Cas9 molecule. A governing gRNA molecule/Cas9 molecule complex may bind to or promote cleavage of a control region sequence, e.g., a promoter, operably linked to a gRNA molecule, or a sequence that encodes the gRNA molecule. The governing gRNA molecule, *e.g.,* a Cas9-targeting governing gRNA molecule, or a target gene gRNA-targeting governing gRNA molecule may limit the effect of the Cas9 molecule/target gene gRNA molecule complex-mediated gene targeting. A governing gRNA may place temporal, level of expression, or other limits, on activity of the Cas9 molecule/target gene gRNA molecule complex. A governing gRNA may reduce off-target or other unwanted activity. A governing gRNA molecule may inhibit, e.g., entirely or substantially entirely inhibit, the production of a component of the Cas9 system and thereby limit, or govern, its activity.

"HDR", or homology-directed repair, as used herein, refers to the process of repairing DNA damage using a homologous nucleic acid (*e.g.,* an endogenous nucleic acid, *e.g.,* a sister chromatid, or an exogenous nucleic acid, *e.g.,* a template nucleic acid). HDR typically occurs when there has been significant resection at a double-strand break, forming at least one single stranded portion of DNA. HDR is a category that includes, for example, single-strand annealing (SSA), homologous recombination (HR), single strand template repair (SST-R), and a third, not yet fully characterized alternative homologous recombination (alt-HR) DNA repair pathway. HDR may include gene conversion and gene correction. The term HDR may in some cases not encompass canonical NHEJ (C-NHEJ). The term HDR may in some cases not encompass alternative non-homologous end joining (Alt-NHEJ) (e.g., blunt end-joining (blunt EJ), (micro homology mediated end joining (MMEJ), and synthesis dependent microhomology-mediated end joining (SD-MMEJ)).

The terms "homology" or "identity," as used interchangeably herein, refer to sequence identity between two amino acid sequences or two nucleic acid sequences, with identity being a more strict comparison. The phrases "percent identity or homology" and "% identity or homology" refer to the percentage of sequence identity found in a comparison of two or more amino acid sequences or nucleic acid sequences. Two or more sequences can be anywhere from 0-100% identical, or any value there between. Identity can be determined by comparing a position in each sequence that can be aligned for purposes of comparison to a reference sequence. When a position in the compared sequence is occupied by the same nucleotide base or amino acid, then the molecules are identical at that position. A degree of identity of amino acid sequences is a function of the number of identical amino acids at positions shared by the amino ac id sequences. A degree of identity between nucleic acid sequences is a function of the number of identical or matching nucleotides at positions shared by the nucleic acid sequences. A degree of homology of amino acid sequences is a function of the number of amino acids at positions shared by the polypeptide sequences.

Calculations of homology or sequence identity between two sequences (the terms are used interchangeably herein) are performed as follows. The sequences are aligned for optimal comparison purposes (*e.g.,* gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frame shift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences.

"Gene conversion", as used herein, refers to the process of repairing DNA damage by homology directed recombination (HDR) using an endogenous nucleic acid, *e.g.,* a sister chromatid or a plasmid, as a template nucleic acid. Without being bound by theory, BRCA1, BRCA2 and/or RAD51 are in some cases believed to be involved in gene conversion. The endogenous nucleic acid may be a nucleic acid sequence having homology, *e.g.,* significant homology, with a fragment of DNA proximal to the site of the DNA lesion or mutation. The template may in some cases not be an exogenous nucleic acid.

"Gene correction", as used herein, refers to the process of repairing DNA damage by homology directed recombination using an exogenous nucleic acid, *e.g.,* a donor template nucleic acid. The exogenous nucleic acid may be single-stranded or double-stranded.

"Homologous recombination" or "HR" refers to a type of HDR DNA-repair which typically acts occurs when there has been significant resection at the double-strand break, forming at least one single stranded portion of DNA. In a normal cell, HR typically involves a series of steps such as recognition of the break, stabilization of the break, resection, stabilization of single stranded DNA, formation of a DNA crossover intermediate, resolution of the crossover intermediate, and ligation. The process requires RAD51 and BRCA2, and the homologous nucleic acid is typically double-stranded. In some embodiments, homologous recombination includes gene conversion.

"Modulator," as used herein, refers to an entity, *e.g.,* a compound, that can alter the activity (*e.g.,* enzymatic activity, transcriptional activity, or translational activity), amount, distribution, or structure of a subject molecule or genetic sequence. Modulation may comprise cleavage, *e.g.,* breaking of a covalent or non-covalent bond, or the forming of a covalent or non-covalent bond, *e.g.,* the attachment of a moiety, to the subject molecule. A modulator may alter the, three dimensional, secondary, tertiary, or quaternary structure, of a subject molecule. A modulator can increase, decrease, initiate, or eliminate a subject activity.

As used herein, the term "mutation" refers to a change in the sequence of a nucleic acid as compared to a wild-type sequence of the nucleic acid, resulting a variant form of the nucleic acid. A mutation in a nucleic acid may be caused by the alteration of a single base pair in the nucleic acid, or the insertion, deletion, or rearrangement of larger sections of the nucleic acid. A mutation in a gene may result in variants of the protein encoded by the gene which are associated with genetic disorders. For example, a mutation (*e.g.,* GAG → GTG) results in the substitution of valine for glutamic acid at amino acid position 6 in exon 1 of the *HBB* gene. This mutation in the *HBB* gene is associated with beta thalassemia and sickle cell disease.

"Non-homologous end joining" or "NHEJ," as used herein, refers to ligation mediated repair and/or non-template mediated repair including canonical NHEJ (cNHEJ), alternative NHEJ (altNHEJ), microhomology-mediated end joining (MMEJ), single-strand annealing (SSA), and synthesis-dependent microhomology-mediated end joining (SD-MMEJ). Unless indicate otherwise, "NHEJ" as used herein encompasses canonical NHEJ, alt-NHEJ, MMEJ, SSA and SD-MMEJ.

"Polypeptide," as used herein, refers to a polymer of amino acids.

As used herein, the term "processing," with respect to overhangs, refers to either the endonucleolytic processing or the exonucleolytic processing of a break in a nucleic acid molecule. Processing of a 5' overhang in a nucleic acid molecule may result in a 3' overhang. Processing of a 3' overhang in a nucleic acid molecule may result in a 5' overhang.

A "reference molecule," as used herein, refers to a molecule to which a modified or candidate molecule is compared. For example, a reference Cas9 molecule refers to a Cas9 molecule to which a modified or candidate Cas9 molecule is compared. The modified or candidate molecule may me compared to the reference molecule on the basis of sequence (*e.g.,* the modified or candidate may have X% sequence identity or homology with the reference molecule) or activity (*e.g.,* the modified or candidate molecule may have X% of the activity of the reference molecule). For example, where the reference molecule is a Cas9 molecule, a modified or candidate may be characterized as having no more than 10% of the nuclease activity of the reference Cas9 molecule. Examples of reference Cas9 molecules include naturally occurring unmodified Cas9 molecules, *e.g.,* a naturally occurring Cas9 molecule from *S. pyogenes, S. aureus, S. thermophilus* or *N. meningitidis.* The reference Cas9 molecule may be the naturally occurring Cas9 molecule having the closest sequence identity or homology with the modified or candidate Cas9 molecule to which it is being compared. The reference Cas9 molecule may be a parental molecule having a naturally occurring or known sequence on which a mutation has been made to arrive at the modified or candidate Cas9 molecule.

"Replacement," or "replaced," as used herein with reference to a modification of a molecule does not require a process limitation but merely indicates that the replacement entity is present.

"Resection", as used herein, refers to exonuclease-mediated digestion of one strand of a double-stranded DNA molecule, which results in a single-stranded overhang. Resection may occur, *e.g.,* on one or both sides of a double-stranded break. Resection can be measured by, for instance, extracting genomic DNA, digesting it with an enzyme that selectively degrades dsDNA, and performing quantitative PCR using primers spanning the DSB site, *e.g.,* as described herein.

"SSA" or "Single-strand Annealing", as used herein, refers to the process where RAD52 as opposed to RAD51 in the HR pathways, binds to the single stranded portion of DNA and promotes annealing of the two single stranded DNA segments at repetitive regions. Once RAD52 binds XFP/ERCC1 removes DNA flaps to make the DNA more suitable for ligation.

"SCD target point position," as used herein, refers to a target position in the *HBB* gene, typically a single nucleotide, which, if mutated, can result in a protein having a mutant amino acid and give rise to SCD. The SCD target position may be the target position at which a change can give rise to an E6 mutant protein, *e.g.,* a protein having an E6V substitution.

"Subject," as used herein, may mean either a human or non-human animal. The term includes, but is not limited to, mammals (*e.g.,* humans, other primates, pigs, rodents (*e.g.,* mice and rats or hamsters), rabbits, guinea pigs, cows, horses, cats, dogs, sheep, and goats). The subject may be a human. The subject may be poultry. The subject may be piscine. The subject may be a human, and in certain cases the human may be an infant, child, young adult, or adult.

As used herein, the terms "target nucleic acid" or "target gene" refer to a nucleic acid which is being targeted for alteration, e.g., by gene conversion, by a Cas9 system described herein. A target nucleic acid may comprise one gene. A target nucleic acid may comprise one or more genes, e.g., two genes, three genes, four genes, or five genes. A target nucleic acid may comprise a promoter region, or control region, of a gene. A target nucleic acid may comprise an intron of a gene. A target nucleic acid may comprise an exon of a gene. A target nucleic acid may comprise a coding region of gene. A target nucleic acid may comprise a non-coding region of a gene. The target gene is an *HBB* gene. Described herein, the target gene may be an *SMN1* gene. Described herein, the target gene may be an *NCF1* (p47-PHOX) gene.

"Target position" as used herein, refers to a site on a target nucleic acid that is modified by a Cas9 molecule-dependent process. For example, the target position can be modified by a Cas9 molecule-mediated cleavage of the target nucleic acid and template nucleic acid directed modification, *e.g.,* correction, of the target position. A target position can be a site between two nucleotides, *e.g.,* adjacent nucleotides, on the target nucleic acid into which one or more nucleotides is added based on homology with a template nucleic acid. The target position may comprise one or more nucleotides that are altered, *e.g.,* corrected, based on homology with a template nucleic acid. The target position may comprise one or more nucleotides that are deleted based on homology with a template nucleic acid. The target position may be within a "target sequence" (*e.g.,* the sequence to which the gRNA binds). A target position may be upstream or downstream of a target sequence (*e.g.,* the sequence to which the gRNA binds).

"Target region," "target domain," or "target sequence," as used herein, is a nucleic acid sequence that comprises a target position and at least one nucleotide position outside the target position. The target position may be flanked by sequences of the target position region, *i.e.,* the target position is disposed in the target position region such that there are target position region sequences both 5' and 3' to the target position. The target position region may provide sufficient sequences on each side (*i.e.,* 5' and 3') of the target position to allow gene conversion of the target position, wherein the gene conversion uses an endogenous sequence homologous with the target position region as a template.

A "template nucleic acid," as the term is used herein, refers to a nucleic acid sequence which can be used in conjunction with a Cas9 molecule and a gRNA molecule to alter the structure of a target position. The target nucleic acid may be modified to have some or all of the sequence of the template nucleic acid, typically at or near cleavage site(s). The template nucleic acid may be single stranded or double stranded. The template nucleic acid may be DNA, *e.g.,* double stranded DNA. The template nucleic acid may be single stranded DNA. The template nucleic acid may be RNA, *e.g.,* double stranded RNA or single stranded RNA. The template nucleic acid may be encoded on the same vector backbone, *e.g.,* AAV genome, plasmid DNA, as the Cas9 and gRNA. The template nucleic acid may be excised from a vector backbone *in vivo, e.g.,* it is flanked by gRNA recognition sequences. The template DNA may be in an ILDV. The template nucleic acid may be an exogenous nucleic acid sequence. The template nucleic acid sequence may be an endogenous nucleic acid sequence, *e.g.,* an endogenhous homologous region. The template nucleic acid may be a single stranded oligonucleotide corresponding to a plus strand of a nucleic acid sequence. The template nucleic acid may be a single stranded oligonucleotide corresponding to a minus strand of a nucleic acid sequence.

"Treat," "treating" and "treatment," as used herein, mean the treatment of a disease in a mammal, *e.g.,* in a human, including (a) inhibiting the disease, *i.e.,* arresting or preventing its development or progression; (b) relieving the disease, *i.e.,* causing regression of the disease state; and (c) relieving one or more symptoms of the disease; and (d) curing the disease.

"Prevent," "preventing" and "prevention," as used herein, means the prevention of a disease in a mammal, *e.g.,* in a human, including (a) avoiding or precluding the disease; (b) affecting the predisposition toward the disease (c) preventing or delaying the onset of at least one symptom of the disease.

An "up-regulator", as used herein, refers to an agent that directly increases the activity of a specified biological pathway. Directly increasing the activity of the pathway refers to (i) the up-regulator binding to a component of that pathway (*e.g.,* a protein that acts in the pathway or an mRNA encoding that protein) and increasing the level or activity of that component, *e.g.,* by increasing the concentration or specific activity of that component, or (ii) the up-regulator is an added amount of a component that is ordinarily present in the pathway at a given level, *e.g.,* an overexpressed protein. An up-regulator may, *e.g.,* speed up one of the steps of that pathway or increase the level or activity of a component in that pathway. An up-regulator may be, *e.g.,* a protein in the pathway, *e.g.,* one may overexpress a protein that is ordinarily in the pathway to increase the overall activity of the pathway. The pathway may be, *e.g.,* a DNA damage repair pathway, for example, HDR, *e.g.,* gene conversion. The increased level or activity may be compared to what would be seen in the absence of the up-regulator.

"Wild type", as used herein, refers to a gene or polypeptide which has the characteristics, *e.g.,* the nucleotide or amino acid sequence, of a gene or polypeptide from a naturally-occurring source. The term "wild type" typically includes the most frequent observation of a particular gene or polypeptide in a population of organisms found in nature.

"X" as used herein in the context of an amino acid sequence, refers to any amino acid (*e.g.,* any of the twenty natural amino acids) unless otherwise specified.

### Sickle Cell Disease and Methods of Repairing Mutation(s) in the HBB Gene

Sickle Cell Disease (SCD), also known as Sickle Cell Anemia (SCA), is a common inherited hematologic disease which affects 80,000 - 90,000 people in the United States. It is common in people of African descent and in Hispanic-Americans, with the prevalence of SCD being 1 in 500 and 1 in 1,000, respectively.

SCD is caused by a mutation in the *beta-globin* (*HBB*) gene. *HBB* is located on chromosome 11 within the *HBB* gene cluster, which includes genes encoding the delta globin chain, A gamma chain, G gamma chain. The alpha-globin gene is located on chromosome 16. A point mutation (e.g., GAG → GTG) results in the substitution of valine for glutamic acid at amino acid position 6 in exon 1 of the *HBB* gene. Beta hemoglobin chains with this mutation are expressed as HbS. The disease is inherited in an autosomal recessive manner, so that only patients with two HbS alleles have SCD. Subjects who have sickle cell trait (are heterozygous for HbS) only display a phenotype if they are severely dehydrated or oxygen deprived.

Normal adult hemoglobin (Hb) is composed of a tetramer made from two alpha-globin chains and two beta-globin chains. In SCD, the valine at position 6 of the beta-chain is hydrophobic and causes a change in conformation of the beta-globin protein when it is not bound to oxygen. HbS is more likely to polymerize and leads to the characteristic sickle shaped red blood cells (RBCs) found in SCD.

Sickle shape RBCs cause multiple manifestations of disease, which include, *e.g.,* anemia, sickle cell crises, vaso-occlusive crises, aplastic crises and acute chest syndrome. The disease has various manifestations, *e.g*., vaso-occlusive crisis, splenic sequestration crisis and anemia. Subjects may also suffer from acute chest crisis and infarcts of extremities, end organs and central nervous system. Current treatments for SCD include, *e.g.,* hydration, transfusion, analgesics, the use of hydroxyurea, supplementation with folic acid, penicillin prophylaxis during childhood, and bone marrow transplants. However, there remains a need for additional methods and compositions that can be used to cure, not just treat, sickle cell disease and other genetic diseases.

One approach to treat or prevent SCD is to repair *(i.e.,* correct) one or more mutations in the *HBB* gene. In this approach, mutant *HBB* allele(s) are corrected and restored to wild type state. While not wishing to be bound by theory, it is believed that correction of the glutamic acid to valine substitution at amino acid 6 in the beta-globin gene restores wild type beta-globin production within erythroid cells. The instant disclosure provides methods for modifying target genes, such as *HBB,* for repair by gene conversion using an endogenous homologous region, such as a region of the *HBD* gene. Specifically, the instant disclosure increases the rates of repair by gene conversion as compared to previously disclosed technologies. The methods described herein can be performed in all cell types, including mammalian and human cells. Specifically with respect to SCD, beta-globin is expressed in cells of erythroid cell lineage. Thus, an erythroid cell may be targeted.

While much of the disclosure herein is presented in the context of the mutation in the *HBB* gene that gives rise to an E6 mutant protein (*e.g.,* E6V mutant protein), the methods and compositions herein are broadly applicable to any mutation, *e.g.,* a point mutation or a deletion, in any gene, including promoters and control regions of a gene, that gives rise to any disease.

One *HBB* allele may be repaired in the subject or both *HBB* alleles may be repaired in the subject. In either situation, the subject can be cured of disease. As the disease only displays a phenotype when both alleles are mutated, repair of a single allele is adequate for a cure.

Methods and compositions discussed herein, provide for the correction of the underlying genetic cause of SCD, *e.g.,* the correction of a mutation at a target position in the *HBB* gene, *e.g.,* correction of a mutation at amino acid position 6, *e.g.,* an E6V substitution in the *HBB* gene.

The method provides for the correction of a mutation at a target position in the *HBB* gene, *e.g.,* correction of a mutation at amino acid position 6, *e.g.,* an E6V substitution in the *HBB* gene. As described herein, the method comprises the introduction of one or more breaks (*e.g.,* single-strand breaks or double-strand breaks) sufficiently close to (*e.g.,* either 5' or 3' to) the target position in the *HBB* gene, *e.g.,* E6V.

The targeting domain of the gRNA molecule may be configured to provide a cleavage event, *e.g.,* a double-strand break or a single-strand break, sufficiently close to (*e.g.,* either 5' or 3' to) the target position in the *HBB* gene, *e.g.,* E6V to allow correction, *e.g.,* an alteration in the *HBB* gene, *e.g.,* an alternation associated with HDR. The targeting domain may be configured such that a cleavage event, *e.g.,* a double-strand or single-strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of a the target position in the *HBB* gene, *e.g.,* E6V. The break, *e.g.,* a double-strand or single-strand break, can be positioned upstream or downstream of the target position in the *HBB* gene, *e.g.,* E6V.

A second, third and/or fourth gRNA molecule may be configured to provide a cleavage event, *e.g.,* a double-strand break or a single-strand break, sufficiently close to *(e.g.,* either 5' or 3' to) the target position in the *HBB* gene, *e.g.,* E6V to allow correction, *e.g.,* an alteration associated with HDR in the *HBB* gene. The targeting domain may be configured such that a cleavage event, *e.g.,* a double-strand or single-strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of a the target position in the *HBB* gene, *e.g.,* E6V. The break, *e.g.,* a double-strand or single-strand break, can be positioned upstream or downstream of the target position in the *HBB* gene, *e.g.,* E6V.

A single-strand break may be accompanied by an additional single-strand break, positioned by a second, third and/or fourth gRNA molecule, as discussed below. For example, the targeting domains bind configured such that a cleavage event, *e.g.,* the two single-strand breaks, are positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position in the *HBB* gene, *e.g.,* E6V. The first and second gRNA molecules may be configured such, that when guiding a Cas9 nickase, a single-strand break is accompanied by an additional single-strand break, positioned by a second gRNA molecule, sufficiently close to one another to result in an alteration of the target position in the *HBB* gene, *e.g.,* E6V. The first and second gRNA molecules may be configured such that a single-strand break positioned by said second gRNA is within 10, 20, 30, 40, or 50 nucleotides of the break positioned by said first gRNA molecule, *e.g.,* when the Cas9 is a nickase. The two gRNA molecules may be configured to position cuts at the same position, or within a few nucleotides of one another, on different strands, *e.g.,* essentially mimicking a double-strand break.

A double-strand break can be accompanied by an additional double-strand break, positioned by a second, third and/or fourth gRNA molecule, as is discussed below. For example, the targeting domain of a first gRNA molecule is configured such that a double-strand break is positioned upstream of the target position in the *HBB* gene, *e.g.,* E6V, *e.g.,* within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domain of a second gRNA molecule is configured such that a double-strand break is positioned downstream the target position in the *HBB* gene, *e.g.,* E6V, *e.g.,* within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

A double-strand break can be accompanied by two additional single-strand breaks, positioned by a second gRNA molecule and a third gRNA molecule. For example, the targeting domain of a first gRNA molecule is configured such that a double-strand break is positioned upstream of the target position in the *HBB* gene, *e.g.,* E6V, *e.g.,* within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a second and third gRNA molecule are configured such that two single-strand breaks are positioned downstream of the target position in the *HBB* gene, *e.g.,* E6V, *e.g.,* within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. The targeting domain of the first, second and third gRNA molecules may be configured such that a cleavage event, *e.g.,* a double-strand or single-strand break, is positioned, independently for each of the gRNA molecules.

A first and second single-strand breaks can be accompanied by two additional single-strand breaks positioned by a third gRNA molecule and a fourth gRNA molecule. For example, the targeting domain of a first and second gRNA molecule are configured such that two single-strand breaks are positioned upstream of the target position in the *HBB* gene, *e.g.,* E6V, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position in the *HBB* gene, *e.g.,* E6V; and the targeting domains of a third and fourth gRNA molecule are configured such that two single-strand breaks are positioned downstream of the target position in the *HBB* gene, *e.g.,* E6V, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position in the *HBB* gene, *e.g.,* E6V.

A mutation in the *HBB* gene, *e.g.,* E6V may be corrected using an exogenously provided template nucleic acid, *e.g.,* by HDR. A mutation in the *HBB* gene, *e.g.,* E6V may be corrected without using an exogenously provided template nucleic acid, *e.g.,* by HDR. According to one aspect of the invention, alteration of the target sequence occurs with an endogenous genomic donor sequence, *e.g.,* by HDR. The endogenous genomic donor sequence comprises one or more nucleotides derived from the *HBD* gene. According to one aspect of the invention, a mutation in the *HBB* gene, *e.g.,* E6V is corrected by an endogenous genomic donor sequence (*e.g., an HBD* gene). An eaCas9 molecule, *e.g.,* an eaCas9 molecule described herein, is used. In an embodiment, the eaCas9 molecule comprises HNH-like domain cleavage activity but has no, or no significant, N-terminal RuvC-like domain cleavage activity. In an embodiment, the eaCas9 molecule is an HNH-like domain nickase. In an embodiment, the eaCas9 molecule comprises a mutation at D10 (*e.g.,* D10A). The eaCas9 molecule may comprise N-terminal RuvC-like domain cleavage activity but have no, or no significant, HNH-like domain cleavage activity. The eaCas9 molecule may be an N-terminal RuvC-like domain nickase. The eaCas9 molecule may comprise a mutation at H840 (*e.g.,* H840A) or N863 (*e.g.,* N863A).

### Methods to Treat or Prevent Sickle Cell Disease (SCD)

Disclosed herein are the approaches to treat or prevent SCD, using the compositions and methods described herein.

One approach to treat or prevent disease, *e.g.,* SCD, is to repair (*i.e.,* correct) one or more mutations in a target gene, *e.g.,* a *HBB* gene, *e.g.,* by gene conversion, using an endogenous homologous region. In this approach, mutant *HBB* allele(s) are corrected and restored to wild type state. While not wishing to be bound by theory, it is believed that correction of the glutamic acid to valine substitution at amino acid 6 in the beta-globin gene restores wild type beta-globin production within erythroid cells. The method described herein can be performed in all cell types. Beta-globin is expressed in cells of erythroid cell lineage. An erythroid cell may be targeted.

One *HBB* allele may be repaired in the subject or both *HBB* alleles may be repaired in the subject. In either situation, the subjects can be cured of disease. As the disease only displays a phenotype when both alleles are mutated, repair of a single allele is adequate for a cure.

The method may comprise initiating treatment of a subject prior to disease onset or after disease onset.

The method may comprise initiating treatment of a subject well after disease onset, *e.g.,* 1, 2, 3, 4, 5, 6, 7, 8, 9, 10, 12, 16, 24, 36, 48 or more months after onset of disease. While not wishing to be bound by theory it is believed that this treatment may be effective if subjects present well into the course of illness.

The method may comprise initiating treatment of a subject in an advanced stage of disease.

Overall, initiation of treatment for subjects at all stages of disease is expected to prevent negative consequences of disease and be of benefit to subjects.

The method may comprise initiating treatment of a subject prior to disease expression. The method may comprise initiating treatment of a subject in an early stage of disease, *e.g.,* when a subject has tested positive for beta-thalassemia mutations but has no signs or symptoms associated with beta-thalassemia major, minor or intermedia.

The method may comprise initiating treatment of a subject at the appearance of microcytic anemia, *e.g.,* in an infant, child, adult or young adult.

The method may comprise initiating treatment of a subject who is transfusion-dependent.

The method may comprise initiating treatment of a subject who has tested positive for a mutation in a beta globin gene.

The method may comprise initiating treatment at the appearance of any one or more of the following findings associated or consistent with beta-thalassemia major or beta-thalassemia minor: anemia, diarrhea, fever, failure to thrive, frontal bossing, broken long bones, hepatomegaly, splenomegaly, thrombosis, pulmonary embolus, stroke, leg ulcer, cardiomyopathy, cardiac arrhythmia, and evidence of extramedullary erythropoiesis.

A cell may be treated, *e.g., ex vivo.* An *ex vivo* treated cell may be returned to a subj ect.

Allogenic or autologous bone marrow or erythroid cells may be treated *ex vivo.* An *ex vivo* treated allogenic or autologous bone marrow or erythroid cells may be administered to the subject. An erythroid cell, *e.g.,* an autologous erythroid cell, may be treated *ex vivo* and returned to the subject. An autologous stem cellmay be treated *ex vivo* and returned to the subject. The modified HSCs may be administered to the patient following no myeloablative pre-conditioning. The modified HSCs may be administered to the patient following mild myeloablative pre-conditioning such that following engraftment, some of the hematopoietic cells may be derived from the modified HSCs. In other aspects, the HSCs may be administered after full myeloablation such that following engraftment, 100% of the hematopoietic cells are derived from the modified HSCs.

The method may comprise delivery of a gRNA molecule and Cas9 molecule by intravenous injection, intramuscular injection, subcutaneous injection, or intra-bone marrow (IBM) injection.

The method may comprise delivery of a gRNA molecule and/or a Cas9 molecule by an AAV. The method may comprise delivery of a gRNA molecule and/or a Cas9 molecule by a lentivirus. The method may comprise delivery of a gRNA molecule and/or a Cas9 molecule by a nanoparticle. The method may comprise delivery of a gRNA molecule by a parvovirus, *e.g.,* a modified parvovirus specifically designed to target bone marrow cells and/or CD4+ cells. Two or more gRNA molecules (*e.g.,* a second, third or fourth gRNA molecules) may be delivered.

### Other Methods to Treat Sickle Cell Disease

In healthy individuals, two beta-globin molecules pair with two alpha-globin molecules to form normal hemoglobin (Hb). In SCD, mutations in the *beta-globin* (*HBB*) gene, *e.g.,* a point mutation (GAG → GTG) that results in the substitution of valine for glutamic acid at amino acid position 6 of the beta-globin molecule, cause production of sickle hemoglobin (HbS). HbS is more likely to polymerize and leads to the characteristic sickle shaped red blood cells (RBCs). Sickle shaped RBCs give rise to multiple manifestations of disease, such as, anemia, sickle cell crises, vaso-occlusive crises, aplastic crises and acute chest syndrome. Alpha-globin can also pair with fetal hemoglobin (HbF), which significantly moderates the severe anemia and other symptoms of SCD. However, the expression of HbF is negatively regulated by the *BCL11A* gene product.

Methods and compositions disclosed herein provide a number of approaches for treating SCD. As is discussed *infra,* methods described herein provide for treating SCD by correcting a target position in the *HBB* gene to provide corrected, or functional, *e.g.,* wild type, beta-globin. Methods and compositions discussed herein can be used in combination with targeting a second gene, *e.g.,* a gene other than the *HBB* gene, *e.g.,* the *BCL11A* gene (also known as B-cell CLL/lymphoma 11A, *BCL11A-L, BCL11A-S, BCL11A-XL, CTIP1, HBFQTL5* and *ZNF*), *e.g.,* by a CRISPR/Cas related method. *BCL11A* encodes a zinc-finger protein that is involved in the regulation of globin gene expression. By altering the *BCL11A* gene *(e.g.,* one or both alleles of the *BCL11A* gene), the levels of gamma globin can be increased. Gamma globin can replace beta globin in the hemoglobin complex and effectively carry oxygen to tissues, thereby ameliorating SCD disease phenotypes. Altering the *BCL11A* gene herein refers to reducing or eliminating (1) *BCL11A* gene expression, (2) *BCL11A* protein function, or (3) the level of *BCL11A* protein. An SCD target knockdown position may be targeted.

"SCD target knockout position", as used herein, refers to a position in the *BCL11A* gene, which if altered, *e.g.,* disrupted by insertion or deletion of one or more nucleotides, *e.g.,* by NHEJ-mediated alteration, results in reduction or elimination of expression of functional *BCL11A* gene product. The position may be in the *BCL11A* coding region, *e.g.,* an early coding region. The position may be in the *BCL11A* non-coding region, *e.g.,* an enhancer region.

"SCD target knockdown position", as used herein, refers to a position, *e.g.,* in the *BCL11A* gene, which if targeted by an eiCas9 or an eiCas9 fusion described herein, results in reduction or elimination of expression of functional *BCL11A* gene product. Transcription may be reduced or eliminated. The position may be in the *BCL11A* promoter sequence. A position in the promoter sequence of the *BCL11A* gene may be targeted by an enzymatically inactive Cas9 (eiCas9) or an eiCas9-fusion protein, as described herein.

### I. Guide RNA (gRNA) Molecules

A gRNA molecule, as that term is used herein, refers to a nucleic acid that promotes the specific targeting or homing of a gRNA molecule/Cas9 molecule complex to a target nucleic acid. gRNA molecules can be unimolecular (having a single RNA molecule) (*e.g.,* chimeric or modular (comprising more than one, and typically two, separate RNA molecules). The gRNA molecules provided herein comprise a targeting domain comprising, consisting of, or consisting essentially of a nucleic acid sequence fully or partially complementary to a target domain. The gRNA molecule may further comprise one or more additional domains, including for example a first complementarity domain, a linking domain, a second complementarity domain, a proximal domain, a tail domain, and a 5' extension domain. Each of these domains is discussed in detail below. Additional details on gRNAs are provided in Section I entitled "gRNA molecules" of PCT Application WO 2015/048577. One or more of the domains in the gRNA molecule may comprise an amino acid sequence identical to or sharing sequence homology with a naturally occurring sequence, *e.g.,* from S. *pyogenes, S. aureus, or S. thermophilus.*

A unimolecular, or chimeric, gRNA may comprise, preferably from 5' to 3':
a targeting domain complementary to a target domain in a *HBB* gene, *e.g.,* a targeting domain from any of SEQ ID NOs: 387-485, 6803-6871, or 16010-16256;
a first complementarity domain;
a linking domain;
a second complementarity domain (which is complementary to the first complementarity domain);
a proximal domain; and
optionally, a tail domain.

A modular gRNA may comprise:
a first strand comprising, preferably from 5' to 3':
   a targeting domain (which is complementary to a target domain in the *HBB* gene), *e.g.,* a targeting domain from any one of SEQ ID NOs: 387-485, 6803-6871, or 16010-16256; and
   a first complementarity domain; and
a second strand, comprising, preferably from 5' to 3':
   optionally, a 5' extension domain;
   a second complementarity domain;
   a proximal domain; and
   optionally, a tail domain.

Each of these domains are described in more detail, below.

### Targeting Domain

The targeting domain (sometimes referred to alternatively as the guide sequence or complementarity region) comprises, consists of, or consists essentially of a nucleic acid sequence that is complementary or partially complementary to a target nucleic acid sequence, *e.g.,* a target nucleic acid sequence in a *HBB* target gene. The nucleic acid sequence in a target gene, *e.g., HBB,* to which all or a portion of the targeting domain is complementary or partially complementary is referred to herein as the target domain. The target domain may comprise a target position within the target gene *HBB.* A target position may lie outside (*i.e.,* upstream or downstream of) the target domain. The target domain may be located entirely within a target gene, *e.g.,* in a coding region, an intron, or an exon. All or part of the target domain may be located outside of a target gene, *e.g.,* in a control region or in a non-coding region.

Methods for selecting targeting domains are known in the art (see, *e.g.,* Fu 2014; Sternberg 2014). Examples of suitable targeting domains for use in the methods, compositions, and kits described herein include those set forth in SEQ ID NOs: 387-485, 6803-6871, or 16010-16256.

The strand of the target nucleic acid comprising the target domain is referred to herein as the "complementary strand" because it is complementary to the targeting domain sequence. Since the targeting domain is part of a gRNA molecule, it comprises the base uracil (U) rather than thymine (T); conversely, any DNA molecule encoding the gRNA molecule will comprise thymine rather than uracil. In a targeting domain/target domain pair, the uracil bases in the targeting domain will pair with the adenine bases in the target domain. In certain embodiments, the degree of complementarity between the targeting domain and target domain is sufficient to allow targeting of a Cas9 molecule to the target nucleic acid.

The targeting domain may comprise a core domain and an optional secondary domain. The core domain may be located 3' to the secondary domain, and the core domain may be located at or near the 3' end of the targeting domain. The core domain may consist of or consist essentially of about 8 to about 13 nucleotides at the 3' end of the targeting domain. Only the core domain may be complementary or partially complementary to the corresponding portion of the target domain, and the core domain may be fully complementary to the corresponding portion of the target domain. The secondary domain may also be complementary or partially complementary to a portion of the target domain. The core domain may be complementary or partially complementary to a core domain target in the target domain, while the secondary domain may be complementary or partially complementary to a secondary domain target in the target domain. The core domain and secondary domain may have the same degree of complementarity with their respective corresponding portions of the target domain. The degree of complementarity between the core domain and its target and the degree of complementarity between the secondary domain and its target may differ. The core domain may have a higher degree of complementarity for its target than the secondary domain, or the secondary domain may have a higher degree of complementarity than the core domain.

The targeting domain and/or the core domain within the targeting domain may be 3 to 100, 5 to 100, 10 to 100, or 20 to 100 nucleotides in length, and in certain of these cases the targeting domain or core domain is 3 to 15, 3 to 20, 5 to 20, 10 to 20, 15 to 20, 5 to 50, 10 to 50, or 20 to 50 nucleotides in length. The targeting domain and/or the core domain within the targeting domain may be 6, 7, 8, 9, 10, 11, 12, 13, 14, 15, 16, 17, 18, 19, 20, 21, 22, 23, 24, 25, or 26 nucleotides in length. The targeting domain and/or the core domain within the targeting domain may be 6 +/-2, 7+/-2, 8+/-2, 9+/-2, 10+/-2, 10+/-4, 10 +/-5, 11+/-2, 12+/-2, 13+/-2, 14+/-2, 15+/-2, or 16+-2, 20+/-5, 30+/-5, 40+/-5, 50+/-5, 60+/-5, 70+/-5, 80+/-5, 90+/-5, or 100+/-5 nucleotides in length.

Wherein the targeting domain may include a core domain, the core domain is 3 to 20 nucleotides in length, and in certain of these cases the core domain 5 to 15 or 8 to 13 nucleotides in length. Wherein the targeting domain includes a secondary domain, the secondary domain may be 0, 1, 2, 3, 4, 5, 6, 7, 8, 9, 10, 11, 12, 13, 14 or 15 nucleotides in length. Wherein the targeting domain comprises a core domain that is 8 to 13 nucleotides in length, the targeting domain may be 26, 25, 24, 23, 22, 21, 20, 19, 18, 17, or 16 nucleotides in length, and the secondary domain may be 13 to 18, 12 to 17, 11 to 16, 10 to 15, 9 to 14, 8 to 13, 7 to 12, 6 to 11, 5 to 10, 4 to 9, or 3 to 8 nucleotides in length, respectively.

The targeting domain may be fully complementary to the target domain. Likewise, where the targeting domain comprises a core domain and/or a secondary domain, one or both of the core domain and the secondary domain may be fully complementary to the corresponding portions of the target domain. The targeting domain may be partially complementary to the target domain, and in certain of these cases where the targeting domain comprises a core domain and/or a secondary domain, one or both of the core domain and the secondary domain may be partially complementary to the corresponding portions of the target domain. In certain of these cases, the nucleic acid sequence of the targeting domain, or the core domain or targeting domain within the targeting domain, may be at least 80%, 85%, 90%, or 95% complementary to the target domain or to the corresponding portion of the target domain. The targeting domain and/or the core or secondary domains within the targeting domain may include one or more nucleotides that are not complementary with the target domain or a portion thereof, and in certain of these cases the targeting domain and/or the core or secondary domains within the targeting domain may include 1, 2, 3, 4, 5, 6, 7, or 8 nucleotides that are not complementary with the target domain. The core domain may include 1, 2, 3, 4, or 5 nucleotides that are not complementary with the corresponding portion of the target domain. Wherein the targeting domain includes one or more nucleotides that are not complementary with the target domain, one or more of said non-complementary nucleotides may be located within five nucleotides of the 5' or 3' end of the targeting domain. In certain of these cases, the targeting domain may include 1, 2, 3, 4, or 5 nucleotides within five nucleotides of its 5' end, 3' end, or both its 5' and 3' ends that are not complementary to the target domain. Wherein the targeting domain includes two or more nucleotides that are not complementary to the target domain, two or more of said non-complementary nucleotides may be adjacent to one another, and in certain of these cases the two or more consecutive non-complementary nucleotides may be located within five nucleotides of the 5' or 3' end of the targeting domain. The two or more consecutive non-complementary nucleotides may both be located more than five nucleotides from the 5' and 3' ends of the targeting domain.

The gRNA molecule, *e.g.,* a gRNA molecule comprising a targeting domain, which is complementary with the *HBB* gene, may be a modular gRNA molecule. The gRNA molecule may be a unimolecular or chimeric gRNA molecule.

The nucleic acid may encode a gRNA molecule, *e.g.,* the first gRNA molecule, comprising a targeting domain comprising a sequence that is the same as, or differs by no more than 1, 2, 3, 4, or 5 nucleotides from, a targeting domain sequence described herein, *e.g.,* a targeting domain sequence from any one of **Tables 1-3.** The nucleic acid may encode a gRNA molecule comprising a targeting domain described herein, *e.g.,* selected from those in **Tables 1-3.**

The targeting domain may comprise 16 nucleotides. The targeting domain may comprise 17 nucleotides. The targeting domain may comprise 18 nucleotides. The targeting domain may comprise 19 nucleotides. The targeting domain may comprise 20 nucleotides. The targeting domain may comprise 21 nucleotides. The targeting domain may comprise 22 nucleotides. The targeting domain may comprise 23 nucleotides. The targeting domain may comprise 24 nucleotides. The targeting domain may comprise 25 nucleotides. The targeting domain may comprise 26 nucleotides.

The targeting domain which is complementary with the *HBB* gene may be 16 nucleotides or more in length. The targeting domain may be 17 nucleotides in length. The targeting domain may be 18 nucleotides in length. The targeting domain may be 19 nucleotides in length. The targeting domain may be 20 nucleotides in length. The targeting domain may be 21 nucleotides in length. The targeting domain may be 22 nucleotides in length. The targeting domain may be 23 nucleotides in length. The targeting domain may be 24 nucleotides in length. The targeting domain may be 25 nucleotides in length. The targeting domain may be 26 nucleotides in length.

A nucleic acid may encode a modular gRNA molecule, *e.g.,* one or more nucleic acids encode a modular gRNA molecule. A nucleic acid may encode a chimeric gRNA molecule. The nucleic acid may encode a gRNA molecule, *e.g.,* the first gRNA molecule, comprising a targeting domain comprising 16 nucleotides or more in length. The nucleic acid may encode a gRNA molecule, *e.g.,* the first gRNA molecule, comprising a targeting domain that is 16 nucleotides in length. The nucleic acid may encode a gRNA molecule, *e.g.,* the first gRNA molecule, comprising a targeting domain that is 17 nucleotides in length. The nucleic acid may encode a gRNA molecule, *e.g.,* the first gRNA molecule, comprising a targeting domain that is 18 nucleotides in length. The nucleic acid may encode a gRNA molecule, *e.g.,* the first gRNA molecule, comprising a targeting domain that is 19 nucleotides in length. The nucleic acid may encode a gRNA molecule, *e.g.,* the first gRNA molecule, comprising a targeting domain that is 20 nucleotides in length. The nucleic acid may encode a gRNA molecule, *e.g.,* the first gRNA molecule, comprising a targeting domain that is 21 nucleotides in length. The nucleic acid may encode a gRNA molecule, *e.g.,* the first gRNA molecule, comprising a targeting domain that is 22 nucleotides in length. The nucleic acid may encode a gRNA molecule, *e.g.,* the first gRNA molecule, comprising a targeting domain that is 23 nucleotides in length. The nucleic acid may encode a gRNA molecule, *e.g.,* the first gRNA molecule, comprising a targeting domain that is 24 nucleotides in length. The nucleic acid may encode a gRNA molecule, *e.g.,* the first gRNA molecule, comprising a targeting domain that is 25 nucleotides in length. The nucleic acid may encode a gRNA molecule, *e.g.,* the first gRNA molecule, comprising a targeting domain that is 26 nucleotides in length.

The targeting domain, core domain, and/or secondary domain in some cases do not comprise any modifications. The targeting domain, core domain, and/or secondary domain, or one or more nucleotides therein, may have a modification, including but not limited to the modifications set forth below. One or more nucleotides of the targeting domain, core domain, and/or secondary domain may comprise a 2' modification (*e.g.,* a modification at the 2' position on ribose), *e.g.,* a 2-acetylation, *e.g.,* a 2' methylation. The backbone of the targeting domain can be modified with a phosphorothioate. Modifications to one or more nucleotides of the targeting domain, core domain, and/or secondary domain may render the targeting domain and/or the gRNA comprising the targeting domain less susceptible to degradation or more bio-compatible, *e.g.,* less immunogenic. The targeting domain and/or the core or secondary domains may include 1, 2, 3, 4, 5, 6, 7, or 8 or more modifications, and in certain of these cases the targeting domain and/or core or secondary domains may include 1, 2, 3, or 4 modifications within five nucleotides of their respective 5' ends and/or 1, 2, 3, or 4 modifications within five nucleotides of their respective 3' ends. The targeting domain and/or the core or secondary domains may comprise modifications at two or more consecutive nucleotides.

Wherein the targeting domain includes core and secondary domains, the core and secondary domains may contain the same number of modifications. In certain of these cases, both domains are free of modifications. The core domain may include more modifications than the secondary domain, or vice versa.

Modifications to one or more nucleotides in the targeting domain, including in the core or secondary domains, may be selected to not interfere with targeting efficacy, which can be evaluated by testing a candidate modification using a system as set forth below. gRNAs having a candidate targeting domain having a selected length, sequence, degree of complementarity, or degree of modification can be evaluated using a system as set forth below. The candidate targeting domain can be placed, either alone or with one or more other candidate changes in a gRNA molecule/Cas9 molecule system known to be functional with a selected target, and evaluated.

All of the modified nucleotides may be complementary to and capable of hybridizing to corresponding nucleotides present in the target domain or 1, 2, 3, 4, 5, 6, 7 or 8 or more modified nucleotides are not complementary to or capable of hybridizing to corresponding nucleotides present in the target domain.

### First and second complementarity domains

The first and second complementarity (sometimes referred to alternatively as the crRNA-derived hairpin sequence and tracrRNA-derived hairpin sequences, respectively) domains are fully or partially complementary to one another. The degree of complementarity may be sufficient for the two domains to form a duplexed region under at least some physiological conditions. The degree of complementarity between the first and second complementarity domains, together with other properties of the gRNA, may be sufficient to allow targeting of a Cas9 molecule to a target nucleic acid.

The first and/or second complementarity domain may include one or more nucleotides that lack complementarity with the corresponding complementarity domain. The first and/or second complementarity domain may include 1, 2, 3, 4, 5, or 6 nucleotides that do not complement with the corresponding complementarity domain. For example, the second complementarity domain may contain 1, 2, 3, 4, 5, or 6 nucleotides that do not pair with corresponding nucleotides in the first complementarity domain. The nucleotides on the first or second complementarity domain that do not complement with the corresponding complementarity domain may loop out from the duplex formed between the first and second complementarity domains. In certain of these cases, the unpaired loop-out is located on the second complementarity domain, and in certain of these cases the unpaired region begins 1, 2, 3, 4, 5, or 6 nucleotides from the 5' end of the second complementarity domain.

The first complementarity domain may be 5 to 30, 5 to 25, 7 to 25, 5 to 24, 5 to 23, 7 to 22, 5 to 22, 5 to 21, 5 to 20, 7 to 18, 7 to 15, 9 to 16, or 10 to 14 nucleotides in length, and in certain of these cases the first complementarity domain may be 5, 6, 7, 8, 9, 10, 11, 12, 13, 14, 15, 16, 17, 18, 19, 20, 21, 22, 23, 24, or 25 nucleotides in length. The second complementarity domain may be 5 to 27, 7 to 27, 7 to 25, 5 to 24, 5 to 23, 5 to 22, 5 to 21, 7 to 20, 5 to 20, 7 to 18, 7 to 17, 9 to 16, or 10 to 14 nucleotides in length, and in certain of these cases the second complementarity domain may be 5, 6, 7, 8, 9, 10, 11, 12, 13, 14, 15, 16, 17, 18, 19, 20, 21, 22, 23, 24, 25, or 26 nucleotides in length. The first and second complementarity domains may each be independently 6 +/-2, 7+/-2, 8+/-2, 9+/-2, 10+/-2, 11+/-2, 12+/-2, 13+/-2, 14+/-2, 15+/-2, 16+/-2, 17+/-2, 18+/-2, 19+/-2, or 20+/-2, 21+/-2, 22+/-2, 23+/-2, or 24+/-2 nucleotides in length. The second complementarity domain may be longer than the first complementarity domain, *e.g.,* 2, 3, 4, 5, or 6 nucleotides longer.

The first and/or second complementarity domains may each independently comprise three subdomains, which, in the 5' to 3' direction are: a 5' subdomain, a central subdomain, and a 3' subdomain. The 5' subdomain and 3' subdomain of the first complementarity domain may be fully or partially complementary to the 3' subdomain and 5' subdomain, respectively, of the second complementarity domain.

The 5' subdomain of the first complementarity domain may be 4 to 9 nucleotides in length, and in certain of these cases the 5' domain may be 4, 5, 6, 7, 8, or 9 nucleotides in length. The 5' subdomain of the second complementarity domain may be 3 to 25, 4 to 22, 4 to 18, or 4 to 10 nucleotides in length, and in certain of these cases the 5' domain may be 3, 4, 5, 6, 7, 8, 9, 10, 11, 12, 13, 14, 15, 16, 17, 18, 19, 20, 21, 22, 23, 24, or 25 nucleotides in length. The central subdomain of the first complementarity domain may be 1, 2, or 3 nucleotides in length. The central subdomain of the second complementarity domain may be 1, 2, 3, 4, or 5 nucleotides in length. The 3' subdomain of the first complementarity domain may be 3 to 25, 4 to 22, 4 to 18, or 4 to 10 nucleotides in length, and in certain of these cases the 3' subdomain may be 3, 4, 5, 6, 7, 8, 9, 10, 11, 12, 13, 14, 15, 16, 17, 18, 19, 20, 21, 22, 23, 24, or 25 nucleotides in length. The 3' subdomain of the second complementarity domain may be 4 to 9, *e.g.,* 4, 5, 6, 7, 8 or 9 nucleotides in length.

The first and/or second complementarity domains can share homology with, or be derived from, naturally occurring or reference first and/or second complementarity domain. In certain of these cases, the first and/or second complementarity domains have at least 50%, 60%, 70%, 80%, 85%, 90%, or 95% homology with, or differ by no more than 1, 2, 3, 4, 5, or 6 nucleotides from, the naturally occurring or reference first and/or second complementarity domain. In certain of these cases, the first and/or second complementarity domains may have at least 50%, 60%, 70%, 80%, 85%, 90%, or 95% homology with homology with a first and/or second complementarity domain from *S. pyogenes* or *S. aureus.*

The first and/or second complementarity domains may comprise no modifications. The first and/or second complementarity domains or one or more nucleotides therein may have a modification, including but not limited to a modification set forth below. One or more nucleotides of the first and/or second complementarity domain may comprise a 2' modification (*e.g.,* a modification at the 2' position on ribose), *e.g.,* a 2-acetylation, *e.g.,* a 2' methylation. The backbone of the targeting domain can be modified with a phosphorothioate. Modifications to one or more nucleotides of the first and/or second complementarity domain may render the first and/or second complementarity domain and/or the gRNA comprising the first and/or second complementarity less susceptible to degradation or more bio-compatible, *e.g.,* less immunogenic. The first and/or second complementarity domains may each independently include 1, 2, 3, 4, 5, 6, 7, or 8 or more modifications, and in certain of these cases the first and/or second complementarity domains may each independently include 1, 2, 3, or 4 modifications within five nucleotides of their respective 5' ends, 3' ends, or both their 5' and 3' ends. The first and/or second complementarity domains may each independently contain no modifications within five nucleotides of their respective 5' ends, 3' ends, or both their 5' and 3' ends. One or both of the first and second complementarity domains may comprise modifications at two or more consecutive nucleotides.

Modifications to one or more nucleotides in the first and/or second complementarity domains may be selected to not interfere with targeting efficacy, which can be evaluated by testing a candidate modification in a system as set forth below. gRNAs having a candidate first or second complementarity domain having a selected length, sequence, degree of complementarity, or degree of modification can be evaluated in a system as set forth below. The candidate complementarity domain can be placed, either alone or with one or more other candidate changes in a gRNA molecule/Cas9 molecule system known to be functional with a selected target, and evaluated.

The duplexed region formed by the first and second complementarity domains may be, for example, 6, 7, 8, 9, 10, 11, 12, 13, 14, 15, 16, 17, 18, 19, 20, 21, or 22 bp in length, excluding any looped out or unpaired nucleotides.

The first and second complementarity domains, when
duplexed, may comprise 11 paired nucleotides (see, for *e.g.,* gRNA of SEQ ID NO:5). The first and second complementarity domains, when duplexed, may comprise 15 paired nucleotides (see, *e.g.,* gRNA of SEQ ID NO:27). The first and second complementarity domains, when duplexed, may comprise 16 paired nucleotides (see, *e.g.,* gRNA of SEQ ID NO:28). The first and second complementarity domains, when duplexed, may comprise 21 paired nucleotides (see, *e.g.,* gRNA of SEQ ID NO:29).

One or more nucleotides may be exchanged between the first and second complementarity domains to remove poly-U tracts. For example, nucleotides 23 and 48 or nucleotides 26 and 45 of the gRNA of SEQ ID NO: 5 may be exchanged to generate the gRNA of SEQ ID NOs:30 or 31, respectively. Similarly, nucleotides 23 and 39 of the gRNA of SEQ ID NO:29 may be exchanged with nucleotides 50 and 68 to generate the gRNA of SEQ ID NO:32.

### Linking domain

The linking domain is disposed between and serves to link the first and second complementarity domains in a unimolecular or chimeric gRNA. Part of the linking domain may be from a crRNA-derived region, and another part is from a tracrRNA-derived region.

The linking domain may link the first and second complementarity domains covalently. In certain of these cases, the linking domain consists of or comprises a covalent bond. The linking domain may link the first and second complementarity domains non-covalently. The linking domain may be ten or fewer nucleotides in length, *e.g.,* 1, 2, 3, 4, 5, 6, 7, 8, 9, or 10 nucleotides. The linking domain may be greater than 10 nucleotides in length, *e.g.,* 11, 12, 13, 14, 15, 16, 17, 18, 19, 20, 21, 22, 23, 24, or 25 or more nucleotides. The linking domain may be 2 to 50, 2 to 40, 2 to 30, 2 to 20, 2 to 10, 2 to 5, 10 to 100, 10 to 90, 10 to 80, 10 to 70, 10 to 60, 10 to 50, 10 to 40, 10 to 30, 10 to 20, 10 to 15, 20 to 100, 20 to 90, 20 to 80, 20 to 70, 20 to 60, 20 to 50, 20 to 40, 20 to 30, or 20 to 25 nucleotides in length. The linking domain may be 10 +/-5, 20+/-5, 20+/-10, 30+/-5, 30+/-10, 40+/-5, 40+/- 10, 50+/-5, 50+/-10, 60+/-5, 60+/-10, 70+/-5, 70+/-10, 80+/-5, 80+/-10, 90+/-5, 90+/-10, 100+/-5, or 100+/-10 nucleotides in length.

The linking domain may share homology with, or be derived from,
a naturally occurring sequence, e.g., the sequence of a tracrRNA that is 5' to the second complementarity domain. The linking domain may have at least 50%, 60%, 70%, 80%, 90%, or 95% homology with or differs by no more than 1, 2, 3, 4, 5, or 6 nucleotides from a linking domain disclosed herein.

The linking domain may in certain cases not comprise any modifications. The linking domain or one or more nucleotides therein may have a modification, including but not limited to the modifications set forth below. One or more nucleotides of the linking domain may comprise a 2' modification (*e.g.,* a modification at the 2' position on ribose), *e.g.,* a 2-acetylation, *e.g.,* a 2' methylation. The backbone of the linking domain can be modified with a phosphorothioate. Modifications to one or more nucleotides of the linking domain may render the linking domain and/or the gRNA comprising the linking domain less susceptible to degradation or more bio-compatible, *e.g.,* less immunogenic. The linking domain may include 1, 2, 3, 4, 5, 6, 7, or 8 or more modifications, and in certain of these cases the linking domain includes 1, 2, 3, or 4 modifications within five nucleotides of its 5' and/or 3' end. The linking domain may comprise modifications at two or more consecutive nucleotides.

Modifications to one or more nucleotides in the linking domain may be selected to not interfere with targeting efficacy, which can be evaluated by testing a candidate modification in a system as set forth below. gRNAs having a candidate linking domain having a selected length, sequence, degree of complementarity, or degree of modification can be evaluated in a system as set forth below. The candidate linking domain can be placed, either alone or with one or more other candidate changes in a gRNA molecule/Cas9 molecule system known to be functional with a selected target, and evaluated.

The linking domain may comprise a duplexed region, typically adjacent to or within 1, 2, or 3 nucleotides of the 3' end of the first complementarity domain and/or the 5' end of the second complementarity domain. In certain of these cases, the duplexed region of the linking region is 10+/-5, 15+/-5, 20+/-5, 20+/-10, or 30+/-5 bp in length. The duplexed region of the linking domain may be 1, 2, 3, 4, 5, 6, 7, 8, 9, 10, 11, 12, 13, 14, or 15 bp in length. The sequences forming the duplexed region of the linking domain may be fully complementarity. One or both of the sequences forming the duplexed region may contain one or more nucleotides (*e.g.,* 1, 2, 3, 4, 5, 6, 7, or 8 nucleotides) that are not complementary with the other duplex sequence.

### 5' extension domain

A modular gRNA as disclosed herein may comprise a 5' extension domain, *i.e.,* one or more additional nucleotides 5' to the second complementarity domain. The 5' extension domain may be 2 to 10 or more, 2 to 9, 2 to 8, 2 to 7, 2 to 6, 2 to 5, or 2 to 4 nucleotides in length, and in certain of these cases the 5' extension domain may be 2, 3, 4, 5, 6, 7, 8, 9, or 10 or more nucleotides in length.

The 5' extension domain nucleotides may comprise no modifications, *e.g.,* modifications of the type provided below. However, the 5' extension domain may comprise one or more modifications, *e.g.,* modifications that it render it less susceptible to degradation or more bio-compatible, *e.g.,* less immunogenic. By way of example, the backbone of the 5' extension domain can be modified with a phosphorothioate, or other modification(s) as set forth below. A nucleotide of the 5' extension domain can comprise a 2' modification (*e.g.,* a modification at the 2' position on ribose), *e.g.,* a 2-acetylation, *e.g.,* a 2' methylation, or other modification(s) as set forth below.

The 5' extension domain can comprise as many as 1, 2, 3, 4, 5, 6, 7, or 8 modifications. The 5' extension domain may comprise as many as 1, 2, 3, or 4 modifications within 5 nucleotides of its 5' end, *e.g.,* in a modular gRNA molecule. The 5' extension domain may comprise as many as 1, 2, 3, or 4 modifications within 5 nucleotides of its 3' end, *e.g.,* in a modular gRNA molecule.

The 5' extension domain may comprise modifications at two consecutive nucleotides, *e.g.,* two consecutive nucleotides that are within 5 nucleotides of the 5' end of the 5' extension domain, within 5 nucleotides of the 3' end of the 5' extension domain, or more than 5 nucleotides away from one or both ends of the 5' extension domain. In certain cases, no two consecutive nucleotides are modified within 5 nucleotides of the 5' end of the 5' extension domain, within 5 nucleotides of the 3' end of the 5' extension domain, or within a region that is more than 5 nucleotides away from one or both ends of the 5' extension domain. In certain cases, no nucleotide is modified within 5 nucleotides of the 5' end of the 5' extension domain, within 5 nucleotides of the 3' end of the 5' extension domain, or within a region that is more than 5 nucleotides away from one or both ends of the 5' extension domain.

Modifications in the 5' extension domain can be selected so as to not interfere with gRNA molecule efficacy, which can be evaluated by testing a candidate modification in a system as set forth below. gRNAs having a candidate 5' extension domain having a selected length, sequence, degree of complementarity, or degree of modification, can be evaluated in a system as set forth below. The candidate 5' extension domain can be placed, either alone, or with one or more other candidate changes in a gRNA molecule/Cas9 molecule system known to be functional with a selected target and evaluated.

The 5' extension domain may have at least 60, 70, 80, 85, 90, or 95% homology with, or differs by no more than 1, 2, 3, 4, 5, or 6 nucleotides from, a reference 5' extension domain, *e.g.,* a naturally occurring, *e.g.,* an *S. pyogenes, S. aureus,* or *S. thermophilus,* 5' extension domain, or a 5' extension domain described herein.

### Proximal domain

The proximal domain may be 5 to 20 or more nucleotides in length, *e.g.,* 5, 6, 7, 8, 9, 10, 11, 12, 13, 14, 15, 16, 17, 18, 19, 20, 21, 22, 23, 24, 25, or 26 nucleotides in length. The proximal domain may be 6 +/-2, 7+/-2, 8+/-2, 9+/-2, 10+/-2, 11+/-2, 12+/-2, 13+/-2, 14+/-2, 14+/-2, 16+/-2, 17+/-2, 18+/-2, 19+/-2, or 20+/-2 nucleotides in length. The proximal domain may be 5 to 20, 7, to 18, 9 to 16, or 10 to 14 nucleotides in length.

The proximal domain can share homology with or be derived from a naturally occurring proximal domain. In certain of these cases, the proximal domain has at least 50%, 60%, 70%, 80%, 85%, 90%, or 95% homology with or differs by no more than 1, 2, 3, 4, 5, or 6 nucleotides from a proximal domain disclosed herein, *e.g.,* an *S. pyogenes, S. aureus,* or *S. thermophilus* proximal domain.

The proximal domain either does not comprise any modifications or the proximal domain or one or more nucleotides therein have a modification, including but not limited to the modifications set forth in herein. One or more nucleotides of the proximal domain may comprise a 2' modification (*e.g.,* a modification at the 2' position on ribose), *e.g.,* a 2-acetylation, *e.g.,* a 2' methylation. The backbone of the proximal domain can be modified with a phosphorothioate. Modifications to one or more nucleotides of the proximal domain may render the proximal domain and/or the gRNA comprising the proximal domain less susceptible to degradation or more bio-compatible, *e.g.,* less immunogenic. The proximal domain may include 1, 2, 3, 4, 5, 6, 7, or 8 or more modifications, and in certain of these cases the proximal domain includes 1, 2, 3, or 4 modifications within five nucleotides of its 5' and/or 3' end. The proximal domain may comprise modifications at two or more consecutive nucleotides.

Modifications to one or more nucleotides in the proximal domain may be selected to not interfere with targeting efficacy, which can be evaluated by testing a candidate modification in a system as set forth below. gRNAs having a candidate proximal domain having a selected length, sequence, degree of complementarity, or degree of modification can be evaluated in a system as set forth below. The candidate proximal domain can be placed, either alone or with one or more other candidate changes in a gRNA molecule/Cas9 molecule system known to be functional with a selected target, and evaluated.

### Tail domain

A broad spectrum of tail domains are suitable for use in the gRNA molecules disclosed herein.

The tail domain may be absent. The tail domain may be 1 to 100 or more nucleotides in length, *e.g.,* 1, 2, 3, 4, 5, 6, 7, 8, 9, 10, 20, 30, 40, 50, 60, 70, 80, 90, or 100 nucleotides in length. The tail domain may be 1 to 5, 1 to 10, 1 to 15, 1 to 20, 1 to 50, 10 to 100, 20 to 100, 10 to 90, 20 to 90, 10 to 80, 20 to 80, 10 to 70, 20 to 70, 10 to 60, 20 to 60, 10 to 50, 20 to 50, 10 to 40, 20 to 40, 10 to 30, 20 to 30, 20 to 25, 10 to 20, or 10 to 15 nucleotides in length. The tail domain may be 5 +/-5, 10 +/-5, 20+/-10, 20+/-5, 25+/-10, 30+/-10, 30+/-5, 40+/-10, 40+/-5, 50+/-10, 50+/-5, 60+/-10, 60+/-5, 70+/-10, 70+/-5, 80+/-10, 80+/-5, 90+/-10, 90+/-5, 100+/-10, or 100+/-5 nucleotides in length.

The tail domain can share homology with or be derived from a naturally occurring tail domain or the 5' end of a naturally occurring tail domain. In certain of these cases, the proximal domain has at least 50%, 60%, 70%, 80%, 85%, 90%, or 95% homology with or differs by no more than 1, 2, 3, 4, 5, or 6 nucleotides from a naturally occurring tail domain disclosed herein, *e.g.,* an *S. pyogenes, S. aureus,* or *S. thermophilus* tail domain.

The tail domain may include sequences that are complementary to each other and which, under at least some physiological conditions, form a duplexed region. In certain of these cases, the tail domain comprises a tail duplex domain which can form a tail duplexed region. The tail duplexed region may be 3, 4, 5, 6, 7, 8, 9, 10, 11, or 12 bp in length. The tail domain may comprise a single stranded domain 3' to the tail duplex domain that does not form a duplex. In certain of these cases, the single stranded domain is 3 to 10 nucleotides in length, *e.g.,* 3, 4, 5, 6, 7, 8, 9, 10, or 4 to 6 nucleotides in length.

The tail domain may comprise no modifications or one or more nucleotides therein may have a modification, including but not limited to the modifications set forth herein. One or more nucleotides of the tail domain may comprise a 2' modification (*e.g.,* a modification at the 2' position on ribose), *e.g.,* a 2-acetylation, *e.g.,* a 2' methylation. The backbone of the tail domain can be modified with a phosphorothioate. Modifications to one or more nucleotides of the tail domain may render the tail domain and/or the gRNA comprising the tail domain less susceptible to degradation or more bio-compatible, *e.g.,* less immunogenic. The tail domain may include 1, 2, 3, 4, 5, 6, 7, or 8 or more modifications, and in certain of these cases the tail domain includes 1, 2, 3, or 4 modifications within five nucleotides of its 5' and/or 3' end. The tail domain may comprise modifications at two or more consecutive nucleotides.

Modifications to one or more nucleotides in the tail domain may be selected to not interfere with targeting efficacy, which can be evaluated by testing a candidate modification as set forth below. gRNAs having a candidate tail domain having a selected length, sequence, degree of complementarity, or degree of modification can be evaluated using a system as set forth below. The candidate tail domain can be placed, either alone or with one or more other candidate changes in a gRNA molecule/Cas9 molecule system known to be functional with a selected target, and evaluated.

The tail domain can include nucleotides at the 3' end that are related to the method of *in vitro* or *in vivo* transcription. When a T7 promoter is used for *in vitro* transcription of the gRNA, these nucleotides may be any nucleotides present before the 3' end of the DNA template. When a U6 promoter is used for *in vivo* transcription, these nucleotides may be the sequence UUUUUU. When an HI promoter is used for transcription, these nucleotides may be the sequence UUUU. When alternate pol-III promoters are used, these nucleotides may be various numbers of uracil bases depending on, *e.g.,* the termination signal of the pol-III promoter, or they may include alternate bases.

The proximal and tail domain taken together may comprise, consist of, or consist essentially of the sequence set forth in SEQ ID NOs: 33, 34, 35, 36, or 38.

### Exemplary unimolecular/chimeric gRNAs

A gRNA as disclosed herein may have the structure: 5' [targeting domain]-[first complementarity domain]-[linking domain]-[second complementarity domain]-[proximal domain]-[tail domain]-3', wherein:
the targeting domain comprises a core domain and optionally a secondary domain, and is 10 to 50 nucleotides in length;
the first complementarity domain is 5 to 25 nucleotides in length and, in certain embodiments has at least 50, 60, 70, 80, 85, 90, or 95% homology with a reference first complementarity domain disclosed herein;
the linking domain is 1 to 5 nucleotides in length;
the second complementarity domain is 5 to 27 nucleotides in length and may have at least 50, 60, 70, 80, 85, 90, or 95% homology with a reference second complementarity domain disclosed herein;
the proximal domain is 5 to 20 nucleotides in length and may have at least 50, 60, 70, 80, 85, 90, or 95% homology with a reference proximal domain disclosed herein; and
the tail domain is absent or a nucleotide sequence is 1 to 50 nucleotides in length and may have at least 50, 60, 70, 80, 85, 90, or 95% homology with a reference tail domain disclosed herein.

A unimolecular gRNA as disclosed herein may comprise, preferably from 5' to 3':
a targeting domain, *e.g.,* comprising 10-50 nucleotides;
a first complementarity domain, *e.g.,* comprising 15, 16, 17, 18, 19, 20, 21, 22, 23, 24, 25, or 26 nucleotides;
a linking domain;
a second complementarity domain;
a proximal domain; and
a tail domain,
wherein,
(a) the proximal and tail domain, when taken together, comprise at least 15, 18, 20, 25, 30, 31, 35, 40, 45, 49, 50, or 53 nucleotides;
(b) there are at least 15, 18, 20, 25, 30, 31, 35, 40, 45, 49, 50, or 53 nucleotides 3' to the last nucleotide of the second complementarity domain; or
(c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The sequence from (a), (b), and/or (c) may have at least 50%, 60%, 70%, 75%, 80%, 85%, 90%, 95%, or 99% homology with the corresponding sequence of a naturally occurring gRNA, or with a gRNA described herein.

The proximal and tail domain, when taken together, may comprise at least 15, 18, 20, 25, 30, 31, 35, 40, 45, 49, 50, or 53 nucleotides.

There may be at least 15, 18, 20, 25, 30, 31, 35, 40, 45, 49, 50, or 53 nucleotides 3' to the last nucleotide of the second complementarity domain.

There may be at least 16, 19, 21, 26, 31, 32, 36, 41, 46, 50, 51, or 54 nucleotides 3' to the last nucleotide of the second complementarity domain that are complementary to the corresponding nucleotides of the first complementarity domain.

The targeting domain may consist of, consist essentially of, or comprise 16, 17, 18, 19, 20, 21, 22, 23, 24, 25, or 26 nucleotides (*e.g.,* 16, 17, 18, 19, 20, 21, 22, 23, 24, 25, or 26 consecutive nucleotides) complementary or partially complementary to the target domain or a portion thereof, *e.g.,* the targeting domain is 16, 17, 18, 19, 20, 21, 22, 23, 24, 25, or 26 nucleotides in length. In certain of these cases, the targeting domain is complementary to the target domain over the entire length of the targeting domain, the entire length of the target domain, or both.

A unimolecular or chimeric gRNA molecule disclosed herein (comprising a targeting domain, a first complementary domain, a linking domain, a second complementary domain, a proximal domain and, optionally, a tail domain) may comprise the amino acid sequence set forth in SEQ ID NO:45, wherein the targeting domain is listed as 20 N's (residues 1-20) but may range in length from 16 to 26 nucleotides, and wherein the final six residues (residues 97-102) represent a termination signal for the U6 promoter buy may be absent or fewer in number. The unimolecular, or chimeric, gRNA molecule may be a *S. pyogenes* gRNA molecule.

A unimolecular or chimeric gRNA molecule disclosed herein (comprising a targeting domain, a first complementary domain, a linking domain, a second complementary domain, a proximal domain and, optionally, a tail domain) may comprise the
amino acid sequence set forth in SEQ ID NO:40, wherein the targeting domain is listed as 20 Ns (residues 1-20) but may range in length from 16 to 26 nucleotides, and wherein the final six residues (residues 97-102) represent a termination signal for the U6 promoter but may be absent or fewer in number. The unimolecular or chimeric gRNA molecule may be an *S*. *aureus* gRNA molecule.

### Exemplary modular gRNAs

A modular gRNA disclosed herein may comprise:
a first strand comprising, preferably from 5' to 3';
   a targeting domain, *e.g.,* comprising 15, 16, 17, 18, 19, 20, 21, 22, 23, 24, 25, or 26 nucleotides;
   a first complementarity domain; and
a second strand, comprising, preferably from 5' to 3':
   optionally a 5' extension domain;
   a second complementarity domain;
   a proximal domain; and
   a tail domain,
wherein:
(a) the proximal and tail domain, when taken together, comprise at least 15, 18, 20, 25, 30, 31, 35, 40, 45, 49, 50, or 53 nucleotides;
(b) there are at least 15, 18, 20, 25, 30, 31, 35, 40, 45, 49, 50, or 53 nucleotides 3' to the last nucleotide of the second complementarity domain; or
(c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The sequence from (a), (b), or (c), may have at least 60, 75, 80, 85, 90, 95, or 99% homology with the corresponding sequence of a naturally occurring gRNA, or with a gRNA described herein.

The proximal and tail domain, when taken together, may comprise at least 15, 18, 20, 25, 30, 31, 35, 40, 45, 49, 50, or 53 nucleotides.

There may be at least 15, 18, 20, 25, 30, 31, 35, 40, 45, 49, 50, or 53 nucleotides 3' to the last nucleotide of the second complementarity domain.

There may be at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 16, 17, 18, 19, 20, 21, 22, 23, 24, 25, or 26 nucleotides (*e.g.,* 16, 17, 18, 19, 20, 21, 22, 23, 24, 25, or 26 consecutive nucleotides) having complementarity with the target domain, *e.g.,* the targeting domain is 16, 17, 18, 19, 20, 21, 22, 23, 24, 25, or 26 nucleotides in length.

The targeting domain may consist of, consist essentially of, or comprise 16, 17, 18, 19, 20, 21, 22, 23, 24, 25, or 26 nucleotides (*e.g.,* 16, 17, 18, 19, 20, 21, 22, 23, 24, 25, or 26 consecutive nucleotides) complementary to the target domain or a portion thereof. In certain of these cases, the targeting domain is complementary to the target domain over the entire length of the targeting domain, the entire length of the target domain, or both.

The targeting domain may comprise, have, or consist of, 16 nucleotides (*e.g.,* 16 consecutive nucleotides) having complementarity with the target domain, *e.g.,* the targeting domain is 16 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 16 nucleotides (*e.g.,* 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

The targeting domain may comprise, have, or consist of, 16 nucleotides (*e.g.,* 16 consecutive nucleotides) having complementarity with the target domain, *e.g.,* the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may have, or consist of, 17 nucleotides (*e.g.,* 17 consecutive nucleotides) having complementarity with the target domain, *e.g.,* the targeting domain is 17 nucleotides in length; and the proximal and tail domain, when taken together, comprise at least 15, 18, 20, 25, 30, 31, 35, 40, 45, 49, 50, or 53 nucleotides.

The targeting domain may have, or consist of, 17 nucleotides (*e.g.,* 17 consecutive nucleotides) having complementarity with the target domain, *e.g.,* the targeting domain is 17 nucleotides in length; and there are at least 15, 18, 20, 25, 30, 31, 35, 40, 45, 49, 50, or 53 nucleotides 3' to the last nucleotide of the second complementarity domain.

The targeting domain may have, or consist of, 17 nucleotides (*e.g.,* 17 consecutive nucleotides) having complementarity with the target domain, *e.g.,* the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may have, or consist of, 18 nucleotides (*e.g.,* 18 consecutive nucleotides) having complementarity with the target domain, *e.g.,* the targeting domain is 18 nucleotides in length; and the proximal and tail domain, when taken together, comprise at least 15, 18, 20, 25, 30, 31, 35, 40, 45, 49, 50, or 53 nucleotides.

The targeting domain may have, or consist of, 18 nucleotides (*e.g.,* 18 consecutive nucleotides) having complementarity with the target domain, *e.g.,* the targeting domain is 18 nucleotides in length; and there are at least 15, 18, 20, 25, 30, 31, 35, 40, 45, 49, 50, or 53 nucleotides 3' to the last nucleotide of the second complementarity domain.

The targeting domain may have, or consist of, 18 nucleotides (*e.g.,* 18 consecutive nucleotides) having complementarity with the target domain, *e.g.,* the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 19 nucleotides (*e.g.,* 19 consecutive nucleotides) having complementarity with the target domain, *e.g.,* the targeting domain is 19 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 19 nucleotides (*e.g.,* 19 consecutive nucleotides) having complementarity with the target domain, *e.g.,* the targeting domain is 19 nucleotides in length; and there are at least 15, 18, 20, 25, 30, 31, 35, 40, 45, 49, 50, or 53 nucleotides 3' to the last nucleotide of the second complementarity domain.

The targeting domain may comprise, have, or consist of, 19 nucleotides (*e.g.,* 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 20 nucleotides (*e.g.,* 20 consecutive nucleotides) having complementarity with the target domain, *e.g.,* the targeting domain is 20 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 20 nucleotides (*e.g.,* 20 consecutive nucleotides) having complementarity with the target domain, *e.g.,* the targeting domain is 20 nucleotides in length; and there are at least 15, 18, 20, 25, 30, 31, 35, 40, 45, 49, 50, or 53 nucleotides 3' to the last nucleotide of the second complementarity domain.

The targeting domain may comprise, have, or consist of, 20 nucleotides (*e.g.,* 20 consecutive nucleotides) having complementarity with the target domain, *e.g.,* the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 21 nucleotides (*e.g.,* 21 consecutive nucleotides) having complementarity with the target domain, *e.g.,* the targeting domain is 21 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 21 nucleotides (*e.g.,* 21 consecutive nucleotides) having complementarity with the target domain, *e.g.,* the targeting domain is 21 nucleotides in length; and there are at least 15, 18, 20, 25, 30, 31, 35, 40, 45, 49, 50, or 53 nucleotides 3' to the last nucleotide of the second complementarity domain.

The targeting domain may comprise, have, or consist of, 21 nucleotides (*e.g.,* 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 22 nucleotides (*e.g.,* 22 consecutive nucleotides) having complementarity with the target domain, *e.g.,* the targeting domain is 22 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 22 nucleotides (*e.g.,* 22 consecutive nucleotides) having complementarity with the target domain, *e.g.,* the targeting domain is 22 nucleotides in length; and there are at least 15, 18, 20, 25, 30, 31, 35, 40, 45, 49, 50, or 53 nucleotides 3' to the last nucleotide of the second complementarity domain.

The targeting domain may comprise, have, or consist of, 22 nucleotides (*e.g.,* 22 consecutive nucleotides) having complementarity with the target domain, *e.g.,* the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 23 nucleotides (*e.g.,* 23 consecutive nucleotides) having complementarity with the target domain, *e.g.,* the targeting domain is 23 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 23 nucleotides (*e.g.,* 23 consecutive nucleotides) having complementarity with the target domain, *e.g.,* the targeting domain is 23 nucleotides in length; and there are at least 15, 18, 20, 25, 30, 31, 35, 40, 45, 49, 50, or 53 nucleotides 3' to the last nucleotide of the second complementarity domain.

The targeting domain may comprises, have, or consist of, 23 nucleotides (*e.g.,* 23 consecutive nucleotides) having complementarity with the target domain, *e.g.,* the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 24 nucleotides (*e.g.,* 24 consecutive nucleotides) having complementarity with the target domain, *e.g.,* the targeting domain is 24 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 24 nucleotides (*e.g.,* 24 consecutive nucleotides) having complementarity with the target domain, *e.g.,* the targeting domain is 24 nucleotides in length; and there are at least 15, 18, 20, 25, 30, 31, 35, 40, 45, 49, 50, or 53 nucleotides 3' to the last nucleotide of the second complementarity domain.

The targeting domain may comprise, have, or consist of, 24 nucleotides (*e.g.,* 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 25 nucleotides (*e.g.,* 25 consecutive nucleotides) having complementarity with the target domain, *e.g.,* the targeting domain is 25 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 25 nucleotides (*e.g.,* 25 consecutive nucleotides) having complementarity with the target domain, *e.g.,* the targeting domain is 25 nucleotides in length; and there are at least 15, 18, 20, 25, 30, 31, 35, 40, 45, 49, 50, or 53 nucleotides 3' to the last nucleotide of the second complementarity domain.

The targeting domain may comprise, have, or consist of, 25 nucleotides (*e.g.,* 25 consecutive nucleotides) having complementarity with the target domain, *e.g.,* the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

The targeting domain may comprise, have, or consist of, 26 nucleotides (*e.g.,* 26 consecutive nucleotides) having complementarity with the target domain, *e.g.,* the targeting domain is 26 nucleotides in length; and the proximal and tail domain, when taken together, comprise at least 15, 18, 20, 25, 30, 31, 35, 40, 45, 49, 50, or 53 nucleotides.

The targeting domain may comprise, have, or consist of, 26 nucleotides (*e.g.,* 26 consecutive nucleotides) having complementarity with the target domain, *e.g.,* the targeting domain is 26 nucleotides in length; and there are at least 15, 18, 20, 25, 30, 31, 35, 40, 45, 49, 50, or 53 nucleotides 3' to the last nucleotide of the second complementarity domain.

The targeting domain may comprise, have, or consist of, 26 nucleotides (*e.g.,* 26 consecutive nucleotides) having complementarity with the target domain, *e.g.,* the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

### gRNA delivery

In the methods provided herein, the methods may comprise delivery of one or more *(e.g.,* two, three, or four) gRNA molecules as described herein. The gRNA molecules may be delivered by intravenous injection, intramuscular injection, subcutaneous injection, or inhalation.

### II. Methods for Designing gRNA Molecules

Methods for selecting, designing, and validating targeting domains for use in the gRNAs described herein are provided. Exemplary targeting domains for incorporation into gRNAs are also provided herein.

Methods for selection and validation of target sequences as well as off-target analyses have been described (see, *e.g.,* Mali 2013; Hsu 2013; Fu 2014; Heigwer 2014; Bae 2014; and Xiao 2014). For example, a software tool can be used to optimize the choice of potential targeting domains corresponding to a user's target sequence, *e.g.,* to minimize total off-target activity across the genome. Off-target activity may be other than cleavage. For each possible targeting domain choice using *S. pyogenes* Cas9, the tool can identify all off-target sequences (preceding either NAG or NGG PAMs) across the genome that contain up to certain number (*e.g.,* 1, 2, 3, 4, 5, 6, 7, 8, 9, or 10) of mismatched base-pairs. The cleavage efficiency at each off-target sequence can be predicted, *e.g.,* using an experimentally-derived weighting scheme. Each possible targeting domain is then ranked according to its total predicted off-target cleavage; the top-ranked targeting domains represent those that are likely to have the greatest on-target cleavage and the least off-target cleavage. Other functions, *e.g.,* automated reagent design for CRISPR construction, primer design for the on-target Surveyor assay, and primer design for high-throughput detection and quantification of off-target cleavage via next-gen sequencing, can also be included in the tool. Candidate targeting domains and gRNAs comprising those targeting domains can be functionally evaluated by using methods known in the art and/or as set forth herein.

As a non-limiting example, targeting domains for use in gRNAs for use with S. *pyogenes,* and *S. aureus* Cas9s were identified using a DNA sequence searching algorithm. 17-mer and 20-mer targeting domains were designed for *S. pyogenes* targets, while 18-mer, 19-mer, 20-mer, 21-mer, 22-mer, 23-mer, and 24-mer targeting domains were designed for *S. aureus* targets. gRNA design was carried out using a custom gRNA design software based on the public tool cas-offinder (Bae 2014). This software scores guides after calculating their genome-wide off-target propensity. Typically matches ranging from perfect matches to 7 mismatches are considered for guides ranging in length from 17 to 24. Once the off-target sites are computationally-determined, an aggregate score is calculated for each guide and summarized in a tabular output using a web-interface. In addition to identifying potential target sites adjacent to PAM sequences, the software also identifies all PAM adjacent sequences that differ by 1, 2, 3 or more than 3 nucleotides from the selected target sites. Genomic DNA sequences for a *HBB* gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, targeting domains were ranked into tiers based on their distance to the target site, their orthogonality and presence of a 5' G (based on identification of close matches in the human genome containing a relevant PAM *e.g.,* NGG PAM for S. *pyogenes,* NNGRRT or NNGRRV PAM for S. *aureus.* Orthogonality refers to the number of sequences in the human genome that contain a minimum number of mismatches to the target sequence. A "high level of orthogonality" or "good orthogonality" may, for example, refer to 20-mer targeting domains that have no identical sequences in the human genome besides the intended target, nor any sequences that contain one or two mismatches in the target sequence. Targeting domains with good orthogonality are selected to minimize off-target DNA cleavage.

Targeting domains were identified for both single-gRNA nuclease cleavage and for a dual-gRNA paired "nickase" strategy. Criteria for selecting targeting domains and the determination of which targeting domains can be incorporated into a gRNA and used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNA molecules. Candidate pair members can be tested for how efficiently they remove the entire sequence versus causing indel mutations at the target site of one gRNA molecule.

### Targeting Domains for Use in Correcting the E6V Mutation in the HBB Gene

Targeting domains to target the E6V mutation in the *HBB* gene in conjunction with the methods disclosed herein were identified and ranked into tiers for *S. pyogenes, S. aureus* and *N. meningitidis.*

### A. First Targeting Domain Selection Strategy

For *S. pyogenes* Cas9, targeting domains were identified and ranked into three tiers. Tier 1 targeting domains were selected based on (1) a reasonable distance upstream or downstream from either end of the target site, and (2) a high level of orthogonality. Tier 2 targeting domains were selected based on (1) a reasonable distance upstream or downstream from either end of the target site, and (2) presence of a 5'G. Tier 3 targeting domains were selected based on (1) a reasonable upstream or downstream from either end of the target site.

For *S. aureus* Cas9, targeting domains were identified manually by scanning genomic DNA sequence for the presence of PAM sequences. These gRNAs were not separated into tiers.

Note that tiers are non-inclusive (each targeting domain is listed only once for the strategy). The identified targeting domains are summarized below in Table 1.

**Table 1. Nucleic Acid Sequences of S. pyogenes and S. aureus targeting domains**

| **Tier** | ***S. pyogenes*** | ***S. aureus** ** |
|---|---|---|
| Tier 1 | SEQ ID NOs: 387-390 | SEQ ID NOs: 442-485 |
| Tier 2 | SEQ ID NOs: 391-404 | |
| Tier 3 | SEQ ID NOs: 405-441 | |

| | | |
|---|---|---|
| *Not separated into tiers. | | |

A point mutation in the *HBB* gene, *e.g.,* at E6, *e.g.,* E6V, may be targeted, *e.g.,* for correction by gene conversion. The targeting domain may be selected from those in **Tables 1.** For example, in certain embodiments, the targeting domain is:
AAGGUGAACGUGGAUGAAGU (SEQ ID NO: 387);
GUAACGGCAGACUUCUCCUC (SEQ ID NO: 388);
GUGAACGUGGAUGAAGU (SEQ ID NO: 389);
ACGGCAGACUUCUCCUC (SEQ ID NO: 390); or
GUAACGGCAGACUUCUCCAC (SEQ ID NO: 16318).

When the SCD target point position is E6, *e.g.,* E6V, and two gRNAs are used to position two breaks, *e.g.,* two single stranded breaks, in the target nucleic acid sequence, the targeting domain of each gRNA molecule may be selected from one of **Table 1.**

### B. Second Targeting Domain Selection Strategy

For *S. pyogenes* Cas9, targeting domains were identified and ranked into four tiers. Tier 1 targeting domains were selected based on (1) a short distance upstream or downstream from either end of the target site, *e.g.,* within 100 bp upstream and 100 bp downstream of the E6V mutation, (2) a high level of orthogonality, and (3) the presence of a 5' G. Tier 2 targeting domains were selected based on (1) a short distance upstream or downstream from either end of the target site, *e.g.,* within 100 bp upstream and 100 bp downstream of the E6V mutation, and (2) a high level of orthogonality. Tier 3 targeting domains were selected based on (1) a short distance upstream or downstream from either end of the target site, *e.g.,* within 100 bp upstream and 100 bp downstream of the E6V mutation, (2) the presence of a 5' G. Tier 4 targeting domains were selected based on (1) a short distance upstream or downstream from either end of the target site, *e.g.,* within 100 bp upstream and 100 bp downstream of the E6V mutation.

For *S. aureus* Cas9, targeting domains were identified and ranked into 5 tiers. Tier 1 targeting domains were selected based on (1) a short distance upstream or downstream from either end of the target site, *e.g.,* within 100 bp upstream and 100 bp downstream of the E6V mutation, (2) a high level of orthogonality, and (3) the presence of a 5' G. Tier 2 targeting domains were selected based on (1) a short distance upstream or downstream from either end of the target site, *e.g.,* within 100 bp upstream and 100 bp downstream of the E6V mutation, and (2) a high level of orthogonality. Tier 3 targeting domains were selected based on (1) a short distance upstream or downstream from either end of the target site, *e.g.,* within 100 bp upstream and 100 bp downstream of the E6V mutation, and (2) the presence of a 5' G. Tier 4 targeting domains were selected based on (1) a short distance upstream or downstream from either end of the target site, *e.g.,* within 100 bp upstream and 100 bp downstream of the E6V mutation. Tier 5 targeting domains were selected based on (1) a short distance upstream or downstream from either end of the target site, *e.g.,* within 100 bp upstream and 100 bp downstream of the E6V mutation, and (2) the PAM is NNGRRV.

Note that tiers are non-inclusive (each targeting domain is listed only once for the strategy). In certain instances, no targeting domain was identified based on the criteria of the particular tier. The identified targeting domains are summarized below in Table 2.

**Table 2. Nucleic Acid Sequences of S. pyogenes and S. aureus targeting domains**

| **Tier** | ***S. pyogenes*** | ***S. aureus*** |
|---|---|---|
| Tier 1 | SEQ ID NOs: 6803-6804 | SEQ ID NO: 6855 |
| Tier 2 | SEQ ID NOs: 6805-6810 | SEQ ID NO: 6856 |
| Tier 3 | SEQ ID NOs: 6811-6822 | N/A |
| Tier 4 | SEQ ID NOs: 6823-6854 | N/A |
| Tier 5 | N/A | SEQ ID NOs: 6857-6871 |

Exemplary gRNA pairs can be generated using the following targeting domain pairs: HBB-9 (SEQ ID NO: 6811) and HBB-11 (SEQ ID NO: 6813); HBB-9 (SEQ ID NO: 6811) and HBB-39 (SEQ ID NO: 6841); HBB-20 (SEQ ID NO: 6822) and HBB-11 (SEQ ID NO: 6813); and HBB-20 (SEQ ID NO: 6822) and HBB-39 (SEQ ID NO: 6841).

A point mutation in the *HBB* gene, *e.g.,* at E6, *e.g.,* E6V, may be targeted, *e.g.,* for correction. The targeting domain of a gRNA molecule may comprise a sequence that is the same as, or differs by no more than 1, 2, 3, 4, or 5 nucleotides from, a targeting domain sequence from any one of **Table 2.** The targeting domain may be selected from those in **Tables 2.** For example, in certain embodiments, the targeting domain is:
GGUGCACCUGACUCCUG (SEQ ID NO: 6803); or
GUAACGGCAGACUUCUCCAC (SEQ ID NO: 6804).

When the SCD target point position is E6, *e.g.,* E6V, and two gRNAs are used to position two breaks, *e.g.,* two single stranded breaks, in the target nucleic acid sequence, the targeting domain of each gRNA molecule may be selected from one of **Table 2.**

### C. Third Targeting Domain Selection Strategy

For *S. pyogenes* Cas9, targeting domains were identified and ranked into 3 tiers. Tier 1 targeting domains were selected based on (1) distance upstream or downstream from either end of the target site, *e.g.,* within 200 bp upstream and 200 bp downstream of the E6V mutation and (2) a high level of orthogonality. Tier 2 targeting domains were selected based on (1) distance upstream or downstream from either end of the target site, *e.g.,* within 200 bp upstream and 200 bp downstream of the E6V mutation, and (2) the presence of a 5' G. Tier 3 targeting domains were selected based on (1) distance upstream or downstream from either end of the target site, *e.g.,* within 200 bp upstream and 200 bp downstream of the E6V mutation. For *S. aureus* Cas9, targeting domains were identified and ranked into 4 tiers. Tier 1 targeting domains were selected based on (1) distance upstream or downstream from either end of the target site, *e.g.,* within 200 bp upstream and 200 bp downstream of the E6V mutation, (2) a high level of orthogonality, and (3) the PAM is NNGRRT. Tier 2 targeting domains were selected based on (1) distance upstream or downstream from either end of the target site, *e.g.,* within 200 bp upstream and 200 bp downstream of the E6V mutation, (2) the presence of a 5' G, and (3) the PAM is NNGRRT. Tier 3 targeting domains were selected based on (1) distance upstream or downstream from either end of the target site, *e.g.,* within 200 bp upstream and 200 bp downstream of the E6V mutation and (2) the PAM is NNGRRT. Tier 4 targeting domains were selected based on (1) distance upstream or downstream from either end of the target site, *e.g.,* within 200 bp upstream and 200 bp downstream of the E6V mutation and (2) the PAM is NNGRRT.

For *N. meningitidis* Cas9, targeting domains were identified and ranked into 3 tiers. Tier 1 targeting domains were selected based on (1) distance upstream or downstream from either end of the target site, *e.g.,* within 200 bp upstream and 200 bp downstream of the E6V mutation and (2) a high level of orthogonality. Tier 2 targeting domains were selected based on (1) distance upstream or downstream from either end of the target site, *e.g.,* within 200 bp upstream and 200 bp downstream of the E6V mutation, and (2) the presence of a 5' G. Tier 3 targeting domains were selected based on (1) distance upstream or downstream from either end of the target site, *e.g.,* within 200 bp upstream and 200 bp downstream of the E6V mutation.

Note that tiers are non-inclusive (each targeting domain is listed only once for the strategy). In certain instances, no targeting domain was identified based on the criteria of the particular tier. The identified targeting domains are summarized below in Table 3.

**Table 3. Nucleic Acid Sequences of S. pyogenes and S. aureus targeting domains**

| **Tier** | *S. **pyogenes*** | ***S. aureus*** | ***N. meningitidis*** |
|---|---|---|---|
| Tier 1 | SEQ ID NOs: 16010-16055 | SEQ ID NOs: 16085-16098 | SEQ ID NOs: 16253-16256 |
| Tier 2 | SEQ ID NOs: 16056-16063 | N/A | |
| Tier 3 | SEQ ID NOs: 16064-16084 | N/A | |
| Tier 4 | N/A | SEQ ID NOs: 16099-16252 | |

A point mutation in the *HBB* gene, *e.g.,* at E6, *e.g.,* E6V, may be targeted, *e.g.,* for correction. The targeting domain of a gRNA molecule may comprise a sequence that is the same as, or differs by no more than 1, 2, 3, 4, or 5 nucleotides from, a targeting domain sequence from any one of **Table 3.** The targeting domain may be selected from those in **Table 3.**

When the SCD target point position is E6, *e.g.,* E6V, and two gRNA molecules are used to position two breaks, *e.g.,* two single stranded breaks, in the target nucleic acid sequence, the target domain of each gRNA may be selected from one of **Table 3.**

### Other gRNA Design Strategy

Two or more *(e.g.,* three or four) gRNA molecules may be used with one Cas9 molecule. When two or more *(e.g.,* three or four) gRNAs are used with two or more Cas9 molecules, at least one Cas9 molecule may be from a different species than the other Cas9 molecule(s). For example, when two gRNA molecules are used with two Cas9 molecules, one Cas9 molecule can be from one species and the other Cas9 molecule can be from a different species. Both Cas9 species may be used to generate a single or double-strand break, as desired.

Dual targeting may be used to create two nicks on opposite DNA strands by using Cas9 nickases (*e.g.,* a *S. pyogenes* Cas9 nickase) with two targeting domains that are complementary to opposite DNA strands, *e.g.,* a gRNA molecule comprising any minus strand targeting domain may be paired any gRNA molecule comprising a plus strand targeting domain provided that the two gRNAs are oriented on the DNA such that PAMs face outward and the distance between the 5' ends of the gRNAs is 0-50bp. When selecting gRNA molecules for use in a nickase pair, one gRNA molecule targets a domain in the complementary strand and the second gRNA molecule targets a domain in the non-complementary strand, *e.g.,* a gRNA comprising any minus strand targeting domain may be paired any gRNA molecule comprising a plus strand targeting domain targeting the same target position. In certain embodiments, two 20-mer gRNAs are used to target two Cas9 nucleases (*e.g.,* two *S. pyogenes* Cas9 nucleases) or two Cas9 nickases (*e.g.,* two *S. pyogenes* Cas9 nickases), *e.g.,* two gRNAs comprising the targeting domains of HBB-8 (SEQ ID NO: 388) and HBB-15 (SEQ ID NO: 387) are used. Two 17-mer gRNAs may be used to target two Cas9 nucleases or two Cas9 nickases, *e.g.,* two gRNAs comprising the targeting domains of HBB-35 (SEQ ID NO: 389) and HBB-53 (SEQ ID NO: 390) may be used. Any of the targeting domains in the tables described herein can be used with a Cas9 molecule that generates a single-strand break (*i.e.,* a *S. pyogenes* or *S. aureus* Cas9 nickase) or with a Cas9 molecule that generates a double-strand break (*i.e., S. pyogenes* or *S. aureus* Cas9 nuclease).

The targeting domain may comprise a sequence that is the same as, or differs by no more than 1, 2, 3, 4, or 5 nucleotides from, a targeting domain sequence described herein, *e.g.,* a targeting domain sequence selected from any one of **Tables 1-3.** The targeting domain may be a targeting domain sequence described herein, *e.g.,* a targeting domain sequence selected from those in **Tables 1-3.**

*HBB* gRNA molecules, as described herein, may comprise from 5' to 3': a targeting domain (comprising a "core domain", and optionally a "secondary domain"); a first complementarity domain; a linking domain; a second complementarity domain; a proximal domain; and a tail domain. The proximal domain and tail domain may be taken together as a single domain.

A gRNA molecule may comprise a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26 nucleotides in length.

A gRNA molecule may comprise a linking domain of no more than 25 nucleotides in length; a proximal and tail domain, that taken together, are at least 25 nucleotides in length; and a targeting domain equal to or greater than 16, 17, 18, 19, 20, 21, 22, 23, 24, 25 or 26 nucleotides in length.

A gRNA molecule may comprise a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

A gRNA molecule may comprise a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26 nucleotides in length.

When two gRNAs are designed for use with two Cas9 molecules, the two Cas9 molecules may be from different species. Both Cas9 species may be used to generate a single or double strand break, as desired.

It is contemplated herein that any upstream gRNA described herein may be paired with any downstream gRNA described herein. When an upstream gRNA designed for use with one species of Cas9 molecule is paired with a downstream gRNA designed for use from a different species of Cas9 molecule, both Cas9 species are used to generate a single or double-strand break, as desired.

### III. Cas9 Molecules

Cas9 molecules of a variety of species can be used in the methods and compositions described herein. While *S. pyogenes* and *S. aureus* Cas9 molecules are the subject of much of the disclosure herein, Cas9 molecules of, derived from, or based on the Cas9 proteins of other species listed herein can be used as well. These include, for example, Cas9 molecules from *Acidovorax avenae, Actinobacillus pleuropneumoniae, Actinobacillus succinogenes, Actinobacillus suis, Actinomyces sp., cycliphilus denitrificans, Aminomonaspaucivorans, Bacillus cereus, Bacillus smithii, Bacillus thuringiensis, Bacteroides sp., Blastopirellula marina, Bradyrhizobium sp., Brevibacillus laterosporus, Campylobacter coli, Campylobacter jejuni, Campylobacter lari, Candidatus Puniceispirillum, Clostridium cellulolyticum, Clostridium perfringens, Corynebacterium accolens, Corynebacterium diphtheria, Corynebacterium matruchotii, Dinoroseobacter shibae, Eubacterium dolichum, gamma proteobacterium, Gluconacetobacter diazotrophicus, Haemophilus parainfluenzae, Haemophilus sputorum, Helicobacter canadensis, Helicobacter cinaedi, Helicobacter mustelae, Ilyobacter polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica, Neisseria meningitidis, Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus lugdunensis, Streptococcus sp., Subdoligranulum sp., Tistrella mobilis, Treponema sp.,* or *Verminephrobacter eiseniae.* The amino acid sequences of exemplary Cas9 orthologs are set forth in SEQ ID NOs: 304-386.

### Cas9 Domains

Crystal structures have been determined for two different naturally occurring bacterial Cas9 molecules (Jinek *et al.* 2014) and for S. *pyogenes* Cas9 with a guide RNA *(e.g.,* a synthetic fusion of crRNA and tracrRNA) (Nishimasu *et al.* 2014; and Anders 2014).

A naturally-occurring Cas9 molecule comprises two lobes: a recognition (REC) lobe and a nuclease (NUC) lobe; each of which further comprise domains described herein. The domain nomenclature and the numbering of the amino acid residues encompassed by each domain used throughout this disclosure is as described previously in (Nishimasu 2014). The numbering of the amino acid residues is with reference to Cas9 from *S. pyogenes.*

The REC lobe comprises the arginine-rich bridge helix (BH), the REC1 domain, and the REC2 domain. The REC lobe does not share structural similarity with other known proteins, indicating that it is a Cas9-specific functional domain. The BH domain is a long α helix and arginine rich region and comprises amino acids 60-93 of the sequence of *S. pyogenes* Cas9. The REC1 domain is important for recognition of the repeat:anti-repeat duplex, *e.g.,* of a gRNA or a tracrRNA, and is therefore critical for Cas9 activity by recognizing the target sequence. The REC1 domain comprises two REC1 motifs at amino acids 94 to 179 and 308 to 717 of the sequence of *S. pyogenes* Cas9. These two REC1 domains, though separated by the REC2 domain in the linear primary structure, assemble in the tertiary structure to form the REC1 domain. The REC2 domain, or parts thereof, may also play a role in the recognition of the repeat:anti-repeat duplex. The REC2 domain comprises amino acids 180-307 of the sequence of *S. pyogenes* Cas9.

The NUC lobe comprises the RuvC domain, the HNH domain, and the PAM-interacting (PI) domain. The RuvC domain shares structural similarity to retroviral integrase superfamily members and cleaves a single strand, *e.g.,* the non-complementary strand of the target nucleic acid molecule. The RuvC domain is assembled from the three split RuvC motifs (RuvC I, RuvCII, and RuvCIII, which are often commonly referred to in the art as RuvCI domain, or N-terminal RuvC domain, RuvCII domain, and RuvCIII domain) at amino acids 1-59, 718-769, and 909-1098, respectively, of the sequence of *S. pyogenes* Cas9. Similar to the REC1 domain, the three RuvC motifs are linearly separated by other domains in the primary structure, however in the tertiary structure, the three RuvC motifs assemble and form the RuvC domain. The HNH domain shares structural similarity with HNH endonucleases, and cleaves a single strand, *e.g.,* the complementary strand of the target nucleic acid molecule. The HNH domain lies between the RuvC II-III motifs and comprises amino acids 775-908 of the sequence of *S. pyogenes* Cas9. The PI domain interacts with the PAM of the target nucleic acid molecule, and comprises amino acids 1099-1368 of the sequence of *S. pyogenes* Cas9.

### RuvC-like domain and an HNH-like domain

A Cas9 molecule or Cas9 polypeptide may comprise an HNH-like domain and a RuvC-like domain and in certain of these cases cleavage activity is dependent on the RuvC-like domain and the HNH-like domain. A Cas9 molecule or Cas9 polypeptide can comprise one or more of a RuvC-like domain and an HNH-like domain. A Cas9 molecule or Cas9 polypeptide may comprise a RuvC-like domain, *e.g.,* a RuvC-like domain described below, and/or an HNH-like domain, *e.g.,* an HNH-like domain described below.

### RuvC-like domains

A RuvC-like domain may cleave, a single strand, *e.g.,* the non-complementary strand of the target nucleic acid molecule. The Cas9 molecule or Cas9 polypeptide can include more than one RuvC-like domain (*e.g.,* one, two, three or more RuvC-like domains). A RuvC-like domain may be at least 5, 6, 7, 8 amino acids in length but not more than 20, 19, 18, 17, 16 or 15 amino acids in length. The Cas9 molecule or Cas9 polypeptide may comprise an N-terminal RuvC-like domain of about 10 to 20 amino acids, *e.g.,* about 15 amino acids in length.

### N-terminal RuvC-like domains

Some naturally occurring Cas9 molecules comprise more than one RuvC-like domain with cleavage being dependent on the N-terminal RuvC-like domain. Accordingly, a Cas9 molecule or Cas9 polypeptide can comprise an N-terminal RuvC-like domain. Exemplary N-terminal RuvC-like domains are described below.

A Cas9 molecule or Cas9 polypeptide may comprise an N-terminal RuvC-like domain comprising an amino acid sequence of Formula I:
D-X₁-G-X₂-X₃-X₄-X₅-G-X₆-X₇-X₈-X₉ (SEQ ID NO: 8),
wherein,
X₁ is selected from I, V, M, L and T (*e.g.,* selected from I, V, and L);
X₂ is selected from T, I, V, S, N, Y, E and L (*e.g.,* selected from T, V, and I);
X₃ is selected from N, S, G, A, D, T, R, M and F (*e.g.,* A or N);
X₄ is selected from S, Y, N and F (*e.g.,* S);
X₅ is selected from V, I, L, C, T and F (*e.g.,* selected from V, I and L);
X₆ is selected from W, F, V, Y, S and L (*e.g.,* W);
X₇ is selected from A, S, C, V and G *(e.g.,* selected from A and S);
X₈ is selected from V, I, L, A, M and H (*e.g.,* selected from V, I, M and L); and
X₉ is selected from any amino acid or is absent (*e.g.,* selected from T, V, I, L, Δ, F, S, A, Y, M and R, or, *e.g.,* selected from T, V, I, L and Δ).

The N-terminal RuvC-like domain may differ from a sequence of SEQ ID NO:8, by as many as 1 but no more than 2, 3, 4, or 5 residues.

The N-terminal RuvC-like domain may be cleavage competent.

The N-terminal RuvC-like domain may be cleavage incompetent.

A Cas9 molecule or Cas9 polypeptide may comprise an N-terminal RuvC-like domain comprising an amino acid sequence of Formula II:
D-X₁-G-X₂-X₃-S-X₅-G-X₆-X₇-X₈-X₉, (SEQ ID NO: 9),
wherein
X₁ is selected from I, V, M, L and T (*e.g.,* selected from I, V, and L);
X₂ is selected from T, I, V, S, N, Y, E and L (*e.g.,* selected from T, V, and I);
X₃ is selected from N, S, G, A, D, T, R, M and F (*e.g.,* A or N);
X₅ is selected from V, I, L, C, T and F (*e.g.,* selected from V, I and L);
X₆ is selected from W, F, V, Y, S and L (*e.g.,* W);
X₇ is selected from A, S, C, V and G (*e.g.,* selected from A and S);
X₈ is selected from V, I, L, A, M and H (*e.g.,* selected from V, I, M and L); and
X₉ is selected from any amino acid or is absent (*e.g.,* selected from T, V, I, L, Δ, F, S, A, Y, M and R or selected from *e.g.,* T, V, I, L and Δ).

The N-terminal RuvC-like domain may differ from a sequence of SEQ ID NO:9 by as many as 1 but not more than 2, 3, 4, or 5 residues.

The N-terminal RuvC-like domain may comprise an amino acid sequence of Formula III:
D-I-G-X₂-X₃-S-V-G-W-A-X₈-X₉ (SEQ ID NO: 10),
wherein
X₂ is selected from T, I, V, S, N, Y, E and L (*e.g.,* selected from T, V, and I);
X₃ is selected from N, S, G, A, D, T, R, M and F (*e.g.,* A or N);
X₈ is selected from V, I, L, A, M and H (*e.g.,* selected from V, I, M and L); and
X₉ is selected from any amino acid or is absent (*e.g.,* selected from T, V, I, L, Δ, F, S, A, Y, M and R or selected from *e.g.,* T, V, I, L and Δ).

The N-terminal RuvC-like domain may differ from a sequence of SEQ ID NO:10 by as many as 1 but not more than, 2, 3, 4, or 5 residues.

The N-terminal RuvC-like domain may comprise an amino acid sequence of Formula IV:
D-I-G-T-N-S-V-G-W-A-V-X (SEQ ID NO: 11),
wherein
X is a non-polar alkyl amino acid or a hydroxyl amino acid, *e.g.,* X is selected from V, I, Land T.

The N-terminal RuvC-like domain may differ from a sequence of SEQ ID NO:11 by as many as 1 but not more than, 2, 3, 4, or 5 residues.

The N-terminal RuvC-like domain may differ from a sequence of an N-terminal RuvC like domain disclosed herein, *e.g.,* in any one of SEQ ID Nos: 54-103, as many as 1 but no more than 2, 3, 4, or 5 residues. 1, 2, 3 or all of the highly conserved residues of SEQ ID Nos: 54-103 may be present.

The N-terminal RuvC-like domain may differ from a sequence of an N-terminal RuvC-like domain disclosed herein, *e.g.,* in any one of SEQ ID Nos: 104-177, as many as 1 but no more than 2, 3, 4, or 5 residues. 1, 2, or all of the highly conserved residues identified of SEQ ID Nos: 104-177 may be present.

### Additional RuvC-like domains

In addition to the N-terminal RuvC-like domain, the Cas9 molecule or Cas9 polypeptide can comprise one or more additional RuvC-like domains. The Cas9 molecule or Cas9 polypeptide can comprise two additional RuvC-like domains. Preferably, the additional RuvC-like domain is at least 5 amino acids in length and, *e.g.,* less than 15 amino acids in length, *e.g.,* 5 to 10 amino acids in length, *e.g.,* 8 amino acids in length.

An additional RuvC-like domain can comprise an amino acid sequence of Formula V: I-X₁-X₂-E-X₃-A-R-E (SEQ ID NO: 12), wherein
X₁ is V or H;
X₂ is I, L or V (*e.g.,* I or V); and
X₃ is M or T.

The additional RuvC-like domain may comprise an amino acid sequence of Formula VI:
I-V-X₂-E-M-A-R-E (SEQ ID NO: 13), wherein
X₂ is I, L or V (*e.g.,* I or V).

An additional RuvC-like domain can comprise an amino acid sequence of Formula VII:
H-H-A-X₁-D-A-X₂-X₃ (SEQ ID NO: 14), wherein
X₁ is H or L;
X₂ is R or V; and
X₃ is E or V.

The additional RuvC-like domain may comprise the amino acid sequence: H-H-A-H-D-A-Y-L (SEQ ID NO:15).

The additional RuvC-like domain may differ from a sequence of SEQ ID NOs: 12-15 by as many as 1 but not more than 2, 3, 4, or 5 residues.

The sequence flanking the N-terminal RuvC-like domain may have the amino acid sequence of Formula VIII:
K-X₁'-Y-X₂'-X₃'-X₄'-Z-T-D-X₉'-Y, (SEQ ID NO: 16).
wherein
X₁' is selected from K and P;
X₂' is selected from V, L, I, and F (*e.g.,* V, I and L);
X₃' is selected from G, A and S (*e.g.,* G);
X₄' is selected from L, I, V and F (*e.g.,* L);
X₉' is selected from D, E, N and Q; and
Z is an N-terminal RuvC-like domain, *e.g.,* as described above, *e.g.,* having 5 to 20 amino acids.

### HNH-like domains

A HNH-like domain may cleave a single stranded complementary domain, *e.g.,* a complementary strand of a double stranded nucleic acid molecule. A HNH-like domain may be at least 15, 20, or 25 amino acids in length but not more than 40, 35, or 30 amino acids in length, *e.g.,* 20 to 35 amino acids in length, *e.g.,* 25 to 30 amino acids in length. Exemplary HNH-like domains are described below.

A Cas9 molecule or Cas9 polypeptide may comprise an HNH-like domain having an amino acid sequence of Formula IX:
X₁-X₂-X₃-H-X₄-X₅-P-X₆-X₇-X₈-X₉-X₁₀-X₁₁-X₁₂-X₁₃-X₁₄-X₁₅-N-X₁₆-X₁₇-X₁₈-X₁₉-X₂₀-X₂₁-X₂₂-X₂₃-N (SEQ ID NO: 17), wherein
X₁ is selected from D, E, Q and N (*e.g.,* D and E);
X₂ is selected from L, I, R, Q, V, M and K;
X₃ is selected from D and E;
X₄ is selected from I, V, T, A and L (*e.g.,* A, I and V);
X₅ is selected from V, Y, I, L, F and W (*e.g.,* V, I and L);
X₆ is selected from Q, H, R, K, Y, I, L, F and W;
X₇ is selected from S, A, D, T and K (*e.g.,* S and A);
X₈ is selected from F, L, V, K, Y, M, I, R, A, E, D and Q (*e.g.,* F);
X₉ is selected from L, R, T, I, V, S, C, Y, K, F and G;
X₁₀ is selected from K, Q, Y, T, F, L, W, M, A, E, G, and S;
X₁₁ is selected from D, S, N, R, L and T (*e.g.,* D);
X₁₂ is selected from D, N and S;
X₁₃ is selected from S, A, T, G and R (*e.g.,* S);
X₁₄ is selected from I, L, F, S, R, Y, Q, W, D, K and H (*e.g.,* I, L and F);
X₁₅ is selected from D, S, I, N, E, A, H, F, L, Q, M, G, Y and V;
X₁₆ is selected from K, L, R, M, T and F (*e.g.,* L, R and K);
X₁₇ is selected from V, L, I, A and T;
X₁₈ is selected from L, I, V and A (*e.g.,* L and I);
X₁₉ is selected from T, V, C, E, S and A (*e.g.,* T and V);
X₂₀ is selected from R, F, T, W, E, L, N, C, K, V, S, Q, I, Y, H and A;
X₂₁ is selected from S, P, R, K, N, A, H, Q, G and L;
X₂₂ is selected from D, G, T, N, S, K, A, I, E, L, Q, R and Y; and
X₂₃ is selected from K, V, A, E, Y, I, C, L, S, T, G, K, M, D and F.

A HNH-like domain may differ from a sequence of SEQ ID NO: 17 by at least one but not more than, 2, 3, 4, or 5 residues.

The HNH-like domain may be cleavage competent or cleavage incompetent.

ACas9 molecule or Cas9 polypeptide may comprise an HNH-like domain comprising an amino acid sequence of Formula X:
X₁-X₂-X₃-H-X₄-X₅-P-X₆-S-X₈-X₉-X₁₀-D-D-S-X₁₄-X₁₅-N-K-V-L-X₁₉-X₂₀-X₂₁-X₂₂-X₂₃-N (SEQ ID NO: 18),
wherein
X₁ is selected from D and E;
X₂ is selected from L, I, R, Q, V, M and K;
X₃ is selected from D and E;
X₄ is selected from I, V, T, A and L (*e.g.,* A, I and V);
X₅ is selected from V, Y, I, L, F and W (*e.g.,* V, I and L);
X₆ is selected from Q, H, R, K, Y, I, L, F and W;
X₈ is selected from F, L, V, K, Y, M, I, R, A, E, D and Q (*e.g.,* F);
X₉ is selected from L, R, T, I, V, S, C, Y, K, F and G;
X₁₀ is selected from K, Q, Y, T, F, L, W, M, A, E, G, and S;
X₁₄ is selected from I, L, F, S, R, Y, Q, W, D, K and H (*e.g.,* I, L and F);
X₁₅ is selected from D, S, I, N, E, A, H, F, L, Q, M, G, Y and V;
X₁₉ is selected from T, V, C, E, S and A (*e.g.,* T and V);
X₂₀ is selected from R, F, T, W, E, L, N, C, K, V, S, Q, I, Y, H and A;
X₂₁ is selected from S, P, R, K, N, A, H, Q, G and L;
X₂₂ is selected from D, G, T, N, S, K, A, I, E, L, Q, R and Y; and
X₂₃ is selected from K, V, A, E, Y, I, C, L, S, T, G, K, M, D and F.

The HNH-like domain may differ from a sequence of SEQ ID NO: 18 by 1, 2, 3, 4, or 5 residues.

A Cas9 molecule or Cas9 polypeptide may comprise an HNH-like domain comprising an amino acid sequence of Formula XI:
X₁-V-X₃-H-I-V-P-X₆-S-X₈-X₉-X₁₀-D-D-S-X₁₄-X₁₅-N-K-V-L-T-X₂₀-X₂₁-X₂₂-X₂₃-N (SEQ ID NO:19),
wherein
X₁ is selected from D and E;
X₃ is selected from D and E;
X₆ is selected from Q, H, R, K, Y, I, L and W;
X₈ is selected from F, L, V, K, Y, M, I, R, A, E, D and Q (*e.g.,* F);
X₉ is selected from L, R, T, I, V, S, C, Y, K, F and G;
X₁₀ is selected from K, Q, Y, T, F, L, W, M, A, E, G, and S;
X₁₄ is selected from I, L, F, S, R, Y, Q, W, D, K and H (*e.g.,* I, L and F);
X₁₅ is selected from D, S, I, N, E, A, H, F, L, Q, M, G, Y and V;
X₂₀ is selected from R, F, T, W, E, L, N, C, K, V, S, Q, I, Y, H and A;
X₂₁ is selected from S, P, R, K, N, A, H, Q, G and L;
X₂₂ is selected from D, G, T, N, S, K, A, I, E, L, Q, R and Y; and
X₂₃ is selected from K, V, A, E, Y, I, C, L, S, T, G, K, M, D and F.

The HNH-like domain may differ from a sequence of SEQ ID NO: 19 by 1, 2, 3, 4, or 5 residues.

A Cas9 molecule or Cas9 polypeptide may comprise a HNH-like domain having an amino acid sequence of Formula XII:
D-X₂-D-H-I-X₅-P-Q-X₇-F-X₉-X₁₀-D-X₁₂-S-I-D-N-X₁₆-V-L-X₁₉-X₂₀-S-X₂₂-X₂₃-N(SEQID NO:20),
wherein
X₂ is selected from I and V;
X₅ is selected from I and V;
X₇ is selected from A and S;
X₉ is selected from I and L;
X₁₀ is selected from K and T;
X₁₂ is selected from D and N;
X₁₆ is selected from R, K and L;
X₁₉ is selected from T and V;
X₂₀ is selected from S and R;
X₂₂ is selected from K, D and A; and
X₂₃ is selected from E, K, G and N (*e.g.,* the Cas9 molecule or Cas9 polypeptide can comprise an HNH-like domain as described herein).

The HNH-like domain may differ from a sequence of SEQ ID NO: 20 by as many as 1 but not more than 2, 3, 4, or 5 residues.

A Cas9 molecule or Cas9 polypeptide may comprise the amino acid sequence of formula XIII:
L-Y-Y-L-Q-N-G-X₁'-D-M-Y-X₂'-X₃'-X₄'-X₅'-L-D-I-X₆'-X₇'-L-S-X₈'-Y-Z-N-R-X₉'-K-X₁₀'-D-X₁₁'-V-P (SEQ ID NO: 21),
wherein
X₁' is selected from K and R;
X₂' is selected from V and T;
X₃' is selected from G and D;
X₄' is selected from E, Q and D;
X₅' is selected from E and D;
X₆' is selected from D, N and H;
X₇' is selected from Y, R and N;
X₈' is selected from Q, D and N;
X₉' is selected from G and E;
X₁₀' is selected from S and G;
X₁₁' is selected from D and N; and
Z is an HNH-like domain, *e.g.,* as described above.

A Cas9 molecule or Cas9 polypeptide may comprise an amino acid sequence that differs from a sequence of SEQ ID NO:21 by as many as 1 but not more than 2, 3, 4, or 5 residues.

The HNH-like domain may differ from a sequence of an HNH-like domain disclosed herein, *e.g.,* in SEQ ID Nos: 178-252, as many as 1 but not more than 2, 3, 4, or 5 residues. One or both of the highly conserved residues of SEQ ID Nos: 178-252 may be present.

The HNH-like domain may differ from a sequence of an HNH-like domain disclosed herein, *e.g.,* in SEQ ID Nos: 253-302, as many as 1 but not more than 2, 3, 4, or 5 residues. One, two, or all three of the highly conserved residues of SEQ ID Nos: 253-302 may be present.

### Cas9 Activities

The Cas9 molecule or Cas9 polypeptide may be capable of cleaving a target nucleic acid molecule. Typically wild-type Cas9 molecules cleave both strands of a target nucleic acid molecule. Cas9 molecules and Cas9 polypeptides can be engineered to alter nuclease cleavage (or other properties), *e.g.,* to provide a Cas9 molecule or Cas9 polypeptide which is a nickase, or which lacks the ability to cleave target nucleic acid. A Cas9 molecule or Cas9 polypeptide that is capable of cleaving a target nucleic acid molecule is referred to herein as an eaCas9 (an enzymatically active Cas9) molecule or eaCas9 polypeptide.

An eaCas9 molecule or eaCas9 polypeptide may comprise one or more of the following enzymatic activities:
a nickase activity, *i.e.,* the ability to cleave a single strand, *e.g.,* the non-complementary strand or the complementary strand, of a nucleic acid molecule;
a double stranded nuclease activity, *i.e.,* the ability to cleave both strands of a double stranded nucleic acid and create a double stranded break, which in an embodiment is the presence of two nickase activities;
an endonuclease activity;
an exonuclease activity; and
a helicase activity, *i.e*., the ability to unwind the helical structure of a double stranded nucleic acid.

An enzymatically active Cas9 or eaCas9 molecule or eaCas9 polypeptide may cleave both DNA strands and result in a double stranded break. An eaCas9 molecule or eaCas9 polypeptide may cleave only one strand, *e.g.,* the strand to which the gRNA hybridizes to, or the strand complementary to the strand the gRNA hybridizes with. An eaCas9 molecule or eaCas9 polypeptide may comprise cleavage activity associated with an HNH domain. An eaCas9 molecule or eaCas9 polypeptide may comprise cleavage activity associated with a RuvC domain. An eaCas9 molecule or eaCas9 polypeptide may comprise cleavage activity associated with an HNH domain and cleavage activity associated with a RuvC domain. An eaCas9 molecule or eaCas9 polypeptide may comprise an active, or cleavage competent, HNH domain and an inactive, or cleavage incompetent, RuvC domain. An eaCas9 molecule or eaCas9 polypeptide may comprise an inactive, or cleavage incompetent, HNH domain and an active, or cleavage competent, RuvC domain.

Some Cas9 molecules or Cas9 polypeptides have the ability to interact with a gRNA molecule, and in conjunction with the gRNA molecule localize to a core target domain, but are incapable of cleaving the target nucleic acid, or incapable of cleaving at efficient rates. Cas9 molecules having no, or no substantial, cleavage activity are referred to herein as an eiCas9 molecule or eiCas9 polypeptide. For example, an eiCas9 molecule or eiCas9 polypeptide can lack cleavage activity or have substantially less, *e.g.,* less than 20, 10, 5, 1 or 0.1 % of the cleavage activity of a reference Cas9 molecule or eiCas9 polypeptide, as measured by an assay described herein.

### Targeting and PAMs

A Cas9 molecule or Cas9 polypeptide that can interact with a gRNA molecule and, in concert with the gRNA molecule, localizes to a site which comprises a target domain, and in certain embodiments, a PAM sequence.

The ability of an eaCas9 molecule or eaCas9 polypeptide to interact with and cleave a target nucleic acid may be PAM sequence dependent. A PAM sequence is a sequence in the target nucleic acid. Cleavage of the target nucleic acid may occur upstream from the PAM sequence. eaCas9 molecules from different bacterial species can recognize different sequence motifs (*e.g.,* PAM sequences). An eaCas9 molecule *of S. pyogenes* may recognize the sequence motif NGG and directs cleavage of a target nucleic acid sequence 1 to 10, *e.g.,* 3 to 5, bp upstream from that sequence (see, *e.g.,* Mali 2013). An eaCas9 molecule of *S. thermophilus* may recognize the sequence motif NGGNG and/or NNAGAAW (W = A or T) and directs cleavage of a target nucleic acid sequence 1 to 10, *e.g.,* 3 to 5, bp upstream from these sequences (see, *e.g.,* Horvath 2010; Deveau 2008). An eaCas9 molecule of *S. mutans* may recognize the sequence motif NGG and/or NAAR (R = A or G) and directs cleavage of a target nucleic acid sequence 1 to 10, *e.g.,* 3 to 5, bp upstream from this sequence (see, *e.g.,* Deveau 2008). An eaCas9 molecule *of S. aureus* may recognize the sequence motif NNGRR (R = A or G) and directs cleavage of a target nucleic acid sequence 1 to 10, *e.g.,* 3 to 5, bp upstream from that sequence. An eaCas9 molecule *of S. aureus* may recognize the sequence motif NNGRRN (R = A or G) and directs cleavage of a target nucleic acid sequence 1 to 10, *e.g.,* 3 to 5, bp upstream from that sequence. An eaCas9 molecule of *S. aureus* may recognize the sequence motif NNGRRT (R = A or G) and directs cleavage of a target nucleic acid sequence 1 to 10, *e.g.,* 3 to 5, base pairs upstream from that sequence. An eaCas9 molecule of *S. aureus* may recognize the sequence motif NNGRRV (R = A or G) and directs cleavage of a target nucleic acid sequence 1 to 10, *e.g.,* 3 to 5, bp upstream from that sequence. The ability of a Cas9 molecule to recognize a PAM sequence can be determined, *e.g.,* using a transformation assay as described in Jinek 2012. In the aforementioned embodiments, N can be any nucleotide residue, *e.g.,* any of A, G, C, or T.

As is discussed herein, Cas9 molecules can be engineered to alter the PAM specificity of the Cas9 molecule.

Exemplary naturally occurring Cas9 molecules have been described previously (see, *e.g.,* Chylinski 2013). Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 11 bacterial family, a cluster 12 bacterial family, a cluster 13 bacterial family, a cluster 14 bacterial family, a cluster 15 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family, a cluster 49 bacterial family, a cluster 50 bacterial family, a cluster 51 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: *S. aureus, S. pyogenes* (*e.g.,* strain SF370, MGAS10270, MGAS10750, MGAS2096, MGAS315, MGAS5005, MGAS6180, MGAS9429, NZ131 and SSI-1), *S. thermophilus* (*e.g.,* strain LMD-9), *S. pseudoporcinus* (*e.g.,* strain SPIN 20026), *S. mutans* (*e.g.,* strain UA159, NN2025), *S. macacae* (*e.g.,* strain NCTC11558), *S. gallolyticus* (*e.g.,* strain UCN34, ATCC BAA-2069), *S. equines* (*e.g.,* strain ATCC 9812, MGCS 124), *S. dysdalactiae* (*e.g.,* strain GGS 124), *S. bovis* (*e.g.,* strain ATCC 700338), *S. anginosus* (*e.g.,* strain F0211), *S. agalactiae* (*e.g.,* strain NEM316, A909), *Listeria monocytogenes* (*e.g.,* strain F6854), *Listeria innocua* (*L. innocua, e.g.,* strain Clip11262), *Enterococcus italicus* (*e.g.,* strain DSM 15952), or *Enterococcus faecium* (*e.g.,* strain 1,231,408).

A Cas9 molecule or Cas9 polypeptide may comprise an amino acid sequence: having 60%, 65%, 70%, 75%, 80%, 85%, 90%, 95%, 96%, 97%, 98% or 99% homology with; differs at no more than, 2, 5, 10, 15, 20, 30, or 40% of the amino acid residues when compared with; differs by at least 1, 2, 5, 10 or 20 amino acids, but by no more than 100, 80, 70, 60, 50, 40 or 30 amino acids from; or is identical to any Cas9 molecule sequence described herein, or to a naturally occurring Cas9 molecule sequence, *e.g.,* a Cas9 molecule from a species listed herein (*e.g.,* SEQ ID NO:1-4 or described in Chylinski 2013 or Hou 2013). The Cas9 molecule or Cas9 polypeptide may comprise one or more of the following activities: a nickase activity; a double stranded cleavage activity (*e.g.,* an endonuclease and/or exonuclease activity); a helicase activity; or the ability, together with a gRNA molecule, to localize to a target nucleic acid.

A comparison of the sequence of a number of Cas9 molecules indicate that certain regions are conserved. These are identified below as:
region 1 (residues 1 to 180, or in the case of region 1, residues 120 to 180)
region 2 (residues 360 to 480);
region 3 (residues 660 to 720);
region 4 (residues 817 to 900); and
region 5 (residues 900 to 960).

A Cas9 molecule or Cas9 polypeptide may comprise regions 1-5, together with sufficient additional Cas9 molecule sequence to provide a biologically active molecule, *e.g.,* a Cas9 molecule having at least one activity described herein. Each of regions 1-5, independently, may have 50%, 60%, 70%, 80%, 85%, 90%, 95%, 96%, 97%, 98% or 99% homology with the corresponding residues of a Cas9 molecule or Cas9 polypeptide described herein, *e.g.,* a sequence from SEQ ID Nos: 1-4.

A Cas9 molecule or Cas9 polypeptide may comprise an amino acid sequence referred to as region 1:
having 50%, 60%, 70%, 80%, 85%, 90%, 95%, 96%, 97%, 98% or 99% homology with amino acids 1-180 of the amino acid sequence of Cas9 of *S. pyogenes;*
differs by at least 1, 2, 5, 10 or 20 amino acids but by no more than 90, 80, 70, 60, 50, 40 or 30 amino acids from amino acids 1-180 of the amino acid sequence of Cas9 of *S*. *pyogenes, S. thermophilus, S. mutans,* or *Listeria innocua;* or
is identical to amino acids 1-180 of the amino acid sequence of Cas9 *of S. pyogenes, S. thermophilus, S. mutans,* or *L. innocua.*

A Cas9 molecule or Cas9 polypeptide may comprise an amino acid sequence referred to as region 1':
having 55%, 60%, 65%, 70%, 75%, 80%, 85%, 90%, 95%, 96%, 97%, 98% or 99% homology with amino acids 120-180 of the amino acid sequence of Cas9 *of S. pyogenes, S. thermophilus, S. mutans* or *L. innocua;*
differs by at least 1, 2, or 5 amino acids but by no more than 35, 30, 25, 20 or 10 amino acids from amino acids 120-180 of the amino acid sequence of Cas9 of *S. pyogenes, S. thermophilus, S. mutans,* or *L. innocua* ; or
is identical to amino acids 120-180 of the amino acid sequence of Cas9 of *S*. *pyogenes, S. thermophilus, S. mutans,* or *L. innocua.*

A Cas9 molecule or Cas9 polypeptide may comprise an amino acid sequence referred to as region 2:
having 50%, 55%, 60%, 65%, 70%, 75%, 80%, 85%, 90%, 95%, 96%, 97%, 98% or 99% homology with amino acids 360-480 of the amino acid sequence of Cas9 of *S. pyogenes, S. thermophilus, S. mutans,* or *L. innocua;*
differs by at least 1, 2, or 5 amino acids but by no more than 35, 30, 25, 20 or 10 amino acids from amino acids 360-480 of the amino acid sequence of Cas9 of *S. pyogenes, S. thermophilus, S. mutans,* or *L. innocua;* or
is identical to amino acids 360-480 of the amino acid sequence of Cas9 of *S*. *pyogenes, S. thermophilus, S. mutans,* or *L. innocua.*

A Cas9 molecule or Cas9 polypeptide may comprise an amino acid sequence referred to as region 3:
having 55%, 60%, 65%, 70%, 75%, 80%, 85%, 90%, 95%, 96%, 97%, 98%, or 99% homology with amino acids 660-720 of the amino acid sequence of Cas9 of *S. pyogenes, S. thermophilus, S. mutans* or *L. innocua;*
differs by at least 1, 2, or 5 amino acids but by no more than 35, 30, 25, 20 or 10 amino acids from amino acids 660-720 of the amino acid sequence of Cas9 *of S. pyogenes, S. thermophilus, S. mutans* or *L. innocua;* or
is identical to amino acids 660-720 of the amino acid sequence of Cas9 of *S*. *pyogenes, S. thermophilus, S. mutans* or *L. innocua.*

A Cas9 molecule or Cas9 polypeptide may comprise an amino acid sequence referred to as region 4:
having 50%, 55%, 60%, 65%, 70%, 75%, 80%, 85%, 90%, 95%, 96%, 97%, 98%, or 99% homology with amino acids 817-900 of the amino acid sequence of Cas9 *of S. pyogenes, S. thermophilus, S. mutans,* or *L. innocua;*
differs by at least 1, 2, or 5 amino acids but by no more than 35, 30, 25, 20 or 10 amino acids from amino acids 817-900 of the amino acid sequence of Cas9 of *S. pyogenes, S. thermophilus, S. mutans,* or *L. innocua;* or
is identical to amino acids 817-900 of the amino acid sequence of Cas9 of *S*. *pyogenes, S. thermophilus, S. mutans,* or *L. innocua.*

A Cas9 molecule or Cas9 polypeptide may comprise an amino acid sequence referred to as region 5:
having 50%, 55%, 60%, 65%, 70%, 75%, 80%, 85%, 90%, 95%, 96%, 97%, 98%, or 99% homology with amino acids 900-960 of the amino acid sequence of Cas9 of *S. pyogenes, S. thermophilus, S. mutans,* or *L. innocua;*
differs by at least 1, 2, or 5 amino acids but by no more than 35, 30, 25, 20 or 10 amino acids from amino acids 900-960 of the amino acid sequence of Cas9 *of S. pyogenes, S. thermophilus, S. mutans,* or *L. innocua;* or
is identical to amino acids 900-960 of the amino acid sequence of Cas9 of *S*. *pyogenes, S. thermophilus, S. mutans,* or *L. innocua.*

### Engineered Or Altered Cas9 Molecules And Cas9 Polypeptides

Cas9 molecules and Cas9 polypeptides described herein can possess any of a number of properties, including: nickase activity, nuclease activity (*e.g.,* endonuclease and/or exonuclease activity); helicase activity; the ability to associate functionally with a gRNA molecule; and the ability to target (or localize to) a site on a nucleic acid (*e.g.,* PAM recognition and specificity). A Cas9 molecule or Cas9 polypeptide can include all or a subset of these properties. In a typical case, a Cas9 molecule or Cas9 polypeptide has the ability to interact with a gRNA molecule and, in concert with the gRNA molecule, localize to a site in a nucleic acid. Other activities, *e.g.,* PAM specificity, cleavage activity, or helicase activity can vary more widely in Cas9 molecules and Cas9 polypeptides.

Cas9 molecules include engineered Cas9 molecules and engineered Cas9 polypeptides (engineered, as used in this context, means merely that the Cas9 molecule or Cas9 polypeptide differs from a reference sequences, and implies no process or origin limitation). An engineered Cas9 molecule or Cas9 polypeptide can comprise altered enzymatic properties, *e.g.,* altered nuclease activity, (as compared with a naturally occurring or other reference Cas9 molecule) or altered helicase activity. As discussed herein, an engineered Cas9 molecule or Cas9 polypeptide can have nickase activity (as opposed to double-strand nuclease activity). An engineered Cas9 molecule or Cas9 polypeptide can have an alteration that alters its size, *e.g.,* a deletion of amino acid sequence that reduces its size, *e.g.,* without significant effect on one or more, or any Cas9 activity. An engineered Cas9 molecule or Cas9 polypeptide can comprise an alteration that affects PAM recognition. For example, an engineered Cas9 molecule can be altered to recognize a PAM sequence other than that recognized by the endogenous wild-type PI domain. A Cas9 molecule or Cas9 polypeptide can differ in sequence from a naturally occurring Cas9 molecule but not have significant alteration in one or more Cas9 activities.

Cas9 molecules or Cas9 polypeptides with desired properties can be made in a number of ways, *e.g.,* by alteration of a parental, *e.g.,* naturally occurring, Cas9 molecules or Cas9 polypeptides, to provide an altered Cas9 molecule or Cas9 polypeptide having a desired property. For example, one or more mutations or differences relative to a parental Cas9 molecule, *e.g.,* a naturally occurring or engineered Cas9 molecule, can be introduced. Such mutations and differences comprise: substitutions (*e.g.,* conservative substitutions or substitutions of non-essential amino acids); insertions; or deletions. A Cas9 molecule or Cas9 polypeptide can comprise one or more mutations or differences, *e.g.,* at least 1, 2, 3, 4, 5, 10, 15, 20, 30, 40 or 50 mutations but less than 200, 100, or 80 mutations relative to a reference, *e.g.,* a parental, Cas9 molecule.

A mutation or mutations may or may not have a substantial effect on a Cas9 activity, *e.g.,* a Cas9 activity described herein.

### Non-Cleaving and Modified-Cleavage Cas9 Molecules and Cas9 Polypeptides

A Cas9 molecule or Cas9 polypeptide may comprise a cleavage property that differs from naturally occurring Cas9 molecules, *e.g.,* that differs from the naturally occurring Cas9 molecule having the closest homology. For example, a Cas9 molecule or Cas9 polypeptide can differ from naturally occurring Cas9 molecules, *e.g.,* a Cas9 molecule of *S. pyogenes,* as follows: its ability to modulate, *e.g.,* decreased or increased, cleavage of a double stranded nucleic acid (endonuclease and/or exonuclease activity), *e.g.,* as compared to a naturally occurring Cas9 molecule (*e.g.,* a Cas9 molecule of *S. pyogenes);* its ability to modulate, *e.g.,* decreased or increased, cleavage of a single-strand of a nucleic acid, *e.g.,* a non-complementary strand of a nucleic acid molecule or a complementary strand of a nucleic acid molecule (nickase activity), *e.g.,* as compared to a naturally occurring Cas9 molecule (*e.g.,* a Cas9 molecule of *S. pyogenes);* or the ability to cleave a nucleic acid molecule, *e.g.,* a double stranded or single stranded nucleic acid molecule, can be eliminated.

An eaCas9 molecule or eaCas9 polypeptide may comprise one or more of the following activities: cleavage activity associated with an N-terminal RuvC-like domain; cleavage activity associated with an HNH-like domain; cleavage activity associated with an HNH-like domain and cleavage activity associated with an N-terminal RuvC-like domain.

An eaCas9 molecule or eaCas9 polypeptide may comprise an active, or cleavage competent, HNH-like domain (*e.g.,* an HNH-like domain described herein) and an inactive, or cleavage incompetent, N-terminal RuvC-like domain. An exemplary inactive, or cleavage incompetent N-terminal RuvC-like domain can have a mutation of an aspartic acid in an N-terminal RuvC-like domain, *e.g.,* an aspartic acid at position 10 of SEQ ID NO:2, *e.g.,* can be substituted with an alanine. The eaCas9 molecule or eaCas9 polypeptide may differ from wild-type in the N-terminal RuvC-like domain and does not cleave the target nucleic acid, or cleaves with significantly less efficiency, *e.g.,* less than 20, 10, 5, 1 or .1 % of the cleavage activity of a reference Cas9 molecule, *e.g.,* as measured by an assay described herein. The reference Cas9 molecule can by a naturally occurring unmodified Cas9 molecule, *e.g.,* a naturally occurring Cas9 molecule such as a Cas9 molecule of *S. pyogenes, S. aureus,* or *S*. *thermophilus.* The reference Cas9 molecule may be the naturally occurring Cas9 molecule having the closest sequence identity or homology.

An eaCas9 molecule or eaCas9 polypeptide may comprise an inactive, or cleavage incompetent, HNH domain and an active, or cleavage competent, N-terminal RuvC-like domain (*e.g.,* a RuvC-like domain described herein). Exemplary inactive, or cleavage incompetent HNH-like domains can have a mutation at one or more of: a histidine in an HNH-like domain, for example, at position 856 of the *S. pyogenes* Cas9 sequence (SEQ ID NO:2), *e.g.,* can be substituted with an alanine; and one or more asparagines in an HNH-like domain, for example, at position 870 and/or 879 of the S. *pyogenes* Cas9 sequence (SEQ ID NO:2) *e.g.,* can be substituted with an alanine. The eaCas9 may differ from wild-type in the HNH-like domain and may not cleave the target nucleic acid, or may cleave with significantly less efficiency, *e.g.,* less than 20, 10, 5, 1 or 0.1% of the cleavage activity of a reference Cas9 molecule, *e.g.,* as measured by an assay described herein. The reference Cas9 molecule can by a naturally occurring unmodified Cas9 molecule, *e.g.,* a naturally occurring Cas9 molecule such as a Cas9 molecule *of S. pyogenes, S. aureus,* or *S. thermophilus.* The reference Cas9 molecule may be the naturally occurring Cas9 molecule having the closest sequence identity or homology.

Exemplary Cas9 activities may comprise one or more of PAM specificity, cleavage activity, and helicase activity. A mutation(s) can be present, *e.g.,* in: one or more RuvC domains, *e.g.,* an N-terminal RuvC domain; an HNH domain; a region outside the RuvC domains and the HNH domain. A mutation(s) may be present in a RuvC domain. A mutation(s) may be present in an HNH domain. Mutations may be present in both a RuvC domain and an HNH domain.

Exemplary mutations that may be made in the RuvC domain or HNH domain with reference to the *S. pyogenes* Cas9 sequence include: D10A, E762A, H840A, N854A, N863A and/or D986A. Exemplary mutations that may be made in the RuvC domain with reference to the *S. aureus* Cas9 sequence include N580A.

A Cas9 molecule may be an eiCas9 molecule comprising one or more differences in a RuvC domain and/or in an HNH domain as compared to a reference Cas9 molecule, and the eiCas9 molecule does not cleave a nucleic acid, or cleaves with significantly less efficiency than does wild type, *e.g.,* when compared with wild type in a cleavage assay, *e.g.,* as described herein, cuts with less than 50, 25, 10, or 1% of a reference Cas9 molecule, as measured by an assay described herein.

Whether or not a particular sequence, *e.g.,* a substitution, may affect one or more activity, such as targeting activity, cleavage activity, etc., can be evaluated or predicted, *e.g.,* by evaluating whether the mutation is conservative. A "non-essential" amino acid residue, as used in the context of a Cas9 molecule, may be a residue that can be altered from the wild-type sequence of a Cas9 molecule, *e.g.,* a naturally occurring Cas9 molecule, *e.g.,* an eaCas9 molecule, without abolishing or more preferably, without substantially altering a Cas9 activity (*e.g.,* cleavage activity), whereas changing an "essential" amino acid residue results in a substantial loss of activity (*e.g.,* cleavage activity).

A Cas9 molecule may comprise a cleavage property that differs from naturally occurring Cas9 molecules, *e.g.,* that differs from the naturally occurring Cas9 molecule having the closest homology. For example, a Cas9 molecule can differ from naturally occurring Cas9 molecules, *e.g.,* a Cas9 molecule of *S aureus* or *S. pyogenes* as follows: its ability to modulate, *e.g.,* decreased or increased, cleavage of a double stranded break (endonuclease and/or exonuclease activity), *e.g.,* as compared to a naturally occurring Cas9 molecule *(e.g.,* a Cas9 molecule of *S aureus* or *S. pyogenes*); its ability to modulate, *e.g.,* decreased or increased, cleavage of a single-strand of a nucleic acid, *e.g.,* a non-complimentary strand of a nucleic acid molecule or a complementary strand of a nucleic acid molecule (nickase activity), *e.g.,* as compared to a naturally occurring Cas9 molecule (*e.g.,* a Cas9 molecule of *S aureus* or *S. pyogenes*); or the ability to cleave a nucleic acid molecule, *e.g.,* a double stranded or single stranded nucleic acid molecule, can be eliminated. The nickase may be *S. aureus* Cas9-derived nickase comprising the sequence of SEQ ID NO: 16302 (D10A) or SEQ ID NO: 16303 (N580A) (Friedland 2015).

The altered Cas9 molecule may be an eaCas9 molecule comprising one or more of the following activities: cleavage activity associated with a RuvC domain; cleavage activity associated with an HNH domain; cleavage activity associated with an HNH domain and cleavage activity associated with a RuvC domain.

The altered Cas9 molecule may be an eiCas9 molecule which does not cleave a nucleic acid molecule (either double stranded or single stranded nucleic acid molecules) or cleaves a nucleic acid molecule with significantly less efficiency, *e.g.,* less than 20, 10, 5, 1 or 0.1% of the cleavage activity of a reference Cas9 molecule, *e.g.,* as measured by an assay described herein. The reference Cas9 molecule can be a naturally occurring unmodified Cas9 molecule, *e.g.,* a naturally occurring Cas9 molecule such as a Cas9 molecule of *S. pyogenes, S. thermophilus, S. aureus, C. jejuni* or *N. meningitidis.* The reference Cas9 molecule may be the naturally occurring Cas9 molecule having the closest sequence identity or homology. The eiCas9 molecule may lack substantial cleavage activity associated with a RuvC domain and cleavage activity associated with an HNH domain.

The altered Cas9 molecule or Cas9 polypeptide, *e.g.,* an
eaCas9 molecule or eaCas9 polypeptide, can be a fusion, *e.g.,* of two of more different Cas9 molecules, *e.g.,* of two or more naturally occurring Cas9 molecules of different species. For example, a fragment of a naturally occurring Cas9 molecule of one species can be fused to a fragment of a Cas9 molecule of a second species. As an example, a fragment of a Cas9 molecule of *S. pyogenes* comprising an N-terminal RuvC-like domain can be fused to a fragment of Cas9 molecule of a species other than *S. pyogenes* (*e.g., S. thermophilus*) comprising an HNH-like domain.

### Cas9 with Altered or No PAM Recognition

Naturally-occurring Cas9 molecules can recognize specific PAM sequences, for example the PAM recognition sequences described above for, *e.g., S. pyogenes, S. thermophilus, S. mutans,* and *S. aureus.*

A Cas9 molecule or Cas9 polypeptide may have the same PAM specificities as a naturally occurring Cas9 molecule. A Cas9 molecule or Cas9 polypeptide may have a PAM specificity not associated with a naturally occurring Cas9 molecule, or a PAM specificity not associated with the naturally occurring Cas9 molecule to which it has the closest sequence homology. For example, a naturally occurring Cas9 molecule can be altered, *e.g.,* to alter PAM recognition, *e.g.,* to alter the PAM sequence that the Cas9 molecule or Cas9 polypeptide recognizes in order to decrease off-target sites and/or improve specificity; or eliminate a PAM recognition requirement. A Cas9 molecule or Cas9 polypeptide can be altered, *e.g.,* to increase length of PAM recognition sequence and/or improve Cas9 specificity to high level of identity (*e.g.,* 98%, 99% or 100% match between gRNA and a PAM sequence), *e.g.,* to decrease off-target sites and/or increase specificity. The length of the PAM recognition sequence may be at least 4, 5, 6, 7, 8, 9, 10 or 15 amino acids in length. The Cas9 specificity may require at least 90%, 95%, 96%, 97%, 98%, 99% or more homology between the gRNA and the PAM sequence. Cas9 molecules or Cas9 polypeptides that recognize different PAM sequences and/or have reduced off-target activity can be generated using directed evolution. Exemplary methods and systems that can be used for directed evolution of Cas9 molecules are described (see, *e.g.,* Esvelt 2011). Candidate Cas9 molecules can be evaluated, *e.g.,* by methods described below.

### Size-Optimized Cas9 Molecules

Engineered Cas9 molecules and engineered Cas9 polypeptides described herein include a Cas9 molecule or Cas9 polypeptide comprising a deletion that reduces the size of the molecule while still retaining desired Cas9 properties, *e.g.,* essentially native conformation, Cas9 nuclease activity, and/or target nucleic acid molecule recognition. Provided herein are Cas9 molecules or Cas9 polypeptides comprising one or more deletions and optionally one or more linkers, wherein a linker is disposed between the amino acid residues that flank the deletion. Methods for identifying suitable deletions in a reference Cas9 molecule, methods for generating Cas9 molecules with a deletion and a linker, and methods for using such Cas9 molecules will be apparent to one of ordinary skill in the art upon review of this document.

A Cas9 molecule, *e.g.,* a *S. aureus* or *S. pyogenes* Cas9 molecule, having a deletion is smaller, *e.g.,* has reduced number of amino acids, than the corresponding naturally-occurring Cas9 molecule. The smaller size of the Cas9 molecules allows increased flexibility for delivery methods, and thereby increases utility for genome-editing. A Cas9 molecule can comprise one or more deletions that do not substantially affect or decrease the activity of the resultant Cas9 molecules described herein. Activities that are retained in the Cas9 molecules comprising a deletion as described herein include one or more of the following:
a nickase activity, *i.e.,* the ability to cleave a single strand, *e.g.,* the non-complementary strand or the complementary strand, of a nucleic acid molecule; a double stranded nuclease activity, *i.e.,* the ability to cleave both strands of a double stranded nucleic acid and create a double stranded break, which may be the presence of two nickase activities; an endonuclease activity; an exonuclease activity; a helicase activity, *i.e*., the ability to unwind the helical structure of a double stranded nucleic acid; and recognition activity of a nucleic acid molecule, *e.g.,* a target nucleic acid or a gRNA molecule.

Activity of the Cas9 molecules described herein can be assessed using the activity assays described herein or in the art.

### Identifying regions suitable for deletion

Suitable regions of Cas9 molecules for deletion can be identified by a variety of methods. Naturally-occurring orthologous Cas9 molecules from various bacterial species can be modeled onto the crystal structure of *S. pyogenes* Cas9 (Nishimasu 2014) to examine the level of conservation across the selected Cas9 orthologs with respect to the three-dimensional conformation of the protein. Less conserved or unconserved regions that are spatially located distant from regions involved in Cas9 activity, *e.g.,* interface with the target nucleic acid molecule and/or gRNA, represent regions or domains are candidates for deletion without substantially affecting or decreasing Cas9 activity.

### Nucleic Acids Encoding Cas9 Molecules

Nucleic acids encoding the Cas9 molecules or Cas9 polypeptides, *e.g.,* an eaCas9 molecule or eaCas9 polypeptides are provided herein. Exemplary nucleic acids encoding Cas9 molecules or Cas9 polypeptides have been described previously (see, *e.g.,* Cong 2013; Wang 2013; Mali 2013; Jinek 2012).

A nucleic acid encoding a Cas9 molecule or Cas9 polypeptide can be a synthetic nucleic acid sequence. For example, the synthetic nucleic acid molecule can be chemically modified, *e.g.,* as described herein. The Cas9 mRNA may have one or more (*e.g.,* all of the following properties: it is capped, polyadenylated, substituted with 5-methylcytidine and/or pseudouridine.

In addition, or alternatively, the synthetic nucleic acid sequence can be codon optimized, *e.g.,* at least one non-common codon or less-common codon has been replaced by a common codon. For example, the synthetic nucleic acid can direct the synthesis of an optimized messenger mRNA, *e.g.,* optimized for expression in a mammalian expression system, *e.g.,* described herein.

In addition, or alternatively, a nucleic acid encoding a Cas9 molecule or Cas9 polypeptide may comprise a nuclear localization sequence (NLS). Nuclear localization sequences are known in the art.

An exemplary codon optimized nucleic acid sequence encoding a Cas9 molecule of *S*. *pyogenes* is set forth in SEQ ID NO: 22. The corresponding amino acid sequence of an *S*. *pyogenes* Cas9 molecule is set forth in SEQ ID NO: 23.

Exemplary codon optimized nucleic acid sequence encoding a Cas9 molecule of *S*. *aureus* is set forth in SEQ ID NO: 26, 39, 16300 and 16301.

If any of the above Cas9 sequences are fused with a peptide or polypeptide at the C-terminus, it is understood that the stop codon will be removed.

### Other Cas Molecules and Cas Polypeptides

Various types of Cas molecules or Cas polypeptides can be used to practice the inventions disclosed herein. Cas molecules of Type II Cas systems may be used. Cas molecules of other Cas systems may be used. For example, Type I or Type III Cas molecules may be used. Exemplary Cas molecules (and Cas systems) have been described previously (see, *e.g.,* Haft 2005; Makarova 2011). Exemplary Cas molecules (and Cas systems) are also shown in **Table 4.**

| **Table 4: Cas Systems** | | | | | |
|---|---|---|---|---|---|
| **Gene name^{‡}** | **System type or subtype** | **Name from Haft 2005^{§}** | **Structure of encoded protein (PDB accessions)^{¶}** | **Families (and superfamily) of encoded protein^{#**}** | **Representatives** |
| *cas1* | • Type I | *cas1* | 3GOD, 3LFX and 2YZS | COG1518 | SERP2463, SPy1047 and *ygbT* |
| | • Type II | | | | |
| | • Type III | | | | |
| *cas2* | • Type I | *cas2* | 2IVY, 2I8E and 3EXC | COG1343 and COG3512 | SERP2462, SPy1048, SPyl723 (N-terminal domain) and *ygbF* |
| | • Type II | | | | |
| | • Type III | | | | |
| *cas3'* | • Type 1^{‡} | *cas3* | NA | COG1203 | APE1232 and *ygcB* |
| *cas3"* | • Subtype I-A | NA | NA | COG2254 | APE1231 and BH0336 |
| | • Subtype I-B | | | | |
| *cas4* | • Subtype I-A | *cas4* and *csa1* | NA | COG1468 | APE1239 and BH0340 |
| | • Subtype I-B | | | | |
| | • Subtype I-C | | | | |
| | • Subtype I-D | | | | |
| | • Subtype II-B | | | | |
| *cas5* | • Subtype I-A | *cas5a, cas5d,* | 3KG4 | COG1688 (RAMP) | APE1234, BH0337, *devS* and *ygcI* |
| | • Subtype I-B | *cas5e, cas5h,* | | | |
| | • Subtype I-C | *cas5p, cas5t* | | | |
| | • Subtype I-E | and *cmx5* | | | |
| *cas6* | • Subtype I-A | *cas6* and *cmx6* | 3I4H | COG1583 and COG5551 (RAMP) | PF1131 and slr7014 |
| | • Subtype I-B | | | | |
| | • Subtype I-D | | | | |
| | • Subtype III-A• Subtype III-B | | | | |
| *cas6e* | • Subtype I-E | *cse3* | 1WJ9 | (RAMP) | *ygcH* |
| *cas6f* | • Subtype I-F | *csy4* | 2XLJ | (RAMP) | yl727 |
| *cas7* | • Subtype I-A | *csa2, csd2,* | NA | COG1857 and COG3649 (RAMP) | *devR* and *ygcJ* |
| | • Subtype I-B | *cse4, csh2,* | | | |
| | • Subtype I-C | *csp1* and *cst2* | | | |
| | • Subtype I-E | | | | |
| *cas8a1* | • Subtype I-A^{‡‡} | *cmx1, cst1, csx8, csx13* and CXXC-CXXC | NA | BH0338-like | LA3191^{§§} and PG2018^{§§} |
| *cas8a2* | • Subtype I-A^{‡‡} | *csa4* and *csx9* | NA | PH0918 | AF0070, AF1873, MJ0385, PF0637, PH0918 and SS01401 |
| *cas8b* | • Subtype I-B^{‡‡} | *csh1* and TM1802 | NA | BH0338-like | MTH1090 and TM1802 |
| *cas8c* | • Subtype I-C^{‡‡} | *csd1* and *csp2* | NA | BH0338-like | BH0338 |
| *cas9* | • Type II^{‡‡} | *csn1* and *csx12* | NA | COG3513 | FTN_0757 and SPy1046 |
| *cas10* | • Type III^{‡‡} | *cmr2, csm1* | NA | COG1353 | MTH326, Rv2823c^{§§} |
| | | and *csx11* | | | and TM1794^{§§} |
| *cas10d* | • Subtype I-D^{‡‡} | *csc3* | NA | COG1353 | slr7011 |
| *csy1* | • Subtype IF^{‡‡} | *csy1* | NA | yl724-like | yl724 |
| *csy2* | • Subtype I-F | *csy2* | NA | (RAMP) | yl725 |
| *csy3* | • Subtype I-F | *csy3* | NA | (RAMP) | yl726 |
| *cse1* | • Subtype I-E^{‡‡} | *cse1* | NA | YgcL-like | *ygcL* |
| *cse2* | • Subtype I-E | *cse2* | 2ZCA | YgcK-like | *ygcK* |
| *csc1* | • Subtype I-D | *csc1* | NA | alr1563-like (RAMP) | alr1563 |
| *csc2* | • Subtype I-D | *csc1* and *csc2* | NA | COG1337 (RAMP) | slr7012 |
| *csa5* | • Subtype I-A | *csa5* | NA | AF1870 | AF1870, MJ0380, PF0643 and SSO1398 |
| *csn2* | • Subtype II-A | *csn2* | NA | SPy1049-like | SPy1049 |
| *csm2* | • Subtype III-A^{‡‡} | *csm2* | NA | COG1421 | MTH1081 and SERP2460 |
| *csm3* | • Subtype III-A | *csc2* and *csm3* | NA | COG1337 (RAMP) | MTH1080 and SERP2459 |
| *csm4* | • Subtype III-A | *csm4* | NA | COG1567 (RAMP) | MTH1079 and SERP2458 |
| *csm5* | • Subtype III-A | *csm5* | NA | COG1332 (RAMP) | MTH1078 and SERP2457 |
| *csm6* | • Subtype III-A | APE2256 and *csm6* | 2WTE | COG1517 | APE2256 and SSO1445 |
| *cmr1* | • Subtype III-B | *cmr1* | NA | COG1367 (RAMP) | PF1130 |
| *cmr3* | • Subtype III-B | *cmr3* | NA | COG1769 (RAMP) | PF1128 |
| *cmr4* | • Subtype III-B | *cmr4* | NA | COG1336 (RAMP) | PF1126 |
| *cmr5* | • Subtype III-B^{‡‡} | *cmr5* | 2ZOP and 2OEB | COG3337 | MTH324 and PF1125 |
| *cmr6* | • Subtype III-B | *cmr6* | NA | COG 1604 (RAMP) | PF1124 |
| *csb1* | • Subtype I-U | GSU0053 | NA | (RAMP) | Balac_1306 and GSU0053 |
| *csb2* | • Subtype I-U^{§§} | NA | NA | (RAMP) | Balac_1305 and GSU0054 |
| *csb3* | • Subtype I-U | NA | NA | (RAMP) | Balac_1303^{§§} |
| *csx17* | • Subtype I-U | NA | NA | NA | Btus_2683 |
| *csx14* | • Subtype I-U | NA | NA | NA | GSU0052 |
| *csx10* | • Subtype I-U | *csx10* | NA | (RAMP) | Caur_2274 |
| *csx16* | • Subtype III-U | VVA1548 | NA | NA | VVA1548 |
| *csaX* | • Subtype III-U | *csaX* | NA | NA | SSO1438 |
| *csx3* | • Subtype III-U | *csx3* | NA | NA | AF1864 |
| *csx1* | • Subtype III-U | *csa3, csx1, csx2,* DXTHG, NE0113 and TIGR02710 | 1XMX and 2171 | COG1517 and COG4006 | MJ1666, NE0113, PF1127 and TM1812 |
| *csx15* | • Unknown | NA | NA | TTE2665 | TTE2665 |
| *csf1* | • Type U | *csf1* | NA | NA | AFE_1038 |
| *csf2* | • Type U | *csf2* | NA | (RAMP) | AFE_1039 |
| *csf3* | • Type U | *csf3* | NA | (RAMP) | AFE_1040 |
| *csf4* | • Type U | *csf4* | NA | NA | AFE_1037 |

### IV. Functional Analysis of Candidate Molecules

Candidate Cas9 molecules, candidate gRNA molecules, candidate Cas9 molecule/gRNA molecule complexes, can be evaluated by art-known methods or as described herein. For example, exemplary methods for evaluating the endonuclease activity of Cas9 molecule have been described previously (Jinek 2012).

### Binding and Cleavage Assay: Testing the endonuclease activity of Cas9 molecule

The ability of a Cas9 molecule/gRNA molecule complex to bind to and cleave a target nucleic acid can be evaluated in a plasmid cleavage assay. In this assay, synthetic or *in vitro-*transcribed gRNA molecule is pre-annealed prior to the reaction by heating to 95°C and slowly cooling down to room temperature. Native or restriction digest-linearized plasmid DNA (300 ng (∼8 nM)) is incubated for 60 min at 37°C with purified Cas9 protein molecule (50-500 nM) and gRNA (50-500 nM, 1:1) in a Cas9 plasmid cleavage buffer (20 mM HEPES pH 7.5, 150 mM KCl, 0.5 mM DTT, 0.1 mM EDTA) with or without 10 mM MgCl₂. The reactions are stopped with 5X DNA loading buffer (30% glycerol, 1.2% SDS, 250 mM EDTA), resolved by a 0.8 or 1% agarose gel electrophoresis and visualized by ethidium bromide staining. The resulting cleavage products indicate whether the Cas9 molecule cleaves both DNA strands, or only one of the two strands. For example, linear DNA products indicate the cleavage of both DNA strands. Nicked open circular products indicate that only one of the two strands is cleaved.

Alternatively, the ability of a Cas9 molecule/gRNA molecule complex to bind to and cleave a target nucleic acid can be evaluated in an oligonucleotide DNA cleavage assay. In this assay, DNA oligonucleotides (10 pmol) are radiolabeled by incubating with 5 units T4 polynucleotide kinase and ∼3-6 pmol (∼20-40 mCi) [γ-32P]-ATP in 1X T4 polynucleotide kinase reaction buffer at 37°C for 30 min, in a 50 µL reaction. After heat inactivation (65°C for 20 min), reactions are purified through a column to remove unincorporated label. Duplex substrates (100 nM) are generated by annealing labeled oligonucleotides with equimolar amounts of unlabeled complementary oligonucleotide at 95°C for 3 min, followed by slow cooling to room temperature. For cleavage assays, gRNA molecules are annealed by heating to 95°C for 30 s, followed by slow cooling to room temperature. Cas9 (500 nM final concentration) is pre-incubated with the annealed gRNA molecules (500 nM) in cleavage assay buffer (20 mM HEPES pH 7.5, 100 mM KCl, 5 mM MgC12, 1 mM DTT, 5% glycerol) in a total volume of 9 µL. Reactions are initiated by the addition of 1 µl target DNA (10 nM) and incubated for 1 h at 37°C. Reactions are quenched by the addition of 20 µL of loading dye (5 mM EDTA, 0.025% SDS, 5% glycerol in formamide) and heated to 95°C for 5 min. Cleavage products are resolved on 12% denaturing polyacrylamide gels containing 7 M urea and visualized by phosphorimaging. The resulting cleavage products indicate that whether the complementary strand, the non-complementary strand, or both, are cleaved.

One or both of these assays can be used to evaluate the suitability of a candidate gRNA molecule or candidate Cas9 molecule.

### Binding Assay: Testing the binding of Cas9 molecule to target DNA

Exemplary methods for evaluating the binding of Cas9 molecule to target DNA have been described previously (Jinek 2012).

For example, in an electrophoretic mobility shift assay, target DNA duplexes are formed by mixing of each strand (10 nmol) in deionized water, heating to 95°C for 3 min and slow cooling to room temperature. All DNAs are purified on 8% native gels containing 1X TBE. DNA bands are visualized by UV shadowing, excised, and eluted by soaking gel pieces in DEPC-treated H₂O. Eluted DNA is ethanol precipitated and dissolved in DEPC-treated H₂O. DNA samples are 5' end labeled with [y-32P]-ATP using T4 polynucleotide kinase for 30 min at 37°C. Polynucleotide kinase is heat denatured at 65°C for 20 min, and unincorporated radiolabel is removed using a column. Binding assays are performed in buffer containing 20 mM HEPES pH 7.5, 100 mM KCl, 5 mM MgCl₂, 1 mM DTT and 10% glycerol in a total volume of 10 µL. Cas9 protein molecule is programmed with equimolar amounts of pre-annealed gRNA molecule and titrated from 100 pM to 1 µM. Radiolabeled DNA is added to a final concentration of 20 pM. Samples are incubated for 1 h at 37°C and resolved at 4°C on an 8% native polyacrylamide gel containing 1X TBE and 5 mM MgCl₂. Gels are dried and DNA visualized by phosphorimaging.

### Differential Scanning Flourimetry (DSF)

The thermostability of Cas9 molecule-gRNA ribonucleoprotein (RNP) complexes can be measured via DSF. This technique measures the thermostability of a protein, which can increase under favorable conditions such as the addition of a binding RNA molecule, *e.g.,* a gRNA.

The assay is performed using two different protocols, one to test the best stoichiometric ratio of gRNA:Cas9 protein and another to determine the best solution conditions for RNP formation.

To determine the best solution to form RNP complexes, a 2 µM solution of Cas9 in water + 10x SYPRO Orange® (Life Technologies cat#S-6650) and dispensed into a 384 well plate. An equimolar amount of gRNA diluted in solutions with varied pH and salt is then added. After incubating at room temperature for 10 min. and brief centrifugation to remove any bubbles,a Bio-Rad CFX384™ Real-Time System C1000 Touch™ Thermal Cycler with the Bio-Rad CFX Manager software is used to run a gradient from 20°C to 90°C with a 1°C increase in temperature every 10 seconds.

The second assay consists of mixing various concentrations of gRNA with 2 µM Cas 9 in optimal buffer from the assay above and incubating at RT for 10 min in a 384 well plate. An equal volume of optimal buffer + 10x SYPRO Orange® (Life Technologies cat#S-6650) is added and the plate sealed with Microseal® B adhesive (MSB-1001). Following brief centrifugation to remove any bubbles, a Bio-Rad CFX384™ Real-Time System C1000 Touch™ Thermal Cycler with the Bio-Rad CFX Manager software is used to run a gradient from 20°C to 90°C with a 1°C increase in temperature every 10 seconds.

### Resection Assay: Testing a Cas9 to promote resection

The ability of a Cas9 to promote resection can be evaluated by measuring the levels of single stranded DNA at specific double strand break sites in human cells using quantitative methods (as described in Zhou 2014) . In this assay, a cell line is delivered, *e.g.,* by transfection, a candidate Cas9 or a candidate Cas9 fusion protein. The cells are cultured for a sufficient amount of time to allow nuclease activity and resection to occur. Genomic DNA is carefully extracted using a method in which cells are embedded in low-gelling point agar that protects the DNA from shearing and damage during extraction. The genomic DNA is digested with a restriction enzyme that selectively cuts double-stranded DNA. Primers for quantitative PCR that span up to 5 kb of the double strand break site are designed. The results from the PCR reaction show the levels of single strand DNA detected at each of the primer positions. Thus, the length and the level of resection promoted by the candidate Cas9 or Cas9 fusion protein can be determined from this assay.

Other qualitative assays for identifying the occurrence of resection include the detection of proteins or protein complexes that bind to single-stranded DNA after resection has occurred, *e.g.,* RPA foci, Rad51 foci, or BrDU detection by immunofluorescence. Antibodies for RPA protein and Rad51 are known in the art.

### V. Genome Editing Approaches

Mutations in a target gene may be corrected using one of the approaches discussed herein. A mutation in a target gene may be corrected by homology directed repair (HDR) using an exogenously provided template nucleic acid (see Section V.1), referred to herein as "gene correction". In one embodiment, a mutation in a target gene is corrected by homology directed repair without using an exogenously provided template nucleic acid (see Section V.1), referred to herein as gene correction.

### V.1 HDR Repair and Template Nucleic Acids

Of the methods provided herein, HDR-mediated sequence alteration may be used to alter and/or correct (*e.g.,* repair or edit) the sequence of one or more nucleotides in a genome (*e.g.,* a point mutation at E6 of the *HBB* gene). While not wishing to be bound by theory, it is believed that HDR-mediated alteration of a target sequence within a target gene (*e.g.,* a *HBB* gene) occurs by HDR with an exogenously provided donor template or template nucleic acid in a process referred to herein as gene correction. For example, the donor template or template nucleic acid provides for alteration of the target sequence. It is contemplated that a plasmid donor can be used as a template for homologous recombination. It is further contemplated that a single stranded donor template can be used as a template for alteration of the target sequence by alternate methods bof HDR (*e.g.,* single-strand annealing) between the target sequence and the donor template. Donor template-effected alteration of a target sequence depends on cleavage by a Cas9 molecule. Cleavage by Cas9 can comprise a double-strand break or two single-strand breaks.

In one embodiment, HDR-mediated sequence alteration is used to alter and/or correct (*e.g.,* repair or edit) the sequence of one or more nucleotides in a target sequence in a genome without the use of an exogenously provided donor template or template nucleic acid. While not wishing to be bound by theory, it is believed that alteration of the target sequence occurs by HDR with endogenous genomic donor sequence, in a process referred to herein as gene conversion. For example, the endogenous genomic donor sequence provides for alteration of the target sequence. It is contemplated that the endogenous genomic donor sequence may be located on the same chromosome as the target sequence. It is further contemplated that the endogenous genomic donor sequence may be located on a different chromosome from the target sequence. The endogenous genomic donor sequence may comprise one or more nucleotides derived from the *HBD* gene. The endogenous genomic donor sequence may comprise one or more nucleotides derived from the SMN2 gene. The endogenous genomic donor sequence may comprise one or more nucleotides derived from the p47-PHOX pseudogene. Alteration of a target sequence by endogenous genomic donor sequence depends on cleavage by a Cas9 molecule. Cleavage by Cas9 can comprise a double-strand break or two single-strand breaks.

The target position or target position regions may have at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology with an endogenous homologous sequence.

The target position region, except for the target position, may differ by 1, 2, 3, 4, 5, 10, 25, 50, 100 or fewer, nucleotides with an endogenous homologous sequence.

The target position region may have at least 50%, 60%, 70%, 80%, 90%, 92%, 94%, 96%, 98%, or 99% homology with an endogenous homologous sequence over at least 10, 20, 30, 40, 50, 100, 200, 300, 400, 500, 750, 1,000, 2500, 5000, or 10000 nucleotides.

The target position region, except for the target position, may differ by 1, 2, 3, 4, 5, 10, 25, 50, 100 or fewer, nucleotides with an endogenous homologous sequence over at least 10, 20, 30, 40, 50, 100, 200, 300, 400, 500, 750, 1,000, 2500, 5000, or 10000 nucleotides.

The endogenous homologous sequence may comprise a domain, *e.g.,* a catalytic domain, a domain that binds a target, a structural domain, found in the gene that comprises the target position.

Of the methods provided herein, HDR-mediated alteration may be used to alter a single nucleotide in a target sequence. Either one double-strand break or two single-strand breaks may be utilized. A single nucleotide alteration may be incorporated using (1) one double-strand break, (2) two single-strand breaks, (3) two double-strand breaks with a break occurring on each side of the target position, (4) one double-strand break and two single-strand breaks with the double-strand break and two single-strand breaks occurring on each side of the target position (5) four single-strand breaks with a pair of single stranded breaks occurring on each side of the target position, or (6) one single-strand break.

Wherein a single-stranded template nucleic acid is used, the target position can be altered by alternative HDR.

Donor template-effected alteration of a target position depends on cleavage by a Cas9 molecule. Cleavage by Cas9 can comprise a nick, a double-strand break, or two single-strand breaks, *e.g.,* one on each strand of the target nucleic acid. After introduction of the breaks on the target nucleic acid, resection occurs at the break ends resulting in single stranded overhanging DNA regions.

In canonical HDR, a double-stranded donor template is introduced, comprising homologous sequence to the target nucleic acid that will either be directly incorporated into the target nucleic acid or used as a template to change the sequence of the target nucleic acid. After resection at the break, repair can progress by different pathways, e.g., by the double Holliday junction model (or double-strand break repair, DSBR, pathway) or the synthesis-dependent strand annealing (SDSA) pathway. In the double Holliday junction model, strand invasion by the two single stranded overhangs of the target nucleic acid to the homologous sequences in the donor template occurs, resulting in the formation of an intermediate with two Holliday junctions. The junctions migrate as new DNA is synthesized from the ends of the invading strand to fill the gap resulting from the resection. The end of the newly synthesized DNA is ligated to the resected end, and the junctions are resolved, resulting in the alteration of the target nucleic acid, *e.g.,* incorporation of the altered sequence of the donor template at the corresponding target position. Crossover with the donor template may occur upon resolution of the junctions. In the SDSA pathway, only one single stranded overhang invades the donor template and new DNA is synthesized from the end of the invading strand to fill the gap resulting from resection. The newly synthesized DNA then anneals to the remaining single stranded overhang, new DNA is synthesized to fill in the gap, and the strands are ligated to produce the altered DNA duplex.

In alternative HDR, a single-strand donor template, *e.g.,* template nucleic acid, is introduced. A nick, single-strand break, or double-strand break at the target nucleic acid, for altering a desired target position, is mediated by a Cas9 molecule, *e.g.,* described herein, and resection at the break occurs to reveal single stranded overhangs. Incorporation of the sequence of the template nucleic acid to correct or alter the target position of the target nucleic acid typically occurs by the SDSA pathway, as described above.

Additional details on template nucleic acids are provided in Section IV entitled "Template nucleic acids" in International Application PCT/US2014/057905, now published as WO2015/048577.

Mutations in the *HBB* gene that can be altered (*e.g.,* corrected) by HDR with a template nucleic acid or with endogenous genomic donor sequence include, *e.g.,* point mutation at E6, *e.g.,* E6V.

Double-strand cleavage may be effected by a Cas9 molecule having cleavage activity associated with an HNH-like domain and cleavage activity associated with a RuvC-like domain, *e.g.,* an N-terminal RuvC-like domain, *e.g.,* a wild type Cas9. Such embodiments require only a single gRNA molecule.

One single-strand break, or nick, may be effected by a Cas9 molecule having nickase activity, *e.g.,* a Cas9 nickase as described herein. A nicked target nucleic acid can be a substrate for alt-HDR.

Two single-strand breaks, or nicks, may be effected by a Cas9 molecule having nickase activity, *e.g.,* cleavage activity associated with an HNH-like domain or cleavage activity associated with an N-terminal RuvC-like domain. Such cases usually require two gRNAs, one for placement of each single-strand break. The Cas9 molecule having nickase activity may cleave the strand to which the gRNA hybridizes, but not the strand that is complementary to the strand to which the gRNA hybridizes. The Cas9 molecule having nickase activity may not cleave the strand to which the gRNA hybridizes, but may rather cleave the strand that is complementary to the strand to which the gRNA hybridizes.

The nickase may have HNH activity, *e.g.,* a Cas9 molecule having the RuvC activity inactivated, *e.g.,* a Cas9 molecule having a mutation at D10, *e.g.,* the D10A mutation. D10A inactivates RuvC; therefore, the Cas9 nickase has (only) HNH activity and will cut on the strand to which the gRNA hybridizes (*e.g.,* the complementary strand, which does not have the NGG PAM on it). A Cas9 molecule having an H840, *e.g.,* an H840A, mutation can be used as a nickase. H840A inactivates HNH; therefore, the Cas9 nickase has (only) RuvC activity and cuts on the non-complementary strand (*e.g.,* the strand that has the NGG PAM and whose sequence is identical to the gRNA). A Cas9 molecule having an N863 mutation, *e.g.,* the N863A mutation, mutation can be used as a nickase. N863A inactivates HNH therefore the Cas9 nickase has (only) RuvC activity and cuts on the non-complementary strand (the strand that has the NGG PAM and whose sequence is identical to the gRNA).

Where a nickase and two gRNAs are used to position two single-strand nicks, one nick is on the + strand and one nick is on the - strand of the target nucleic acid. The PAMs can be outwardly facing or inwardly facing. The gRNAs can be selected such that the gRNAs are separated by, from about 0-50, 0-100, or 0-200 nucleotides. There may be no overlap between the target sequences that are complementary to the targeting domains of the two gRNAs. The gRNAs may not overlap and are separated by as much as 50, 100, or 200 nucleotides. The use of two gRNAs can increase specificity, *e.g.,* by decreasing off-target binding (Ran 2013).

A single nick can be used to induce HDR, *e.g.,* alt-HDR. It is contemplated herein that a single nick can be used to increase the ratio of HR to NHEJ at a given cleavage site. A single-strand break may be formed in the strand of the target nucleic acid to which the targeting domain of said gRNA is complementary. A single-strand break may be formed in the strand of the target nucleic acid other than the strand to which the targeting domain of said gRNA is complementary.

### Placement of double-strand or single-strand breaks relative to the target position

A double-strand break or single-strand break in one of the strands should be sufficiently close to target position that an alteration is produced in the desired region, *e.g.,* correction of a mutation occurs. The distance may be not more than 50, 100, 200, 300, 350 or 400 nucleotides. While not wishing to be bound by theory, in certain embodiments, it is believed that the break should be sufficiently close to target position such that the target position is within the region that is subject to exonuclease-mediated removal during end resection. If the distance between the target position and a break is too great, the sequence desired to be altered may not be included in the end resection and, therefore, may not be altered, as donor sequence, either exogenously provided donor sequence or endogenous genomic donor sequence, in some embodiments is only used to alter sequence within the end resection region.

The gRNA targeting domain may be configured such that a cleavage event, *e.g.,* a double-strand or single-strand break, is positioned within 1, 2, 3, 4, 5, 10, 15, 20, 25, 30, 35, 40, 45, 50, 60, 70, 80, 90, 100, 150 or 200 nucleotides of the region desired to be altered, *e.g.,* a mutation. The break, *e.g.,* a double-strand or single-strand break, can be positioned upstream or downstream of the region desired to be altered, *e.g.,* a mutation. A break may be positioned within the region desired to be altered, *e.g.,* within a region defined by at least two mutant nucleotides. A break may be positioned immediately adjacent to the region desired to be altered, *e.g.,* immediately upstream or downstream of a mutation.

A single-strand break may be accompanied by an additional single-strand break, positioned by a second gRNA molecule, as discussed below. For example, the targeting domains bind configured such that a cleavage event, *e.g.,* the two single-strand breaks, are positioned within 1, 2, 3, 4, 5, 10, 15, 20, 25, 30, 35, 40, 45, 50, 60, 70, 80, 90, 100, 150 or 200 nucleotides of a target position. The first and second gRNA molecules may be configured such that when guiding a Cas9 nickase, a single-strand break is accompanied by an additional single-strand break, positioned by a second gRNA, sufficiently close to one another to result in alteration of the desired region. The first and second gRNA molecules may be configured such that a single-strand break positioned by said second gRNA is within 10, 20, 30, 40, or 50 nucleotides of the break positioned by said first gRNA molecule, *e.g.,* when the Cas9 is a nickase. The two gRNA molecules may be configured to position cuts at the same position, or within a few nucleotides of one another, on different strands, *e.g.,* essentially mimicking a double-strand break.

Where a gRNA (unimolecular (or chimeric) or modular gRNA) and Cas9 nuclease induce a double-strand break for the purpose of inducing HDR-mediated alteration, the cleavage site may be between 0-200 bp (*e.g.,* 0-175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, 75 to 100 bp) away from the target position. The cleavage site may be between 0-100 bp (*e.g.,* 0 to 75, 0 to 50, 0 to 25, 25 to 100, 25 to 75, 25 to 50, 50 to 100, 50 to 75 or 75 to 100 bp) away from the target position.

One can promote HDR by using nickases to generate a break with overhangs. While not wishing to be bound by theory, the single stranded nature of the overhangs can enhance the cell's likelihood of repairing the break by HDR as opposed to, *e.g.,* NHEJ. Specifically, HDR may be promoted by selecting a first gRNA that targets a first nickase to a first target sequence, and a second gRNA that targets a second nickase to a second target sequence which is on the opposite DNA strand from the first target sequence and offset from the first nick.

The targeting domain of a gRNA molecule may be configured to position a cleavage event sufficiently far from a preselected nucleotide, *e.g.,* the nucleotide of a coding region, such that the nucleotide is not altered. The targeting domain of a gRNA molecule may be configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

### Placement of a first break and a second break relative to each other

A double-strand break can be accompanied by an additional double-strand break, positioned by a second gRNA molecule, as is discussed below.

A double-strand break can be accompanied by two additional single-strand breaks, positioned by a second gRNA molecule and a third gRNA molecule.

A first and second single-strand breaks can be accompanied by two additional single-strand breaks positioned by a third gRNA molecule and a fourth gRNA molecule.

When two or more gRNAs are used to position two or more cleavage events, *e.g.,* double-strand or single-strand breaks, in a target nucleic acid, it is contemplated that the two or more cleavage events may be made by the same or different Cas9 proteins. For example, when two gRNAs are used to position two double stranded breaks, a single Cas9 nuclease may be used to create both double stranded breaks. When two or more gRNAs are used to position two or more single stranded breaks (nicks), a single Cas9 nickase may be used to create the two or more nicks. When two or more gRNAs are used to position at least one double stranded break and at least one single stranded break, two Cas9 proteins may be used, *e.g.,* one Cas9 nuclease and one Cas9 nickase. It is contemplated that when two or more Cas9 proteins are used that the two or more Cas9 proteins may be delivered sequentially to control specificity of a double stranded versus a single stranded break at the desired position in the target nucleic acid.

The targeting domain of the first gRNA molecule and the targeting domain of the second gRNA molecules may be complementary to opposite strands of the target nucleic acid molecule. The gRNA molecule and the second gRNA molecule may be configured such that the PAMs are oriented outward. The gRNA molecule and the second gRNA molecule may be configured such that the PAMs are oriented inward.

Two gRNA may be selected to direct Cas9-mediated cleavage at two positions that are a preselected distance from each other. The two points of cleavage may be on opposite strands of the target nucleic acid. The two cleavage points may form a blunt ended break, and they may be offset so that the DNA ends comprise one or two overhangs (*e.g.,* one or more 5' overhangs and/or one or more 3' overhangs). Each cleavage event may be a nick. The nicks may be close enough together that they form a break that is recognized by the double stranded break machinery (as opposed to being recognized by, *e.g.,* the SSBr machinery). The nicks may be far enough apart that they create an overhang that is a substrate for HDR, *i.e.,* the placement of the breaks mimics a DNA substrate that has experienced some resection. For instance, in some embodiments the nicks are spaced to create an overhang that is a substrate for processive resection. The two breaks may be spaced within 25-65 nucleotides of each other. The two breaks may be, *e.g.,* about 25, 30, 35, 40, 45, 50, 55, 60 or 65 nucleotides of each other. The two breaks may be, *e.g.,* at least about 25, 30, 35, 40, 45, 50, 55, 60 or 65 nucleotides of each other. The two breaks may be, *e.g.,* at most about 30, 35, 40, 45, 50, 55, 60 or 65 nucleotides of each other. The two breaks may be about 25-30, 30-35, 35-40, 40-45, 45-50, 50-55, 55-60, or 60-65 nucleotides of each other.

The break that mimics a resected break may comprise a 3' overhang (*e.g.,* generated by a DSB and a nick, where the nick leaves a 3' overhang), a 5' overhang (*e.g.,* generated by a DSB and a nick, where the nick leaves a 5' overhang), a 3' and a 5' overhang (*e.g.,* generated by three cuts), two 3' overhangs (*e.g.,* generated by two nicks that are offset from each other), or two 5' overhangs (*e.g.,* generated by two nicks that are offset from each other).

Where two gRNAs (independently, unimolecular (or chimeric) or modular gRNA) complexing with Cas9 nickases induce two single-strand breaks for the purpose of inducing HDR-mediated alteration (*e.g.,* correction), the closer nick may be between 0-200 bp (*e.g.,* 0-175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, or 75 to 100 bp) away from the target position and the two nicks will ideally be within 25-65 bp of each other (*e.g.,* 25 to 50, 25 to 45, 25 to 40, 25 to 35, 25 to 30, 30 to 55, 30 to 50, 30 to 45, 30 to 40, 30 to 35, 35 to 55, 35 to 50, 35 to 45, 35 to 40, 40 to 55, 40 to 50, 40 to 45 bp, 45 to 50 bp, 50 to 55 bp, 55 to 60 bp, or 60 to 65 bp) and no more than 100 bp away from each other (*e.g.,* no more than 90, 80, 70, 60, 50, 40, 30, 20, 10, or 5 bp away from each other). The cleavage site may be between 0-100 bp (*e.g.,* 0 to 75, 0 to 50, 0 to 25, 25 to 100, 25 to 75, 25 to 50, 50 to 100, 50 to 75, or 75 to 100 bp) away from the target position.

Two gRNAs, *e.g.,* independently, unimolecular (or chimeric) or modular gRNA, may be configured to position a double-strand break on both sides of a target position. Three gRNAs, *e.g.,* independently, unimolecular (or chimeric) or modular gRNA, may be configured to position a double-strand break (*i.e.,* one gRNA complexes with a cas9 nuclease) and two single-strand breaks or paired single stranded breaks (*i.e*., two gRNAs complex with Cas9 nickases) on either side of the target position. Four gRNAs, *e.g.,* independently, unimolecular (or chimeric) or modular gRNA, may be configured to generate two pairs of single stranded breaks (*i.e.,* two pairs of two gRNA molecules complex with Cas9 nickases) on either side of the target position. The double-strand break(s) or the closer of the two single-strand nicks in a pair will ideally be within 0-500 bp of the target position (*e.g.,* no more than 450, 400, 350, 300, 250, 200, 150, 100, 50 or 25 bp from the target position). When nickases are used, the two nicks in a pair may be within 25-65 bp of each other (*e.g.,* between 25 to 55, 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50, 45 to 50, 35 to 45, 40 to 45 bp, 45 to 50 bp, 50 to 55 bp, 55 to 60 bp, or 60 to 65 bp) and no more than 100 bp away from each other (*e.g.,* no more than 90, 80, 70, 60, 50, 40, 30, 20, or 10 bp).

When two gRNAs are used to target Cas9 molecules to breaks, different combinations of Cas9 molecules are envisioned. A first gRNA may be used to target a first Cas9 molecule to a first target position, and a second gRNA may be used to target a second Cas9 molecule to a second target position. The first Cas9 molecule may create a nick on the first strand of the target nucleic acid, and the second Cas9 molecule may create a nick on the opposite strand, resulting in a double stranded break (*e.g.,* a blunt ended cut or a cut with overhangs).

Different combinations of nickases can be chosen to target one single stranded break to one strand and a second single stranded break to the opposite strand. When choosing a combination, one can take into account that there are nickases having one active RuvC-like domain, and nickases having one active HNH domain. A RuvC-like domain may cleave the non-complementary strand of the target nucleic acid molecule. An HNH-like domain may cleave a single stranded complementary domain, *e.g.,* a complementary strand of a double stranded nucleic acid molecule. Generally, if both Cas9 molecules have the same active domain (*e.g.,* both have an active RuvC domain or both have an active HNH domain), one will choose two gRNAs that bind to opposite strands of the target. In more detail, a first gRNA may be complementary with a first strand of the target nucleic acid and bind a nickase having an active RuvC-like domain and cause that nickase to cleave the strand that is non-complementary to that first gRNA, *i.e.,* a second strand of the target nucleic acid; and a second gRNA may be complementary with a second strand of the target nucleic acid and bind a nickase having an active RuvC-like domain and cause that nickase to cleave the strand that is non-complementary to that second gRNA, *i.e.,* the first strand of the target nucleic acid. Conversely, a first gRNA may be complementary with a first strand of the target nucleic acid and bind a nickase having an active HNH domain and cause that nickase to cleave the strand that is complementary to that first gRNA, *i.e.,* a first strand of the target nucleic acid; and a second gRNA may be complementary with a second strand of the target nucleic acid and bind a nickase having an active HNH domain and cause that nickase to cleave the strand that is complementary to that second gRNA, *i.e*., the second strand of the target nucleic acid. In another arrangement, if one Cas9 molecule has an active RuvC-like domain and the other Cas9 molecule has an active HNH domain, the gRNAs for both Cas9 molecules can be complementary to the same strand of the target nucleic acid, so that the Cas9 molecule with the active RuvC-like domain will cleave the non-complementary strand and the Cas9 molecule with the HNH domain will cleave the complementary strand, resulting in a double stranded break.

The cleavage event may comprise one or more breaks, *e.g.,* one or more single-strand breaks, one or more double-strand breaks, or a combination thereof.

The cleavage event may comprise any one of the following: (a) one single-strand break; (b) two single-strand breaks; (c) three single-strand breaks; (d) four single-strand breaks; (e) one double-strand break; (f) two double-strand breaks; (g) one single-strand break and one double-strand break; (h) two single-strand breaks and one double-strand break; or (i) any combination thereof.

The gRNA molecule and the second gRNA molecule may position a cleavage event on each strand of a target nucleic acid.

The cleavage event may flank the target position, and wherein the terminus (created by the cleavage event) closest to the target position, for each cleavage event, may be a 5' terminus, *e*.*g*., resulting in a 5' overhang.

While not wishing to be bound by theory, it believed that the sequence exposed by a cleavage event (*e.g*., a single-strand cleavage event) mediated by a gRNA molecule and a Cas9 molecule (*e.g*., a Cas9 nickase, *e.g*., a Cas9 molecule containing D10A or N863A mutation) may affect (*e*.*g*., increase or decrease) gene conversion efficiency. For example, the sequence exposed by the cleavage event can include a 5' overhang, a 3' overhang, a product of the nucleolytic processing of a 5' overhang, a product of the nucleolytic processing of a 3' overhang, or any combination thereof. The exposed sequence may comprise or consist of a 5' overhang, a 3' overhang, a product of the nucleolytic processing of a 5' overhang, or a product of the nucleolytic processing of a 3' overhang.

The 5' overhang may be between 1 and 20000 nucleotides, 5 and 20000 nucleotides, 10 and 20000 nucleotides, 20 and 20000 nucleotides, 30 and 20000 nucleotides, between 35 and 20000 nucleotides, between 40 and 20000 nucleotides, between 50 and 20000 nucleotides, between 1000 and 10000 nucleotides, or between 500 and 5000 nucleotides in length, *e*.*g*., between 1 and 100 nucleotides, between 1 and 50 nucleotides, between 1 and 25 nucleotides, between 40 and 60 nucleotides, between 40 and 55 nucleotides, or between 45 and 50 nucleotides in length, *e.g.,* at least about 1, 5, 10, 20, 30, 35, 40, 45, 50, 75, 100, 200, 300, 400, 500, 750, 1000, 2000, 3000, 4000, 5000, 6000, 7000, 8000, 9000, 10000, or 15000 nucleotides in length. The sequence exposed by the Cas9 molecule/gRNA molecule mediated cleavage event can constitute a substrate used for homology search in gene conversion.

By way of example, when a pair of gRNA molecules targets a Cas9 nickase to mediate a cleavage event at a *HBB* locus, the sequence exposed by said cleavage event can probe any genomic locus for possible homology. While not to be bound by theory, it is believed that higher sequence identity of the exposed *HBB* region with a genomic region can promote gene conversion more effectively. For example, the *HBD* gene has approximately 92% sequence homology with the *HBB* gene, therefore the *HBD* gene may be used as a donor template for effective gene conversion. If the exposed sequence contains a mutation with respect to the *HBD* sequence, the mutation may decrease gene conversion efficiency. Therefore, different gRNA molecules may give rise to exposed sequences having different homologies to a donor template.

The exposed sequence may differ by 1, 2, 3, 4, 5, 10, 25, 50, 100 or fewer, nucleotides with an endogenous homologous sequence. The exposed sequence may have at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology with an endogenous homologous sequence over at least 10, 20, 30, 40, 50, 100, 200, 300, 400, 500, 750, 1000, 2500, 5000, or 10000 nucleotides. The exposed sequence may differ by 1, 2, 3, 4, 5, 10, 25, 50, 100 or fewer, nucleotides with an endogenous homologous sequence over at least 10, 20, 30, 40, 50, 100, 200, 300, 400, 500, 750, 1000, 2500, 5000, or 10000 nucleotides.

The cleavage event may flank the target position, and the terminus (created by a cleavage event) closest to the target position, for each cleavage event, may be a 3' terminus, *e*.*g*., resulting a 3' overhang.

The 3' overhang may be between 1 and 20000 nucleotides, 5 and 20000 nucleotides, 10 and 20000 nucleotides, 20 and 20000 nucleotides, between 30 and 20000 nucleotides, between 35 and 20000 nucleotides, between 40 and 20000 nucleotides, between 50 and 20000 nucleotides, between 1000 and 10000 nucleotides, or between 500 and 5000 nucleotides in length, *e*.*g*., between 1 and 100 nucleotides, between 1 and 50 nucleotides, between 1 and 25 nucleotides, between 40 and 60 nucleotides, between 40 and 55 nucleotides, or between 45 and 50 nucleotides in length, *e.g.,* at least about 30, 35, 40, 45, 50, 75, 100, 200, 300, 400, 500, 750, 1000, 2000, 3000, 4000, 5000, 6000, 7000, 8000, 9000, 10000, or 15000 nucleotides in length.

The distance between the cleavage event and the target position may be between 10 and 10000 nucleotides in length, *e*.*g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

The cleavage event may comprise a single-strand break, and wherein the distance between the single-strand break and the target position is between 10 and 10000 nucleotides in length, *e*.*g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

The cleavage event may comprise two, three or four single-strand breaks, and wherein the distance between each of the single-strand breaks and the target position may be between 10 and 10000 nucleotides in length, *e*.*g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

The cleavage event may comprise a double-strand break, and wherein the distance between the double-strand break and the target position may be between 10 and 10000 nucleotides in length, *e*.*g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

The cleavage event may comprise two double-strand breaks, and wherein the distance between each of the double-strand breaks and the target position may be between 10 and 10000 nucleotides in length, *e*.*g*., between 50 and 5000 nucleotides, between 100 and 1000 nucleotides, between 200 and 800 nucleotides, between 400 and 600 nucleotides, between 100 and 500 nucleotides, or between 500 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

The cleavage event may comprise a single-strand break and a double-strand break, wherein the distance between the single-strand break and the target position may be between 10 and 10000 nucleotides in length, *e*.*g*., between 50 and 5000 nucleotides or between 100 and 1000 nucleotides in length, *e*.*g*., about 20, 30, 40, 50, 75, 100, 200, 300, 400, 500, 750, 1000, 2000, 3000, 4000, 5000, 6000, 7000, 8000, 9000, or 10000 nucleotides in length, and
wherein the distance between the double-strand break and the target position may be between 10 and 10000 nucleotides in length, *e*.*g*., between 50 and 5000 nucleotides or between 100 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

The cleavage event may comprise two single-strand breaks and
a double-strand break,
wherein the distance between each of the single-strand breaks and the target position may be between 10 and 10000 nucleotides in length, *e*.*g*., between 50 and 5000 nucleotides or between 100 and 1000 nucleotides in length, *e*.*g*., about 20, 30, 40, 50, 75, 100, 200, 300, 400, 500, 750, 1000, 2000, 3000, 4000, 5000, 6000, 7000, 8000, 9000, or 10000 nucleotides in length, and
wherein the distance between the double-strand break and the target position may be between 10 and 10000 nucleotides in length, *e*.*g*., between 50 and 5000 nucleotides or between 100 and 1000 nucleotides in length, *e.g.,* at least about 20, 30, 40, 50, 75, 100, 200, 300, 400, 500, 750, 1000, 2000, 3000, 4000, 5000, 6000, 7000, 8000, 9000, or 10000 nucleotides in length.

The cleavage event may comprise two or more single-strand breaks, two or more double-strand breaks, or two single-strand breaks and one double-strand breaks,
wherein the distance between any of the two breaks that are present on the same strand may be between 30 and 20000 nucleotides, 40 and 20000 nucleotides, or 50 and 20000 nucleotides in length, *e*.*g*., between 1000 and 10000 nucleotides or between 500 and 5000 nucleotides in length, *e*.*g*., between 40 and 60 nucleotides, between 40 and 55 nucleotides, or between 45 and 50 nucleotides in length, *e.g.,* at least about 30, 35, 40, 45, 50, 75, 100, 200, 300, 400, 500, 750, 1000, 2000, 3000, 4000, 5000, 6000, 7000, 8000, 9000, 10000, or 15000 nucleotides in length.

The cleavage event may comprise two or more single-strand breaks, two or more double-strand breaks, or two single-strand breaks and one double-strand breaks,
wherein the distance between at least two breaks that are present on different strands may be between 30 and 20000 nucleotides, 40 and 20000 nucleotides, or 50 and 20000 nucleotides in length, *e*.*g*., between 1000 and 10000 nucleotides or between 500 and 5000 nucleotides in length, *e*.*g*., between 40 and 60 nucleotides, between 40 and 55 nucleotides, or between 45 and 50 nucleotides in length, *e.g.,* at least about 30, 35, 40, 45, 50, 75, 100, 200, 300, 400, 500, 750, 1000, 2000, 3000, 4000, 5000, 6000, 7000, 8000, 9000, 10000, or 15000 nucleotides in length.

The cleavage event may comprise two single-strand breaks, wherein the distance between the two single breaks may be between 30 and 20000 nucleotides, 40 and 20000 nucleotides, or 50 and 20000 nucleotides in length, *e*.*g*., between 1000 and 10000 nucleotides or between 500 and 5000 nucleotides in length, *e*.*g*., between 40 and 60 nucleotides, between 40 and 55 nucleotides, or between 45 and 50 nucleotides in length, *e.g.,* at least about 30, 35, 40, 45, 50, 75, 100, 200, 300, 400, 500, 750, 1000, 2000, 3000, 4000, 5000, 6000, 7000, 8000, 9000, 10000, or 15000 nucleotides in length. The single-strand breaks may be present on different strands. The single-strand breaks may be present on the same strand. The cleavage event may further comprise one or more (*e*.*g*., two) of single-strand break, double-strand break, or both.

The Cas9 molecule may comprise HNH-like domain cleavage activity but have no, or no significant, N-terminal RuvC-like domain cleavage activity. The eaCas9 molecule may be an HNH-like domain nickase, *e.g.,* the Cas9 molecule may comprise a mutation at D10, *e.g.,* D10A. The eaCas9 molecule may comprise N-terminal RuvC-like domain cleavage activity but have no, or no significant, HNH-like domain cleavage activity. The Cas9 molecule may be an N-terminal RuvC-like domain nickase, *e*.*g*., the eaCas9 molecule comprises a mutation at N863, *e.g.,* N863A.

The first gRNA molecule may position a cleavage event on a strand that does not bind to the first gRNA molecule.

The second gRNA molecule may position a cleavage event on a strand that does not bind to the second gRNA molecule.

The first gRNA molecule may position a cleavage event on a strand that does not bind to the first gRNA and the second gRNA molecule may position a cleavage event on a strand that does not bind to the second gRNA molecule, and wherein the gRNA molecule and the second gRNA molecule bind to different strands, *e*.*g*., resulting in a 3' overhang on each strand.

The first gRNA molecule may position a cleavage event 5' to the target position on the first strand. The first gRNA molecule may position a cleavage event 5' to the target position on the first strand, as shown in the diagram below:
5'-X-M- 3'
3'-5',
wherein X is the cleavage event and M is the target position.

The second gRNA molecule may position a cleavage event 3' to the target position (relative to the target position on the first strand) on the second strand. The second gRNA molecule may position a cleavage event 3' to the target position (relative to the target position on the first strand) on the second strand, as shown in the diagram below:
5'-M-3'
3'-X-5',
wherein X is the cleavage event and M is the target position.

The second gRNA molecule may position a cleavage event 5' to the target position on the second strand. The second gRNA molecule may position a cleavage event 5' to the target position on the second strand, as shown in the diagram below:
5'-M-3'
3'-M-X-5',
wherein X is the cleavage event and M is the target position.

The first gRNA molecule may position a cleavage event 5' to the target position on the first strand, and wherein the second gRNA molecule may position a cleavage event 3' to the target position (relative to the target position on the first strand) on the second strand. The first gRNA molecule may position a cleavage event 5' to the target position on the first strand, and wherein the second gRNA molecule may position a cleavage event 3' to the target position (relative to the target position on the first strand) on the second strand, as shown in the diagram below:
5'-X-M-3'
3'-X-5',
wherein X is the cleavage event and M is the target position.

The first gRNA molecule may position a cleavage event 3' to the target position on the first strand. The first gRNA molecule may position a cleavage event 3' to the target position on the first strand, as shown in the diagram below:
5'-M-X-3'
3'-5',
wherein X is the cleavage event and M is the target position.

The second gRNA molecule may position a cleavage event 5' to the target position (relative to the target position on the first strand) on the second strand. The second gRNA molecule may position a cleavage event 5' to the target position (relative to the target position on the first strand) on the second strand, as shown in the diagram below:
5'-M-3'
3'-X-5',
wherein X is the cleavage event and M is the target position.

The first gRNA molecule may position a cleavage event 3' to the target position on the first strand, and wherein the second gRNA molecule positions a cleavage event 5' to the target position (relative to the target position on the first strand) on the second strand. The first gRNA molecule may position a cleavage event 3' to the target position on the first strand, and wherein the second gRNA molecule may position a cleavage event 5' to the target position (relative to the target position on the first strand) on the second strand, as shown in the diagram below:
5'-M-X-3'
3'-X-5',
wherein X is the cleavage event and M is the target position.

The first gRNA molecule may position a cleavage event 5' to the target position on the first strand, and wherein the second gRNA molecule may position a cleavage event 5' to the target position (relative to the target position on the first strand) on the second strand, *e.g.,* to produce a 5' overhang. The first gRNA molecule may position a cleavage event 5' to the target position on the first strand, and wherein the second gRNA molecule may position a cleavage event 5' to the target position (relative to the target position on the first strand) on the second strand, *e.g.,* to produce a 5' overhang, as shown in the diagram below:
5'-X-M-3'
3'-X-5',
wherein X is the cleavage event and M is the target position.

The first gRNA molecule may position a cleavage event 3' to the target position on the first strand, and wherein the second gRNA molecule may position a cleavage event 3' to the target position (relative to the target position on the first strand) on the second strand, *e.g.,* to produce a 5' overhang. The first gRNA molecule may position a cleavage event 3' to the target position on the first strand, and wherein the second gRNA molecule may position a cleavage event 3' to the target position (relative to the target position on the first strand) on the second strand, *e.g.,* to produce a 5' overhang, as shown in the diagram below:
5'-M-X-3'
3'-X-5',
wherein X is the cleavage event and M is the target position.

The first gRNA molecule may position a cleavage event 5' to the target position on the first strand, and wherein the second gRNA molecule may position a cleavage event 5' to the target position (relative to the target position on the first strand) on the second strand, *e.g.,* to produce a 3' overhang. The first gRNA molecule may position a cleavage event 5' to the target position on the first strand, and wherein the second gRNA molecule may position a cleavage event 5' to the target position (relative to the target position on the first strand) on the second strand, *e.g.,* to produce a 3' overhang, as shown in the diagram below:
5'-X-M-3'
3'-X-5',
wherein X is the cleavage event and M is the target position.

The first gRNA molecule may position a cleavage event 3' to the target position on the first strand, and wherein the second gRNA molecule may position a cleavage event 3' to the target position (relative to the target position on the first strand) on the second strand, *e.g.,* to produce a 3' overhang. The first gRNA molecule may position a cleavage event 3' to the target position on the first strand, and wherein the second gRNA molecule may position a cleavage event 3' to the target position (relative to the target position on the first strand) on the second strand, *e.g.,* to produce a 3' overhang, as shown in the diagram below:
5'-M-X-3'
3'-X-5',
wherein X is the cleavage event and M is the target position.

The target position may comprise a mutation. The mutation may be associated with a disease phenotype.

The first gRNA molecule may position a cleavage event on a strand that binds to the gRNA molecule.

The second gRNA molecule may position a cleavage event on a strand that binds to the second gRNA molecule.

The first gRNA molecule may position a cleavage event on a strand that binds to the gRNA and the second gRNA molecule may position a cleavage event on a strand that binds to the second gRNA molecule, and wherein the first gRNA molecule and the second gRNA molecule may bind to different strands, *e*.*g*., resulting in a 5' overhang on each strand.

The gRNA molecule, together with the Cas9 molecule *(e.g.,* a nickase), may position a cleavage event on a strand *(e.g.,* a first strand or a second strand).

The gRNA molecule may position a cleavage event 5' to the target position on the first strand. This allows the use of a single Cas9 molecule, *e.g.,* a single Cas9 molecule that is a nickase *(e.g.,* a Cas9 molecule with a D10A mutation), *e.g.,* to place a single-strand cleavage event sufficiently close to the target position (*e*.*g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this can be illustrated as shown in the diagram below: 
—gRNA—
5' ———X——————M———————————— 3'
 3'-5',
wherein X is the cleavage event, M is the target position.

The gRNA molecule may position a cleavage event 3' to the target position (relative to the target position on the first strand) on the second strand. This allows the use of a single Cas9 molecule, *e.g.,* a single Cas9 molecule that is a nickase *(e.g.,* a Cas9 molecule with a D10A mutation), *e*.*g*., to place a single-strand cleavage event sufficiently close to the target position (*e*.*g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this can be illustrated as shown in the diagram below:
5'-M-3' 
3' ——————————————————X——— 5',
—gRNA—
 wherein X is the cleavage event, M is the target position.

The gRNA molecule may position a cleavage event 3' to the target position on the first strand. This allows the use of a single Cas9 molecule, *e.g.,* a single Cas9 molecule that is a nickase *(e.g.,* a Cas9 molecule with a D10A mutation), *e.g.,* to place a single-strand cleavage event sufficiently close to the target position (*e*.*g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this can be illustrated as shown in the diagram below: 
—gRNA—
5' ——————————————M——————————X—————— 3'
 3'-5',
wherein X is the cleavage event, M is the target position.

The gRNA molecule may position a cleavage event 5' to the target position (relative to the target position on the first strand) on the second strand. This allows the use of a single Cas9 molecule, *e.g.,* a single Cas9 molecule that is a nickase *(e.g.,* a Cas9 molecule with a D10A mutation), *e.g.,* to place a single-strand cleavage event sufficiently close to the target position (*e*.*g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this can be illustrated as shown in the diagram below:
5'-M-3' 
3' ————X———————————————————— 5',
—gRNA—
 wherein X is the cleavage event, M is the target position.

The gRNA molecule may position a cleavage event 5' to the target position on the first strand. This allows the use of a single Cas9 molecule, *e.g.,* a single Cas9 molecule that is a nickase *(e.g.,* a Cas9 molecule with an N863A mutation), *e.g.,* to place a single-strand cleavage event sufficiently close to the target position (*e*.*g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this can be illustrated as shown in the diagram below:
5'-X-M-3' 
3’ ——————————————————————————— 5’,
—gRNA—
 wherein X is the cleavage event, M is the target position.

The gRNA molecule may position a cleavage event 3' to the target position (relative to the target position on the first strand) on the second strand. This allows the use of a single Cas9 molecule, *e.g.,* a single Cas9 molecule that is a nickase *(e.g.,* a Cas9 molecule with an N863A mutation), *e.g.,* to place a single-strand cleavage event sufficiently close to the target position (*e*.*g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this can be illustrated as shown in the diagram below: 
—gRNA—
5' ——————————M—————————— 3'
 3'-X-5',
wherein X is the cleavage event, M is the target position.

The gRNA molecule may position a cleavage event 3' to the target position on the first strand. This allows the use of a single Cas9 molecule, *e.g.,* a single Cas9 molecule that is a nickase *(e.g.,* a Cas9 molecule with an N863A mutation), *e.g.,* to place a single-strand cleavage event sufficiently close to the target position (*e*.*g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this can be illustrated as shown in the diagram below:
5'-M-X-3' 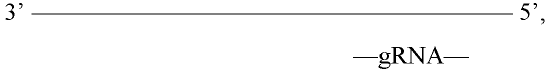
3' 5',
—gRNA—
 wherein X is the cleavage event, M is the target position.

The gRNA molecule may position a cleavage event 5' to the target position (relative to the target position on the first strand) on the second strand. This allows the use of a single Cas9 molecule, *e.g.,* a single Cas9 molecule that is a nickase *(e.g.,* a Cas9 molecule with an N863A mutation), *e*.*g*., to place a single-strand cleavage event sufficiently close to the target position (*e*.*g*., within 10000, 9000, 8000, 7000, 6000, 5000, 4000, 3000, 2000, 1000, 800, 600, 500, 400, 300, 200, 100, 75, 50, 40, 30, 20, 10, 5, or 1 bp to the target position). For example, this can be illustrated as shown in the diagram below: 
—gRNA—
5' ——————————————M—————————————— 3'
 3'-X-5',
wherein X is the cleavage event, M is the target position.

The gRNA molecule, together with the Cas9 molecule *(e.g.,* a nickase), may positions a cleavage event on the strand that binds to the gRNA molecule; and the second gRNA molecule, together with the Cas9 molecule, positions a cleavage event on the strand that binds to the second gRNA molecule, wherein the gRNA molecule and the second gRNA molecule may bind to different strands, the gRNA molecule may position a cleavage event 5' to the target position on the first strand, and the second gRNA molecule may position a cleavage event 3' to the target position (relative to the target position on the first strand) on the second strand. This example allows the use of a single Cas9 molecule, *e.g.,* a single Cas9 molecule that is a nickase *(e.g.,* a Cas9 molecule with a D10A mutation), *e.g.,* to place single-strand cleavage events on each side of the target position.

This example can be illustrated as shown in the diagram below: 
—gRNA—
5' ———X——————M——————————— 3'
3' ——————————————————X——— 5'
—gRNA—
 wherein X is the cleavage event, M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
the gRNA molecule and the second gRNA molecule bind to different strands,
the gRNA molecule positions a cleavage event 5' to the target position on the first strand, and
the second gRNA molecule positions a cleavage event 3' to the target position (relative to the target position on the first strand) on the second strand. This example allows the use of a single Cas9 molecule, *e.g.,* a single Cas9 molecule that is a nickase *(e.g.,* a Cas9 molecule with an N863A mutation), *e*.*g*., to place single-strand cleavage events on each side of the target position.

This example can be illustrated as shown in the diagram below: 
—gRNA—
5' ———X——————————M———————————— 3'
3' ——————————————————————X——— 5'
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule (a nickase), positions a cleavage event on the strand that binds to the gRNA molecule; and
the second gRNA molecule, together with the Cas9 molecule, positions a cleavage event on the strand that binds to the second gRNA molecule,
wherein:
the gRNA molecule and the second gRNA molecule bind to different strands,
the gRNA molecule positions a cleavage event 3' to the target position on the first strand, and
the second gRNA molecule positions a cleavage event 5' to the target position (relative to the target position on the first strand) on the second strand. This allows the use of a single Cas9 molecule, *e.g.,* a single Cas9 molecule that is a nickase *(e.g.,* a Cas9 molecule with a D10A mutation), *e*.*g*., to place single-strand cleavage events on each side of the target position.

This example can be illustrated as shown in the diagram below: 
—gRNA—
5' ——————————————M——————————X———— 3'
3' ———X——————————————————————————— 5'
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
the gRNA molecule and the second gRNA molecule bind to different strands,
the gRNA molecule positions a cleavage event 3' to the target position on the first strand, and
the second gRNA molecule positions a cleavage event 5' to the target position (relative to the target position on the first strand) on the second strand. This allows the use of a single Cas9 molecule, *e.g.,* a single Cas9 molecule that is a nickase *(e.g.,* a Cas9 molecule with a N863A mutation), *e*.*g*., to place single-strand cleavage events on each side of the target position. This example can be illustrated as shown in the diagram below: 
—gRNA—
5’ ——————————————M——————————X———— 3’
3’ ———X——————————————————————————— 5’
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule *(e.g.,* a nickase), positions a cleavage event on the strand that binds to the gRNA molecule; and
the second gRNA molecule, together with the Cas9 molecule, positions a cleavage event on the strand that binds to the second gRNA molecule,
wherein:
the gRNA molecule and the second gRNA molecule bind to different strands,
the gRNA molecule positions a cleavage event 5' to the target position on the first strand, and
the second gRNA molecule positions a cleavage event 5' to the target position (relative to the target position on the first strand) on the second strand, *e.g.,* to produce a 5' overhang. This example allows the use of a single Cas9 molecule, *e.g.,* a single Cas9 molecule that is a nickase *(e.g.,* a Cas9 molecule with a D10A mutation), *e.g.,* to place single-strand cleavage events on one side of the target position, *e.g.,* to produce a 5' overhang.

This example can be illustrated as shown in the diagram below: 
—gRNA—
5' ———X——————M—————— 3'
 
3’ ——————X—————————————— 5’,
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
the gRNA molecule and the second gRNA molecule bind to different strands,
the gRNA molecule positions a cleavage event 5' to the target position on the first strand, and
the second gRNA molecule positions a cleavage event 5' to the target position (relative to the target position on the first strand) on the second strand, *e.g.,* to produce a 5' overhang. This example allows the use of a single Cas9 molecule, *e.g.,* a single Cas9 molecule that is a nickase *(e.g.,* a Cas9 molecule with an N863A mutation), *e.g.,* to place single-strand cleavage events on each side of the target position, *e.g.,* to produce a 5' overhang.

This example can be illustrated as shown in the diagram below: 
—gRNA—
5' ——X——————M—————————— 3'
3' ——————X———————————————— 5',
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule *(e.g.,* a nickase), positions a cleavage event on the strand that binds to the gRNA molecule; and
the second gRNA molecule, together with the Cas9 molecule, positions a cleavage event on the strand that binds to the second gRNA molecule,
wherein:
the gRNA molecule and the second gRNA molecule bind to different strands,
the gRNA molecule positions a cleavage event 3' to the target position on the first strand, and
the second gRNA molecule positions a cleavage event 3' to the target position (relative to the target position on the first strand) on the second strand, *e.g.,* to produce a 5' overhang. This example allows the use of a single Cas9 molecule, *e.g.,* a single Cas9 molecule that is a nickase *(e.g.,* a Cas9 molecule with a D10A mutation), *e.g.,* to place single-strand cleavage events on one side of the target position, *e.g.,* to produce a 5' overhang.

This example can be illustrated as shown in the diagram below: 
—gRNA—
5’ ——————————————M————X—————————— 3’
3’ ——————————————————————————X———— 5’,
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
the gRNA molecule and the second gRNA molecule bind to different strands,
the gRNA molecule positions a cleavage event 3' to the target position on the first strand, and
the second gRNA molecule positions a cleavage event 3' to the target position (relative to the target position on the first strand) on the second strand, *e.g.,* to produce a 5' overhang. This example allows the use of a single Cas9 molecule, *e.g.,* a single Cas9 molecule that is a nickase *(e.g.,* a Cas9 molecule with an N863A mutation), *e.g.,* to place single-strand cleavage events on each side of the target position, *e.g.,* to produce a 5' overhang.

This example can be illustrated as shown in the diagram below: 
—gRNA—
5' ——————————M———X——————— 3'
3' ————————————————————X——— 5',
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule *(e.g.,* a nickase), positions a cleavage event on the strand that binds to the gRNA molecule; and
the second gRNA molecule, together with the Cas9 molecule, positions a cleavage event on the strand that binds to the second gRNA molecule,
wherein:
the gRNA molecule and the second gRNA molecule bind to different strands,
the gRNA molecule positions a cleavage event 5' to the target position on the first strand, and
the second gRNA molecule positions a cleavage event 5' to the target position (relative to the target position on the first strand) on the second strand, *e.g.,* to produce a 3' overhang. This example allows the use of a single Cas9 molecule, *e.g.,* a single Cas9 molecule that is a nickase *(e.g.,* a Cas9 molecule with a D10A mutation), *e.g.,* to place single-strand cleavage events on one side of the target position, *e.g.,* to produce a 3' overhang.

This example can be illustrated as shown in the diagram below: 
—gRNA—
5' ——————X———M—————————— 3'
3' ———X———————————————— 5',
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
the gRNA molecule and the second gRNA molecule bind to different strands,
the gRNA molecule positions a cleavage event 5' to the target position on the first strand, and
the second gRNA molecule positions a cleavage event 5' to the target position (relative to the target position on the first strand) on the second strand, *e.g.,* to produce a 3' overhang. This example allows the use of a single Cas9 molecule, *e.g.,* a single Cas9 molecule that is a nickase *(e.g.,* a Cas9 molecule with an N863A mutation), *e.g.,* to place single-strand cleavage events on each side of the target position, *e.g.,* to produce a 3' overhang.

This example can be illustrated as shown in the diagram below: 
—gRNA—
5’ ——————X———M——————————— 3’
3’ ———X——————————————————— 5’,
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule *(e.g.,* a nickase), positions a cleavage event on the strand that binds to the gRNA molecule; and
the second gRNA molecule, together with the Cas9 molecule, positions a cleavage event on the strand that binds to the second gRNA molecule,
wherein:
the gRNA molecule and the second gRNA molecule bind to different strands,
the gRNA molecule positions a cleavage event 3' to the target position on the first strand, and
the second gRNA molecule positions a cleavage event 3' to the target position (relative to the target position on the first strand) on the second strand, *e.g.,* to produce a 3' overhang. This example allows the use of a single Cas9 molecule, *e.g.,* a single Cas9 molecule that is a nickase *(e.g.,* a Cas9 molecule with a D10A mutation), *e.g.,* to place single-strand cleavage events on one side of the target position, *e.g.,* to produce a 3' overhang.

This example can be illustrated as shown in the diagram below: 
—gRNA—
5’ ——————————M——————X———— 3’
3’ ————————————————X——————— 5’,
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

In an example:
the gRNA molecule, together with the Cas9 molecule (a nickase), positions a cleavage event on the strand other than the strand that binds to the gRNA molecule; and
the second gRNA molecule, together with the Cas9 molecule, positions a cleavage event on the strand other than the strand that binds to the second gRNA molecule,
wherein:
the gRNA molecule and the second gRNA molecule bind to different strands,
the gRNA molecule positions a cleavage event 3' to the target position on the first strand, and
the second gRNA molecule positions a cleavage event 3' to the target position (relative to the target position on the first strand) on the second strand, *e.g.,* to produce a 3' overhang. This example allows the use of a single Cas9 molecule, *e.g.,* a single Cas9 molecule that is a nickase *(e.g.,* a Cas9 molecule with an N863A mutation), *e.g.,* to place single-strand cleavage events on each side of the target position, *e.g.,* to produce a 3' overhang.

This example can be illustrated as shown in the diagram below: 
—gRNA—
5' ——————————M——————X———— 3'
3' ——————————————X——————— 5',
—gRNA—
 wherein X is the cleavage event and M is the target position.

The cleavage event positioned by the gRNA molecule and the cleavage event positioned by the second gRNA molecule may be separated by 10 to 10000, 10 to 5000, 10 to 2500, 10 to 1000, 10 to 750, 10 to 500, 10 to 400, 10 to 300, 10 to 200, 10 to 100, 10 to 75, 10 to 50, or 10 to 25 base pairs.

### Homology arms of the donor template

A homology arm should extend at least as far as the region in which end resection may occur, *e.g.,* in order to allow the resected single stranded overhang to find a complementary region within the donor template. The overall length could be limited by parameters such as plasmid size or viral packaging limits. In an example, a homology arm does not extend into repeated elements, *e*.*g*., Alu repeats or LINE repeats.

Exemplary homology arm lengths include at least 50, 100, 250, 500, 750, 1000, 2000, 3000, 4000, or 5000 nucleotides. The homology arm length may be 50-100, 100-250, 250-500, 500-750, 750-1000, 1000-2000, 2000-3000, 3000-4000, or 4000-5000 nucleotides.

Target position, as used herein, refers to a site on a target nucleic acid *(e.g.,* the chromosome) that is modified by a Cas9 molecule-dependent process. For example, the target position can be a modified Cas9 molecule cleavage of the target nucleic acid and template nucleic acid directed modification, *e*.*g*., correction, of the target position. A target position can be a site between two nucleotides, *e*.*g*., adjacent nucleotides, on the target nucleic acid into which one or more nucleotides is added. The target position may comprise one or more nucleotides that are altered, *e*.*g*., corrected, by a template nucleic acid. The target position may be within a target sequence (*e*.*g*., the sequence to which the gRNA binds). A target position may be upstream or downstream of a target sequence (*e*.*g*., the sequence to which the gRNA binds).

A template nucleic acid, as that term is used herein, refers to a nucleic acid sequence which can be used in conjunction with a Cas9 molecule and a gRNA molecule to alter the structure of a target position. The target nucleic acid may be modified to have the some or all of the sequence of the template nucleic acid, typically at or near cleavage site(s). The template nucleic acid may be single stranded. The template nucleic acid may be double stranded. The template nucleic acid may be DNA, *e.g.,* double stranded DNA. The template nucleic acid may be single stranded DNA. The template nucleic acid may be encoded on the same vector backbone, *e*.*g*., AAV genome, plasmid DNA, as the Cas9 and gRNA. The template nucleic acid may be excised from a vector backbone *in vivo*, *e.g*., it is flanked by gRNA recognition sequences. The template nucleic acid may comprise endogenous genomic sequence.

The template nucleic acid may alter the structure of the target position by participating in an HDR event. The template nucleic acid may alter the sequence of the target position. The template nucleic acid may result in the incorporation of a modified, or non-naturally occurring base into the target nucleic acid.

Typically, the template sequence undergoes a breakage mediated or catalyzed recombination with the target sequence. The template nucleic acid may include sequence that corresponds to a site on the target sequence that is cleaved by an eaCas9 mediated cleavage event. The template nucleic acid may include sequence that corresponds to both, a first site on the target sequence that is cleaved in a first Cas9 mediated event, and a second site on the target sequence that is cleaved in a second Cas9 mediated event.

The template nucleic acid can include sequence which results in an alteration in the coding sequence of a translated sequence, *e*.*g*., one which results in the substitution of one amino acid for another in a protein product, *e*.*g*., transforming a mutant allele into a wild type allele, transforming a wild type allele into a mutant allele, and/or introducing a stop codon, insertion of an amino acid residue, deletion of an amino acid residue, or a nonsense mutation.

The template nucleic acid can include sequence which results in an alteration in a non-coding sequence, *e*.*g*., an alteration in an exon or in a 5' or 3' non-translated or non-transcribed region. Such alterations include an alteration in a control element, *e.g.,* a promoter, enhancer, and an alteration in a cis-acting or trans-acting control element.

A template nucleic acid having homology with a target position in the *HBB* gene can be used to alter the structure of a target sequence *(e.g.,* to correct a mutation present in a target position of an endogenous *HBB* gene). The template sequence can be used to alter an unwanted structure, *e*.*g*., an unwanted or mutant nucleotide.

A template nucleic acid typically comprises the following components:
[5' homology arm]-[replacement sequence]-[3' homology arm].

The homology arms provide for recombination into the chromosome, thus replacing the undesired element, *e.g.,* a mutation or signature, with the replacement sequence. The homology arms may flank the most distal cleavage sites.

The 3' end of the 5' homology arm may be the position next to the 5' end of the replacement sequence. The 5' homology arm can extend at least 10, 20, 30, 40, 50, 100, 200, 300, 400, 500, 600, 700, 800, 900, 1000, 1500, 2000, 3000, 4000, or 5000 nucleotides 5' from the 5' end of the replacement sequence.

The 5' end of the 3' homology arm may be the position next to the 3' end of the replacement sequence. The 3' homology arm can extend at least 10, 20, 30, 40, 50, 100, 200, 300, 400, 500, 600, 700, 800, 900, 1000, 1500, 2000, 3000, 4000, or 5000 nucleotides 3' from the 3' end of the replacement sequence.

To alter one or more nucleotides at a target position *(e.g.,* to correct a mutation), the homology arms, *e.g.,* the 5' and 3' homology arms, may each comprise about 1000 bp of sequence flanking the most distal gRNAs (*e.g*., 1000 bp of sequence on either side of the target position (*e*.*g*., the mutation).

It is contemplated herein that one or both homology arms may be shortened to avoid including certain sequence repeat elements, *e*.*g*., Alu repeats or LINE elements. For example, a 5' homology arm may be shortened to avoid a sequence repeat element or a 3' homology arm may be shortened to avoid a sequence repeat element or both the 5' and the 3' homology arms may be shortened to avoid including certain sequence repeat elements.

It is contemplated herein that template nucleic acids for altering the sequence (*e*.*g*., correcting a mutation) of a target position may be designed for use as a single-stranded oligonucleotide, *e*.*g*., a single-stranded oligodeoxynucleotide (ssODN). When using a ssODN, 5' and 3' homology arms may range up to about 200 bp in length, *e.g.,* at least 25, 50, 75, 100, 125, 150, 175, or 200 bp in length. Longer homology arms are also contemplated for ssODNs as improvements in oligonucleotide synthesis continue to be made. A longer homology arm may be made by a method other than chemical synthesis, *e*.*g*., by denaturing a long double stranded nucleic acid and purifying one of the strands, *e*.*g*., by affinity for a strand-specific sequence anchored to a solid substrate.

While not wishing to be bound by theory, alt-HDR may proceed more efficiently when the template nucleic acid has extended homology 5' to the nick *(i.e.,* in the 5' direction of the nicked strand). Accordingly, the template nucleic acid may have a longer homology arm and a shorter homology arm, wherein the longer homology arm can anneal 5' of the nick. The arm that can anneal 5' to the nick may be at least 25, 50, 75, 100, 125, 150, 175, or 200, 300, 400, 500, 600, 700, 800, 900, 1000, 1500, 2000, 3000, 4000, or 5000 nucleotides from the nick or the 5' or 3' end of the replacement sequence. The arm that can anneal 5' to the nick may be at least 10%, 20%, 30%, 40%, or 50% longer than the arm that can anneal 3' to the nick. The arm that can anneal 5' to the nick may be at least 2x, 3x, 4x, or 5x longer than the arm that can anneal 3' to the nick. Depending on whether a ssDNA template can anneal to the intact strand or the nicked strand, the homology arm that anneals 5' to the nick may be at the 5' end of the ssDNA template or the 3' end of the ssDNA template, respectively.

Similarly, the template nucleic acid may have a 5' homology arm, a replacement sequence, and a 3' homology arm, such that the template nucleic acid has extended homology to the 5' of the nick. For example, the 5' homology arm and 3' homology arm may be substantially the same length, but the replacement sequence may extend farther 5' of the nick than 3' of the nick. The replacement sequence may extend at least 10%, 20%, 30%, 40%, 50%, 2x, 3x, 4x, or 5x further to the 5' end of the nick than the 3' end of the nick.

While not wishing to be bound by theory, alt-HDR may proceed more efficiently when the template nucleic acid is centered on the nick. Accordingly, the template nucleic acid may have two homology arms that are essentially the same size. For instance, the first homology arm of a template nucleic acid may have a length that is within 10%, 9%, 8%, 7%, 6%, 5%, 4%, 3%, 2%, or 1% of the second homology arm of the template nucleic acid.

Similarly, the template nucleic acid may have a 5' homology arm, a replacement sequence, and a 3' homology arm, such that the template nucleic acid extends substantially the same distance on either side of the nick. For example, the homology arms may have different lengths, but the replacement sequence may be selected to compensate for this. For example, the replacement sequence may extend further 5' from the nick than it does 3' of the nick, but the homology arm 5' of the nick is shorter than the homology arm 3' of the nick, to compensate. The converse is also possible, *e*.*g*., that the replacement sequence may extend further 3' from the nick than it does 5' of the nick, but the homology arm 3' of the nick is shorter than the homology arm 5' of the nick, to compensate.

### Exemplary template nucleic acids

In order to increase DNA repair via gene conversion, the template nucleic acid is an endogenous homologous region. The template nucleic acid may be double stranded or single stranded. The template nucleic acid may comprise a single stranded portion and a double stranded portion. The template nucleic acid may comprise about 50 to 100, *e.g.,* 55 to 95, 60 to 90, 65 to 85, or 70 to 80 bp, homology on either side of the nick and/or replacement sequence. The template nucleic acid may comprise about 50, 55, 60, 65, 70, 75, 80, 85, 90, 95, or 100 bp homology 5' of the nick or replacement sequence, 3' of the nick or replacement sequence, or both 5' and 3' of the nick or replacement sequences.

The template nucleic acid may comprise about 150 to 200 bp, *e.g.,* 155 to 195, 160 to 190, 165 to 185, or 170 to 180 bp, homology 3' of the nick and/or replacement sequence. The template nucleic acid may comprise about 150, 155, 160, 165, 170, 175, 180, 185, 190, 195, or 200 bp homology 3' of the nick or replacement sequence. The template nucleic acid may comprise less than about 100, 90, 80, 70, 60, 50, 40, 30, 20, 15, or 10 bp homology 5' of the nick or replacement sequence.

The template nucleic acid may comprise about 150 to 200 bp, *e.g.,* 155 to 195, 160 to 190, 165 to 185, or 170 to 180 bp, homology 5' of the nick and/or replacement sequence. The template nucleic acid may comprise about 150, 155, 160, 165, 170, 175, 180, 185, 190, 195, or 200 bp homology 5' of the nick or replacement sequence. The template nucleic acid may comprise less than about 100, 90, 80, 70, 60, 50, 40, 30, 20, 15, or 10 bp homology 3' of the nick or replacement sequence.

The template nucleic acid may comprise a nucleotide sequence, *e.g.,* of one or more nucleotides, that will be added to or will template a change in the target nucleic acid. The template nucleic acid may comprise a nucleotide sequence that may be used to modify the target position. The template nucleic acid may comprises a nucleotide sequence, *e.g.,* of one or more nucleotides, that corresponds to wild type sequence of the target nucleic acid, *e.g.,* of the target position.

The template nucleic acid may comprise a replacement sequence. The template nucleic acid may comprise a 5' homology arm. The template nucleic acid may comprise a 3' homology arm.

The template nucleic acid may be linear double stranded DNA. The length may be, *e.g.,* about 150-200 bp, *e.g.,* about 150, 160, 170, 180, 190, or 200 bp. The length may be, *e.g.,* at least 150, 160, 170, 180, 190, or 200 bp. The length may be no greater than 150, 160, 170, 180, 190, or 200 bp. A double stranded template nucleic acid may have a length of about 160 bp, *e.g.,* about 155-165, 150-170, 140-180, 130-190, 120-200, 110-210, 100-220, 90-230, or 80-240 bp.

The template nucleic acid can be linear single stranded DNA. The template nucleic acid may be (i) linear single stranded DNA that can anneal to the nicked strand of the target nucleic acid, (ii) linear single stranded DNA that can anneal to the intact strand of the target nucleic acid, (iii) linear single stranded DNA that can anneal to the plus strand of the target nucleic acid, (iv) linear single stranded DNA that can anneal to the minus strand of the target nucleic acid, or more than one of the preceding. The length may be, *e.g.,* about 150-200 nucleotides, *e*.*g*., about 150, 160, 170, 180, 190, or 200 nucleotides. The length may be at least, or no greater than, 150, 160, 170, 180, 190, or 200 nucleotides. A single stranded template nucleic acid may have a length of about 160 nucleotides, *e*.*g*., about 155-165, 150-170, 140-180, 130-190, 120-200, 110-210, 100-220, 90-230, or 80-240 nucleotides.

The template nucleic acid may be circular double stranded DNA, *e.g.,* a plasmid. The template nucleic acid may comprise about 500 to 1000 bp of homology on either side of the replacement sequence and/or the nick. The template nucleic acid may comprise about 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. The template nucleic acid may comprise at least 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. The template nucleic acid may comprise no more than 300, 400, 500, 600, 700, 800, 900, 1000, 1500, or 2000 bp of homology 5' of the nick or replacement sequence, 3' of the nick or replacement sequence, or both 5' and 3' of the nick or replacement sequence.

One or both homology arms may be shortened to avoid including certain sequence repeat elements, *e*.*g*., Alu repeats, LINE elements. For example, a 5' homology arm may be shortened to avoid a sequence repeat element, while a 3' homology arm may be shortened to avoid a sequence repeat element. Both the 5' and the 3' homology arms may be shortened to avoid including certain sequence repeat elements.

The template nucleic acid may be an adenovirus vector, *e.g.,* an AAV vector, *e.g.,* a ssDNA molecule of a length and sequence that allows it to be packaged in an AAV capsid. The vector may be, *e.g.,* less than 5 kb and may contain an ITR sequence that promotes packaging into the capsid. The vector may be integration-deficient. The template nucleic acid may comprise about 150 to 1000 nucleotides of homology on either side of the replacement sequence and/or the nick. The template nucleic acid may comprise about 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. The template nucleic acid may comprise at least 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence. The template nucleic acid may comprise at most 100, 150, 200, 300, 400, 500, 600, 700, 800, 900, 1000, 1500, or 2000 nucleotides 5' of the nick or replacement sequence, 3' of the nick or replacement sequence, or both 5' and 3' of the nick or replacement sequence.

The template nucleic acid may be a lentiviral vector, *e.g.,* an IDLV (integration deficiency lentivirus). The template nucleic acid may comprise about 500 to 1000 base pairs of homology on either side of the replacement sequence and/or the nick. The template nucleic acid may comprise about 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. The template nucleic acid may comprise at least 300, 400, 500, 600, 700, 800, 900, 1000, 1500, or 2000 bp of homology 5' of the nick or replacement sequence, 3' of the nick or replacement sequence, or both 5' and 3' of the nick or replacement sequence. The template nucleic acid may comprise no more than 300, 400, 500, 600, 700, 800, 900, 1000, 1500, or 2000 bp of homology 5' of the nick or replacement sequence, 3' of the nick or replacement sequence, or both 5' and 3' of the nick or replacement sequence.

The template nucleic acid may comprise one or more mutations, *e*.*g*., silent mutations, that prevent Cas9 from recognizing and cleaving the template nucleic acid. The template nucleic acid may comprise, *e*.*g*., at least 1, 2, 3, 4, 5, 10, 20, 30, 40, or 50 silent mutations relative to the corresponding sequence in the genome of the cell to be altered. The template nucleic acid may comprise at most 2, 3, 4, 5, 10, 20, 30, 40, or 50 silent mutations relative to the corresponding sequence in the genome of the cell to be altered. The template nucleic acid may comprise one or more mutations, *e*.*g*., silent mutations that prevent Cas9 from recognizing and cleaving the template nucleic acid. The template nucleic acid may comprise, *e.g.,* at least 1, 2, 3, 4, 5, 10, 20, 30, 40, or 50 silent mutations relative to the corresponding sequence in the genome of the cell to be altered. The template nucleic acid may comprise at most 2, 3, 4, 5, 10, 20, 30, 40, or 50 silent mutations relative to the corresponding sequence in the genome of the cell to be altered.

The template nucleic acid may alter the structure of the target position by participating in an HDR event. The template nucleic acid may alter the sequence of the target position. The template nucleic acid may result in the incorporation of a modified, or non-naturally occurring nucleotide base into the target nucleic acid.

Typically, the template sequence undergoes a breakage mediated or catalyzed recombination with the target sequence. The template nucleic acid may include sequence that corresponds to a site on the target sequence that is cleaved by an eaCas9 mediated cleavage event. The template nucleic acid may include sequence that corresponds to both, a first site on the target sequence that is cleaved in a first Cas9 mediated event, and a second site on the target sequence that is cleaved in a second Cas9 mediated event.

The template nucleic acid can include sequence which results in an alteration in the coding sequence of a translated sequence, *e*.*g*., one which results in the substitution of one amino acid for another in a protein product, *e*.*g*., transforming a mutant allele into a wild type allele, transforming a wild type allele into a mutant allele, and/or introduction of a stop codon, insertion of an amino acid residue, deletion of an amino acid residue, or a nonsense mutation.

The template nucleic acid can include sequence which results in an alteration in a non-coding sequence, *e*.*g*., an alteration in an exon or in a 5' or 3' non-translated or non-transcribed region. Such alterations include an alteration in a control element, *e.g.,* a promoter or enhancer, or an alteration in a cis-acting or trans-acting control element.

A template nucleic acid having homology with a target position can be used to alter the structure of a target sequence. The template nucleic acid sequence can be used to alter an unwanted structure, *e*.*g*., an unwanted or mutant nucleotide.

Exemplary template nucleic acids (also referred to herein as donor constructs) to correction a mutation, *e.g.,* at E6, *e.g.,* E6V, in the *HBB* gene, are provided.

Suitable sequence for a 5' homology arm can be selected from (*e*.*g*., includes a portion of) or include the nucleic acid sequence of SEQ ID NO: 16257 (5'H arm).

Suitable sequence for the 3' homology arm can be selected from (*e*.*g*., includes a portion of) or include the nucleic acid sequence of SEQ ID NO: 16258 (3'H arm).

The replacement sequence may comprise or consist of an adenine (A) residue to correct the amino acid sequence to a glutamic acid (E) residue.

To correct a mutation, *e.g.,* at E6, *e.g.,* E6V, in the *HBB* gene, the homology arms, *e.g.,* the 5' and 3' homology arms, may each comprise about 1000 base pairs (bp) of sequence flanking the most distal gRNAs *(e.g.,* 1100 bp of sequence on either side of the mutation). The 5' homology arm is shown as bold sequence, codon 6 is shown as underlined sequence, the inserted base to correct the mutation at E6, *e.g.,* E6V, is shown as boxed sequence, and the 3' homology arm is shown as no emphasis sequence:

ATAGGAACTTGAATCAAGGAAATGATTTAAAACGCAGTATTCTTAGTGGACTAGAG
GAAAAAAATAATCTGAGCCAAGTAGAAGACCTTTTCCCCTCCTACCCCTACTTTCTA
AGTCACAGAGGCTTTTGTTCCCCCAGACACTCTTGCAGATTAGTCCAGGCAGAAA
CAGTTAGATGTCCCCAGTTAACCTCCTATTTGACACCACTGATTACCCCATTGATAG
TCACACTTTGGGTTGTAAGTGACTTTTTATTTATTTGTATTTTTGACTGCATTAAGAG
GTCTCTAGTTTTTTATCTCTTGTTTCCCAAAACCTAATAAGTAACTAATGCACAGAG
CACATTGATTTGTATTTATTCTATTTTTAGACATAATTTATTAGCATGCATGAGCAAA
TTAAGAAAAACAACAACAAATGAATGCATATATATATGTATATGTATGTGTGTATATAT
ACACACATATATATATATATATTTTTTCTTTTCTTACCAGAAGGTTTTAATCCAAATAAG

GAGAAGATATGCTTAGAACCGAGGTAGAGTTTTCATCCATTCTGTCCTGTAAGTATT
TTGCATATTCTGGAGACGCAGGAAGAGATCCATCTACATATCCCAAAGCTGAATTAT
GGTAGACAAAACTCTTCCACTTTTAGTGCATCAACTTCTTATTTGTGTAATAAGAAA
ATTGGGAAAACGATCTTCAATATGCTTACCAAGCTGTGATTCCAAATATTACGTAAA
TACACTTGCAAAGGAGGATGTTTTTAGTAGCAATTTGTACTGATGGTATGGGGCCA
AGAGATATATCTTAGAGGGAGGGCTGAGGGTTTGAAGTCCAACTCCTAAGCCAGTG
CCAGAAGAGCCAAGGACAGGTACGGCTGTCATCACTTAGACCTCACCCTGTGGAGC
CACACCCTAGGGTTGGCCAATCTACTCCCAGGAGCAGGGAGGGCAGGAGCCAGGG
CTGGGCATAAAAGTCAGGGCAGAGCCATCTATTGCTTACATTTGCTTCTGACACAAC
TGTGTTCACTAGCAACCTCAAACAGACACCATGGTGCATCTGACTCCTGAGGAGAAG
TCTGCCGTTACTGCCCTGTGGGGCAAGGTGAACGTGGATGAAGTTGGTGGTGAGGCCCTG
GGCAGGTTGGTATCAAGGTTACAAGACAGGTTTAAGGAGACCAATAGAAACTGGGCATG
TGGAGACAGAGAAGACTCTTGGGTTTCTGATAGGCACTGACTCTCTCTGCCTATTGGTCT
ATTTTCCCACCCTTAGGCTGCTGGTGGTCTACCCTTGGACCCAGAGGTTCTTTGAGTCCTT
TGGGGATCTGTCCACTCCTGATGCTGTTATGGGCAACCCTAAGGTGAAGGCTCATGGCAA
GAAAGTGCTCGGTGCCTTTAGTGATGGCCTGGCTCACCTGGACAACCTCAAGGGCACCTT
TGCCACACTGAGTGAGCTGCACTGTGACAAGCTGCACGTGGATCCTGAGAACTTCAGGG
TGAGTCTATGGGACGCTTGATGTTTTCTTTCCCCTTCTTTTCTATGGTTAAGTTCATGTCAT
AGGAAGGGGATAAGTAACAGGGTACAGTTTAGAATGGGAAACAGACGAATGATTGCAT
CAGTGTGGAAGTCTCAGGATCGTTTTAGTTTCTTTTATTTGCTGTTCATAACAATTGTTTT
CTTTTGTTTAATTCTTGCTTTCTTTTTTTTTCTTCTCCGCAATTTTTACTATTATACTTAATG
CCTTAACATTGTGTATAACAAAAGGAAATATCTCTGAGATACATTAAGTAACTTAAAAAA
AAACTTTACACAGTCTGCCTAGTACATTACTATTTGGAATATATGTGTGCTTATTTGCATA
TTCATAATCTCCCTACTTTATTTTCTTTTATTTTTAATTGATACATAATCATTATACATATT
TATGGGTTAAAGTGTAATGTTTTAATATGTGTACACATATTGACCAAATCAGGGTAATTT
TGCATTTGTAATTTTAAAAAATGCTTTCTTCTTTTAATATACTTTTTTGTTTATCTTATTTCT
AATACTTTCCCTAATCTCTTTCTTTCAGGGCAATAATGATACAATGTATCATGCCTCTTTG
CACCATTCTAAAGAATAACAGTGATAATTTCTGGGTTAAGGCAATAGCAATATCTCTGCA
TATAAATATTTCTGCATATAAATTGTAACTG (Template Construct 1; SEQ ID NO: 16259).

Shorter homology arms, *e.g.,* 5' and/or 3' homology arms may be used. The length of the 5' homology arm may be about 5 to about 100 nucleotidesor about 10 to about 150 nucleotidesor about 20 to about 150 nucleotides. The length of the 5' homology arm may be about 10, 20, 50, 100, 150, 200, 250, 300, 350, 400, 450, 500, 550, 600, 650, 700, 750, 800, 850, 900, 950, 1000, 1100, 1200, or more nucleotides in length.

The length of the 3' homology arm may be about 5 to about 100 nucleotidesor about 10 to about 150 nucleotidesor about 20 to about 150 nucleotides. The length of the 3' homology arm may be about 10, 20, 50, 100, 150, 200, 250, 300, 350, 400, 450, 500, 550, 600, 650, 700, 750, 800, 850, 900, 950, 1000, 1100, 1200, or more nucleotides in length.

It is contemplated herein that one or both homology arms may be shortened to avoid including certain sequence repeat elements, *e*.*g*., Alu repeats, LINE elements. For example, a 5' homology arm may be shortened to avoid a sequence repeat element. A 3' homology arm may be shortened to avoid a sequence repeat element. Both the 5' and the 3' homology arms may be shortened to avoid including certain sequence repeat elements. The length of the 5' homology arm may be at least 50 nucleotides in length, but not long enough to include a repeated element. The length of the 5' homology arm may be at least 100 or at least 150 nucleotides in length, but not long enough to include a repeated element. The length of the 3' homology arm may be at least 50, at least 100, or at least 150 nucleotides in length, but not long enough to include a repeated element.

It is contemplated herein that template nucleic acids for correcting a mutation may be designed for use as a single-stranded oligonucleotide (ssODN), *e.g.,* a single-stranded oligodeoxynucleotide. When using a ssODN, 5' and 3' homology arms may range up to about 200 bp in length, *e.g.,* at least 25, 50, 75, 100, 125, 150, 175, or 200 bp in length. Longer homology arms are also contemplated for ssODNs as improvements in oligonucleotide synthesis continue to be made.

An ssODN may be used to correct a mutation, *e.g.,* E6V in the *HBB* gene. For example, the ssODN may include 50 bp 5' and 3' homology arms as shown below. The 5' homology arm is shown as bold sequence, codon 6 is shown as underlined sequence, the inserted base to correct the E6V mutation is shown as boxed sequence, and the 3' homology arm is shown as no emphasis sequence.

ACTGTGTTCACTAGCAACCTCAAACAGACACCATGGTGCATCTGACTC
CTGGGGAGAAGTCTGCCGTTACTGCCCTGTGGGGCAAGGTGAACGTGGATGAAG
T[?](Template Construct 2; SEQ ID NO: 16260)

### Silent mutations in the template nucleic acid

It is contemplated herein that Cas9 could potentially cleave donor constructs either prior to or following homology directed repair (*e*.*g*., homologous recombination), resulting in a possible non-homologous-end-joining event and further DNA sequence mutation at the chromosomal locus of interest. Therefore, to avoid cleavage of the donor sequence before and/or after Cas9-mediated homology directed repair, alternate versions of the donor sequence may be used where silent mutations are introduced. These silent mutations may disrupt Cas9 binding and cleavage, but not disrupt the amino acid sequence of the repaired gene. For example, mutations may include those made to a donor sequence to repair the HBB gene, the mutant form of which can cause Sickle Cell Disease. If gRNA HBB-6 with the 20-base target sequence CGUUACUGCCCUGUGGGGCA is used to insert a donor sequence including **CTCCTGAGGAGAAGTCTGCICGTTACTGCCCTGTGGGGCAAGgGTGAACGTG** GATGAAGT (SEQ ID NO: 16297), where the *italic* A is the base being corrected and the bracketed bases are those that match the guide RNA, the donor sequence may be changed to **CTCCTGAGGAGAAGTCTGCICGTTACTGCCCTGTGGGGCAAGaTGAACGTGG** ATGAAGT (SEQ ID NO: 16298), where the lowercase a has been changed from a G (lower case g) at that position so that codon 15 still codes for the amino acid arginine but the PAM sequence AGG has been modified to AGA to reduce or eliminate Cas9 cleavage at that locus.

### Upregulators of Gene Conversion

Without being bound by theory, the methods provided herein may involve up-regulating a gene conversion pathway(s). For instance, the methods may involve modulating *(e.g.,* stimulating or overexpressing) a component *(e.g.,* exactly one component, or one or more components, *e.g.,* two or three components) of a gene conversion pathway, *e.g.,* a BRCA1, BRCA2 and/or RAD51. The up-regulator of gene conversion may be a BRCA1. The up-regulator of gene conversion may be a BRCA2. The up-regulator of gene conversion may be a RAD51.

The up-regulator of gene conversion may be selected from the group consisting of a polypeptide of Table 5, or a polypeptide that comprises at least 60, 70, 80, 81, 82, 83, 84, 85, 86, 87, 88, 89, 90, 91, 92, 93, 94, 95, 96, 97, 98, 99 or 100% homology with, or differs by no more than 50, 40, 30, 20, 15, 14, 13, 12, 11, 10, 9, 8, 7, 6, 5, 4, 3, 2, or 1, amino acid residues from a naturally occurring polypeptide of Table 5.

**Table 5. Polypeptides that Promote Gene Conversion**

| **Factor** | **Sequence** | **SEQ ID NOs** |
|---|---|---|
| BRCA2 | MPIGSKERPTFFEIFKTRCNKADLGPISLNWFEELSSEAPPYNSEPAEESEHKNNNYEPNLFKTPQRKPS YNQLASTPIIFKEQGLTLPLYQSPVKELDKFKLDLGRNVPNSRHKSLRTVKTKMDQADDVSCPLLNSCLS ESPVVLQCTHVTPQRDKSVVCGSLFHTPKFVKGRQTPKHISESLGAEVDPDMSWSSSLATPPTLSSTVLI VRNEEASETVFPHDTTANVKSYFSNHDESLKKNDRFIASVTDSENTNQREAASHGFGKTSGNSFKVNSCK DHIGKSMPNVLEDEVYETVVDTSEEDSFSLCFSKCRTKNLQKVRTSKTRKKIFHEANADECEKSKNQVKE KYSFVSEVEPNDTDPLDSNVANQKPFESGSDKISKEVVPSLACEWSQLTLSGLNGAQMEKIPLLHISSCD QNISEKDLLDTENKRKKDFLTSENSLPRISSLPKSEKPLNEETVVNKRDEEQHLESHTDCILAVKQAISG | 16304 |
| | TSPVASSFQGIKKSIFRIRESPKETFNASFSGHMTDPNFKKETEASESGLEIHTVCSQKEDSLCPNLIDN GSWPATTTQNSVALKNAGLISTLKKKTNKFIYAIHDETSYKGKKIPKDQKSELINCSAQFEANAFEAPLT FANADSGLLHSSVKRSCSQNDSEEPTLSLTSSFGTI[?]RKCSRNETCSNNTVISQDLDYKEAKCNKEKLQL FITPEADSLSCLQEGQCENDPKSKKVSDIKEEVLAAACHPVQHSKVEYSDTDFQSQKSLLYDHENASTLI LTPTSKDVLSNLVMISRGKESYKMSDKLKGNNYESDVELTKNIPMEKNQDVCALNENYKNVELLPPEKYM RVASPSRKVQFNQNTNLRVIQKNQEETTSISKITVNPDSEELFSDNENNFVFQVANERNNLALGNTKELH ETDLTCVNEPIFKNSTMVLYGDTGDKQATQVSIKKD[?]VYVLAEENKNSVKQHIKMTLGQDLKSDISLNID KIPEKNNDYMNKWAGLLGPISNHSFGGSFRTASNKEIKLSEHNIKKSKMFFKDIEEQYPTSLACVEIVNT LALDNQKKLSKPQSINTVSAHLQSSVVVSDCKNSHITPQMLFSKQDFNSNHNLTPSQKAEITELSTILEE SGSQFEFTQFRKPSYILQKSTFEVPENQMTILKTTSEECRDADLHVIMNAPSIGQVDSSKQFEGTVEIKR KFAGLLKNDCNKSASGYLTDENEVGFRGFYSAHGTK[?]NVSTEALQKAVKLFSDIENISEETSAEVHPISL SSSKCHDSVVSMFKIENHNDKTVSEKNNKCQLILQNNIEMTTGTFVEEITENYKRNTENEDNKYTAASRN SHNLEFDGSDSSKNDTVCIHKDETDLLFTDQHNICLKLSGQFMKEGNTQIKEDLSDLTFLEVAKAQEACH GNTSNKEQLTATKTEQNIKDFETSDTFFQTASGKNISVAKESFNKIVNFFDQKPEELHNFSLNSELHSDI RKNKMDILSYEETDIVKHKILKESVPVGTGNQLVTFQGQPERDEKIKEPTLLGFHTASGKKVKIAKESLD KVKNLFDEKEQGTSEITSFSHQWAKTLKYREACKDLELACETIEITAAPKCKEMQNSLNNDKNLVSIETV VPPKLLSDNLCRQTENLKTSKSIFLKVKVHENVEKETAKSPATCYTNQSPYSVIENSALAFYTSCSRKTS VSQTSLLEAKKWLREGIFDGQPERINTADYVGNYLYENNSNSTIAENDKNHLSEKQDTYLSNSSMSNSYS YHSDEVYNDSGYLSKNKLDSGIEPVLKNVEDQKNTSFSKVISNVKDANAYPQTVNEDICVEELVTSSSPC KNKNAAIKLSISNSNNFEVGPPAFRIASGKIVCVSHETIKKVKDIFTDSFSKVIKENNENKSKICQTKIM AGCYEALDDSEDILHNSLDNDECSTHSHKVFADIQSEEILQHNQNMSGLEKVSKISPCDVSLETSDICKC SIGKLHKSVSSANTCGIFSTASGKSVQVSDASLQNARQVFSEIEDSTKQVFSKVLFKSNEHSDQLTREEN TAIRTPEHLISQKGFSYNVVNSSAFSGFSTASGKQVSILESSLHKVKGVLEEFDLIRTEHSLHYSPTSRQ NVSKILPRVDKRNPEHCVNSEMEKTCSKEFKLSNNLNVEGGSSENNHSIKVSPYLSQFQQDKQQLVLGTK VSLVENIHVLGKEQASPKNVKMEIGKTETFSDVPVKTNIEVCSTYSKDSENYFETEAVEIAKAFMEDDEL TDSKLPSHATHSLFTCPENEEMVLSNSRIGKRRGEP[?]ILVGEPSIKRNLLNEFDRIIENQEKSLKASKST PDGTIKDRRLFMHHVSLEPITCVPFRTTKERQEIQNPNFTAPGQEFLSKSHLYEHLTLEKSSSNLAVSGH PFYQVSATRNEKMRHLITTGRPTKVFVPPFKTKSHFHRVEQCVRNINLEENRQKQNIDGHGSDDSKNKIN DNEIHQFNKNNSNQAVAVTFTKCEEEPLDLITSLQNARDIQDMRIKKKQRQRVFPQPGSLYLAKTSTLPR ISLKAAVGGQVPSACSHKQLYTYGVSKHCIKINSKNAESFQFHTEDYFGKESLWTGKGIQLADGGWLIPS NDGKAGKEEFYRALCDTPGVDPKLISRIWVYNHYRWIIWKLAAMECAFPKEFANRCLSPERVLLQLKYRY DTEIDRSRRSAIKKIMERDDTAAKTLVLCVSDIISLSANISETSSNKTSSADTQKVAIIELTDGWYAVKA QLDPPLLAVLKNGRLTVGQKIILHGAELVGSPDACTPLEAPESLMLKISANSTRPARWYTKLGFFPDPRP FPLPLSSLFSDGGNVGCVDVIIQRAYPIQWMEKTSSGLYIFRNEREEEKEAAKYVEAQQKRLEALFTKIQ EEFEEHEENTTKPYLPSRALTRQQVRALQDGAELYEAVKNAADPAYLEGYFSEEQLRALNNHRQMLNDKK QAQIQLEIRKAMESAEQKEQGLSRDVTTVWKLRIVSYSKKEKDSVILSIWRPSSDLYSLLTEGKRYRIYH LATSKSKSKSERANIQLAATKKTQYQQLPVSDEILFQIYQPREPLHFSKFLDPDFQPSCSEVDLIGFVVS VVKKTGLAPFVYLSDECYNLLAIKFWIDLNEDIIKPHMLIAASNLQWRPESKSGLLTLFAGDFSVFSASP KEGHFQETFNKMKNTVENIDILCNEAENKLMHILHANDPKWSTPTKDCTSGPYTAQIIPGTGNKLLMSSP NCEIYYQSPLSLCMAKRKSVSTPVSAQMTSKSCKGEKEIDDQKNCKKRRALDFLSRLPLPPPVSPICTFV SPAAQKAFQPPRSCGTKYETPIKKKELNSPQMTPFKKFNEISLLESNSIADEELALINTQALLSGSTGEK QFISVSESTRTAPTSSEDYLRLKRRCTTSLIKEQESSQASTEECEKNKQDTITTKKYI (breast cancer 2, early onset, isoform CRA_c [Homo sapiens] CCDS 9344.1) | |
| BRCA1 |  (breast cancer type 1 susceptibility protein isoform 1 [Homo sapiens] CCDS 11453.1) MLKLLNQKKGPSQCPLCKNDITKRSLQESTRFSQLVEELLKIICAFQLDTGLEYANSYNFAKKENNSPEH LKDEVSIIQSMGYRNRAKRLLQSEPENPSLQETSLSVQLSNLGTVRTLRTKQRIQPQKTSVYIELGSDSS EDTVNKATYCSVGDQELLQITPQGTRDEISLDSAKKAACEFSETDVTNTEHHQPSNNDLNTTEKRAAERH PEKYQGSSVSNLHVEPCGTNTHASSLQHENSSLLLTKDRMNVEKAEFCNKSKQPGLARSQHNRWAGSKET | 16305-16309 |
| | CNDRRTPSTEKKVDLNADPLCERKEWNKQKLPCSENPRDTEDVPWITLNSSIQKVNEWFSRSDELLGSDD SHDGESESNAKVADVLDVLNEVDEYSGSSEKIDLLASDPHEALICKSERVHSKSVESNIEDKIFGKTYRK KASLPNLSHVTENLIIGAFVTEPQIIQERPLTNKLKRKRRPTSGLHPEDFIKKADLAVQKTPEMINQGTN QTEQNGQVMNITNSGHENKTKGDSIQNEKNPNPIESLEKESAFKTKAEPISSSISNMELELNIHNSKAPK KNRLRRKSSTRHIHALELVVSRNLSPPNCTELQIDSCSSSEEIKKKKYNQMPVRHSRNLQLMEGKEPATG AKKSNKPNEQTSKRHDSDTFPELKLTNAPGSFTKCSNTSELKEFVNPSLPREEKEEKLETVKVSNNAEDP KDLMLSGERVLQTERSVESSSISLVPGTDYGTQESISLLEVSTLGKAKTEPNKCVSQCAAFENPKGLIHG CSKDNRNDTEGFKYPLGHEVNHSRETSIEMEESELDAQYLQNTFKVSKRQSFAPFSNPGNAEEECATFSA HSGSLKKQSPKVTFECEQKEENQGKNESNIKPVQTVNITAGFPVVGQKDKPVDNAKCSIKGGSRFCLSSQ FRGNETGLITPNKHGLLQNPYRIPPLFPIKSFVKTKCKKNLLEENFEEHSMSPEREMGNENIPSTVSTIS RNNIRENVFKEASSSNINEVGSSTNEVGSSINEIGSSDENIQAELGRNRGPKLNAMLRLGVLQPEVYKQS LPGSNCKHPEIKKQEYEEVVQTVNTDFSPYLISDNLEQPMGSSHASQVCSETPDDLLDDGEIKEDTSFAE NDIKESSAVFSKSVQKGELSRSPSPFTHTHLAQGYRRGAKKLESSEENLSSEDEELPCFQHLLFGKVNNI PSQSTRHSTVATECLSKNTEENLLSLKNSLNDCSNQVILAKASQEHHLSEETKCSASLFSSQCSELEDLT ANTNTQDPFLIGSSKQMRHQSESQGVGLSDKELVSDDEERGTGLEENNQEEQSMDSNLGEAASGCESETS VSEDCSGLSSQSDILTTQQRDTMQHNLIKLQQEMAELEAVLEQHGSQPSNSYPSIISDSSALEDLRNPEQ STSEKAVLTSQKSSEYPISQNPEGLSADKFEVSADSSTSKNKEPGVERSSPSKCPSLDDRWYMHSCSGSL QNRNYPSQEELIKVVDVEEQQLEESGPHDLTETSYLPRQDLEGTPYLESGISLFSDDPESDPSEDRAPES ARVGNIPSSTSALKVPQLKVAESAQSPAAAHTTDTAGYNAMEESVSREKPELTASTERVNKRMSMVVSGL TPEEFMLVYKFARKHHITLTNLITEETTHVVMKTDAEFVCERTLKYFLGIAGGKWVVSYFWVTQSIKERK MLNEHDFEVRGDVVNGRNHQGPKRARESQDRKIFRGLEICCYGPFTNMPTDQLEWMVQLCGASVVKELSS FTLGTGVHPIVVVQPDAWTEDNGFHAIGQMCEAPVVTREWVLDSVALYQCQELDTYLIPQIPHSHY (breast cancer type 1 susceptibility protein isoform 2 [Homo sapiens] CCDS 11459.2) | |
| | MDLSALRVEEVQNVINAMQKILECPICLELIKEPVSTKCDHIFCKFCMLKLLNQKKGPSQCPLCKNDITK RSLQESTRFSQLVEELLKIICAFQLDTGLEYANSYNFAKKENNSPEHLKDEVSIIQSMGYRNRAKRLLQS EPENPSLQETSLSVQLSNLGTVRTLRTKQRIQPQKTSVYIELGSDSSEDTVNKATYCSVGDQELLQITPQ GTRDEISLDSAKKAACEFSETDVTNTEHHQPSNNDLNTTEKRAAERHPEKYQGSSVSNLHVEPCGTNTHA SSLQHENSSLLLTKDRMNVEKAEFCNKSKQPGLARSQHNRWAGSKETCNDRRTPSTEKKVDLNADPLCER KEWNKQKLPCSENPRDTEDVPWITLNSSIQKVNEWFSRSDELLGSDDSHDGESESNAKVADVLDVLNEVD EYSGSSEKIDLLASDPHEALICKSERVHSKSVESNIEDKIFGKTYRKKASLPNLSHVTENLIIGAFVTEP QIIQERPLTNKLKRKRRPTSGLHPEDFIKKADLAVQKTPEMINQGTNQTEQNGQVMNITNSGHENKTKGD SIQNEKNPNPIESLEKESAFKTKAEPISSSISNMELELNIHNSKAPKKNRLRRKSSTRHIHALELVVSRN LSPPNCTELQIDSCSSSEEIKKKKYNQMPVRHSRNLQLMEGKEPATGAKKSNKPNEQTSKRHDSDTFPEL KLTNAPGSFTKCSNTSELKEFVNPSLPREEKEEKLETVKVSNNAEDPKDLMLSGERVLQTERSVESSSIS LVPGTDYGTQESISLLEVSTLGKAKTEPNKCVSQCAAFENPKGLIHGCSKDNRNDTEGFKYPLGHEVNHS RETSIEMEESELDAQYLQNTFKVSKRQSFAPFSNPGNAEEECATFSAHSGSLKKQSPKVTFECEQKEENQ GKNESNIKPVQTVNITAGFPVVGQKDKPVDNAKCSIKGGSRFCLSSQFRGNETGLITPNKHGLLQNPYRI PPLFPIKSFVKTKCKKNLLEENFEEHSMSPEREMGNENIPSTVSTISRNNIRENVFKEASSSNINEVGSS TNEVGSSINEIGSSDENIQAELGRNRGPKLNAMLRLGVLQPEVYKQSLPGSNCKHPEIKKQEYEEVVQTV NTDFSPYLISDNLEQPMGSSHASQVCSETPDDLLDDGEIKEDTSFAENDIKESSAVFSKSVQKGELSRSP SPFTHTHLAQGYRRGAKKLESSEENLSSEDEELPCFQHLLFGKVNNIPSQSTRHSTVATECLSKNTEENL LSLKNSLNDCSNQVILAKASQEHHLSEETKCSASLFSSQCSELEDLTANTNTQDPFLIGSSKQMRHQSES QGVGLSDKELVSDDEERGTGLEENNQEEQSMDSNLGEAASGCESETSVSEDCSGLSSQSDILTTQQRDTM QHNLIKLQQEMAELEAVLEQHGSQPSNSYPSIISDSSALEDLRNPEQSTSEKDSHIHGQRNNSMFSKRPR EHISVLTSQKSSEYPISQNPEGLSADKFEVSADSSTSKNKEPGVERSSPSKCPSLDDRWYMHSCSGSLQN RNYPSQEELIKVVDVEEQQLEESGPHDLTETSYLPRQDLEGTPYLESGISLFSDDPESDPSEDRAPESAR VGNIPSSTSALKVPQLKVAESAQSPAAAHTTDTAGYNAMEESVSREKPELTASTERVNKRMSMVVSGLTP EEFMLVYKFARKHHITLTNLITEETTHVVMKTDAEFVCERTLKYFLGIAGGKWVVSYFWVTQSIKERKML NEHDFEVRGDVVNGRNHQGPKRARESQDRKIFRGLEICCYGPFTNMPTDQLEWMVQLCGASVVKELSSFT LGTGVHPIVVVQPDAWTEDNGFHAIGQMCEAPVVTREWVLDSVALYQCQELDTYLIPQIPHSHY (breast cancer type 1 susceptibility protein isoform 2 [Homo sapiens], CCDS 11456.2) | |
| | MDLSALRVEEVQNVINAMQKILECPICLELIKEPVSTKCDHIFCKFCMLKLLNQKKGPSQCPLCKNDITK RSLQESTRFSQLVEELLKIICAFQLDTGLEYANSYNFAKKENNSPEHLKDEVSIIQSMGYRNRAKRLLQS EPENPSLQETSLSVQLSNLGTVRTLRTKQRIQPQKTSVYIELGSDSSEDTVNKATYCSVGDQELLQITPQ GTRDEISLDSAKKAACEFSETDVTNTEHHQPSNNDLNTTEKRAAERHPEKYQGEAASGCESETSVSEDCS GLSSQSDILTTQQRDTMQHNLIKLQQEMAELEAVLEQHGSQPSNSYPSIISDSSALEDLRNPEQSTSEKV LTSQKSSEYPISQNPEGLSADKFEVSADSSTSKNKEPGVERSSPSKCPSLDDRWYMHSCSGSLQNRNYPS QEELIKVVDVEEQQLEESGPHDLTETSYLPRQDLEGTPYLESGISLFSDDPESDPSEDRAPESARVGNIP SSTSALKVPQLKVAESAQSPAAAHTTDTAGYNAMEESVSREKPELTASTERVNKRMSMVVSGLTPEEFML VYKFARKHHITLTNLITEETTHVVMKTDAEFVCERTLKYFLGIAGGKWVVSYFWVTQSIKERKMLNEHDF EVRGDVVNGRNHQGPKRARESQDRKIFRGLEICCYGPFTNMPTDQLEWMVQLCGASVVKELSSFTLGTGV HPIVVVQPDAWTEDNGFHAIGQMCEAPVVTREWVLDSVALYQCQELDTYLIPQIPHSHY (breast cancer type 1 susceptibility protein isoform 2 [Homo sapiens] CCDS 11454.2) | |
| | MDLSALRVEEVQNVINAMQKILECPICLELIKEPVSTKCDHIFCKFCMLKLLNQKKGPSQCPLCKNDITK RSLQESTRFSQLVEELLKIICAFQLDTGLEYANSYNFAKKENNSPEHLKDEVSIIQSMGYRNRAKRLLQS EPENPSLQETSLSVQLSNLGTVRTLRTKQRIQPQKTSVYIELGSDSSEDTVNKATYCSVGDQELLQITPQ GTRDEISLDSAKKAACEFSETDVTNTEHHQPSNNDLNTTEKRAAERHPEKYQGEAASGCESETSVSEDCS GLSSQSDILTTQQRDTMQHNLIKLQQEMAELEAVLEQHGSQPSNSYPSIISDSSALEDLRNPEQSTSEKV LTSQKSSEYPISQNPEGLSADKFEVSADSSTSKNKEPGVERSSPSKCPSLDDRWYMHSCSGSLQNRNYPS QEELIKVVDVEEQQLEESGPHDLTETSYLPRQDLEGTPYLESGISLFSDDPESDPSEDRAPESARVGNIP | |
| | SSTSALKVPQLKVAESAQSPAAAHTTDTAGYNAMEESVSREKPELTASTERVNKRMSMVVSGLTPEEFML VYKFARKHHITLTNLITEETTHVVMKTDAEFVCERTLKYFLGIAGGKWVVSYFWVTQSIKERKMLNEHDF EVRGDVVNGRNHQGPKRARESQDRKIFRGLEICCYGPFTNMPTGCPPNCGCAARCLDRGQWLPCNWADV (breast cancer type 1 susceptibility protein isoform 2 [Homo sapiens] CCDS 11455.2) | |
| RAD51 |  (RAD51 [Homo sapiens], CCDS 10062.1) | 16310-16312 |
| | MAMQMQLEANADTSVEEESFGPQPISRLEQCGINANDVKKLEEAGFHTVEAVAYAPKKELINIKGISEAK ADKILTESRSVARLECNSVILVYCTLRLSGSSDSPASASRVVGTTGGIETGSITEMFGEFRTGKTQICHT LAVTCQLPIDRGGGEGKAMYIDTEGTFRPERLLAVAERYGLSGSDVLDNVAYARAFNTDHQTQLLYQASA MMVESRYALLIVDSATALYRTDYSGRGELSARQMHLARFLRMLLRLADEFGVAVVITNQVVAQVDGAAMF AADPKKPIGGNIIAHASTTRLYLRKGRGETRICKIYDSPCLPEAEAMFAINADGVGDAKD (RAD51 [Homo sapiens], CCDS 53931.1) | |
| | MAMQMQLEANADTSVEEESFGPQPISRLEQCGINANDVKKLEEAGFHTVEAVAYAPKKELINIKGISEAK ADKILAEAAKLVPMGFTTATEFHQRRSEIIQITTGSKELDKLLQGGIETGSITEMFGEFRTGKTQICHTL AVTCQLPIDRGGGEGKAMYIDTEGTFRPERLLAVAERYGLSGSDVLDNVAYARAFNTDHQTQLLYQASAM MVESRYALLIVDSATALYRTDYSGRGELSARQMHLARFLRMLLRLADEIVSEERKRGNQNLQNLRLSLSS (CCDS 53932.1) | |

In the methods provided herein, the frequency of preferred repair outcomes generated using a Cas9 molecule described herein may be increased by modulating (*e*.*g*., stimulating or overexpressing) a component (*e*.*g*., exactly one component, or one or more components, *e.g.,* two or three components) of a gene conversion pathway, *e*.*g*., a BRCA1, BRCA2 and/or RAD51. The frequency of gene conversion resulting from a Cas9 nuclease (*e*.*g*., wild type Cas9 nuclease) induced-lesion in a target position of a target cell overexpressing a gene conversion pathway component may be increased at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, or more, as compared to the frequency of gene conversion resulting from a Cas9 nickase *(e.g.,* a Cas9 D10A nickase) induced-lesion in a target position in the absence of overexpression of a gene conversion pathway.

The frequency of gene conversion resulting from a Cas9 nuclease *(e.g.,* wild type Cas9 nuclease) induced-lesion in a target position of a target cell overexpressing a gene conversion pathway component may be increased at least 5% (*e*.*g*., at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 150%, at least about 200%, at least about 300%, at least about 400%, at least about 500%, at least about 600%, at least about 700%, at least about 800%, at least about 900%, or more, as compared to the frequency of gene conversion resulting from a Cas9 nickase (*e*.*g*., a Cas9 D10A nickase) induced-lesion in a target position in the absence of overexpression of a gene conversion pathway.

In the methods provided herein, the frequency of preferred repair outcomes generated using a Cas9 molecule described herein may be increased by modulating (*e*.*g*., stimulating or overexpressing) a component (*e*.*g*., exactly one component, or one or more components, *e.g.,* two or three components) of a gene conversion pathway, *e*.*g*., a BRCA1, BRCA2 and/or RAD51. The frequency of gene conversion resulting from a Cas9 nickase *(e.g.,* a Cas9 D10A nickase) induced-lesion in a target position of a target cell overexpressing a gene conversion pathway component may be increased at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, or more, as compared to the frequency of gene conversion resulting from a Cas9 nickase *(e.g.,* a Cas9 D10A nickase) induced-lesion in a target position in the absence of overexpression of a gene conversion pathway.

The frequency of gene conversion resulting from a Cas9 nickase *(e.g.,* a Cas9 D10A nickase) induced-lesion in a target position of a target cell overexpressing a gene conversion pathway component may be increased at least 5% (*e.g*., at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 150%, at least about 200%, at least about 300%, at least about 400%, at least about 500%, at least about 600%, at least about 700%, at least about 800%, at least about 900%, or more, as compared to the frequency of gene conversion resulting from a Cas9 nickase *(e.g.,* a Cas9 D10A nickase) induced-lesion in a target position in the absence of overexpression of a gene conversion pathway.

### V.2 NHEJ Approaches for Gene Targeting

In the methods provided herein, NHEJ-mediated deletion may be used to delete all or part of a target gene. As described herein, nuclease-induced NHEJ can also be used to remove (*e*.*g*., delete) sequences in a gene of interest.

While not wishing to be bound by theory, it is believed that
the genomic alterations associated with the methods described herein may rely on nuclease-induced NHEJ and the error-prone nature of the NHEJ repair pathway. NHEJ repairs a double-strand break in the DNA by joining together the two ends; however, generally, the original sequence is restored only if two compatible ends, exactly as they were formed by the double-strand break, are perfectly ligated. The DNA ends of the double-strand break are frequently the subject of enzymatic processing, resulting in the addition or removal of nucleotides, *e*.*g*., resection, at one or both strands, prior to rejoining of the ends. This results in the presence of insertion and/or deletion (indel) mutations in the DNA sequence at the site of the NHEJ repair. Two-thirds of these mutations typically alter the reading frame and, therefore, produce a non-functional protein. Additionally, mutations that maintain the reading frame, but which insert or delete a significant amount of sequence, can destroy functionality of the protein. This is locus dependent as mutations in critical functional domains are likely less tolerable than mutations in non-critical regions of the protein.

The indel mutations generated by NHEJ are unpredictable in nature; however, at a given break site certain indel sequences are favored and are over represented in the population, likely due to small regions of microhomology. The lengths of deletions can vary widely; most commonly in the 1-50 bp range, but they can easily reach greater than 100-200 bp. In some embodiments, the deletion is at least about 1, 2, 3, 4, 5, 6, 7, 8, 9, 10, 11, 12, 13, 14, 15, 16, 17, 18, 19, 20, 30, 40, 47, 50, 75, 100, 200, 300, 400, 500, 750, 1000, 2000, 3000, 4000, 5000, 6000, 7000, 8000, 9000, 10000, 15000, 20000, 25000, 30000, 40000, 50000, 60000, 70000, 80000, 90000, 100000, 200000, 300000, 400000, 500000, 600000, 700000, 800000, 900000, 1000000 or more nucleotides in length. Insertions tend to be shorter and often include short duplications of the sequence immediately surrounding the break site. However, it is possible to obtain large insertions, and in these cases, the inserted sequence has often been traced to other regions of the genome or to plasmid DNA present in the cells.

Because NHEJ is a mutagenic process, it can also be used to delete small sequence motifs as long as the generation of a specific final sequence is not required. If a double-strand break is targeted near to a short target sequence, the deletion mutations caused by the NHEJ repair often span, and therefore remove, the unwanted nucleotides. For the deletion of larger DNA segments, introducing two double-strand breaks, one on each side of the sequence, can result in NHEJ between the ends with removal of the entire intervening sequence. Both of these approaches can be used to delete specific DNA sequences; however, the error-prone nature of NHEJ may still produce indel mutations at the site of repair.

Both double-strand cleaving eaCas9 molecules and single strand, or nickase, eaCas9 molecules can be used in the methods and compositions described herein to generate NHEJ-mediated indels. NHEJ-mediated indels targeted to the gene, *e.g.,* a coding region, *e.g.,* an early coding region of a gene of interest can be used to knockout *(i.e.,* eliminate expression of) a gene of interest. For example, early coding region of a gene of interest includes sequence immediately following a transcription start site, within a first exon of the coding sequence, or within 500 bp of the transcription start site *(e.g.,* less than 500, 450, 400, 350, 300, 250, 200, 150, 100 or 50 bp).

### Placement of double-strand or single-strand breaks relative to the target position

When a gRNA and Cas9 nuclease generate a double-strand break for the purpose of inducing NHEJ-mediated indels, a gRNA, *e.g.,* a unimolecular (or chimeric) or modular gRNA molecule, may be configured to position one double-strand break in close proximity to a nucleotide of the target position. The cleavage site may be between 0-30 bp away from the target position (*e*.*g*., less than 30, 25, 20, 15, 10, 9, 8, 7, 6, 5, 4, 3, 2 or 1 bp from the target position).

When two gRNAs complexing with Cas9 nickases induce two single-strand breaks for the purpose of inducing NHEJ-mediated indels, two gRNAs, *e*.*g*., independently, unimolecular (or chimeric) or modular gRNA, may be configured to position two single-strand breaks to provide for NHEJ repair a nucleotide of the target position. The gRNAs may be configured to position cuts at the same position, or within a few nucleotides of one another, on different strands, essentially mimicking a double-strand break. The closer nick may be between 0-30 bp away from the target position (*e*.*g*., less than 30, 25, 20, 15, 10, 9, 8, 7, 6, 5, 4, 3, 2, or 1 bp from the target position), and the two nicks are within 25-55 bp of each other (*e*.*g*., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50, 45 to 50, 35 to 45, or 40 to 45 bp) and no more than 100 bp away from each other *(e.g.,* no more than 90, 80, 70, 60, 50, 40, 30, 20, or 10 bp). The gRNAs may be configured to place a single-strand break on either side of a nucleotide of the target position.

Both double-strand cleaving eaCas9 molecules and single strand, or nickase, eaCas9 molecules can be used in the methods and compositions described herein to generate breaks both sides of a target position. Double-strand or paired single-strand breaks may be generated on both sides of a target position to remove the nucleic acid sequence between the two cuts *(e.g.,* the region between the two breaks in deleted). Two gRNAs, *e.g.,* independently, unimolecular (or chimeric) or modular gRNA, may be configured to position a double-strand break on both sides of a target position. Three gRNAs, *e*.*g*., independently, unimolecular (or chimeric) or modular gRNA, may be configured to position a double-strand break (*i.e.,* one gRNA complexes with a Cas9 nuclease) and two single-strand breaks or paired single-strand breaks (*i.e*., two gRNAs complex with Cas9 nickases) on either side of the target position. Four gRNAs, *e.g.,* independently, unimolecular (or chimeric) or modular gRNA, may be configured to generate two pairs of single-strand breaks *(i.e.,* two pairs of two gRNAs complex with Cas9 nickases) on either side of the target position. The double-strand break(s) or the closer of the two single-strand nicks in a pair will ideally be within 0-500 bp of the target position (*e*.*g*., no more than 450, 400, 350, 300, 250, 200, 150, 100, 50, or 25 bp from the target position). When nickases are used, the two nicks in a pair are within 25-55 bp of each other (*e.g*., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50, 45 to 50, 35 to 45, or 40 to 45 bp) and no more than 100 bp away from each other *(e.g.,* no more than 90, 80, 70, 60, 50, 40, 30, 20, or 10 bp).

### V.3 Targeted Knockdown

Unlike CRISPR/Cas-mediated gene knockout, which permanently eliminates expression by mutating the gene at the DNA level, CRISPR/Cas knockdown allows for temporary reduction of gene expression through the use of artificial transcription factors. Mutating key residues in both DNA cleavage domains of the Cas9 molecule *(e.g.,* the D10A and H840A mutations) results in the generation of a catalytically inactive Cas9 (referred to herein as "eiCas9", which is also known as dead Cas9 or dCas9) molecule. An eiCas9 complexes with a gRNA and localizes to the DNA sequence specified by that gRNA's targeting domain, however, it does not cleave the target DNA. Fusion of the eiCas9 to an effector domain, *e.g.,* a transcription repression domain, enables recruitment of the effector to any DNA site specified by the gRNA. Although an eiCas9 itself can block transcription when recruited to early regions in the coding sequence, more robust repression can be achieved by fusing a transcriptional repression domain (for example KRAB, SID or ERD) to the eiCas9, referred to herein as a "Cas9-repressor", and recruiting the transcriptional repression domain to the target knockdown position, *e*.*g*., within 1000 bp of sequence 3' of the start codon or within 500 bp of a promoter region 5' of the start codon of a gene. It is likely that targeting DNAse I hypersensitive sites (DHSs) of the promoter may yield more efficient gene repression or activation because these regions are more likely to be accessible to the eiCas9 and are also more likely to harbor sites for endogenous transcription factors. Especially for gene repression, it is contemplated herein that blocking the binding site of an endogenous transcription factor would aid in downregulating gene expression. One or more eiCas9 molecules may be used to block binding of one or more endogenous transcription factors. An eiCas9 molecule can be fused to a chromatin modifying protein. Altering chromatin status can result in decreased expression of the target gene. One or more eiCas9 molecules fused to one or more chromatin modifying proteins may be used to alter chromatin status.

A gRNA molecule can be targeted to a known transcription response elements (*e*.*g*., promoters, enhancers, etc.), a known upstream activating sequences (UAS), and/or sequences of unknown or known function that are suspected of being able to control expression of the target DNA.

CRISPR/Cas-mediated gene knockdown can be used to reduce expression of an unwanted allele or transcript. Contemplated herein are scenarios wherein permanent destruction of the gene is not ideal. In these scenarios, site-specific repression may be used to temporarily reduce or eliminate expression. It is also contemplated herein that the off-target effects of a Cas9-repressor may be less severe than those of a Cas9-nuclease as a nuclease can cleave any DNA sequence and cause mutations whereas a Cas9-repressor may only have an effect if it targets the promoter region of an actively transcribed gene. However, while nuclease-mediated knockout is permanent, repression may only persist as long as the Cas9-repressor is present in the cells. Once the repressor is no longer present, it is likely that endogenous transcription factors and gene regulatory elements would restore expression to its natural state.

### V.4 Single-Strand Annealing

Single-strand annealing (SSA) is another DNA repair process that repairs a double-strand break between two repeat sequences present in a target nucleic acid. Repeat sequences utilized by the SSA pathway are generally greater than 30 nucleotides in length. Resection at the break ends occurs to reveal repeat sequences on both strands of the target nucleic acid. After resection, single-strand overhangs containing the repeat sequences are coated with RPA protein to prevent the repeats sequences from inappropriate annealing, *e*.*g.*, to themselves. RAD52 binds to and each of the repeat sequences on the overhangs and aligns the sequences to enable the annealing of the complementary repeat sequences. After annealing, the single-strand flaps of the overhangs are cleaved. New DNA synthesis fills in any gaps, and ligation restores the DNA duplex. As a result of the processing, the DNA sequence between the two repeats is deleted. The length of the deletion can depend on many factors including the location of the two repeats utilized, and the pathway or processivity of the resection.

In contrast to HDR pathways, SSA does not require a template nucleic acid to alter or correct a target nucleic acid sequence. Instead, the complementary repeat sequence is utilized.

### V. 5 Other DNA Repair Pathways

### SSBR (single-strand break repair)

Single-stranded breaks (SSB) in the genome are repaired by the SSBR pathway, which is a distinct mechanism from the DSB repair mechanisms discussed above. The SSBR pathway has four major stages: SSB detection, DNA end processing, DNA gap filling, and DNA ligation. A more detailed explanation is given in Caldecott 2008, and a summary is given here.

In the first stage, when a SSB forms, PARP1 and/or PARP2 recognize the break and recruit repair machinery. The binding and activity of PARP1 at DNA breaks is transient and it seems to accelerate SSBr by promoting the focal accumulation or stability of SSBr protein complexes at the lesion. Arguably the most important of these SSBr proteins is XRCC1, which functions as a molecular scaffold that interacts with, stabilizes, and stimulates multiple enzymatic components of the SSBr process including the protein responsible for cleaning the DNA 3' and 5' ends. For instance, XRCC1 interacts with several proteins (DNA polymerase beta, PNK, and three nucleases, APE1, APTX, and APLF) that promote end processing. APE1 has endonuclease activity. APLF exhibits endonuclease and 3' to 5' exonuclease activities. APTX has endonuclease and 3' to 5' exonuclease activity.

This end processing is an important stage of SSBR since the 3'- and/or 5'-termini of most, if not all, SSBs are damaged. End processing generally involves restoring a damaged 3'-end to a hydroxylated state and and/or a damaged 5' end to a phosphate moiety, so that the ends become ligation-competent. Enzymes that can process damaged 3' termini include PNKP, APE1, and TDP1. Enzymes that can process damaged 5' termini include PNKP, DNA polymerase beta, and APTX. LIG3 (DNA ligase III) can also participate in end processing. Once the ends are cleaned, gap filling can occur.

At the DNA gap filling stage, the proteins typically present are PARP1, DNA polymerase beta, XRCC1, FEN1 (flap endonuclease 1), DNA polymerase delta/epsilon, PCNA, and LIG1. There are two ways of gap filling, the short patch repair and the long patch repair. Short patch repair involves the insertion of a single nucleotide that is missing. At some SSBs, "gap filling" might continue displacing two or more nucleotides (displacement of up to 12 bases have been reported). FEN1 is an endonuclease that removes the displaced 5'-residues. Multiple DNA polymerases, including Polβ, are involved in the repair of SSBs, with the choice of DNA polymerase influenced by the source and type of SSB.

In the fourth stage, a DNA ligase such as LIG1 (Ligase I) or LIG3 (Ligase III) catalyzes joining of the ends. Short patch repair uses Ligase III and long patch repair uses Ligase I.

Sometimes, SSBR is replication-coupled. This pathway can involve one or more of CtIP, MRN, ERCC1, and FEN1. Additional factors that may promote SSBR include: aPARP, PARP1, PARP2, PARG, XRCC1, DNA polymerase β, DNA polymerase delta, DNA polymerase epsilon, PCNA, LIG1, PNK, PNKP, APE1, APTX, APLF, TDP1, LIG3, FEN1, CtIP, MRN, and ERCC1.

### MMR (mismatch repair)

Cells contain three excision repair pathways: MMR, BER, and NER. The excision repair pathways have a common feature in that they typically recognize a lesion on one strand of the DNA, then exo/endonucleases remove the lesion and leave a 1-30 nucleotide gap that is sub-sequentially filled in by DNA polymerase and finally sealed with ligase. A more complete picture is given in Li 2008, and a summary is provided here.

Mismatch repair (MMR) operates on mispaired DNA bases.

The MSH2/6 or MSH2/3 complexes both have ATPase activity that plays an important role in mismatch recognition and the initiation of repair. MSH2/6 preferentially recognizes base-base mismatches and identifies mispairs of 1 or 2 nucleotides, while MSH2/3 preferentially recognizes larger ID mispairs.

hMLH1 heterodimerizes with hPMS2 to form hMutLα which possesses an ATPase activity and is important for multiple steps of MMR. It possesses a PCNA/replication factor C (RFC)-dependent endonuclease activity which plays an important role in 3' nick-directed MMR involving EXO1 (EXO1 is a participant in both HR and MMR). It regulates termination of mismatch-provoked excision. Ligase I is the relevant ligase for this pathway. Additional factors that may promote MMR include: EXO1, MSH2, MSH3, MSH6, MLH1, PMS2, MLH3, DNA Pol delta, RPA, HMGB1, RFC, and DNA ligase I.

### Base excision repair (BER)

The base excision repair (BER) pathway is active throughout the cell cycle; it is responsible primarily for removing small, non-helix-distorting base lesions from the genome. In contrast, the related Nucleotide Excision Repair pathway (discussed in the next section) repairs bulky helix-distorting lesions. A more detailed explanation is given in Caldecott 2008, and a summary is given here.

Upon DNA base damage, base excision repair (BER) is initiated and the process can be simplified into five major steps: (a) removal of the damaged DNA base; (b) incision of the subsequent a basic site; (c) clean-up of the DNA ends; (d) insertion of the desired nucleotide into the repair gap; and (e) ligation of the remaining nick in the DNA backbone. These last steps are similar to the SSBR.

In the first step, a damage-specific DNA glycosylase excises the damaged base through cleavage of the N-glycosidic bond linking the base to the sugar phosphate backbone. Then AP endonuclease-1 (APE1) or bifunctional DNA glycosylases with an associated lyase activity incises the phosphodiester backbone to create a DNA single-strand break (SSB). The third step of BER involves cleaning-up of the DNA ends. The fourth step in BER is conducted by Pol β that adds a new complementary nucleotide into the repair gap and in the final step XRCC1/Ligase III seals the remaining nick in the DNA backbone. This completes the short-patch BER pathway in which the majority (∼80%) of damaged DNA bases are repaired. However, if the 5'-ends in step 3 are resistant to end processing activity, following one nucleotide insertion by Pol β there is then a polymerase switch to the replicative DNA polymerases, Pol δ/ε, which then add ∼2-8 more nucleotides into the DNA repair gap. This creates a 5'-flap structure, which is recognized and excised by flap endonuclease-1 (FEN-1) in association with the processivity factor proliferating cell nuclear antigen (PCNA). DNA ligase I then seals the remaining nick in the DNA backbone and completes long-patch BER. Additional factors that may promote the BER pathway include: DNA glycosylase, APE1, Polβ, Pol delta, Pol epsilon, XRCC1, Ligase III, FEN-1, PCNA, RECQL4, WRN, MYH, PNKP, and APTX.

### Nucleotide excision repair (NER)

Nucleotide excision repair (NER) is an important excision mechanism that removes bulky helix-distorting lesions from DNA. Additional details about NER are given in Marteijn *et al.* 2014, and a summary is given here. NER a broad pathway encompassing two smaller pathways: global genomic NER (GG-NER) and transcription coupled repair NER (TC-NER). GG-NER and TC-NER use different factors for recognizing DNA damage. However, they utilize the same machinery for lesion incision, repair, and ligation.

Once damage is recognized, the cell removes a short single-stranded DNA segment that contains the lesion. Endonucleases XPF/ERCC1 and XPG (encoded by ERCC5) remove the lesion by cutting the damaged strand on either side of the lesion, resulting in a single-strand gap of 22-30 nucleotides. Next, the cell performs DNA gap filling synthesis and ligation. Involved in this process are: PCNA, RFC, DNA Pol δ, DNA Pol ε or DNA Pol κ, and DNA ligase I or XRCC1/Ligase III. Replicating cells tend to use DNA pol ε and DNA ligase I, while non-replicating cells tend to use DNA Pol δ, DNA Pol κ, and the XRCC1/ Ligase III complex to perform the ligation step.

NER can involve the following factors: XPA-G, POLH, XPF, ERCC1, XPA-G, and LIG1. Transcription-coupled NER (TC-NER) can involve the following factors: CSA, CSB, XPB, XPD, XPG, ERCC1, and TTDA. Additional factors that may promote the NER repair pathway include XPA-G, POLH, XPF, ERCC1, XPA-G, LIG1, CSA, CSB, XPA, XPB, XPC, XPD, XPF, XPG, TTDA, UVSSA, USP7, CETN2, RAD23B, UV-DDB, CAK subcomplex, RPA, and PCNA.

### Interstrand Crosslink (ICL)

A dedicated pathway called the ICL repair pathway repairs interstrand crosslinks. Interstrand crosslinks, or covalent crosslinks between bases in different DNA strand, can occur during replication or transcription. ICL repair involves the coordination of multiple repair processes, in particular, nucleolytic activity, translesion synthesis (TLS), and HDR. Nucleases are recruited to excise the ICL on either side of the crosslinked bases, while TLS and HDR are coordinated to repair the cut strands. ICL repair can involve the following factors: endonucleases, *e*.*g*., XPF and RAD51C, endonucleases such as RAD51, translesion polymerases, *e*.*g*., DNA polymerase zeta and Rev1, and the Fanconi anemia (FA) proteins, *e.g.,* FancJ.

### Other pathways

Several other DNA repair pathways exist in mammals.

Translesion synthesis (TLS) is a pathway for repairing a single stranded break left after a defective replication event and involves translesion polymerases, *e*.*g*., DNA pol ζ and Rev1.

Error-free postreplication repair (PRR) is another pathway for repairing a single stranded break left after a defective replication event.

### V.6 Examples of gRNAs in Genome Editing Methods

gRNA molecules as described herein can be used with Cas9 molecules that generate a double-strand break or a single-strand break to alter the sequence of a target nucleic acid, *e.g.,* a target position or target genetic signature. gRNA molecules useful in these methods are described below.

The gRNA, *e.g.,* a chimeric gRNA, may be configured such that it comprises one or more of the following properties;
a) it can position, *e*.*g*., when targeting a Cas9 molecule that makes double-strand breaks, a double-strand break (i) within 50, 100, 150, 200, 250, 300, 350, 400, 450, or 500 nucleotides of a target position, or (ii) sufficiently close that the target position is within the region of end resection;
b) it has a targeting domain of at least 16 nucleotides, *e*.*g*., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
(c)(i) the proximal and tail domain, when taken together, comprise at least 15, 18, 20, 25, 30, 31, 35, 40, 45, 49, 50, or 53 nucleotides, *e*.*g*., at least 15, 18, 20, 25, 30, 31, 35, 40, 45, 49, 50, or 53 nucleotides from a naturally occurring *S. pyogenes* or *S. aureus,* tail and proximal domain, or a sequence that differs by no more than 1, 2, 3, 4, 5, 6, 7, 8, 9, or 10 nucleotides therefrom;
(c)(ii) there are at least 15, 18, 20, 25, 30, 31, 35, 40, 45, 49, 50, or 53 nucleotides 3' to the last nucleotide of the second complementarity domain, *e.g.,* at least 15, 18, 20, 25, 30, 31, 35, 40, 45, 49, 50, or 53 nucleotides from the corresponding sequence of a naturally occurring *S. pyogenes* or *S. aureus* gRNA, or a sequence that differs by no more than 1, 2, 3, 4, 5, 6, 7, 8, 9, or 10 nucleotides therefrom;
(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, *e.g.,* at least 16, 19, 21, 26, 31, 32, 36, 41, 46, 50, 51, or 54 nucleotides from the corresponding sequence of a naturally occurring *S. pyogenes* or *S. aureus* gRNA, or a sequence that differs by no more than 1, 2, 3, 4, 5, 6, 7, 8, 9, or 10 nucleotides therefrom;
(c)(iv) the tail domain is at least 10, 15, 20, 25, 30, 35 or 40 nucleotides in length, *e.g.,* it comprises at least 10, 15, 20, 25, 30, 35 or 40 nucleotides from a naturally occurring *S*. *pyogenes* or *S. aureus* tail domain, or a sequence that differs by no more than 1, 2, 3, 4, 5, 6, 7, 8, 9, or 10 nucleotides therefrom; or
(c)(v) the tail domain comprises 15, 20, 25, 30, 35, 40 nucleotides or all of the corresponding portions of a naturally occurring tail domain, *e.g.,* a naturally occurring *S*. *pyogenes* or *S. aureus* tail domain.

The gRNA may be configured such that it comprises properties a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(ii), and c(i); a(i), b(ii), and c(ii); a(i), b(iii), and c(i); a(i), b(iii), and c(ii); a(i), b(iv), and c(i); a(i), b(iv), and c(ii); a(i), b(v), and c(i); a(i), b(v), and c(ii); a(i), b(vi), and c(i); a(i), b(vi), and c(ii); a(i), b(vii), and c(i); a(i), b(vii), and c(ii); a(i), b(viii), and c(i); a(i), b(viii), and c(ii); a(i), b(ix), and c(i); a(i), b(ix), and c(ii); a(i), b(x), and c(i); a(i), b(x), and c(ii); a(i), b(xi), or c(i); a(i), b(xi), and c(ii).

The gRNA, *e.g.,* a chimeric gRNA, may be configured such that it comprises one or more of the following properties:
(a) one or both of the gRNAs can position, *e*.*g*., when targeting a Cas9 molecule that makes single-strand breaks, a single-strand break within (i) 50, 100, 150, 200, 250, 300, 350, 400, 450, or 500 nucleotides of a target position, or (ii) sufficiently close that the target position is within the region of end resection;
(b) one or both have a targeting domain of at least 16 nucleotides, *e*.*g*., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
(c)(i) the proximal and tail domain, when taken together, comprise at least 15, 18, 20, 25, 30, 31, 35, 40, 45, 49, 50, or 53 nucleotides, *e*.*g*., at least 15, 18, 20, 25, 30, 31, 35, 40, 45, 49, 50, or 53 nucleotides from a naturally occurring *S. pyogenes* or *S. aureus* tail and proximal domain, or a sequence that differs by no more than 1, 2, 3, 4, 5, 6, 7, 8, 9, or 10 nucleotides therefrom;
(c)(ii) there are at least 15, 18, 20, 25, 30, 31, 35, 40, 45, 49, 50, or 53 nucleotides 3' to the last nucleotide of the second complementarity domain, *e.g.,* at least 15, 18, 20, 25, 30, 31, 35, 40, 45, 49, 50, or 53 nucleotides from the corresponding sequence of a naturally occurring *S. pyogenes,* or *S. aureus* gRNA, or a sequence that differs by no more than 1, 2, 3, 4, 5, 6, 7, 8, 9, or 10 nucleotides therefrom;
(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, *e.g.,* at least 16, 19, 21, 26, 31, 32, 36, 41, 46, 50, 51, or 54 nucleotides from the corresponding sequence of a naturally occurring *S. pyogenes* or *S. aureus* gRNA, or a sequence that differs by no more than 1, 2, 3, 4, 5, 6, 7, 8, 9, or 10 nucleotides therefrom;
(c)(iv) the tail domain is at least 10, 15, 20, 25, 30, 35 or 40 nucleotides in length, *e.g.,* it comprises at least 10, 15, 20, 25, 30, 35 or 40 nucleotides from a naturally occurring *S*. *pyogenes,* or *S. aureus* tail domain, or a sequence that differs by no more than 1, 2, 3, 4, 5, 6, 7, 8, 9, or 10 nucleotides therefrom; or
(c)(v) the tail domain comprises 15, 20, 25, 30, 35, 40 nucleotides or all of the corresponding portions of a naturally occurring tail domain, *e.g.,* a naturally occurring *S*. *pyogenes* or *S. aureus* tail domain.

The gRNA may be configured such that it comprises properties: a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(ii), and c(i); a(i), b(ii), and c(ii); a(i), b(iii), and c(i); a(i), b(iii), and c(ii); a(i), b(iv), and c(i); a(i), b(iv), and c(ii); a(i), b(v), and c(i); a(i), b(v), and c(ii); a(i), b(vi), and c(i); a(i), b(vi), and c(ii); a(i), b(vii), and c(i); a(i), b(vii), and c(ii); a(i), b(viii), and c(i); a(i), b(viii), and c(ii); a(i), b(ix), and c(i); a(i), b(ix), and c(ii); a(i), b(x), and c(i); a(i), b(x), and c(ii); a(i), b(xi), and c(i); or a(i), b(xi), and c(ii).

The gRNA may be used with a Cas9 nickase molecule having HNH activity, *e.g*., a Cas9 molecule having the RuvC activity inactivated, *e.g*., a Cas9 molecule having a mutation at D10, *e.g.,* the D10A mutation.

The gRNA may be used with a Cas9 nickase molecule having RuvC activity, *e.g*., a Cas9 molecule having the HNH activity inactivated, *e.g*., a Cas9 molecule having a mutation at 840, *e.g.,* the H840A.

The gRNAs may be used with a Cas9 nickase molecule having RuvC activity, *e.g*., a Cas9 molecule having the HNH activity inactivated, *e.g.*, a Cas9 molecule having a mutation at N863, *e.g.,* the N863A mutation.

A pair of gRNAs, *e.g.,* a pair of chimeric gRNAs, comprising a first and a second gRNA, may be configured such that they comprises one or more of the following properties:
a) one or both of the gRNA molecules can position, *e.g*., when targeting a Cas9 molecule that makes single-strand breaks, a single-strand break within (i) 50, 100, 150, 200, 250, 300, 350, 400, 450, or 500 nucleotides of a target position, or (ii) sufficiently close that the target position is within the region of end resection;
b) one or both have a targeting domain of at least 16 nucleotides, *e.g*., a targeting domain of (i) 16, (ii), 17, (iii) 18, (iv) 19, (v) 20, (vi) 21, (vii) 22, (viii) 23, (ix) 24, (x) 25, or (xi) 26 nucleotides;
(c)(i) the proximal and tail domain, when taken together, comprise at least 15, 18, 20, 25, 30, 31, 35, 40, 45, 49, 50, or 53 nucleotides, *e.g*., at least 15, 18, 20, 25, 30, 31, 35, 40, 45, 49, 50, or 53 nucleotides from a naturally occurring *S. pyogenes* or *S. aureus* tail and proximal domain, or a sequence that differs by no more than 1, 2, 3, 4, 5, 6, 7, 8, 9, or 10 nucleotides therefrom;
(c)(ii) there are at least 15, 18, 20, 25, 30, 31, 35, 40, 45, 49, 50, or 53 nucleotides 3' to the last nucleotide of the second complementarity domain, *e.g.,* at least 15, 18, 20, 25, 30, 31, 35, 40, 45, 49, 50, or 53 nucleotides from the corresponding sequence of a naturally occurring *S. pyogenes* or *S. aureus* gRNA, or a sequence that differs by no more than 1, 2, 3, 4, 5, 6, 7, 8, 9, or 10 nucleotides therefrom;
(c)(iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, *e.g.,* at least 16, 19, 21, 26, 31, 32, 36, 41, 46, 50, 51, or 54 nucleotides from the corresponding sequence of a naturally occurring *S. pyogenes* or *S. aureus* gRNA, or a sequence that differs by no more than 1, 2, 3, 4, 5, 6, 7, 8, 9, or 10 nucleotides therefrom;
(c)(iv) the tail domain is at least 10, 15, 20, 25, 30, 35 or 40 nucleotides in length, *e.g.,* it comprises at least 10, 15, 20, 25, 30, 35 or 40 nucleotides from a naturally occurring *S*. *pyogenes* or *S. aureus* tail domain; or, or a sequence that differs by no more than 1, 2, 3, 4, 5, 6, 7, 8, 9, or 10 nucleotides therefrom; or
(c)(v) the tail domain comprises 15, 20, 25, 30, 35, or 40 nucleotides or all of the corresponding portions of a naturally occurring tail domain, *e.g.,* a naturally occurring *S*. *pyogenes* or *S. aureus* tail domain;
(d) the gRNAs are configured such that, when hybridized to target nucleic acid, they are separated by 0-50, 0-100, 0-200, at least 10, at least 20, at least 30 or at least 50 nucleotides;
(e) the breaks made by the first gRNA and second gRNA are on different strands; and
(f) the PAMs are facing outwards.

One or both of the gRNAs may be configured such that it
comprises properties a and b(i); a and b(ii); a and b(iii); a and b(iv); a and b(v); a and b(vi); a and b(vii); a and b(viii); a and b(ix); a and b(x); a and b(xi); a and c; a, b, and c; a(i), b(i), and c(i); a(i), b(i), and c(ii); a(i), b(i), c, and d; a(i), b(i), c, and e; a(i), b(i), c, d, and e; a(i), b(ii), and c(i); a(i), b(ii), and c(ii); a(i), b(ii), c, and d; a(i), b(ii), c, and e; a(i), b(ii), c, d, and e; a(i), b(iii), and c(i); a(i), b(iii), and c(ii); a(i), b(iii), c, and d; a(i), b(iii), c, and e; a(i), b(iii), c, d, and e; a(i), b(iv), and c(i); a(i), b(iv), and c(ii); a(i), b(iv), c, and d; a(i), b(iv), c, and e; a(i), b(iv), c, d, and e; a(i), b(v), and c(i); a(i), b(v), and c(ii); a(i), b(v), c, and d; a(i), b(v), c, and e; a(i), b(v), c, d, and e; a(i), b(vi), and c(i); a(i), b(vi), and c(ii); a(i), b(vi), c, and d; a(i), b(vi), c, and e; a(i), b(vi), c, d, and e; a(i), b(vii), and c(i); a(i), b(vii), and c(ii); a(i), b(vii), c, and d; a(i), b(vii), c, and e; a(i), b(vii), c, d, and e; a(i), b(viii), and c(i); a(i), b(viii), and c(ii); a(i), b(viii), c, and d; a(i), b(viii), c, and e; a(i), b(viii), c, d, and e; a(i), b(ix), and c(i); a(i), b(ix), and c(ii); a(i), b(ix), c, and d; a(i), b(ix), c, and e; a(i), b(ix), c, d, and e; a(i), b(x), and c(i); a(i), b(x), and c(ii); a(i), b(x), c, and d; a(i), b(x), c, and e; a(i), b(x), c, d, and e; a(i), b(xi), and c(i); a(i), b(xi), and c(ii); a(i), b(xi), c, and d; a(i), b(xi), c, and e; or a(i), b(xi), c, d, and e.

The gRNAs may be used with a Cas9 nickase molecule having HNH activity, *e.g*., a Cas9 molecule having the RuvC activity inactivated, *e.g.*, a Cas9 molecule having a mutation at D10, *e.g.,* the D10A mutation.

The gRNAs may be used with a Cas9 nickase molecule having RuvC activity, *e.g*., a Cas9 molecule having the HNH activity inactivated, *e.g*., a Cas9 molecule having a mutation at H840, *e.g.,* the H840A mutation.

The gRNAs may be used with a Cas9 nickase molecule having RuvC activity, *e.g*., a Cas9 molecule having the HNH activity inactivated, *e.g*., a Cas9 molecule having a mutation at N863, *e.g.,* the N863A mutation.

### VI. Target Cells

Cas9 molecules and gRNA molecules, *e.g.,* a Cas9 molecule/gRNA molecule complex, can be used to manipulate a cell, *e.g.,* to edit a target nucleic acid, in a wide variety of cells. Additional details on types of cells that can be manipulated may be found in the section entitled "VIIA. TARGETS: CELLS" of PCT Publication WO 2015/048577.

A cell may be manipulated by editing (*e.g*., introducing a mutation in) a target gene *(e.g.,* a *HBB* gene) as described herein. A cell, or a population of cells, may be manipulated by editing one or more non-coding sequences, *e.g*., an alteration in an intron or in a 5' or 3' non-translated or non-transcribed region. A cell, or a population of cells, may be manipulated by editing the sequence of a control element, *e.g.,* a promoter, enhancer, or a cis-acting or trans-acting control element. A cell, or a population of cells, may be manipulated by editing one or more coding sequences, *e.g*., an alteration in an exon. A cell, or a population of cells, may be manipulated *in vitro.* A cell, or a population of cells, may be manipulated *ex vivo.* A cell, or a population of cells, may be manipulated *in vivo.* The expression of one or more target genes *(e.g.,* one or more target genes described herein) may be modulated, *e.g., in vivo.* The expression of one or more target genes *(e.g.,* one or more target genes described herein) may be modulated, *e.g., ex vivo.* The expression of one or more target genes *(e.g.,* one or more target genes described herein) may be modulated, *e.g., in vitro.*

A cell, or a population of cells, may be manipulated by editing (*e.g*., inducing a mutation in) the *HBB* target gene, *e.g.,* as described herein. The expression of the *HBB* target gene may be modulated, *e.g., in vivo.* The expression of the *HBB* target gene may be modulated, *e.g., ex vivo.*

The Cas9 and gRNA molecules described herein can be delivered to a target cell. The target cell may be an erythroid cell, *e.g.,* an erythroblast. Erythroid cells may be preferentially targeted, *e.g.,* at least about 90%, 95%, 96%, 97%, 98%, 99%, or 100% of the targeted cells are erythroid cells. For example, in the case of *in vivo* delivery, erythroid cells are preferentially targeted, and if cells are treated *ex vivo* and returned to the subject, erythroid cells are preferentially modified. The target cell may be a circulating blood cell, *e.g.,* a reticulocyte, megakaryocyte erythroid progenitor (MEP) cell, myeloid progenitor cell (CMP/GMP), lymphoid progenitor (LP) cell, hematopoietic stem/progenitor cell (HSC), or endothelial cell (EC). The target cell may be a bone marrow cell *(e.g.,* a reticulocyte, an erythroid cell (*e.g*., erythroblast), an MEP cell, myeloid progenitor cell (CMP/GMP), LP cell, erythroid progenitor (EP) cell, HSC, multipotent progenitor (MPP) cell, endothelial cell (EC), hemogenic endothelial (HE) cell, or mesenchymal stem cell). The target cell may be a myeloid progenitor cell *(e.g.,* a common myeloid progenitor (CMP) cell or granulocyte macrophage progenitor (GMP) cell). The target cell may be a lymphoid progenitor cell, *e.g.,* a common lymphoid progenitor (CLP) cell. The target cell may be an erythroid progenitor cell *(e.g.,* an MEP cell). The target cell may be a hematopoietic stem/progenitor cell *(e.g.,* a long term HSC (LT-HSC), short term HSC (ST-HSC), MPP cell, or lineage restricted progenitor (LRP) cell). The target cell may be a CD34⁺ cell, CD34⁺CD90⁺ cell, CD34⁺CD38⁻ cell, CD34⁺CD90⁺CD49⁺CD38⁻CD45RA⁻ cell, CD105⁺ cell, CD31⁺, or CD133⁺ cell, or a CD34⁺CD90⁺ CD133⁺ cell. The target cell may be an umbilical cord blood CD34⁺ HSPC, umbilical cord venous endothelial cell, umbilical cord arterial endothelial cell, amniotic fluid CD34⁺ cell, amniotic fluid endothelial cell, placental endothelial cell, or placental hematopoietic CD34⁺ cell. The target cell may be a mobilized peripheral blood hematopoietic CD34⁺ cell (after the patient is treated with a mobilization agent, *e.g.,* G-CSF or Plerixafor). The target cell may be a peripheral blood endothelial cell.

A target cell may be manipulated *ex vivo* by editing *(e.g.,* inducing a mutation in) the *HBB* gene and/or modulating the expression of the *HBB* target gene, then the target cell is administered to the subject. Sources of target cells for *ex vivo* manipulation may include, for example, the subject's blood, cord blood, or marrow. Other sources of target cells for *ex vivo* manipulation may include, for example, heterologous donor blood, cord blood, or bone marrow.

An erythrocyte may be removed from a subject, manipulated *ex vivo* as described above, and the erythrocyte is returned to the subject. A hematopoietic stem cell may be removed from a subject, manipulated *ex vivo* as described above, and the hematopoietic stem cell is returned to the subject. An erythroid progenitor cell may be removed from a subject, manipulated *ex vivo* as described above, and the erythroid progenitor cell is returned to the subject. A myeloid progenitor cell may be removed from a subject, manipulated *ex vivo* as described above, and the myeloid progenitor cell is returned to the subject. A hematopoietic stem/progenitor cell (HSC) may be removed from a subject, manipulated *ex vivo* as described above, and returned to the subject. A CD34⁺HSC may be removed from a subject, manipulated *ex vivo* as described above, and returned to the subject.

Modified HSCs generated *ex vivo* may be administered to a subject without myeloablative pre-conditioning. The modified HSCs may be administered after mild myeloblative conditioning such that, followed engraftment, some of the hematopoietic cells are derived from the modified HSCs. The modified HSCs may be administered after full myeloblation such that, following engraftment, 100% of the hematopoietic cells are derived from the modified HSCs.

A suitable cell can also include a stem cell such as, by way of example, an embryonic stem cell, induced pluripotent stem cell, hematopoietic stem cell, or hemogenic endothelial (HE) cell (precursor to both hematopoietic stem cells and endothelial cells). The cell may be an induced pluripotent stem (iPS) cell or a cell derived from an iPS cell, *e.g.,* an iPS cell generated from the subject, modified using methods disclosed herein and differentiated into a clinically relevant cell such as *e.g.,* an erythrocyte. AAV may be used to transduce the target cells, *e.g.,* the target cells described herein.

Cells produced by the methods described herein may be used immediately. Alternatively, the cells may be frozen *(e.g.,* in liquid nitrogen) and stored for later use. The cells will usually be frozen in 10% dimethylsulfoxide (DMSO), 50% serum, 40% buffered medium, or some other such solution as is commonly used in the art to preserve cells at such freezing temperature and thawed in such a manner as commonly known in the art for thawing frozen cultured cells. Cells may also be thermostabilized for prolonged storage at 4°C.

### VII. Delivery, Formulations and Routes of Administration

The components, *e.g.,* a Cas9 molecule and gRNA molecule *(e.g.,* a Cas9 molecule/gRNA molecule complex), and a donor template nucleic acid, or all three, can be delivered, formulated, or administered, in a variety of forms, see, *e.g.,* **Tables 6-7.** In certain embodiments, one Cas9 molecule and two or more *(e.g.,* 2, 3, 4, or more) different gRNA molecules are delivered, *e.g*., by an AAV vector. The sequence encoding the Cas9 molecule and the sequence(s) encoding the two or more *(e.g.,* 2, 3, 4, or more) different gRNA molecules may be present on the same nucleic acid molecule, *e.g.,* an AAV vector. When a Cas9 or gRNA component is delivered encoded in DNA, the DNA will typically include a control region, *e*.*g*., comprising a promoter, to effect expression. Useful promoters for Cas9 molecule sequences include CMV, SFFV, EFS, EF-1a, PGK, CAG, and CBH promoters, or a blood cell specific promoter. The promoter may be a constitutive promoter. The promoter may be a tissue specific promoter. Useful promoters for gRNAs include T7, H1, EF-1a, U6, U1, and tRNA promoters. Promoters with similar or dissimilar strengths can be selected to tune the expression of components. Sequences encoding a Cas9 molecule can comprise a nuclear localization signal (NLS), *e.g.,* an SV40 NLS. The sequence encoding a Cas9 molecule may comprise at least two nuclear localization signals. A promoter for a Cas9 molecule or a gRNA molecule can be, independently, inducible, tissue specific, or cell specific.

**Table 6** provides examples of how the components can be formulated, delivered, or administered.

**Table 6**

| Elements | | | |
|---|---|---|---|
| Cas9 Molecule(s) | gRNA Molecule(s) | Optional Donor Template Nucleic Acid | Comments |
| DNA | DNA | DNA | A Cas9 molecule, typically an eaCas9 molecule, and a gRNA molecule are transcribed from DNA. They are encoded on separate molecules. The donor template is provided as a separate DNA molecule. |
| DNA | DNA | | A Cas9 molecule, typically an eaCas9 molecule, and a gRNA molecule are transcribed from DNA. They are encoded on separate molecules. The donor template is provided on the same DNA molecule that encodes the gRNA molecule. |
| DNA | | DNA | A Cas9 molecule, typically an eaCas9 molecule, and a gRNA molecule are transcribed from DNA, here from a single molecule. The donor template is provided as a separate DNA molecule. |
| DNA | DNA | DNA | A Cas9 molecule, typically an eaCas9 molecule, and a gRNA molecule are transcribed from DNA. They are encoded on separate molecules. The donor template is provided on the same DNA molecule that encodes the Cas9 molecule. |
| DNA | RNA | DNA | A Cas9 molecule, typically an eaCas9 molecule, is transcribed from DNA, and a gRNA molecule is provided as *in vitro* transcribed or synthesized RNA. The donor template is provided as a separate DNA molecule. |
| DNA | RNA | DNA | A Cas9 molecule, typically an eaCas9 molecule, is transcribed from DNA, and a gRNA molecule is provided as *in vitro* transcribed or synthesized RNA. The donor template is provided on the same DNA molecule that encodes the Cas9 molecule. |
| mRNA | RNA | DNA | A Cas9 molecule, typically an eaCas9 molecule, is translated from *in vitro* transcribed mRNA, and a gRNA molecule is provided as *in vitro* transcribed or synthesized RNA. The donor template is provided as a DNA molecule. |
| mRNA | DNA | DNA | A Cas9 molecule, typically an eaCas9 molecule, is translated from *in vitro* |
| | | | transcribed mRNA, and a gRNA molecule is transcribed from DNA. The donor template is provided as a separate DNA molecule. |
| mRNA | DNA | | A Cas9 molecule, typically an eaCas9 molecule, is translated from *in vitro* transcribed mRNA, and a gRNA molecule is transcribed from DNA. The donor template is provided on the same DNA molecule that encodes the gRNA molecule. |
| Protein | DNA | DNA | A Cas9 molecule, typically an eaCas9 molecule, is provided as a protein, and a gRNA molecule is transcribed from DNA. The donor template is provided as a separate DNA molecule. |
| Protein | DNA | | A Cas9 molecule, typically an eaCas9 molecule, is provided as a protein, and a gRNA is transcribed from DNA. The donor template is provided on the same DNA molecule that encodes the gRNA molecule. |
| Protein | RNA | DNA | An eaCas9 molecule is provided as a protein, and a gRNA molecule is provided as transcribed or synthesized RNA. The donor template is provided as a DNA molecule. |

**Table 7** summarizes various delivery methods for the components of a Cas system, *e.g.,* the Cas9 molecule component and the gRNA molecule component, as described herein.

**Table 7**

| **Delivery Vector/Mode** | | **Delivery into Non-Dividing Cells** | **Duration of Expression** | **Genome Integration** | **Type of Molecule Delivered** |
|---|---|---|---|---|---|
| **Physical (*e.g*., electroporation, particle gun, calcium phosphate transfection, cell compression or squeezing)** | | YES | Transient | NO | Nucleic Acids and Proteins |
| ***Viral*** | **Retrovirus** | NO | Stable | YES | RNA |
| | **Lentivirus** | YES | Stable | YES/NO with modifications | RNA |
| | **Adenovirus** | YES | Transient | NO | DNA |
| | **Adeno-Associated Virus (AAV)** | YES | Stable | NO | DNA |
| | **Vaccinia Virus** | YES | Very Transient | NO | DNA |
| | **Herpes Simplex Virus** | YES | Stable | NO | DNA |
| ***Non-Viral*** | **Cationic Liposomes** | YES | Transient | Depends on what is delivered | Nucleic Acids and Proteins |
| | **Polymeric Nanoparticles** | YES | Transient | Depends on what is delivered | Nucleic Acids and Proteins |
| ***Biological Non-Viral Delivery Vehicles*** | **Attenuated Bacteria** | YES | Transient | NO | Nucleic Acids |
| | **Engineered Bacteriophages** | YES | Transient | NO | Nucleic Acids |
| | **Mammalian Virus-like Particles** | YES | Transient | NO | Nucleic Acids |
| | **Biological liposomes: Erythrocyte Ghosts and Exosomes** | YES | Transient | NO | Nucleic Acids |

### DNA-based Delivery of a Cas9 molecule and/or one or more gRNA molecule and/or a donor template

Nucleic acids encoding Cas9 molecules (*e.g*., eaCas9 molecules), gRNA molecules, donor template nucleic acid, or any combination (*e.g*., two or all) thereof, can be administered to subjects or delivered into cells by art-known methods or as described herein. For example, Cas9-encoding and/or gRNA-encoding DNA, as well as donor template nucleic acids, can be delivered by, *e.g.,* vectors (*e.g.,* viral or non-viral vectors), non-vector based methods (*e.g*., using naked DNA or DNA complexes), or a combination thereof.

Nucleic acids encoding Cas9 molecules (*e.g*., eaCas9 molecules) and/or gRNA molecules can be conjugated to molecules (*e.g*., N-acetylgalactosamine) promoting uptake by the target cells (*e.g*., erythrocytes, HSCs). Donor template molecules can likewise be conjugated to molecules (*e.g*., N-acetylgalactosamine) promoting uptake by the target cells (*e.g*., erythrocytes, HSCs).

The Cas9- and/or gRNA-encoding DNA may be delivered by a vector (*e.g*., viral vector/virus or plasmid).

Vectors can comprise a sequence that encodes a Cas9 molecule and/or a gRNA molecule and/or a donor template with high homology to the region (*e.g.,* target sequence) being targeted. The donor template may comprise all or part of a target sequence. Exemplary donor templates are a repair template, *e.g.,* a gene correction template, or a gene mutation template, *e.g.,* point mutation (*e.g.,* single nucleotide (nt) substitution) template. A vector can also comprise a sequence encoding a signal peptide (*e.g.,* for nuclear localization, nucleolar localization, or mitochondrial localization), fused, *e.g.,* to a Cas9 molecule sequence. For example, the vectors can comprise a nuclear localization sequence (*e.g*., from SV40) fused to the sequence encoding the Cas9 molecule.

One or more regulatory/control elements, *e.g*., promoters, enhancers, introns, polyadenylation signals, Kozak consensus sequences, internal ribosome entry sites (IRES), can be included in the vectors. The promoter may be recognized by RNA polymerase II (*e.g.,* a CMV promoter). The promoter may be recognized by RNA polymerase III (*e.g.,* a U6 promoter). The promoter may be a regulated promoter (*e.g*., inducible promoter). The promoter may be a constitutive promoter. The promoter may be a tissue specific promoter. The promoter may be a viral promoter. The promoter may be a non-viral promoter.

The vector may be a viral vector (*e.g.,* for generation of recombinant viruses). The virus may be a DNA virus (*e.g*., dsDNA or ssDNA virus). The virus may be an RNA virus (*e.g.,* an ssRNA virus). The virus may infect dividing cells. The virus may infect non-dividing cells. Exemplary viral vectors/viruses include, *e.g*., retroviruses, lentiviruses, adenovirus, adeno-associated virus (AAV), vaccinia viruses, poxviruses, and herpes simplex viruses.

The virus may infect both dividing and non-dividing cells. The virus can integrate into the host genome. The virus may be engineered to have reduced immunity, *e.g*., in human. The virus may be replication-competent. The virus may be replication-defective, *e.g*., having one or more coding regions for the genes necessary for additional rounds of virion replication and/or packaging replaced with other genes or deleted. The virus may cause transient expression of the Cas9 molecule and/or the gRNA molecule. The virus may cause long-lasting, *e.g*., at least 1 week, 2 weeks, 1 month, 2 months, 3 months, 6 months, 9 months, 1 year, 2 years, or permanent expression, of the Cas9 molecule and/or the gRNA molecule. The packaging capacity of the viruses may vary, *e.g*., from at least about 4 kb to at least about 30 kb, *e.g.,* at least about 5 kb, 10 kb, 15 kb, 20 kb, 25 kb, 30 kb, 35 kb, 40 kb, 45 kb, or 50 kb.

The viral vector may recognize a specific cell type or tissue. For example, the viral vector can be pseudotyped with a different/alternative viral envelope glycoprotein; engineered with a cell type-specific receptor (*e.g*., genetic modification(s) of one or more viral envelope glycoproteins to incorporate a targeting ligand such as a peptide ligand, a single chain antibody, or a growth factor); and/or engineered to have a molecular bridge with dual specificities with one end recognizing a viral glycoprotein and the other end recognizing a moiety of the target cell surface (*e.g.,* a ligand-receptor, monoclonal antibody, avidin-biotin and chemical conjugation).

The Cas9- and/or gRNA-encoding nucleic acid sequence may be delivered by a recombinant retrovirus. The retrovirus (*e.g*., Moloney murine leukemia virus) may comprise a reverse transcriptase, *e.g*., that allows integration into the host genome. The retrovirus may be replication-competent. The retrovirus may be replication-defective, *e.g*., having one of more coding regions for the genes necessary for additional rounds of virion replication and packaging replaced with other genes, or deleted.

The Cas9- and/or gRNA-encoding nucleic acid sequence may be delivered by a recombinant lentivirus. The donor template nucleic acid may be delivered by a recombinant lentivirus. For example, the lentivirus is replication-defective, *e.g.,* does not comprise one or more genes required for viral replication.

The Cas9- and/or gRNA-encoding nucleic acid sequence may be delivered by a recombinant adenovirus. The donor template nucleic acid may be delivered by a recombinant adenovirus. The adenovirus may be engineered to have reduced immunity in human.

The Cas9- and/or gRNA-encoding nucleic acid sequence may be delivered by a recombinant AAV. The donor template nucleic acid may be delivered by a recombinant AAV. The AAV may not incorporate its genome into that of a host cell, *e.g.,* a target cell as described herein, or the AAV can incorporate its genome into that of a host cell. The AAV may be a self-complementary adeno-associated virus (scAAV), *e.g.,* a scAAV that packages both strands which anneal together to form double stranded DNA.

An AAV capsid that can be used in the methods described herein may be a capsid sequence from serotype AAV1, AAV2, AAV3, AAV4, AAV5, AAV6, AAV7, AAV8, AAV9, AAV.rh8, AAV.rh10, AAV.rh32/33, AAV.rh43, AAV.rh64R1, or AAV7m8.

The Cas9- and/or gRNA-encoding DNA may be delivered in a re-engineered AAV capsid, *e.g.,* with 50% or greater, *e.g.,* 60% or greater, 70% or greater, 80% or greater, 90% or greater, or 95% or greater, sequence homology with a capsid sequence from serotypes AAV1, AAV2, AAV3, AAV4, AAV5, AAV6, AAV7, AAV8, AAV9, AAV.rh8, AAV.rh10, AAV.rh32/33, AAV.rh43, or AAV.rh64R1.

The Cas9- and/or gRNA-encoding DNA may be delivered by a chimeric AAV capsid. The donor template nucleic acid may be delivered by a chimeric AAV capsid. Exemplary chimeric AAV capsids include, but are not limited to, AAV9i1, AAV2i8, AAV-DJ, AAV2G9, AAV2i8G9, or AAV8G9.

The AAV may be a self-complementary adeno-associated virus (scAAV), *e.g.,* a scAAV that packages both strands which anneal together to form double stranded DNA.

The Cas9- and/or gRNA-encoding DNA may be delivered by a hybrid virus, *e.g.,* a hybrid of one or more of the viruses described herein. The hybrid virus may be hybrid of an AAV (*e.g.,* of any AAV serotype), with a Bocavirus, B19 virus, porcine AAV, goose AAV, feline AAV, canine AAV, or MVM

A packaging cell is used to form a virus particle that is capable of infecting a target cell. Exemplary packaging cells include 293 cells, which can package adenovirus, and ψ2 or PA317 cells, which can package retrovirus. A viral vector used in gene therapy is usually generated by a producer cell line that packages a nucleic acid vector into a viral particle. The vector typically contains the minimal viral sequences required for packaging and subsequent integration into a host or target cell (if applicable), with other viral sequences being replaced by an expression cassette encoding the protein to be expressed, e.g. Cas9. For example, an AAV vector used in gene therapy typically only possesses inverted terminal repeat (ITR) sequences from the AAV genome which are required for packaging and gene expression in the host or target cell. The missing viral functions can be supplied in *trans* by the packaging cell line and/or plasmid containing E2A, E4, and VA genes from adenovirus, and plasmid encoding *Rep* and *Cap* genes from AAV, as described in "Triple Transfection Protocol." Henceforth, the viral DNA is packaged in a cell line, which contains a helper plasmid encoding the other AAV genes, namely rep and cap, but lacking ITR sequences. The viral DNA may be packaged in a producer cell line, which contains E1A and/or E1B genes from adenovirus. The cell line is also infected with adenovirus as a helper. The helper virus (*e.g*., adenovirus or HSV) or helper plasmid promotes replication of the AAV vector and expression of AAV genes from the helper plasmid with ITRs. The helper plasmid is not packaged in significant amounts due to a lack of ITR sequences. Contamination with adenovirus can be reduced by, *e.g.,* heat treatment to which adenovirus is more sensitive than AAV.

The viral vector may be capable of cell type and/or tissue type recognition. For example, the viral vector can be pseudotyped with a different/alternative viral envelope glycoprotein; engineered with a cell type-specific receptor (*e.g*., genetic modification of the viral envelope glycoproteins to incorporate targeting ligands such as a peptide ligand, single chain antibody, or growth factor); and/or engineered to have a molecular bridge with dual specificities with one end recognizing a viral glycoprotein and the other end recognizing a moiety of the target cell surface (*e.g*., ligand-receptor, monoclonal antibody, avidin-biotin and chemical conjugation).

The viral vector may achieve cell type specific expression. For example, a tissue-specific promoter can be constructed to restrict expression of the transgene (Cas9 and gRNA) to only the target cell. The specificity of the vector can also be mediated by microRNA-dependent control of transgene expression. The viral vector may have increased efficiency of fusion of the viral vector and a target cell membrane. For example, a fusion protein such as fusion-competent hemagglutin (HA) can be incorporated to increase viral uptake into cells. The viral vector may have the ability of nuclear localization. For example, a virus that requires the breakdown of the nuclear envelope (during cell division) and therefore will not infect a non-diving cell can be altered to incorporate a nuclear localization peptide in the matrix protein of the virus thereby enabling the transduction of non-proliferating cells.

The Cas9- and/or gRNA-encoding DNA may be delivered by a non-vector based method (*e.g*., using naked DNA or DNA complexes). For example, the DNA can be delivered, *e.g*., by organically modified silica or silicate (Ormosil), electroporation, transient cell compression or squeezing (see, *e.g.,* Lee 2012), gene gun, sonoporation, magnetofection, lipid-mediated transfection, dendrimers, inorganic nanoparticles, calcium phosphates, or a combination thereof.

Delivery via electroporation may comprise mixing the cells with the Cas9-and/or gRNA-encoding DNA in a cartridge, chamber or cuvette and applying one or more electrical impulses of defined duration and amplitude. Delivery via electroporation may be performed using a system in which cells are mixed with the Cas9-and/or gRNA-encoding DNA in a vessel connected to a device (*e.g.,* a pump) which feeds the mixture into a cartridge, chamber or cuvette wherein one or more electrical impulses of defined duration and amplitude are applied, after which the cells are delivered to a second vessel.

The Cas9- and/or gRNA-encoding DNA may be delivered by a combination of a vector and a non-vector based method. The donor template nucleic acid may be delivered by a combination of a vector and a non-vector based method. For example, virosomes combine liposomes with an inactivated virus (*e.g*., HIV or influenza virus), which can result in more efficient gene transfer, *e.g*., in respiratory epithelial cells than either viral or liposomal methods alone.

As described above, a nucleic acid may comprise (a) a sequence encoding a gRNA molecule comprising a targeting domain that is complementary with a target domain in the *HBB* gene, and (b) a sequence encoding a Cas9 molecule. (a) and (b) may be present on the same nucleic acid molecule, *e.g.,* the same vector, *e.g.,* the same viral vector, *e.g.,* the same adeno-associated virus (AAV) vector. The nucleic acid molecule may be an AAV vector. Exemplary AAV vectors that may be used in any of the described compositions and methods include an AAV2 vector, a modified AAV2 vector, an AAV3 vector, a modified AAV3 vector, an AAV6 vector, a modified AAV6 vector, an AAV8 vector and an AAV9 vector. (a) may be present on a first nucleic acid molecule, *e.g.,* a first vector, *e.g.,* a first viral vector, *e.g.,* a first AAV vector; and (b) is present on a second nucleic acid molecule, *e.g.,* a second vector, *e.g.,* a second vector, *e.g.,* a second AAV vector. The first and second nucleic acid molecules may be AAV vectors. The nucleic acid may further comprise (c) a sequence that encodes a second, third and/or fourth gRNA molecule as described herein. The nucleic acid may comprise (a), (b) and (c). Each of (a) and (c) may be present on the same nucleic acid molecule, *e.g.,* the same vector, *e.g.,* the same viral vector, *e.g.,* the same adeno-associated virus (AAV) vector. The nucleic acid molecule may be an AAV vector.
(a) and (c) may be on different vectors. For example, (a) may be present on a first nucleic acid molecule, *e.g.,* a first vector, *e.g.,* a first viral vector, *e.g.,* a first AAV vector; and (c) may be present on a second nucleic acid molecule, *e.g.,* a second vector, *e.g.,* a second vector, *e.g.,* a second AAV vector. The first and second nucleic acid molecules may be AAV vectors. Each of (a), (b), and (c) may be present on the same nucleic acid molecule, *e.g.,* the same vector, *e.g.,* the same viral vector, *e.g.,* an AAV vector. The nucleic acid molecule may be an AAV vector. One of (a), (b), and (c) may be encoded on a first nucleic acid molecule, *e.g.,* a first vector, *e.g.,* a first viral vector, *e.g.,* a first AAV vector; and a second and third of (a), (b), and (c) is encoded on a second nucleic acid molecule, *e.g.,* a second vector, *e.g.,* a second vector, *e.g.,* a second AAV vector. The first and second nucleic acid molecule may be AAV vectors.
(a) may be present on a first nucleic acid molecule, *e.g.,* a first vector, *e.g.,* a first viral vector, a first AAV vector; and (b) and (c) may be present on a second nucleic acid molecule, *e.g.,* a second vector, *e.g.,* a second vector, *e.g.,* a second AAV vector. The first and second nucleic acid molecule may be AAV vectors. (b) may be present on a first nucleic acid molecule, *e.g.,* a first vector, *e.g.,* a first viral vector, *e.g.,* a first AAV vector; and (a) and (c) may be present on a second nucleic acid molecule, *e.g.,* a second vector, *e.g.,* a second vector, *e.g.,* a second AAV vector. The first and second nucleic acid molecule may be AAV vectors.
(c) may be present on a first nucleic acid molecule, *e.g.,* a first vector, *e.g.,* a first viral vector, *e.g.,* a first AAV vector; and (b) and (a) may be present on a second nucleic acid molecule, *e.g.,* a second vector, *e.g.,* a second vector, *e.g.,* a second AAV vector. The first and second nucleic acid molecule may be AAV vectors.

Each of (a), (b) and (c) may be present on different nucleic acid molecules, *e.g*., different vectors, *e.g.,* different viral vectors, *e.g.,* different AAV vector. For example, (a) may be on a first nucleic acid molecule, (b) on a second nucleic acid molecule, and (c) on a third nucleic acid molecule. The first, second and third nucleic acid molecule may be AAV vectors.

When a third and/or fourth gRNA molecule are present, each of (a), (b), (c)(i), (c)(ii) and (c)(iii) may be present on the same nucleic acid molecule, *e.g.,* the same vector, *e.g.,* the same viral vector, *e.g.,* an AAV vector. The nucleic acid molecule may be an AAV vector. Each of (a), (b), (c)(i), (c)(ii) and (c)(iii) may be present on the different nucleic acid molecules, *e.g.,* different vectors, *e.g.,* the different viral vectors, *e.g.,* different AAV vectors. Each of (a), (b), (c)(i), (c)(ii) and (c)(iii) may be present on more than one nucleic acid molecule, but fewer than five nucleic acid molecules, *e.g*., AAV vectors.

When (d) a template nucleic acid is present, each of (a), (b), and (d) may be present on the same nucleic acid molecule, *e.g.,* the same vector, *e.g.,* the same viral vector, *e.g.,* an AAV vector. The nucleic acid molecule may be an AAV vector. Each of (a), (b), and (d) may be present on the different nucleic acid molecules, *e.g.,* different vectors, *e.g.,* the different viral vectors, *e.g*., different AAV vectors. Each of (a), (b), and (d) may be present on more than one nucleic acid molecule, but fewer than three nucleic acid molecules, *e.g*., AAV vectors.

When (d) a template nucleic acid is present, each of (a), (b), (c)(i) and (d) may be present on the same nucleic acid molecule, *e.g.,* the same vector, *e.g.,* the same viral vector, *e.g.,* an AAV vector. The nucleic acid molecule may be an AAV vector. Each of (a), (b), (c)(i) and (d) may be present on the different nucleic acid molecules, *e.g*., different vectors, *e.g.,* the different viral vectors, *e.g.,* different AAV vectors. Each of (a), (b), (c)(i) and (d) may be present on more than one nucleic acid molecule, but fewer than four nucleic acid molecules, *e.g*., AAV vectors.

When (d) a template nucleic acid is present, each of (a), (b), (c)(i), (c)(ii) and (d) may be present on the same nucleic acid molecule, *e.g.,* the same vector, *e.g.,* the same viral vector, *e.g.,* an AAV vector. The nucleic acid molecule may be an AAV vector. Each of (a), (b), (c)(i), (c)(ii) and (d) may be present on the different nucleic acid molecules, *e.g*., different vectors, *e.g.,* the different viral vectors, *e.g.,* different AAV vectors. Each of (a), (b), (c)(i), (c)(ii) and (d) may be present on more than one nucleic acid molecule, but fewer than five nucleic acid molecules, *e.g*., AAV vectors.

When (d) a template nucleic acid is present, each of (a), (b), (c)(i), (c)(ii), (c)(iii) and (d) may be present on the same nucleic acid molecule, *e.g.,* the same vector, *e.g.,* the same viral vector, *e.g.,* an AAV vector. The nucleic acid molecule may be an AAV vector. Each of (a), (b), (c)(i), (c)(ii), (c)(iii) and (d) may be present on the different nucleic acid molecules, *e.g.,* different vectors, *e.g.,* the different viral vectors, *e.g.,* different AAV vectors. Each of (a), (b), (c)(i), (c)(ii), (c)(iii) and (d) may be present on more than one nucleic acid molecule, but fewer than six nucleic acid molecules, *e.g*., AAV vectors.

The nucleic acids described herein may comprise a promoter operably linked to the sequence that encodes the gRNA molecule of (a), *e.g.,* a promoter described herein. The nucleic acid may further comprise a second promoter operably linked to the sequence that encodes the second, third and/or fourth gRNA molecule of (c), *e.g.,* a promoter described herein. The promoter and second promoter differ from one another. The promoter and second promoter may be the same.

The nucleic acids described herein may further comprise a promoter operably linked to the sequence that encodes the Cas9 molecule of (b), *e.g.,* a promoter described herein.

The delivery vehicle may be a non-viral vector, and the non-viral vector may be an inorganic nanoparticle. Exemplary inorganic nanoparticles include, *e.g*., magnetic nanoparticles (*e.g*., Fe₃MnO₂) or silica. The outer surface of the nanoparticle can be conjugated with a positively charged polymer (*e.g*., polyethylenimine, polylysine, polyserine) which allows for attachment (*e.g*., conjugation or entrapment) of payload. The non-viral vector may be an organic nanoparticle (*e.g*., entrapment of the payload inside the nanoparticle). Exemplary organic nanoparticles include, *e.g*., SNALP liposomes that contain cationic lipids together with neutral helper lipids which are coated with polyethylene glycol (PEG) and protamine and nucleic acid complex coated with lipid coating.

Exemplary lipids for gene transfer are shown below in **Table 8.**

**Table 8. Lipids Used for Gene Transfer**

| **Lipid** | **Abbreviation** | **Feature** |
|---|---|---|
| 1,2- Dioleoyl-sn-glycero-3 -phosphatidylcholine | DOPC | Helper |
| 1,2-Dioleoyl-sn-glycero-3-phosphatidylethanolamine | DOPE | Helper |
| Cholesterol | | Helper |
| N-[1-(2,3-Dioleyloxy)propyl]N,N,N-trimethylammonium chloride | DOTMA | Cationic |
| 1,2-Dioleoyloxy-3-trimethylammonium-propane | DOTAP | Cationic |
| Dioctadecylamidoglycylspermine | DOGS | Cationic |
| N-(3-Aminopropyl)-N,N-dimethyl-2,3-bis(dodecyloxy)-1-propanaminium bromide | GAP-DLRIE | Cationic |
| Cetyltrimethylammonium bromide | CTAB | Cationic |
| 6-Lauroxyhexyl ornithinate | LHON | Cationic |
| 1-(2,3-Dioleoyloxypropyl)-2,4,6-trimethylpyridinium | 2Oc | Cationic |
| 2,3-Dioleyloxy-N-[2(sperminecarboxamido-ethyl]-N,N-dimethyl-1-propanaminium trifluoroacetate | DOSPA | Cationic |
| 1,2-Dioleyl-3-trimethylammonium-propane | DOPA | Cationic |
| N-(2-Hydroxyethyl)-N,N-dimethyl-2,3-bis(tetradecyloxy)-1-propanaminium bromide | MDRIE | Cationic |
| Dimyristooxypropyl dimethyl hydroxyethyl ammonium bromide | DMRI | Cationic |
| 3β-[N-(N',N'-Dimethylaminoethane)-carbamoyl]cholesterol | DC-Chol | Cationic |
| Bis-guanidium-tren-cholesterol | BGTC | Cationic |
| 1,3-Diodeoxy-2-(6-carboxy-spermyl)-propylamide | DOSPER | Cationic |
| Dimethyloctadecylammonium bromide | DDAB | Cationic |
| Dioctadecylamidoglicylspermidin | DSL | Cationic |
| rac-[(2,3-Dioctadecyloxypropyl)(2-hydroxyethyl)]-dimethylammonium chloride | CLIP-1 | Cationic |
| rac-[2(2,3-Dihexadecyloxypropyl-oxymethyloxy)ethyl]trimethylammonium bromide | CLIP-6 | Cationic |
| Ethyldimyristoylphosphatidylcholine | EDMPC | Cationic |
| 1,2-Distearyloxy-N,N-dimethyl-3-aminopropane | DSDMA | Cationic |
| 1,2-Dimyristoyl-trimethylammonium propane | DMTAP | Cationic |
| O,O'-Dimyristyl-N-lysyl aspartate | DMKE | Cationic |
| 1,2-Distearoyl-sn-glycero-3-ethylphosphocholine | DSEPC | Cationic |
| N-Palmitoyl D-erythro-sphingosyl carbamoyl-spermine | CCS | Cationic |
| N-t-Butyl-N0-tetradecyl-3-tetradecylaminopropionamidine | diC14-amidine | Cationic |
| Octadecenolyoxy[ethyl-2-heptadecenyl-3 hydroxyethyl] imidazolinium chloride | DOTIM | Cationic |
| N1-Cholesteryloxycarbonyl-3,7-diazanonane-1,9-diamine | CDAN | Cationic |
| 2-(3-[Bis(3-amino-propyl)-amino]propylamino)-N-ditetradecylcarbamoylme-ethyl-acetamide | RPR209120 | Cationic |
| 1,2-dilinoleyloxy-3-dimethylaminopropane | DLinDMA | Cationic |
| 2,2-dilinoleyl-4-dimethylaminoethyl- [1,3] - dioxolane | DLin-KC2-DMA | Cationic |
| dilinoleyl-methyl-4-dimethylaminobutyrate | DLin-MC3-DMA | Cationic |

Exemplary polymers for gene transfer are shown below in **Table 9.**

**Table 9. Polymers Used for Gene Transfer**

| **Polymer** | **Abbreviation** |
|---|---|
| Poly(ethylene)glycol | PEG |
| Polyethylenimine | PEI |
| Dithiobis(succinimidylpropionate) | DSP |
| Dimethyl-3,3' -dithiobispropionimidate | DTBP |
| Poly(ethylene imine) biscarbamate | PEIC |
| Poly(L-lysine) | PLL |
| Histidine modified PLL | |
| Poly(*N*-vinylpyrrolidone) | PVP |
| Poly(propylenimine) | PPI |
| Poly(amidoamine) | PAMAM |
| Poly(amido ethylenimine) | SS-PAEI |
| Triethylenetetramine | TETA |
| Poly(β-aminoester) | |
| Poly(4-hydroxy-L-proline ester) | PHP |
| Poly(allylamine) | |
| Poly(α-[4-aminobutyl]-L-glycolic acid) | PAGA |
| Poly(D,L-lactic-co-glycolic acid) | PLGA |
| Poly(*N*-ethyl-4-vinylpyridinium bromide) | |
| Poly(phosphazene)s | PPZ |
| Poly(phosphoester)s | PPE |
| Poly(phosphoramidate)s | PPA |
| Poly(*N*-2-hydroxypropylmethacrylamide) | pHPMA |
| Poly (2-(dimethylamino)ethyl methacrylate) | pDMAEMA |
| Poly(2-aminoethyl propylene phosphate) | PPE-EA |
| Chitosan | |
| Galactosylated chitosan | |
| *N*-Dodacylated chitosan | |
| Histone | |
| Collagen | |
| Dextran-spermine | D-SPM |

The vehicle may have targeting modifications to increase target cell update of nanoparticles and liposomes, *e.g*., cell specific antigens, monoclonal antibodies, single chain antibodies, aptamers, polymers, sugars (*e.g*., N-acetylgalactosamine (GalNAc)), and cell penetrating peptides. The vehicle may use fusogenic and endosome-destabilizing peptides/polymers. The vehicle may undergo acid-triggered conformational changes (*e.g.,* to accelerate endosomal escape of the cargo). A stimuli-cleavable polymer may be used, *e.g.,* for release in a cellular compartment. For example, disulfide-based cationic polymers that are cleaved in the reducing cellular environment can be used.

The delivery vehicle may be a biological non-viral delivery vehicle. The vehicle may be an attenuated bacterium (*e.g*., naturally or artificially engineered to be invasive but attenuated to prevent pathogenesis and expressing the transgene (*e.g., Listeria monocytogenes,* certain *Salmonella strains, Bifidobacterium longum,* and modified *Escherichia coli*), bacteria having nutritional and tissue-specific tropism to target specific tissues, bacteria having modified surface proteins to alter target tissue specificity). The vehicle may be a genetically modified bacteriophage (*e.g*., engineered phages having large packaging capacity, less immunogenic, containing mammalian plasmid maintenance sequences and having incorporated targeting ligands). The vehicle may be a mammalian virus-like particle. For example, modified viral particles can be generated (*e.g*., by purification of the "empty" particles followed by *ex vivo* assembly of the virus with the desired cargo). The vehicle can also be engineered to incorporate targeting ligands to alter target tissue specificity. The vehicle may be a biological liposome. For example, the biological liposome is a phospholipid-based particle derived from human cells (*e.g.,* erythrocyte ghosts, which are red blood cells broken down into spherical structures derived from the subject (*e.g*., tissue targeting can be achieved by attachment of various tissue or cell-specific ligands), or secretory exosomes -subject (*i.e.,* patient) derived membrane-bound nanovesicle (30 -100 nm) of endocytic origin (*e.g.,* can be produced from various cell types and can therefore be taken up by cells without the need of for targeting ligands).

One or more nucleic acid molecules (*e.g*., DNA molecules) other than the components of a Cas system, *e.g*., the Cas9 molecule component and/or the gRNA molecule component described herein, may be delivered. The nucleic acid molecule may be delivered at the same time as one or more of the components of the Cas system are delivered. The nucleic acid molecule may be delivered before or after (*e.g.,* less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas system are delivered. The nucleic acid molecule may be delivered by a different means than one or more of the components of the Cas system, *e.g*., the Cas9 molecule component and/or the gRNA molecule component, are delivered. The nucleic acid molecule can be delivered by any of the delivery methods described herein. For example, the nucleic acid molecule can be delivered by a viral vector, *e.g.,* an integration-deficient lentivirus, and the Cas9 molecule component and/or the gRNA molecule component can be delivered by electroporation, *e.g.,* such that the toxicity caused by nucleic acids (*e.g.,* DNAs) can be reduced. The nucleic acid molecule may encode a therapeutic protein, *e.g.,* a protein described herein. The nucleic acid molecule may encode an RNA molecule, *e.g.,* an RNA molecule described herein.

### Delivery of RNA encoding a Cas9 molecule

RNA encoding Cas9 molecules and/or gRNA molecules, can be delivered into cells, *e.g.,* target cells described herein, by art-known methods or as described herein. For example, Cas9-encoding and/or gRNA-encoding RNA can be delivered, *e.g*., by microinjection, electroporation, transient cell compression or squeezing (see, *e.g.,* Lee 2012), lipid-mediated transfection, peptide-mediated delivery, or a combination thereof. Cas9-encoding and/or gRNA-encoding RNA can be conjugated to molecules) promoting uptake by the target cells (*e.g.,* target cells described herein).

Delivery via electroporation may comprise mixing the cells with the RNA encoding Cas9 molecules and/or gRNA molecules, with or without donor template nucleic acid molecules, in a cartridge, chamber or cuvette and applying one or more electrical impulses of defined duration and amplitude. Delivery via electroporation may be performed using a system in which cells are mixed with the RNA encoding Cas9 molecules and/or gRNA molecules, with or without donor template nucleic acid molecules in a vessel connected to a device (*e.g.,* a pump) which feeds the mixture into a cartridge, chamber or cuvette wherein one or more electrical impulses of defined duration and amplitude are applied, after which the cells are delivered to a second vessel. Cas9-encoding and/or gRNA-encoding RNA can be conjugated to molecules to promote uptake by the target cells (*e.g.,* target cells described herein).

### Delivery of Cas9

Cas9 molecules can be delivered into cells by art-known methods or as described herein. For example, Cas9 protein molecules can be delivered, *e.g*., by microinjection, electroporation, transient cell compression or squeezing (see, *e.g.,* Lee 2012), lipid-mediated transfection, peptide-mediated delivery, or a combination thereof. Delivery can be accompanied by DNA encoding a gRNA or by a gRNA. Cas9 protein can be conjugated to molecules promoting uptake by the target cells (*e.g.,* target cells described herein).

Delivery via electroporation may comprise mixing the cells with the Cas9 molecules and/or gRNA molecules, with or without donor nucleic acid, in a cartridge, chamber or cuvette and applying one or more electrical impulses of defined duration and amplitude. Delivery via electroporation may be performed using a system in which cells are mixed with the Cas9 molecules and/or gRNA molecules, with or without donor nucleic acid in a vessel connected to a device (*e.g.,* a pump) which feeds the mixture into a cartridge, chamber or cuvette wherein one or more electrical impulses of defined duration and amplitude are applied, after which the cells are delivered to a second vessel. Cas9-encoding and/or gRNA-encoding RNA can be conjugated to molecules to promote uptake by the target cells (*e.g.,* target cells described herein).

### Route of Administration

Systemic modes of administration include oral and parenteral routes. Parenteral routes include, by way of example, intravenous, intramarrow, intrarterial, intramuscular, intradermal, subcutaneous, intranasal and intraperitoneal routes. Components administered systemically may be modified or formulated to target, *e.g*., HSCs, hematopoetic stem/progenitor cells, or erythroid progenitors or precursor cells.

Local modes of administration include, by way of example, intramarrow injection into the trabecular bone or intrafemoral injection into the marrow space, and infusion into the portal vein. In an embodiment, significantly smaller amounts of the components (compared with systemic approaches) may exert an effect when administered locally (for example, directly into the bone marrow) compared to when administered systemically (for example, intravenously). Local modes of administration can reduce or eliminate the incidence of potentially toxic side effects that may occur when therapeutically effective amounts of a component are administered systemically.

Administration may be provided as a periodic bolus (*e.g*., intravenously) or as continuous infusion from an internal reservoir or from an external reservoir (for example, from an intravenous bag or implantable pump). Components may be administered locally, for example, by continuous release from a sustained release drug delivery device.

In addition, components may be formulated to permit release over a prolonged period of time. A release system can include a matrix of a biodegradable material or a material which releases the incorporated components by diffusion. The components can be homogeneously or heterogeneously distributed within the release system. A variety of release systems may be useful, however, the choice of the appropriate system will depend upon rate of release required by a particular application. Both non-degradable and degradable release systems can be used. Suitable release systems include polymers and polymeric matrices, non-polymeric matrices, or inorganic and organic excipients and diluents such as, but not limited to, calcium carbonate and sugar (for example, trehalose). Release systems may be natural or synthetic. However, synthetic release systems are preferred because generally they are more reliable, more reproducible and produce more defined release profiles. The release system material can be selected so that components having different molecular weights are released by diffusion through or degradation of the material.

Representative synthetic, biodegradable polymers include, for example: polyamides such as poly(amino acids) and poly(peptides); polyesters such as poly(lactic acid), poly(glycolic acid), poly(lactic-co-glycolic acid), and poly(caprolactone); poly(anhydrides); polyorthoesters; polycarbonates; and chemical derivatives thereof (substitutions, additions of chemical groups, for example, alkyl, alkylene, hydroxylations, oxidations, and other modifications routinely made by those skilled in the art), copolymers and mixtures thereof. Representative synthetic, non-degradable polymers include, for example: polyethers such as poly(ethylene oxide), poly(ethylene glycol), and poly(tetramethylene oxide); vinyl polymers-polyacrylates and polymethacrylates such as methyl, ethyl, other alkyl, hydroxyethyl methacrylate, acrylic and methacrylic acids, and others such as poly(vinyl alcohol), poly(vinyl pyrolidone), and poly(vinyl acetate); poly(urethanes); cellulose and its derivatives such as alkyl, hydroxyalkyl, ethers, esters, nitrocellulose, and various cellulose acetates; polysiloxanes; and any chemical derivatives thereof (substitutions, additions of chemical groups, for example, alkyl, alkylene, hydroxylations, oxidations, and other modifications routinely made by those skilled in the art), copolymers and mixtures thereof.

Poly(lactide-co-glycolide) microsphere can also be used for injection. Typically the microspheres are composed of a polymer of lactic acid and glycolic acid, which are structured to form hollow spheres. The spheres can be approximately 15-30 microns in diameter and can be loaded with components described herein.

### Bi-Modal or Differential Delivery of Components

Separate delivery of the components of a Cas system, *e.g*., the Cas9 molecule component and the gRNA molecule component, and more particularly, delivery of the components by differing modes, can enhance performance, *e.g*., by improving tissue specificity and safety.

The Cas9 molecule and the gRNA molecule may be delivered by different modes, or as sometimes referred to herein as differential modes. Different or differential modes, as used herein, refer modes of delivery that confer different pharmacodynamic or pharmacokinetic properties on the subject component molecule, *e.g.,* a Cas9 molecule, gRNA molecule, template nucleic acid, or payload. For example, the modes of delivery can result in different tissue distribution, different half-life, or different temporal distribution, *e.g*., in a selected compartment, tissue, or organ.

Some modes of delivery, *e.g*., delivery by a nucleic acid vector that persists in a cell, or in progeny of a cell, *e.g.,* by autonomous replication or insertion into cellular nucleic acid, result in more persistent expression of and presence of a component. Examples include viral, *e.g.,* AAV or lentivirus, delivery.

By way of example, the components, *e.g*., a Cas9 molecule and a gRNA molecule, can be delivered by modes that differ in terms of resulting half-life or persistent of the delivered component the body, or in a particular compartment, tissue or organ. A gRNA molecule can be delivered by such modes. The Cas9 molecule component can be delivered by a mode which results in less persistence or less exposure to the body or a particular compartment or tissue or organ.

More generally, a first mode of delivery may be used to deliver a first component and a second mode of delivery may be used to deliver a second component. The first mode of delivery confers a first pharmacodynamic or pharmacokinetic property. The first pharmacodynamic property can be, *e.g*., distribution, persistence, or exposure, of the component, or of a nucleic acid that encodes the component, in the body, a compartment, tissue or organ. The second mode of delivery confers a second pharmacodynamic or pharmacokinetic property. The second pharmacodynamic property can be, *e.g*., distribution, persistence, or exposure, of the component, or of a nucleic acid that encodes the component, in the body, a compartment, tissue or organ.

The first pharmacodynamic or pharmacokinetic property, *e.g*., distribution, persistence or exposure, may be more limited than the second pharmacodynamic or pharmacokinetic property.

The first mode of delivery may be selected to optimize, *e.g*., minimize, a pharmacodynamic or pharmacokinetic property, *e.g*., distribution, persistence or exposure.

The second mode of delivery may be selected to optimize, *e.g*., maximize, a pharmacodynamic or pharmacokinetic property, *e.g*., distribution, persistence or exposure.

The first mode of delivery may comprise the use of a relatively persistent element, *e.g.,* a nucleic acid, *e.g.,* a plasmid or viral vector, *e.g.,* an AAV or lentivirus. As such vectors are relatively persistent product transcribed from them would be relatively persistent.

The second mode of delivery may comprise a relatively transient element, *e.g.,* an RNA or protein.

The first component may comprise gRNA molecule, and the delivery mode is relatively persistent, *e.g.,* the gRNA is transcribed from a plasmid or viral vector, *e.g.,* an AAV or lentivirus. Transcription of these genes would be of little physiological consequence because the genes do not encode for a protein product, and the gRNAs are incapable of acting in isolation. The second component, a Cas9 molecule, is delivered in a transient manner, for example as mRNA or as protein, ensuring that the full Cas9 molecule/gRNA molecule complex is only present and active for a short period of time.

Furthermore, the components can be delivered in different molecular form or with different delivery vectors that complement one another to enhance safety and tissue specificity.

Use of differential delivery modes can enhance performance, safety and/or efficacy, *e.g.,* the likelihood of an eventual off-target modification can be reduced. Delivery of immunogenic components, *e.g*., Cas9 molecules, by less persistent modes can reduce immunogenicity, as peptides from the bacterially-derived Cas enzyme are displayed on the surface of the cell by MHC molecules. A two-part delivery system can alleviate these drawbacks.

Differential delivery modes can be used to deliver components to different, but overlapping target regions. The formation active complex is minimized outside the overlap of the target regions. Thus, a first component, *e.g*., a gRNA molecule may be delivered by a first delivery mode that results in a first spatial, *e.g*., tissue, distribution. A second component, *e.g*., a Cas9 molecule may be delivered by a second delivery mode that results in a second spatial, *e.g*., tissue, distribution. The first mode may comprise a first element selected from a liposome, nanoparticle, *e.g.,* polymeric nanoparticle, and a nucleic acid, *e.g.,* viral vector. The second mode may comprise a second element selected from the group. The first mode of delivery may comprise a first targeting element, *e.g.,* a cell specific receptor or an antibody, and the second mode of delivery may not include that element. The second mode of delivery may comprise a second targeting element, *e.g.,* a second cell specific receptor or second antibody.

When the Cas9 molecule is delivered in a virus delivery vector, a liposome, or polymeric nanoparticle, there is the potential for delivery to and therapeutic activity in multiple tissues, when it may be desirable to only target a single tissue. A two-part delivery system can resolve this challenge and enhance tissue specificity. If the gRNA molecule and the Cas9 molecule are packaged in separated delivery vehicles with distinct but overlapping tissue tropism, the fully functional complex is only be formed in the tissue that is targeted by both vectors.

Disclosed herein are methods of altering a cell, *e.g.,* altering the structure, *e.g.,* altering the sequence, of a target nucleic acid of a cell, comprising contacting said cell with: (a) a gRNA molecule that targets the *HBB* gene, *e.g.,* a gRNA molecule as described herein; (b) a Cas9 molecule, *e.g.,* a Cas9 molecule as described herein; and optionally, (c) a second, third and/or fourth gRNA that targets *HBB* gene, *e.g.,* a gRNA molecule; and optionally, (d) a template nucleic acid, as described herein. The method may comprise contacting said cell with (a) and (b). The method may comprise contacting said cell with (a), (b), and (c). The method may comprise contacting said cell with (a), (b), (c) and (d). The gRNA may target the *HBB* gene and no exogenous template nucleic acid is contacted with the cell. The targeting domain of the gRNA molecule of (a) and optionally (c) may be selected from a targeting domain sequence described herein, *e.g.,* a targeting domain sequence selected from any of **Tables 1-3,** or a targeting domain sequence that differs by no more than 1, 2, 3, 4, or 5 nucleotides from, a targeting domain sequence described herein, *e.g.,* a targeting domain sequence from any of **Tables 1-3.**

The method may comprise contacting a cell from a subject suffering from or likely to develop SCD. The cell may be from a subject having a mutation at an SCD target position in the *HBB* gene. The cell being contacted in the disclosed method may be an erythroid cell. The contacting may be performed *ex vivo* and the contacted cell may be returned to the subject's body after the contacting step. The contacting step may be performed *in vivo.* The method of altering a cell as described herein may comprise acquiring knowledge of the sequence at an SCD target position in said cell, prior to the contacting step. Acquiring knowledge of the sequence at an SCD target position in the cell may be by sequencing the *HBB* gene, or a portion of the *HBB* gene. The contacting step of the method may comprise contacting the cell with a nucleic acid, *e.g.,* a vector, *e.g.,* an AAV vector, that expresses at least one of (a), (b), and (c). The contacting step of the method may comprise contacting the cell with a nucleic acid, *e.g.,* a vector, *e.g.,* an AAV vector, that expresses each of (a), (b), and (c). The contacting step of the method may comprise delivering to the cell a Cas9 molecule of (b) and a nucleic acid which encodes a gRNA molecule (a) and optionally, a second gRNA molecule (c)(i) (and further optionally, a third gRNA molecule (c)(iv) and/or fourth gRNA molecule (c)(iii).

The contacting step of the method may comprise contacting the cell with a nucleic acid, *e.g.,* a vector, *e.g.,* an AAV vector, that expresses at least one of (a), (b), (c) and (d). The contacting step of the method may comprise contacting the cell with a nucleic acid, *e.g.,* a vector, *e.g.,* an AAV vector, that expresses each of (a), (b), and (c). The contacting step of the method may comprise delivering to the cell a Cas9 molecule of (b), a nucleic acid which encodes a gRNA molecule of (a) and a template nucleic acid of (d), and optionally, a second gRNA molecule (c)(i) (and further optionally, a third gRNA molecule (c)(iv) and/or fourth gRNA molecule (c)(iii).

Contacting may comprise contacting the cell with a nucleic acid, *e.g.,* a vector, *e.g.,* an AAV vector, *e.g.,* an AAV2 vector, a modified AAV2 vector, an AAV3 vector, a modified AAV3 vector, an AAV6 vector, a modified AAV6 vector, an AAV8 vector or an AAV9 vector.

Contacting may comprise delivering to the cell a Cas9 molecule of (b), as a protein or an mRNA, and a nucleic acid which encodes (a) and optionally a second, third and/or fourth gRNA molecule of (c).

Contacting may comprise delivering to the cell a Cas9 molecule of (b), as a protein or an mRNA, said gRNA molecule of (a), as an RNA, and optionally said second, third and/or fourth gRNA molecule of (c), as an RNA.

Contacting may comprise delivering to the cell a gRNA of (a) as an RNA, optionally said second, third and/or fourth gRNA molecule of (c) as an RNA, and a nucleic acid that encodes the Cas9 molecule of (b).

Disclosed herein are also methods of treating or preventing a subject suffering from or likely to develop SCD, *e.g.,* altering the structure, *e.g.,* sequence, of a target nucleic acid of the subject, comprising contacting the subject (or a cell from the subject) with:
(a) a gRNA that targets the *HBB* gene, *e.g.,* a gRNA disclosed herein;
(b) a Cas9 molecule, *e.g.,* a Cas9 molecule disclosed herein; and
optionally, (c)(i) a second gRNA that targets the *HBB* gene, *e.g.,* a second gRNA disclosed herein, and further optionally, (c)(ii) a third gRNA molecule, and still further optionally, (c)(iii) a fourth gRNA molecule that targets the *HBB* gene, *e.g.,* a third and fourth gRNA disclosed herein. The method of treating a subject may further comprise contacting the subject (or a cell from the subject) with (d) a template nucleic acid (where an exogenous template is used), *e.g.,* a template nucleic acid disclosed herein. A template nucleic acid may be used when the method of treating a subject uses HDR to alter the sequence of the target nucleic acid of the subject. The gRNA molecule may target the *HBB* gene and no exogenous template nucleic acid is contacted with the subject (or a cell from the subject). Contacting may comprise contacting with (a) and (b). Contacting may comprise contacting with (a), (b), and (c)(i). Contacting may comprise contacting with (a), (b), (c)(i) and (c)(ii). Contacting may comprise contacting with (a), (b), (c)(i), (c)(ii) and (c)(iii). Contacting may comprise contacting with (a), (b), (c)(i) and (d). Contacting may comprise contacting with (a), (b), (c)(i), (c)(ii) and (d). Contacting may comprise contacting with (a), (b), (c)(i), (c)(ii), (c)(iii) and (d).

The targeting domain of the gRNA molecule of (a) or (c) *(e.g.,* (c)(i), (c)(ii), or (c)(iii)) may be selected from a targeting domain sequence described herein, *e.g.,* a targeting domain sequence selected from any of **Tables 1-3,** or a targeting domain sequence that differs by no more than 1, 2, 3, 4, or 5 nucleotides from a targeting domain sequence described herein, *e.g.,* a targeting domain sequence from any of **Tables 1-3**.

The method may comprise acquiring knowledge of the sequence (*e.g.,* a mutation) of an SCD target position in said subject. The method may comprise acquiring knowledge of the sequence (*e.g.,* a mutation) of an SCD target position in said subject by sequencing the *HBB* gene or a portion of the *HBB* gene. The method may comprise correcting a mutation at an SCD target position in the *HBB* gene. The method may comprise correcting a mutation at an SCD target position in the *HBB* gene by HDR.

When the method comprises correcting the mutation at an SCD target position by HDR, a Cas9 of (b), at least one gRNA molecule, *e.g.,* a gRNA molecule of (a) and a template nucleic acid of (d) are included in the contacting step. A cell of the subject may be contacted *ex vivo* with (a), (b), (d) and optionally (c)(i), further optionally (c)(ii), and still further optionally (c)(iii). Said cell may be returned to the subject's body. A cell of the subject may be contacted *in vivo* with (a), (b), (d) and optionally (c)(i), further optionally (c)(ii), and still further optionally (c)(iii). The cell of the subject may be contacted *in vivo* by intravenous delivery of (a), (b), (d) and optionally (c)(i), further optionally (c)(ii), and still further optionally (c)(iii). The cell of the subject may be contacted *in vivo* by intramuscular delivery of (a), (b), (d) and optionally (c)(i), further optionally (c)(ii), and still further optionally (c)(iii). The cell of the subject may be contacted *in vivo* by subcutaneous delivery of (a), (b), (d) and optionally (c)(i), further optionally (c)(ii), and still further optionally (c)(iii). The cell of the subject may be contacted *in vivo* by intra-bone marrow (IBM) delivery of (a), (b), (d) and optionally (c)(i), further optionally (c)(ii), and still further optionally (c)(iii).

Contacting may comprise contacting the subject with a nucleic acid, *e.g.,* a vector, *e.g.,* an AAV vector, described herein, *e.g.,* a nucleic acid that encodes at least one of (a), (b), (d) and optionally (c)(i), further optionally (c)(ii), and still further optionally (c)(iii).

Contacting may comprise delivering to said subject said Cas9 molecule of (b), as a protein or mRNA, and a nucleic acid which encodes (a), a nucleic acid of (d) and optionally (c)(i), further optionally (c)(ii), and still further optionally (c)(iii).

Contacting may comprise delivering to the subject the Cas9 molecule of (b), as a protein or mRNA, the gRNA molecule of (a), as an RNA, a nucleic acid of (d) and optionally the second, third and/or fourth gRNA molecule of (c), as an RNA.

Contacting may comprise delivering to the subject the gRNA molecule of (a), as an RNA, optionally said second, third and/or fourth gRNA molecule of (c), as an RNA, a nucleic acid that encodes the Cas9 molecule of (b), and a nucleic acid of (d).

A cell of the subject may be contacted *ex vivo* with (a), (b) and optionally (c)(i), further optionally (c)(ii), and still further optionally (c)(iii). Said cell may be returned to the subject's body.

A population of cells from a subject may be contacted *ex vivo* with (a), (b) and optionally (c) to correct the E6V mutation in the *HBB* gene and a second population of cells from the subject may be contacted *ex vivo* with (a), (b) and optionally (c) to introduce a mutation in a second gene, *e.g.,* the *BCL11A* gene, or to knockout or knock down a second gene, *e.g.,* the *BCL11A* gene. A mixture of the two cell populations may be returned to the subject's body to treat or prevent SCD.

A cell of the subject may be contacted *in vivo* with (a), (b) and optionally (c)(i), further optionally (c)(ii), and still further optionally (c)(iii). The cell of the subject may be contacted *in vivo* by intravenous delivery of (a), (b) and optionally (c)(i), further optionally (c)(ii), and still further optionally (c)(iii). The cell of the subject may be contacted *in vivo* by intramuscular delivery of (a), (b) and optionally (c)(i), further optionally (c)(ii), and still further optionally (c)(iii). The cell of the subject may be contacted *in vivo* by subcutaneous delivery of (a), (b) and optionally (c)(i), further optionally (c)(ii), and still further optionally (c)(iii). The cell of the subject may be contacted *in vivo* by intra-bone marrow (IBM) delivery of (a), (b) and optionally (c)(i), further optionally (c)(ii), and still further optionally (c)(iii).

Contacting may comprise contacting the subject with a nucleic acid, *e.g.,* a vector, *e.g.,* an AAV vector, described herein, *e.g.,* a nucleic acid that encodes at least one of (a), (b), and optionally (c)(i), further optionally (c)(ii), and still further optionally (c)(iii).

Contacting may comprise delivering to said subject said Cas9 molecule of (b), as a protein or mRNA, and a nucleic acid which encodes (a) and optionally (c)(i), further optionally (c)(ii), and still further optionally (c)(iii).

Contacting may comprise delivering to the subject the Cas9 molecule of (b), as a protein or mRNA, the gRNA molecule of (a), as an RNA, and optionally the second, third and/or fourth gRNA molecule of (c), as an RNA.

Contacting may comprise delivering to the subject the gRNA molecule of (a), as an RNA, optionally said second, third and/or fourth gRNA molecule of (c), as an RNA, and a nucleic acid that encodes the Cas9 molecule of (b).

Disclosed herein are kits comprising compositions of the invention and instructions for use.

### Ex vivo delivery

Components described in **Table 6** may be introduced into cells which may then be introduced into the subject. Methods of introducing the components can include, *e.g*., any of the delivery methods described in **Table 7**.

### VIII. Modified Nucleosides, Nucleotides, and Nucleic Acids

Modified nucleosides and modified nucleotides can be present in nucleic acids, *e.g.,* particularly gRNA molecule, but also other forms of RNA, *e.g.,* mRNA, RNAi, or siRNA. As described herein, "nucleoside" is defined as a compound containing a five-carbon sugar molecule (a pentose or ribose) or derivative thereof, and an organic base, purine or pyrimidine, or a derivative thereof. As described herein, "nucleotide" is defined as a nucleoside further comprising a phosphate group.

Modified nucleosides and nucleotides can include one or more of:
(i) alteration, *e.g*., replacement, of one or both of the non-linking phosphate oxygens and/or of one or more of the linking phosphate oxygens in the phosphodiester backbone linkage;
(ii) alteration, *e.g.,* replacement, of a constituent of the ribose sugar, *e.g.,* of the 2' hydroxyl on the ribose sugar;
(iii) wholesale replacement of the phosphate moiety with "dephospho" linkers;
(iv) modification or replacement of a naturally occurring nucleobase;
(v) replacement or modification of the ribose-phosphate backbone;
(vi) modification of the 3' end or 5' end of the oligonucleotide, *e.g*., removal, modification or replacement of a terminal phosphate group or conjugation of a moiety; and
(vii) modification of the sugar.

The modifications listed above can be combined to provide modified nucleosides and nucleotides that can have two, three, four, or more modifications. For example, a modified nucleoside or nucleotide can have a modified sugar and a modified nucleobase. Every base of a gRNA may be modified, *e.g.,* all bases have a modified phosphate group, *e.g.,* all are phosphorothioate groups. All, or substantially all, of the phosphate groups of a unimolecular (or chimeric) or modular gRNA molecule may be replaced with phosphorothioate groups.

Modified nucleotides, *e.g*., nucleotides having modifications as described herein, can be incorporated into a nucleic acid, *e.g.,* a "modified nucleic acid." The modified nucleic acids may comprise one, two, three or more modified nucleotides. At least 5% (*e.g.,* at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified nucleic acid may be modified nucleotides.

Unmodified nucleic acids can be prone to degradation by, *e.g.,* cellular nucleases. For example, nucleases can hydrolyze nucleic acid phosphodiester bonds. Accordingly, in one aspect the modified nucleic acids described herein can contain one or more modified nucleosides or nucleotides, *e.g*., to introduce stability toward nucleases.

The modified nucleosides, modified nucleotides, and modified nucleic acids described herein can exhibit a reduced innate immune response when introduced into a population of cells, both *in vivo* and *ex vivo.* The term "innate immune response" includes a cellular response to exogenous nucleic acids, including single stranded nucleic acids, generally of viral or bacterial origin, which involves the induction of cytokine expression and release, particularly the interferons, and cell death. The modified nucleosides, modified nucleotides, and modified nucleic acids described herein can disrupt binding of a major groove interacting partner with the nucleic acid. The modified nucleosides, modified nucleotides, and modified nucleic acids described herein can exhibit a reduced innate immune response when introduced into a population of cells, both *in vivo* and *ex vivo,* and also disrupt binding of a major groove interacting partner with the nucleic acid.

### Definitions of Chemical Groups

As used herein, "alkyl" is meant to refer to a saturated hydrocarbon group which is straight-chained or branched. Example alkyl groups include methyl (Me), ethyl (Et), propyl (*e.g.,* n-propyl and isopropyl), butyl (*e.g.,* n-butyl, isobutyl, t-butyl), pentyl (*e.g.,* n-pentyl, isopentyl, neopentyl), and the like. An alkyl group can contain from 1 to about 20, from 2 to about 20, from 1 to about 12, from 1 to about 8, from 1 to about 6, from 1 to about 4, or from 1 to about 3 carbon atoms.

As used herein, "aryl" refers to monocyclic or polycyclic (*e.g*., having 2, 3 or 4 fused rings) aromatic hydrocarbons such as, for example, phenyl, naphthyl, anthracenyl, phenanthrenyl, indanyl, indenyl, and the like. In an example, aryl groups have from 6 to about 20 carbon atoms.

As used herein, "alkenyl" refers to an aliphatic group containing at least one double bond.

As used herein, "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl.

As used herein, "arylalkyl" or "aralkyl" refers to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

As used herein, "cycloalkyl" refers to a cyclic, bicyclic, tricyclic, or polycyclic nonaromatic hydrocarbon groups having 3 to 12 carbons. Examples of cycloalkyl moieties include, but are not limited to, cyclopropyl, cyclopentyl, and cyclohexyl.

As used herein, "heterocyclyl" refers to a monovalent radical of a heterocyclic ring system. Representative heterocyclyls include, without limitation, tetrahydrofuranyl, tetrahydrothienyl, pyrrolidinyl, pyrrolidonyl, piperidinyl, pyrrolinyl, piperazinyl, dioxanyl, dioxolanyl, diazepinyl, oxazepinyl, thiazepinyl, and morpholinyl.

As used herein, "heteroaryl" refers to a monovalent radical of a heteroaromatic ring system. Examples of heteroaryl moieties include, but are not limited to, imidazolyl, oxazolyl, thiazolyl, triazolyl, pyrrolyl, furanyl, indolyl, thiophenyl pyrazolyl, pyridinyl, pyrazinyl, pyridazinyl, pyrimidinyl, indolizinyl, purinyl, naphthyridinyl, quinolyl, and pteridinyl.

### Phosphate Backbone Modifications

### Phosphate Group

The phosphate group of a modified nucleotide can be modified by replacing one or more of the oxygens with a different substituent. Further, the modified nucleotide, *e.g*., modified nucleotide present in a modified nucleic acid, can include the wholesale replacement of an unmodified phosphate moiety with a modified phosphate as described herein. The modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. One of the non-bridging phosphate oxygen atoms in the phosphate backbone moiety can be replaced by any of the following groups: sulfur (S), selenium (Se), BR₃ (wherein R can be, *e.g*., hydrogen, alkyl, or aryl), C (*e.g.,* an alkyl group, an aryl group, and the like), H, NR₂ (wherein R can be, *e.g.,* hydrogen, alkyl, or aryl), or OR (wherein R can be, *e.g.,* alkyl or aryl). The phosphorous atom in an unmodified phosphate group is achiral. However, replacement of one of the non-bridging oxygens with one of the above atoms or groups of atoms can render the phosphorous atom chiral; that is to say that a phosphorous atom in a phosphate group modified in this way is a stereogenic center. The stereogenic phosphorous atom can possess either the "R" configuration (herein Rp) or the "S" configuration (herein Sp).

Phosphorodithioates have both non-bridging oxygens replaced by sulfur. The phosphorus center in the phosphorodithioates is achiral which precludes the formation of oligoribonucleotide diastereomers. In an embodiment, modifications to one or both non-bridging oxygens can also include the replacement of the non-bridging oxygens with a group independently selected from S, Se, B, C, H, N, and OR (R can be, *e.g.,* alkyl or aryl).

The phosphate linker can also be modified by replacement of a bridging oxygen, (*i.e.,* the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

### Replacement of the Phosphate Group

The phosphate group can be replaced by non-phosphorus containing connectors. In an embodiment, the charge phosphate group can be replaced by a neutral moiety.

Examples of moieties which can replace the phosphate group can include, without limitation, *e.g*., methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

### Replacement of the Ribophosphate Backbone

Scaffolds that can mimic nucleic acids can also be constructed wherein the phosphate linker and ribose sugar are replaced by nuclease resistant nucleoside or nucleotide surrogates. In an embodiment, the nucleobases can be tethered by a surrogate backbone. Examples can include, without limitation, the morpholino, cyclobutyl, pyrrolidine and peptide nucleic acid (PNA) nucleoside surrogates.

### Sugar Modifications

The modified nucleosides and modified nucleotides can include one or more modifications to the sugar group. For example, the 2' hydroxyl group (OH) can be modified or replaced with a number of different "oxy" or "deoxy" substituents. Modifications to the 2' hydroxyl group can enhance the stability of the nucleic acid since the hydroxyl can no longer be deprotonated to form a 2'-alkoxide ion. The 2'-alkoxide can catalyze degradation by intramolecular nucleophilic attack on the linker phosphorus atom.

Examples of "oxy"-2' hydroxyl group modifications can include alkoxy or aryloxy (OR, wherein "R" can be, *e.g.,* alkyl, cycloalkyl, aryl, aralkyl, heteroaryl or a sugar); polyethyleneglycols (PEG), O(CH₂CH₂O)ₙCH₂CH₂OR wherein R can be, *e.g.,* H or optionally substituted alkyl, and n can be an integer from 0 to 20 (*e.g.,* from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). The "oxy"-2' hydroxyl group modification can include "locked" nucleic acids (LNA) in which the 2' hydroxyl can be connected, *e.g*., by a C₁₋₆ alkylene or C₁₋₆ heteroalkylene bridge, to the 4' carbon of the same ribose sugar, where exemplary bridges can include methylene, propylene, ether, or amino bridges; O-amino (wherein amino can be, *e.g.,* NH₂; alkylamino, dialkylamino, heterocyclyl, arylamino, diarylamino, heteroarylamino, or diheteroarylamino, ethylenediamine, or polyamino) and aminoalkoxy, O(CH₂)ₙ-amino, (wherein amino can be, *e.g.,* NH₂; alkylamino, dialkylamino, heterocyclyl, arylamino, diarylamino, heteroarylamino, or diheteroarylamino, ethylenediamine, or polyamino). The "oxy"-2' hydroxyl group modification can include the methoxyethyl group (MOE), (OCH₂CH₂OCH₃, *e.g.,* a PEG derivative).

"Deoxy" modifications can include hydrogen (i.e. deoxyribose sugars, *e.g.,* at the overhang portions of partially ds RNA); halo (*e.g*., bromo, chloro, fluoro, or iodo); amino (wherein amino can be, *e.g.,* NH₂; alkylamino, dialkylamino, heterocyclyl, arylamino, diarylamino, heteroarylamino, diheteroarylamino, or amino acid); NH(CH₂CH₂NH)ₙCH₂CH₂-amino (wherein amino can be, *e.g.,* as described herein), - NHC(O)R (wherein R can be, *e.g.,* alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g.,* an amino as described herein.

The sugar group can also contain one or more carbons that possess the opposite stereochemical configuration than that of the corresponding carbon in ribose. Thus, a modified nucleic acid can include nucleotides containing *e.g*., arabinose, as the sugar. The nucleotide "monomer" can have an alpha linkage at the 1' position on the sugar, *e.g.,* alpha-nucleosides. The modified nucleic acids can also include "abasic" sugars, which lack a nucleobase at C-1'. These abasic sugars can also be further modified at one or more of the constituent sugar atoms. The modified nucleic acids can also include one or more sugars that are in the L form, *e.g*., L-nucleosides.

Generally, RNA includes the sugar group ribose, which is a 5-membered ring having an oxygen. Exemplary modified nucleosides and modified nucleotides can include, without limitation, replacement of the oxygen in ribose (*e.g*., with sulfur (S), selenium (Se), or alkylene, such as, *e.g.,* methylene or ethylene); addition of a double bond (*e.g.,* to replace ribose with cyclopentenyl or cyclohexenyl); ring contraction of ribose (*e.g.,* to form a 4-membered ring of cyclobutane or oxetane); ring expansion of ribose (*e.g.,* to form a 6- or 7-membered ring having an additional carbon or heteroatom, such as for example, anhydrohexitol, altritol, mannitol, cyclohexanyl, cyclohexenyl, and morpholino that also has a phosphoramidate backbone). The modified nucleotides can include multicyclic forms (*e.g.,* tricyclo; and "unlocked" forms, such as glycol nucleic acid (GNA) (*e.g.,* R-GNA or S-GNA, where ribose is replaced by glycol units attached to phosphodiester bonds), threose nucleic acid (TNA, where ribose is replaced with α-L-threofuranosyl-(3'→2')).

### Modifications on the Nucleobase

The modified nucleosides and modified nucleotides described herein, which can be incorporated into a modified nucleic acid, can include a modified nucleobase. Examples of nucleobases include, but are not limited to, adenine (A), guanine (G), cytosine (C), and uracil (U). These nucleobases can be modified or wholly replaced to provide modified nucleosides and modified nucleotides that can be incorporated into modified nucleic acids. The nucleobase of the nucleotide can be independently selected from a purine, a pyrimidine, a purine or pyrimidine analog. The nucleobase can include, for example, naturally-occurring and synthetic derivatives of a base.

### Uracil

The modified nucleobase may be a modified uracil. Exemplary nucleobases and nucleosides having a modified uracil include without limitation pseudouridine (ψ), pyridin-4-one ribonucleoside, 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho⁵U), 5-aminoallyl-uridine, 5-halo-uridine (*e.g*., 5-iodo-uridine or 5-bromo-uridine), 3-methyl-uridine (m³U), 5-methoxy-uridine (mo⁵U), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s2U), 5-aminomethyl-2-thio-uridine (nm⁵s2U), 5-methylaminomethyl-uridine (mnm⁵U), 5-methylaminomethyl-2-thio-uridine (mnm⁵s2U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm ⁵s2U), 5-propynyl-uridine, 1-propynyl-pseudouridine, 5-taurinomethyl-uridine (τcm⁵U), 1-taurinomethyl-pseudouridine, 5-taurinomethyl-2-thio-uridine(τm⁵s2U), 1-taurinomethyl-4-thio-pseudouridine, 5-methyl-uridine (m⁵U, *i.e.,* having the nucleobase deoxythymine), 1-methyl-pseudouridine (m¹ψ), 5-methyl-2-thio-uridine (m⁵s2U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ψ), 5-(isopentenylaminomethyl)uridine (inm⁵U), 5-(isopentenylaminomethyl)-2-thio-uridine (inm⁵s2U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s2Um), 5-methoxycarbonylmethyl-2'-O-methyl-uridine (mcm ⁵Um), 5-carbamoylmethyl-2'-O-methyl-uridine (ncm ⁵Um), 5-carboxymethylaminomethyl-2'-O-methyl-uridine (cmnm ⁵Um), 3,2'-O-dimethyl-uridine (m³Um), 5-(isopentenylaminomethyl)-2'-O-methyl-uridine (inm ⁵Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, pyrazolo[3,4-d]pyrimidines, xanthine, and hypoxanthine.

### Cytosine

The modified nucleobase may be a modified cytosine. Exemplary nucleobases and nucleosides having a modified cytosine include without limitation 5-aza-cytidine, 6-aza-cytidine, pseudoisocytidine, 3-methyl-cytidine (m³C), N4-acetyl-cytidine (act), 5-formyl-cytidine (f⁵C), N4-methyl-cytidine (m⁴C), 5-methyl-cytidine (m⁵C), 5-halo-cytidine (*e.g*., 5-iodo-cytidine), 5-hydroxymethyl-cytidine (hm⁵C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k²C), α-thio-cytidine, 2'-O-methyl-cytidine (Cm), 5,2'-O-dimethyl-cytidine (m⁵Cm), N4-acetyl-2'-O-methyl-cytidine (ac⁴Cm), N4,2'-O-dimethyl-cytidine (m⁴Cm), 5-formyl-2'-O-methyl-cytidine (f⁵Cm), N4,N4,2'-O-trimethyl-cytidine (m⁴₂Cm), 1-thio-cytidine, 2'-F-ara-cytidine, 2'-F-cytidine, and 2'-OH-ara-cytidine.

### Adenine

The modified nucleobase may be a modified adenine. Exemplary nucleobases and nucleosides having a modified adenine include without limitation 2-amino-purine, 2,6-diaminopurine, 2-amino-6-halo-purine (*e.g.,* 2-amino-6-chloro-purine), 6-halo-purine (*e.g.,* 6-chloro-purine), 2-amino-6-methyl-purine, 8-azido-adenosine, 7-deaza-adenosine, 7-deaza-8-aza-adenosine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m¹A), 2-methyl-adenosine (m²A), N6-methyl-adenosine (m⁶A), 2-methylthio-N6-methyl-adenosine (ms2m⁶A), N6-isopentenyl-adenosine (i⁶A), 2-methylthio-N6-isopentenyl-adenosine (ms²i⁶A), N6-(cis-hydroxyisopentenyl)adenosine (io⁶A), 2-methylthio-N6-(cis-hydroxyisopentenyl)adenosine (ms2io⁶A), N6-glycinylcarbamoyl-adenosine (g⁶A), N6-threonylcarbamoyl-adenosine (t⁶A), N6-methyl-N6-threonylcarbamoyl-adenosine (m⁶t⁶A), 2-methylthio-N6-threonylcarbamoyl-adenosine (ms²g⁶A), N6,N6-dimethyl-adenosine (m⁶₂A), N6-hydroxynorvalylcarbamoyl-adenosine (hn⁶A), 2-methylthio-N6-hydroxynorvalylcarbamoyl-adenosine (ms2hn⁶A), N6-acetyl-adenosine (ac⁶A), 7-methyl-adenosine, 2-methylthio-adenosine, 2-methoxy-adenosine, α-thio-adenosine, 2'-O-methyl-adenosine (Am), N⁶,2'-O-dimethyl-adenosine (m⁶Am), N⁶-Methyl-2'-deoxyadenosine, N6,N6,2'-O-trimethyl-adenosine (m⁶₂Am), 1,2'-O-dimethyl-adenosine (m¹Am), 2'-O-ribosyladenosine (phosphate) (Ar(p)), 2-amino-N6-methyl-purine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

### Guanine

The modified nucleobase may be a modified guanine. Exemplary nucleobases and nucleosides having a modified guanine include without limitation inosine (I), 1-methyl-inosine (m¹I), wyosine (imG), methylwyosine (mimG), 4-demethyl-wyosine (imG-14), isowyosine (imG2), wybutosine (yW), peroxywybutosine (o₂yW), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-queuosine (manQ), 7-cyano-7-deaza-guanosine (preQo), 7-aminomethyl-7-deaza-guanosine (preQ₁), archaeosine (G⁺), 7-deaza-8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1-methyl-guanosine (m'G), N2-methyl-guanosine (m²G), N2,N2-dimethyl-guanosine (m²₂G), N2,7-dimethyl-guanosine (m²,7G), N2, N2,7-dimethyl-guanosine (m²,2,7G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m²Gm), N2,N2-dimethyl-2'-O-methyl-guanosine (m²₂Gm), 1-methyl-2'-O-methyl-guanosine (m'Gm), N2,7-dimethyl-2'-O-methyl-guanosine (m²,7Gm), 2'-O-methyl-inosine (Im), 1,2'-O-dimethyl-inosine (m'Im), O⁶-phenyl-2'-deoxyinosine, 2'-O-ribosylguanosine (phosphate) (Gr(p)), 1-thio-guanosine, O⁶-methyl-guanosine, O⁶-Methyl-2'-deoxyguanosine, 2'-F-ara-guanosine, and 2'-F-guanosine.

### Exemplary Modified gRNAs

The modified nucleic acids can be modified gRNAs. It is to be understood that any of the gRNAs described herein can be modified in accordance with this section, including any gRNAs that comprises a targeting domain from SEQ ID NOs: 387-485, 6803-6871, and 16010-16256.

As discussed above, transiently expressed or delivered nucleic acids can be prone to degradation by, *e.g.,* cellular nucleases. Accordingly, in one aspect the modified gRNAs described herein can contain one or more modified nucleosides or nucleotides which introduce stability toward nucleases. While not wishing to be bound by theory it is also believed that certain modified gRNAs described herein can exhibit a reduced innate immune response when introduced into a population of cells, particularly the cells of the present disclosure. As noted above, the term "innate immune response" includes a cellular response to exogenous nucleic acids, including single stranded nucleic acids, generally of viral or bacterial origin, which involves the induction of cytokine expression and release, particularly the interferons, and cell death.

While some of the exemplary modification discussed in this section may be included at any position within the gRNA sequence, in some embodiments, a gRNA molecule comprises a modification at or near its 5' end (*e.g*., within 1-10, 1-5, or 1-2 nucleotides of its 5' end). A gRNA may comprise a modification at or near its 3' end (*e.g*., within 1-10, 1-5, or 1-2 nucleotides of its 3' end). A gRNA molecule may comprise both a modification at or near its 5' end and a modification at or near its 3' end.

The 5' end of a gRNA may be modified by the inclusion of a eukaryotic mRNA cap structure or cap analog (*e.g.,* a *G(5')ppp(5')G* cap analog, a *m7G(5')ppp(5')G* cap analog, or a *3'-O-Me-m7G(5')ppp(5')G* anti reverse cap analog (ARCA)). The cap or cap analog can be included during either chemical synthesis or *in vitro* transcription of the gRNA.

An *in vitro* transcribed gRNA may be modified by treatment with a phosphatase (*e.g.,* calf intestinal alkaline phosphatase) to remove the 5' triphosphate group.

The 3' end of a gRNA may be modified by the addition of one or more (*e.g.,* 25-200) adenine (A) residues. The polyA tract can be contained in the nucleic acid (*e.g*., plasmid, PCR product, viral genome) encoding the gRNA, or can be added to the gRNA during chemical synthesis, or following *in vitro* transcription using a polyadenosine polymerase (*e.g., E. coli* Poly(A)Polymerase).

In another aspect, methods and compositions discussed herein provide methods and compositions for gene editing by using a gRNA molecule which comprises a polyA tail. A polyA tail of undefined length ranging from 1 to 1000 nucleotide may be added enzymatically using a polymerase such as *E. coli* polyA polymerase (E-PAP). The polyA tail of a specified length (*e.g.,* 1, 5, 10, 20, 30, 40, 50, 60, 100, or 150 nucleotides) may be encoded on a DNA template and transcribed with the gRNA via an RNA polymerase (*e.g*., T7 RNA polymerase). A polyA tail of defined length (*e.g.,* 1, 5, 10, 20, 30, 40, 50, 60, 100, or 150 nucleotides) may be synthesized as a synthetic oligonucleotide and ligated on the 3' end of the gRNA with either an RNA ligase or a DNA ligase with our without a splinted DNA oligonucleotide complementary to the guide RNA and the polyA oligonucleotide. The entire gRNA including a defined length of polyA tail may be made synthetically, in one or several pieces, and ligated together by either an RNA ligase or a DNA ligase with or without a splinted DNA oligonucleotide.

An *in vitro* transcribed gRNA molecule may contain both a 5' cap structure or cap analog and a 3' polyA tract. An *in vitro* transcribed gRNA may be modified by treatment with a phosphatase (*e.g*., calf intestinal alkaline phosphatase) to remove the 5' triphosphate group and comprises a 3' polyA tract.

gRNAs can be modified at a 3' terminal U ribose. For example, the two terminal hydroxyl groups of the U ribose can be oxidized to aldehyde groups and a concomitant opening of the ribose ring to afford a modified nucleoside as shown below: 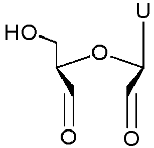 wherein "U" can be an unmodified or modified uridine.

The 3' terminal U can be modified with a 2'3' cyclic phosphate as shown below: 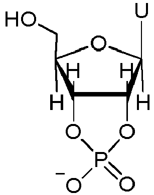 wherein "U" can be an unmodified or modified uridine.

The gRNA molecules may contain 3' nucleotides which can be stabilized against degradation, *e.g*., by incorporating one or more of the modified nucleotides described herein. In this case, *e.g.,* uridines can be replaced with modified uridines, *e.g.,* 5-(2-amino)propyl uridine, and 5-bromo uridine, or with any of the modified uridines described herein; adenosines and guanosines can be replaced with modified adenosines and guanosines, *e.g*., with modifications at the 8-position, *e.g*., 8-bromo guanosine, or with any of the modified adenosines or guanosines described herein.

Sugar-modified ribonucleotides can be incorporated into the gRNA molecule, *e.g.,* wherein the 2' OH-group is replaced by a group selected from H, -OR, -R (wherein R can be, *e.g.,* alkyl, cycloalkyl, aryl, aralkyl, heteroaryl or sugar), halo, -SH, -SR (wherein R can be, *e.g.,* alkyl, cycloalkyl, aryl, aralkyl, heteroaryl or sugar), amino (wherein amino can be, *e.g.,* NH₂; alkylamino, dialkylamino, heterocyclyl, arylamino, diarylamino, heteroarylamino, diheteroarylamino, or amino acid); or cyano (-CN). The phosphate backbone can be modified as described herein, *e.g*., with a phosphothioate group. One or more of the nucleotides of the gRNA can each independently be a modified or unmodified nucleotide including, but not limited to 2'-sugar modified, such as, 2'-O-methyl, 2'-O-methoxyethyl, or 2'-Fluoro modified including, *e.g.,* 2'-F or 2'-O-methyl, adenosine (A), 2'-F or 2'-O-methyl, cytidine (C), 2'-F or 2'-O-methyl, uridine (U), 2'-F or 2'-O-methyl, thymidine (T), 2'-F or 2'-O-methyl, guanosine (G), 2'-O-methoxyethyl-5-methyluridine (Teo), 2'-O-methoxyethyladenosine (Aeo), 2'-O-methoxyethyl-5-methylcytidine (m5Ceo), and any combinations thereof.

A gRNA can include "locked" nucleic acids (LNA) in which the 2' OH-group can be connected, *e.g*., by a C1-6 alkylene or C1-6 heteroalkylene bridge, to the 4' carbon of the same ribose sugar, where exemplary bridges can include methylene, propylene, ether, or amino bridges; O-amino (wherein amino can be, *e.g.,* NH₂; alkylamino, dialkylamino, heterocyclyl, arylamino, diarylamino, heteroarylamino, or diheteroarylamino, ethylenediamine, or polyamino) and aminoalkoxy or O(CH₂)ₙ-amino (wherein amino can be, *e.g.,* NH₂; alkylamino, dialkylamino, heterocyclyl, arylamino, diarylamino, heteroarylamino, or diheteroarylamino, ethylenediamine, or polyamino).

A gRNA can include a modified nucleotide which is multicyclic (*e.g*., tricyclo; and "unlocked" forms, such as glycol nucleic acid (GNA) (*e.g.,* R-GNA or S-GNA, where ribose is replaced by glycol units attached to phosphodiester bonds), or threose nucleic acid (TNA, where ribose is replaced with α-L-threofuranosyl-(3'→2')).

Generally, gRNA molecules include the sugar group ribose, which is a 5-membered ring having an oxygen. Exemplary modified gRNAs can include, without limitation, replacement of the oxygen in ribose (*e.g*., with sulfur (S), selenium (Se), or alkylene, such as, *e.g.,* methylene or ethylene); addition of a double bond (*e.g.,* to replace ribose with cyclopentenyl or cyclohexenyl); ring contraction of ribose (*e.g.,* to form a 4-membered ring of cyclobutane or oxetane); ring expansion of ribose (*e.g.,* to form a 6- or 7-membered ring having an additional carbon or heteroatom, such as for example, anhydrohexitol, altritol, mannitol, cyclohexanyl, cyclohexenyl, and morpholino that also has a phosphoramidate backbone). Although the majority of sugar analog alterations are localized to the 2' position, other sites are amenable to modification, including the 4' position. A gRNA may comprise a 4'-S, 4'-Se or a 4'-C-aminomethyl-2'-O-Me modification.

Deaza nucleotides, *e.g*., 7-deaza-adenosine, can be incorporated into the gRNA molecule. O- and N-alkylated nucleotides, *e.g*., N6-methyl adenosine, can be incorporated into the gRNA molecule. One or more or all of the nucleotides in a gRNA molecule are deoxynucleotides.

### miRNA binding sites

microRNAs (or miRNAs) are naturally occurring cellular 19-25 nucleotide long noncoding RNAs. They bind to nucleic acid molecules having an appropriate miRNA binding site, *e.g.,* in the 3' UTR of an mRNA, and down-regulate gene expression. While not wishing to be bound by theory it is believed that this down regulation occurs either by reducing nucleic acid molecule stability or by inhibiting translation. An RNA species disclosed herein, *e.g.,* an mRNA encoding Cas9 can comprise an miRNA binding site, *e.g.,* in its 3'UTR. The miRNA binding site can be selected to promote down regulation of expression is a selected cell type. By way of example, the incorporation of a binding site for miR-122, a microRNA abundant in liver, can inhibit the expression of the gene of interest in the liver.

### EXAMPLES

### Example 1: Cloning and Initial Screening of gRNA Molecules

The suitability of candidate gRNA molecules can be evaluated as described in this example. Although described for a chimeric gRNA molecule, the approach can also be used to evaluate modular gRNA molecules.

### Cloning gRNAs into Vectors

For each gRNA molecule, a pair of overlapping oligonucleotides is designed and obtained. Oligonucleotides are annealed and ligated into a digested vector backbone containing an upstream U6 promoter and the remaining sequence of a long chimeric gRNA molecule. Plasmid is sequence-verified and prepped to generate sufficient amounts of transfection-quality DNA. Alternate promoters maybe used to drive *in vivo* transcription (*e.g.,* H1 promoter) or for *in vitro* transcription (*e.g.,* a T7 promoter).

### Cloning gRNAs in linear dsDNA molecule (STITCHR)

For each gRNA molecule, a single oligonucleotide is designed and obtained. The U6 promoter and the gRNA scaffold (*e.g.,* including everything except the targeting domain, *e.g.,* including sequences derived from the crRNA and tracrRNA, *e.g*., including a first complementarity domain; a linking domain; a second complementarity domain; a proximal domain; and a tail domain) are separately PCR amplified and purified as dsDNA molecules. The gRNA-specific oligonucleotide is used in a PCR reaction to stitch together the U6 and the gRNA scaffold, linked by the targeting domain specified in the oligonucleotide. Resulting dsDNA molecule (STITCHR product) is purified for transfection. Alternate promoters may be used to drive *in vivo* transcription (*e.g.*, H1 promoter) or for *in vitro* transcription (*e.g*., T7 promoter). Any gRNA scaffold may be used to create gRNAs compatible with Cas9s from any bacterial species.

### Initial gRNA Screen

Each gRNA to be tested is transfected, along with a plasmid expressing Cas9 and a small amount of a GFP-expressing plasmid into human cells. In preliminary experiments, these cells can be immortalized human cell lines such as 293T, K562 or U2OS. Alternatively, primary human cells may be used. In this case, cells may be relevant to the eventual therapeutic cell target (for example, an erythroid cell). The use of primary cells similar to the potential therapeutic target cell population may provide important information on gene targeting rates in the context of endogenous chromatin and gene expression.

Transfection may be performed using lipid transfection (such as Lipofectamine or Fugene) or by electroporation (such as Lonza Nucleofection). Following transfection, GFP expression can be determined either by fluorescence microscopy or by flow cytometry to confirm consistent and high levels of transfection. These preliminary transfections can comprise different gRNAs and different targeting approaches (17-mers, 20-mers, nuclease, dual-nickase, etc.) to determine which gRNAs/combinations of gRNAs give the greatest activity.

Efficiency of cleavage with each gRNA may be assessed by measuring NHEJ-induced indel formation at the target locus by a T7E1-type assay or by sequencing. Alternatively, other mismatch-sensitive enzymes, such as CelI/Surveyor nuclease, may also be used.

For the T7E1 assay, PCR amplicons are approximately 500-700bp with the intended cut site placed asymmetrically in the amplicon. Following amplification, purification and size-verification of PCR products, DNA is denatured and re-hybridized by heating to 95°C and then slowly cooling. Hybridized PCR products are then digested with T7 Endonuclease I (or other mismatch-sensitive enzyme) which recognizes and cleaves non-perfectly matched DNA. If indels are present in the original template DNA, when the amplicons are denatured and re-annealed, this results in the hybridization of DNA strands harboring different indels and therefore lead to double-stranded DNA that is not perfectly matched. Digestion products may be visualized by gel electrophoresis or by capillary electrophoresis. The fraction of DNA that is cleaved (density of cleavage products divided by the density of cleaved and uncleaved) may be used to estimate a percent NHEJ using the following equation: %NHEJ = (1-(1-fraction cleaved)^{½}). The T7E1 assay is sensitive down to about 2-5% NHEJ.

Sequencing may be used instead of, or in addition to, the T7E1 assay. For Sanger sequencing, purified PCR amplicons are cloned into a plasmid backbone, transformed, miniprepped and sequenced with a single primer. Sanger sequencing may be used for determining the exact nature of indels after determining the NHEJ rate by T7E1.

Sequencing may also be performed using next generation sequencing techniques. When using next generation sequencing, amplicons may be 300-500bp with the intended cut site placed asymmetrically. Following PCR, next generation sequencing adapters and barcodes (for example Illumina multiplex adapters and indexes) may be added to the ends of the amplicon, *e.g*., for use in high throughput sequencing (for example on an Illumina MiSeq). This method allows for detection of very low NHEJ rates.

### Example 2: Assessment of Gene Targeting by NHEJ

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency to generate the desired mutations (either knockout of a target gene or removal of a target sequence motif) may be determined by sequencing. For Sanger sequencing, PCR amplicons may be 500-700 bp long. For next generation sequencing, PCR amplicons may be 300-500 bp long. If the goal is to knockout gene function, sequencing may be used to assess what percent of alleles have undergone NHEJ-induced indels that result in a frameshift or large deletion or insertion that would be expected to destroy gene function. If the goal is to remove a specific sequence motif, sequencing may be used to assess what percent of alleles have undergone NHEJ-induced deletions that span this sequence.

### Example 3: Assessment of Gene Targeting by HDR

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency can be determined by several methods.

Determination of gene targeting frequency involves measuring the percentage of alleles that have undergone homologous directed repair (HDR) with the exogenously provided donor template or endogenous genomic donor sequence and which therefore have incorporated the desired correction. If the desired HDR event creates or destroys a restriction enzyme site, the frequency of gene targeting may be determined by a RFLP assay. If no restriction site is created or destroyed, sequencing may be used to determine gene targeting frequency. If a RFLP assay is used, sequencing may still be used to verify the desired HDR event and ensure that no other mutations are present. If an exogenously provided donor template is employed, at least one of the primers is placed in the endogenous gene sequence outside of the region included in the homology arms, which prevents amplification of donor template still present in the cells. Therefore, the length of the homology arms present in the donor template may affect the length of the PCR amplicon. PCR amplicons can either span the entire donor region (both primers placed outside the homology arms) or they can span only part of the donor region and a single junction between donor and endogenous DNA (one internal and one external primer). If the amplicons span less than the entire donor region, two different PCRs should be used to amplify and sequence both the 5' and the 3' junction.

If the PCR amplicon is short (less than 600bp) it is possible to use next generation sequencing. Following PCR, next generation sequencing adapters and barcodes (for example Illumina multiplex adapters and indexes) may be added to the ends of the amplicon, *e.g.,* for use in high throughput sequencing (for example on an Illumina MiSeq). This method allows for detection of very low gene targeting rates.

If the PCR amplicon is too long for next generation sequencing, Sanger sequencing can be performed. For Sanger sequencing, purified PCR amplicons are cloned into a plasmid backbone (for example, TOPO cloned using the LifeTech Zero Blunt® TOPO® cloning kit), transformed, miniprepped and sequenced.

The same or similar assays described above can be used to measure the percentage of alleles that have undergone HDR with endogenous genomic donor sequence and which therefore have incorporated the desired correction.

### Example 4: Gene Targeting of the HBB Locus by CRISPR/Cas9 to Investigate Repair Pathway Choice in Response to Different Types of DNA Lesions

The CRISPR/Cas9 system was used to target the human *HBB* gene in the region of the sickle cell anemia-causing mutation.

To examine how the nature of the targeted break affects the frequency of different DNA repair outcomes, blunt double-strand breaks, single-strand nicks, and dual-nicks in which the nicks are placed on opposite strands and leave either 3' or 5' overhangs of varying lengths, were introduced by utilizing the wild type Cas9 nuclease, as well as two different Cas9 nickases. Several different DNA repair outcomes including indel mutations resulting from non-homologous end-joining, homology-dependent repair (HDR) using an exogenous donor as a template (*i.e.*, gene correction), and HDR using the closely related *HBD* gene as an endogenous template (*i.e.*, gene conversion), were characterized using either single-strand oligonucleotide (ssODN) or plasmid DNA donors. The frequency of these various repair outcomes under different conditions offer insight into the mechanisms of DNA repair and how it is impacted by the nature of the DNA break. The data also indicates a therapeutic approach in which correction of the sickle-cell mutation is efficiently mediated through HDR with either a donor template or with the *HBD* gene.

In this study different gRNA molecules for the *HBB* region that surrounds the nucleotides encoding the amino acid most commonly mutated in sickle cell disease had been tested in 293T cells with wild type Cas9 molecule. The gRNAs that induced similar high rates of NHEJ and had PAMs facing in opposite orientations (*e.g*., each PAM is oriented 5' relative to a mutation on the first and second strands, respectively) were selected to test as pairs with Cas9 D10A and Cas9 N863A nickases.

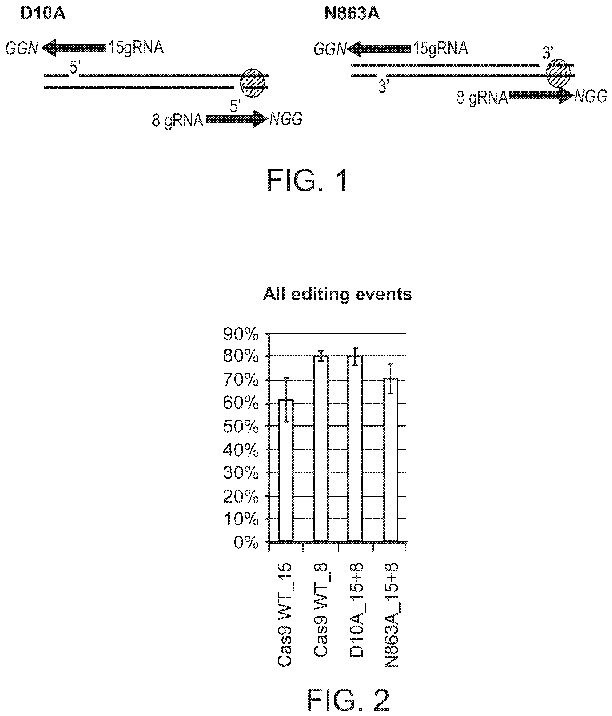

As shown in **Fig. 1**, the gRNA pair 8/15 ("8gRNA"/ "15gRNA" pair) was selected as one of the best pairs of gRNA molecules. "8gRNA" has the targeting domain sequence of GUAACGGCAGACUUCUCCUC (SEQ ID NO: 388) and "15gRNA" has the targeting domain sequence of AAGGUGAACGUGGAUGAAGU (SEQ ID NO: 387). This pair of gRNAs in combination with the mutant Cas9 D10A would generate a 5' overhang of 47 bp, and in combination with the mutant N863A would generate a 3' overhang of 47 bp. Different overhang structures are believed to have different properties for engaging different repair mechanisms. To address repair efficacy and repair outcomes in a systematic manner, U2OS cells with respective Cas9 variants and gRNAs were used.

U2OS cells were electroporated with 200 ng of each gRNA and 750 ng of plasmid that encodes wild type Cas9 or mutant Cas9. Cells were collected 6 days after electroporation and genomic DNA was extracted. PCR amplification of the *HBB* locus was performed and subcloned into a Topo Blunt Vector. For each condition in each experiment 96 colonies were sequenced with Sanger sequencing. In the experiments assessing HDR efficacy, cells were electroporated with 2.5 ug of single stranded oligo or double stranded oligo in addition to the gRNA and the Cas9-encoding plasmid.

As shown in **Fig. 2**, the similar rates of overall modification at the HBB locus were generated using wild type Cas9 or Cas9 nickases (D10A, N863A), as detected by Sanger sequencing, suggesting a similar rate in error prone repair independent of the Cas9-induced DNA lesion.

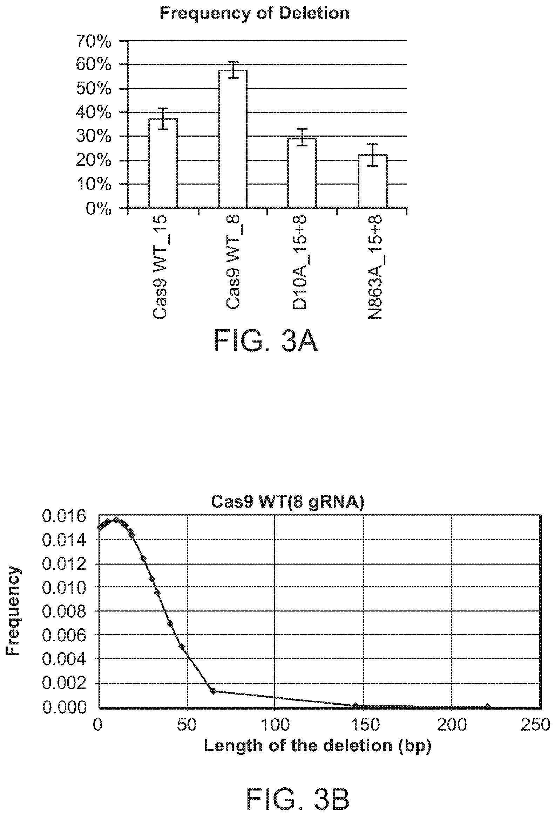

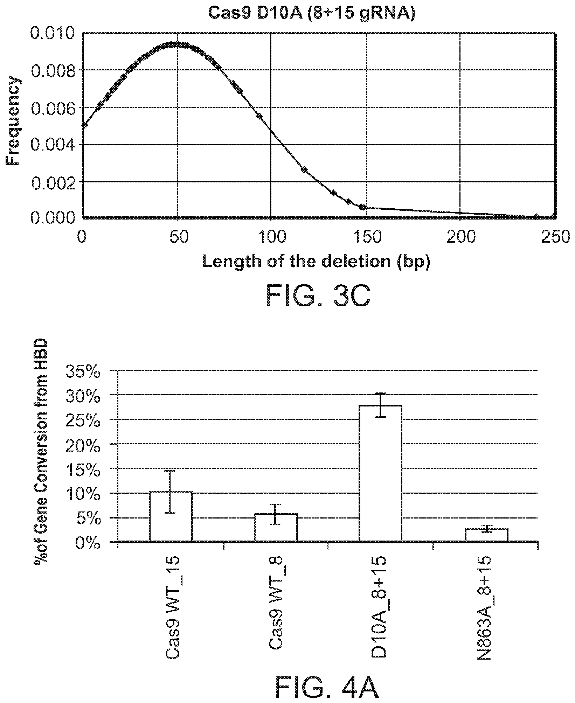

**Figs. 3A-3B** show that a majority of the total gene editing events (about 3/4 of the total) were small deletions (<10 bp). This is consistent with the notion that wild type Cas9 generates a blunt end which are preferentially repaired by canonical NHEJ. In contrast, deletions represented only about a quarter of the total events using either nickase (D10A or N863A). Moreover, larger deletions of -50 bp that can be mapped to the region between the two nickase sites were observed (**Fig. 3A** and **3C**). The remaining gene-editing events were substantially different between the two nickases.

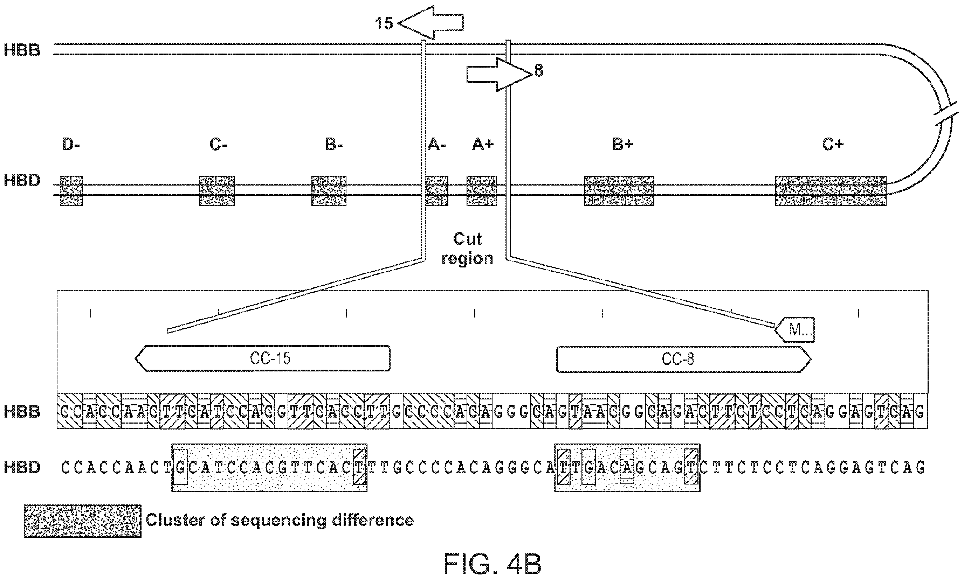

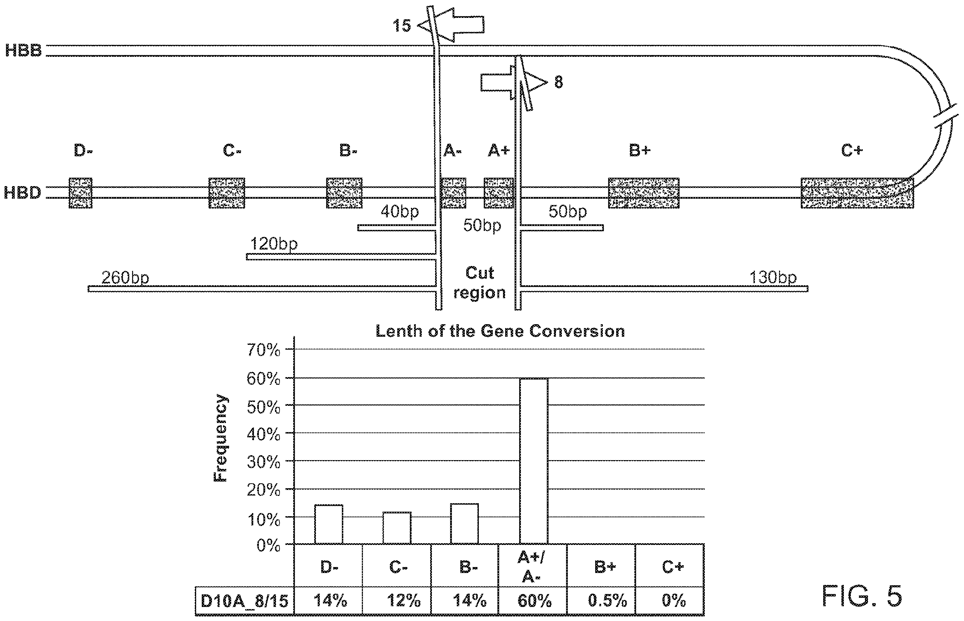

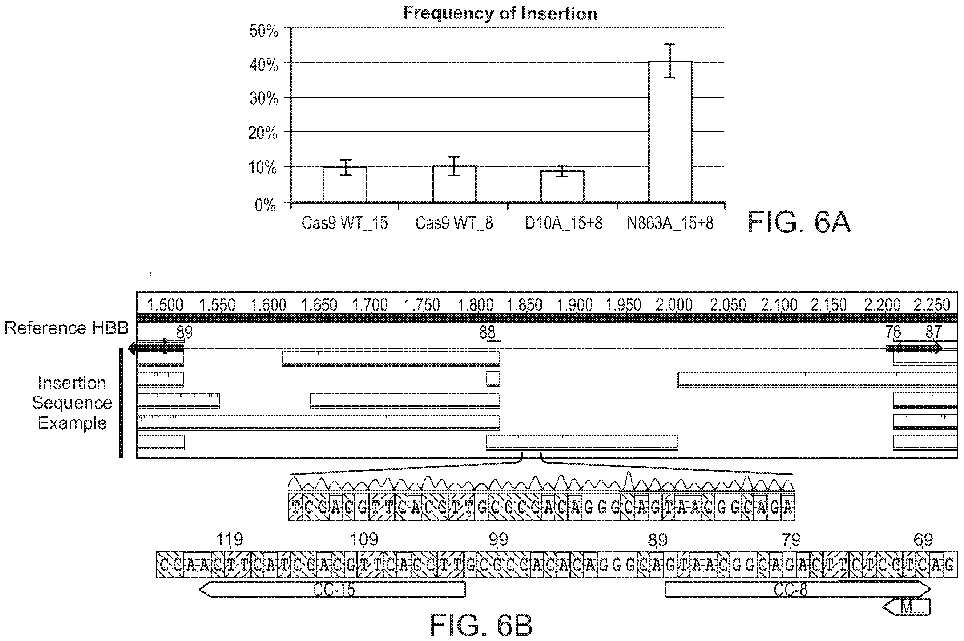

As shown in **Fig. 4A**, in the case of Cas9 D10A nickase, which leaves a 5' protruding end, the lesion is mostly repaired through a mechanism defined as gene conversion. In gene conversion, the *HBD* locus will serve as a template to repair the *HBB* gene. *HBD* is a highly similar gene (92% identity with *HBB*) that does not carry the sickle-cell mutation (**Fig. 4B**). **Fig. 5** shows that the majority of the *HBD* sequence that were incorporated in the *HBB* locus were in the region between the nickase cuts. In contrast, a low frequency of gene conversion was observed when the Cas9 N863 nickase was used (**Fig. 4A**). In the case of Cas9 N863A nickase, a majority of the gene editing events were insertions in which the inserted part was a duplication of the overhangs (**Figs. 6A-6B**).

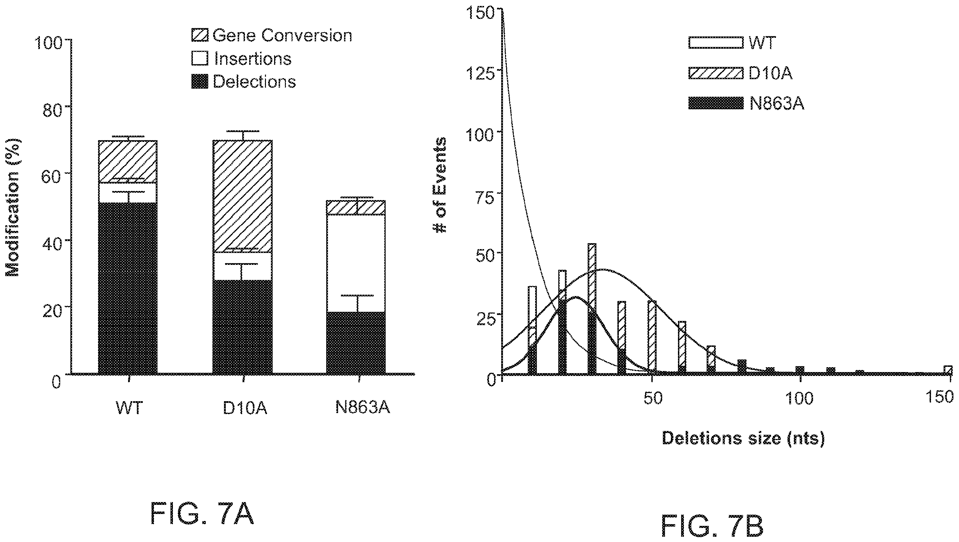

**Figure 7A** shows compiled data of the gene editing events resulting from DNA lesions generated by the three different Cas9 variants. While similar rates of overall modification at the *HBB* locus were observed, the distribution of the resulting type of repair outcome dramatically differed. 12.4% of WT Cas9-induced modifications harbored the footprint of the highly homologous *HBD* gene (a phenomenon known as "gene conversion"). In gene conversion, the *HBD* locus will serve as a template to repair the *HBB* gene. *HBD* is a highly similar gene (92% identity with *HBB*) that does not carry the sickle-cell mutation (**Fig. 6B**). The *HBD* gene lies approximately 7.6 kb upstream of the *HBB* gene on chromosome 11. The rate of gene conversion from the *HBD* gene was significantly enhanced when lesions were induced using the Cas9 D10A nickase. 32.8% of D10A Cas9-induced modifications were resolved by gene conversion. In contrast, 3.5% of N863A Cas9-induced modifications were resolved by gene conversion. Overall, this data suggested that DNA cleavage induced by the Cas9 D10A nickase are particularly amenable for gene conversion from the endogenous *HBD* gene. These data also suggest that the different DNA ends generated with different Cas9 variants activate different DNA repair pathway responses.

**Figure 7B** shows the distribution of deletion size resulting from DNA lesions generated by the three different Cas9 variants. Specifically, WT Cas9-induced lesions were predominantly resolved as deletions. Deletions were also frequently observed when using the gRNA pair 8/15 with either the Cas9 D10A nickase or Cas9 N863A nickase. WT Cas9 induced deletions were predominantly small in size (median deletion size = 3 nucleotides (nts)). Cas9 D10A nickase-induced deletions and Cas9 N863A nickase-induced deletions were significantly larger in size (median deletion size = 28 nts and 36 nts, for Cas9 N863A and Cas9 D10A, respectively).

To determine whether the DNA repair events observed were representative of events occurring in single cells, and not the result of PCR bias during amplification of the DNA from a population of cells, repair outcomes were analyzed from a single U2Os cell clones (∼ 80 single clones per condition) after nucleofection with each of the three Cas9 variants. As shown in **Figure 8A**, the repair event distribution was very similar in individual clones to the distribution observed in parental population of mixed cells. Similar repair event distribution was observed using the myeloid leukemia cell line K562 (**Fig. 8B**). These data suggest that the DNA ends generated with the different Cas9 variants activate different DNA repair pathway responses.

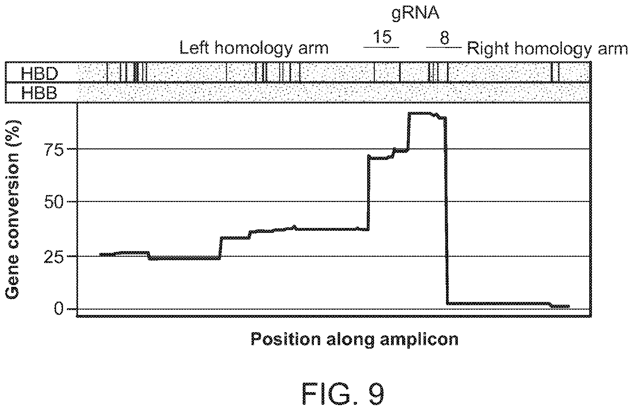

To better understand the molecular pathways responsible for the gene conversion pathway, Cas9 D10A nickase-induced gene conversion tracts were analyzed in detail. The region located between the 8gRNA/ 15gRNA pair was converted to the *HBD* sequence in approximately 80% of all gene conversion cases, as detected by analyzing single nucleotide polymorphisms (SNPs) that perfectly matched the *HBD* sequence (**Fig. 9**). Directionality in the gene conversion tracts was observed, suggesting that perhaps the homology arm to the right of 8gRNA more frequently engages in homology pairing with the *HBD* locus due to the presence of longer stretches of uninterrupted homology, resulting in effective gene conversion (**Fig. 9**). In contrast, the arm to the left of 15gRNA harbors more homology-disrupting SNPs, which we speculated could result in a higher frequency of heteroduplex rejection at the *HBD* site.

Gene conversion is a highly precise mechanism that repairs DSBs during the S/G2 phases of the cell cycle through the homologous recombination (HR) pathway. The genetic requirements of HR have been characterized. Briefly, initially 3-5' resection leads to the exposure of a single stranded 3' overhang. Subsequently, BRCA2-dependent RAD51 loading onto the single stranded DNA initiates homology search and strand invasion. To determine whether gene conversion proceeds through the HR pathway, BRCA2 and RAD51 were knocked-down using siRNA in U2OS cells, and gene conversion analyzed following Cas9 D10A nickase-induced lesions (**Fig. 10A**). Gene conversion outcomes were significantly decreased when either BRCA2 or RAD51 were knocked down, indicating that both genes are required for efficient gene conversion and suggesting that gene conversion from the *HBD* locus proceeds through the HR pathway (**Fig. 10B**).

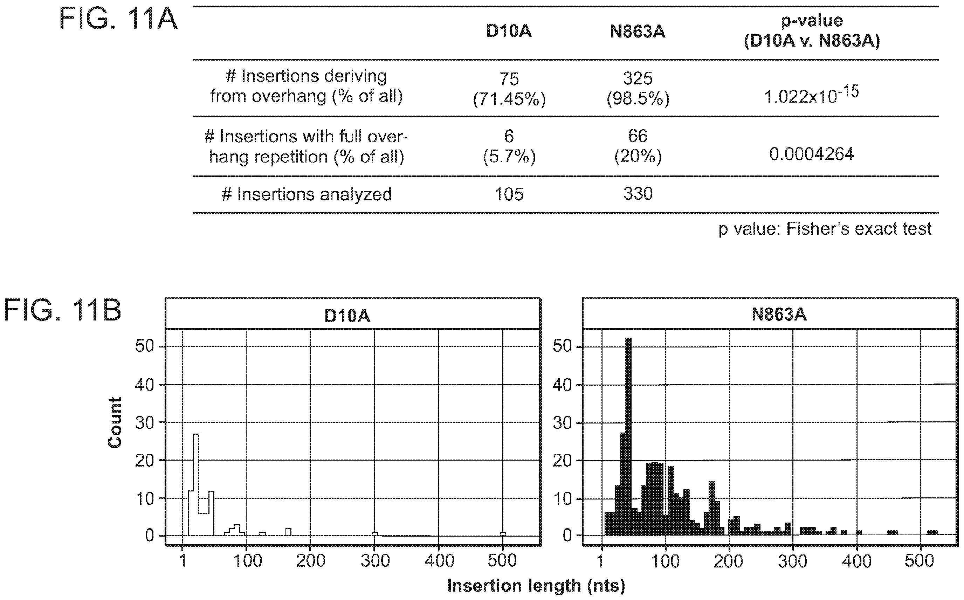

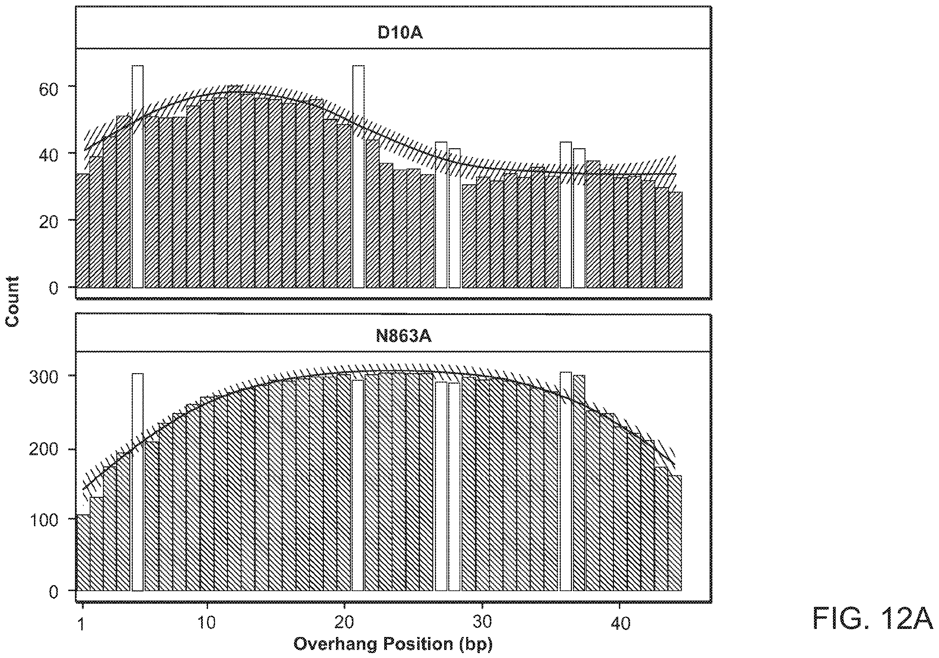

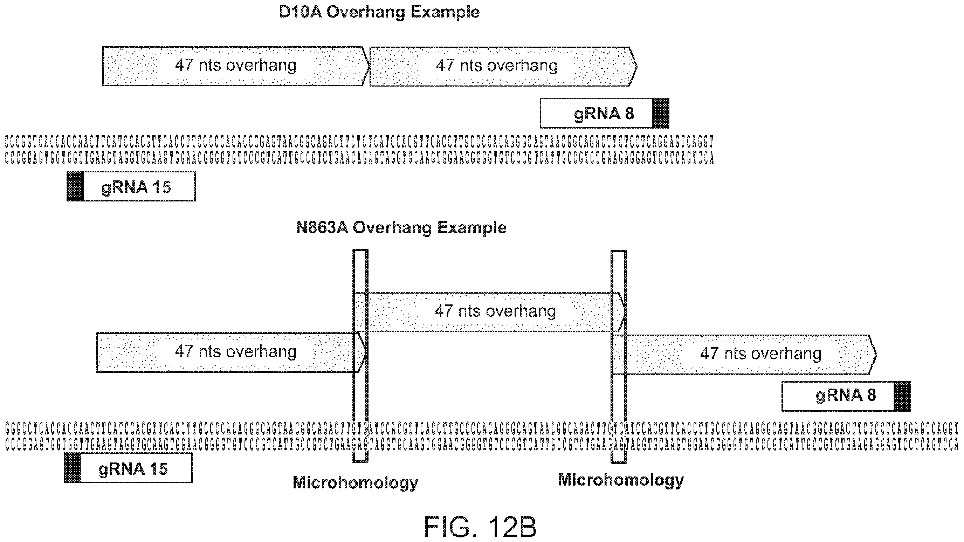

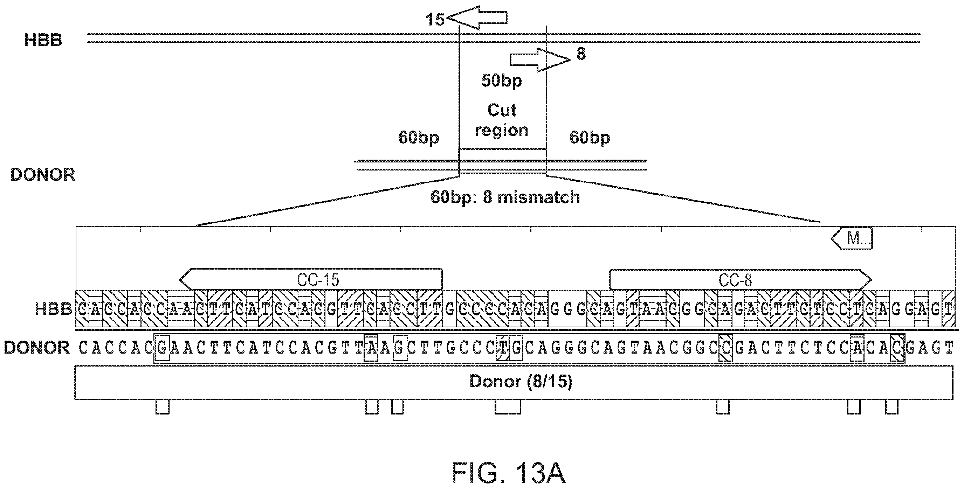

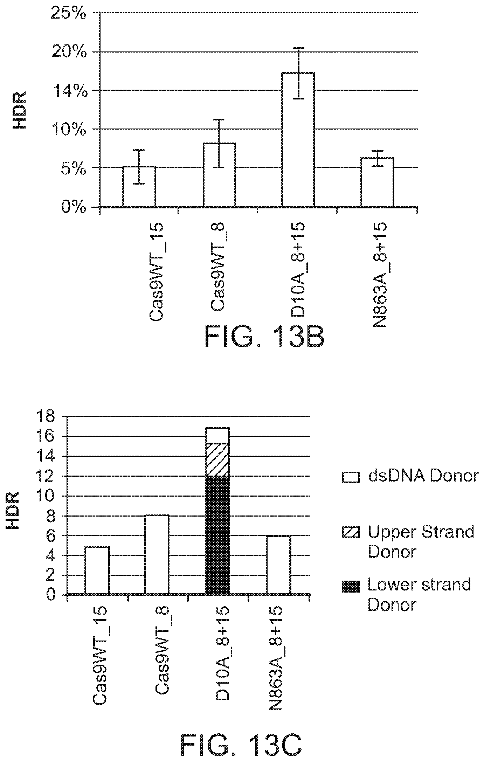

To investigate the fate of the predicted Cas9 N863A nickase-induced 3' overhang structure, the repair outcome of these lesions was analyzed in detail. While the predominant repair outcome for these lesions was insertions (29.9% of all modifications, as compared to 9.0% for modifications induced by the Cas9 D10A nickase), the majority of the insertions mapped to the overhang (98.5% of insertions), while only 71.4% of insertions induced by the Cas9 D10 nickase mapped to the overhang (**Fig. 11A**). Cas9 N863A nickase-induced insertions are significantly longer than the insertions induced by Cas9 D10A nickase (**Fig. 11B**). Cas9 N863A nickase-induced insertions also predominantly stem from the central part of the overhang, which is different from the Cas9 D10A nickase-induced insertions, which mostly derive from the first 20 nts of the overhang structure (**Fig. 12A**). Moreover, Cas9 N863A nickase-induced insertions more frequently contained several (up to seven) repetitions of the entire 47 nts overhang structure generated upon cleavage, and in all cases of full overhang repetition, microhomology usage was evident (**Fig. 11A** **and** **12B**). These observations suggest a mechanistic difference between the Cas9 N863A nickase- and Cas9 D10A nickase-induced insertions. To test the effect that different lesions had on the engagement of HDR, a donor template was provided as a single-strand oligo or as ds DNA donor. In both cases the length of the donor is approximately 170 bp with 60 bp of homology outside the nicks and with 8 mismatches (**Fig. 13A**). As shown in **Fig. 13B****,** the Cas9 D10A nickase that resulted in a 5' overhang resulted in a significantly higher rate of HDR, especially when using the upper stand as a single-strand oligo donor. **Fig. 13C** shows different forms of donors (dsDNA, upper stand, and lower strand) and there contribution to HDR.

To further investigate the different repair outcomes resulting from the use of the different Cas9 variants, gene correction efficiency using a 179 nt ssODN that targets the *HBB* locus in the area of the sickle mutation was tested. Similar to the outcome observed during gene conversion, Cas9 D10A nickase, which creates a 5' overhang, yielded the highest rate of gene correction (23.8% for Cas9 D10A nickase, as compared to 7.7% for WT Cas9 (p=0.0024) and 7.5% for Cas9 N863A nickase (p=0.0016)) (**Fig. 14**). To further dissect the genetic requirements of the ssODN gene correction approach, HR components BRCA2 and RAD51 were knocked down using siRNA, No change in gene correction frequency was observed, indicating that gene correction using a ssODN proceeds through a pathway that is independent of BRCA2 and RAD51 (**Fig. 10C**).

Given the differences in repair outcome and repair pathway engagement observed using the different Cas9 variants using a paired nickase strategy, repair outcome and repair pathway engagement resulting from a single nicking strategy was investigated.

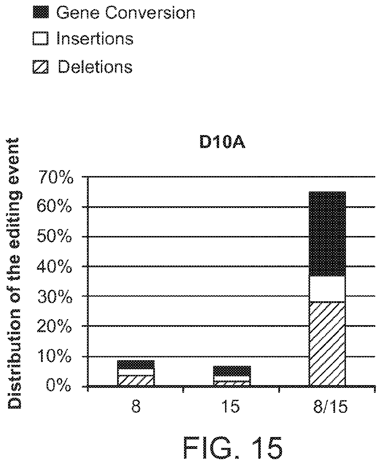

First, the repair outcome in cells treated with the Cas9 D10A nickase with either a single gRNA or two gRNA molecules was analyzed. As shown in **Fig. 15**, gene conversion was observed with Cas9 D10A nickase with a single gRNA molecule (*i.e.*, 8gRNA or 15 gRNA), as compared to two gRNA molecules (8gRNA/ 15gRNA pair). With a single gRNA, a reduction in the overall frequency of gene editing events was observed, as compared to the use of a pair of gRNA molecules. However, a similar distribution of the different types of editing events was maintained.

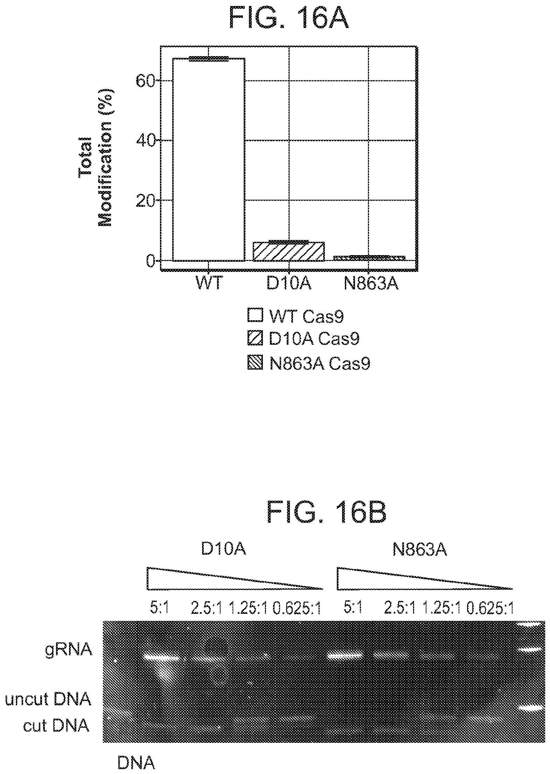

In additional experiments, the repair outcome and repair pathway engagement was examined in cells treated with the 8gRNA in combination with either the Cas9 D10A nickase, which results in a single DNA nick in the target strand, with the Cas9 N863A nickase, which results in a single DNA nick in the non-target strand, or with the control WT Cas9, which results in a blunt double stranded break. The overall modification frequency observed for WT Cas9-induced lesions was significantly higher than that observed for the Cas9 D10A nickase- and Cas9 863A nickase-generated single nicks (66.3% for WT Cas9, as compated to 4.6% for Cas9 D10A nickase and 1.3% for Cas9 N863A nickase) (**Fig. 16A**). This difference suggests the presence of a highly efficient single nick repair mechanism. To determine whether the difference in detectable repair events between Cas9 D10A nickase-induced single nick lesions and Cas9 N863A nickase-induced single nick lesions (approximately 4 fold; 4.6% for Cas9 D10A nickase vs. 1.3% for Cas9 N863A nickase, p=3.1 x 10⁻⁶) was attributable to differences in cutting efficiency between the two nickase variants, an *in vitro* DNA cutting assay was performed in which a gel-shifted DNA band indicates successful DNA cleavage. No significant differences in cutting efficiency between the Cas9 D10A nickase or the Cas9 N863A nickase were observed (**Fig. 16B**), suggesting that either the DNA repair of Cas9 N863A nickase-induced single nick of the non-target strand proceeds more efficiently than the repair of the cleaved target DNA strand, or that Cas9 D10A nickase-induced nick results in a more mutagenic intermediate.

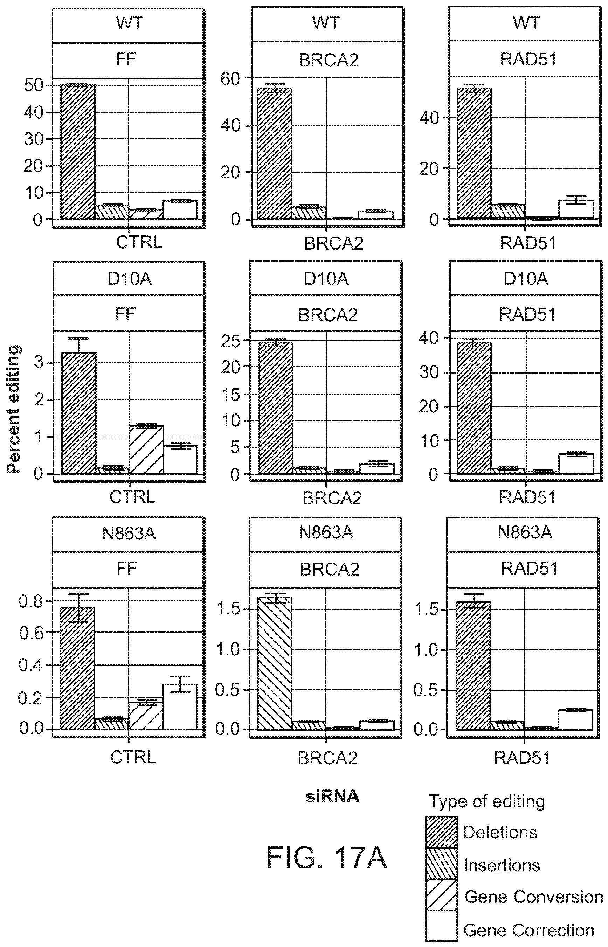

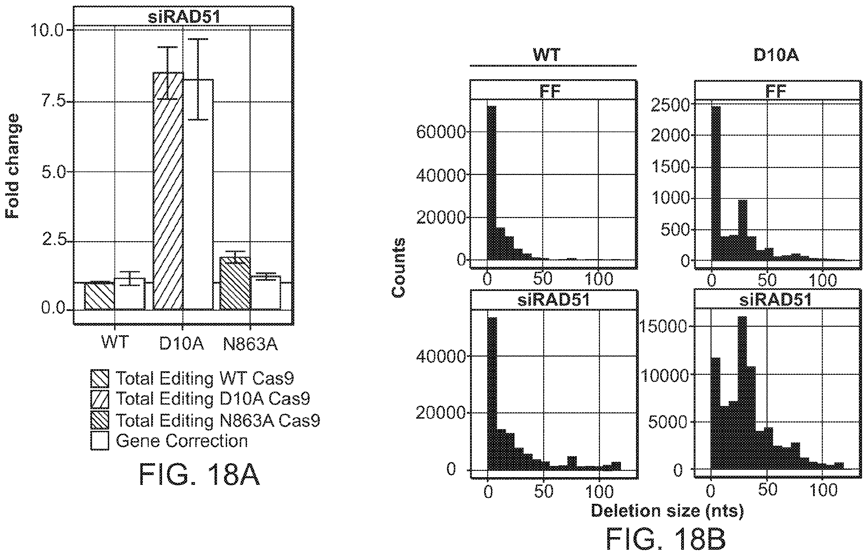

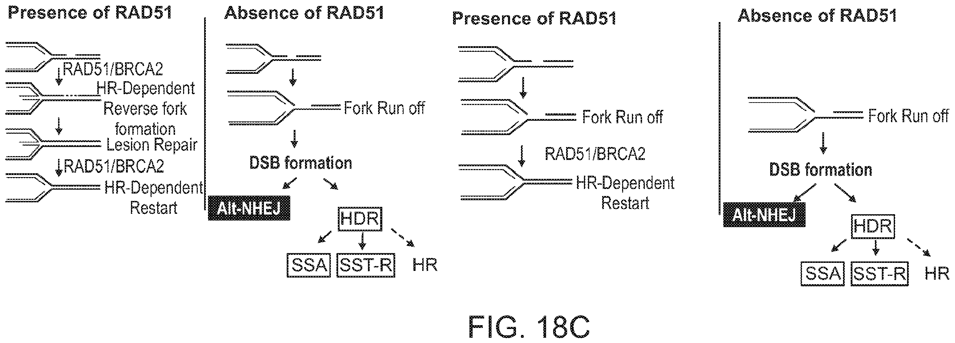

To determine whether gene correction and gene conversion events that result from single nicking using a Cas9 nickase variant proceed through similar pathways as described above for the paired nickase approach (i.e., utilizing a gRNA pair and Cas9 nickase), BRCA2 and RAD51 were knocked-down using siRNA in U2OS cells, and repair outcomes analyzed. Gene conversion was dependent on RAD51 for WT Cas9-, Cas9 D10A nickase-, and Cas9 N863A nickase-induced single nicks (**Fig. 17A** **and** **18A**). An approximately 6-fold increase in gene correction was observed upon RAD51 knockdown for Cas9 D10A nickase-induced single nicks (from 1.0% in FF control to 6.24% in siRAD51-treated cells). However, an increase in gene correction was not observed upon RAD51 knockdown for Cas9 N863A nickase-induced single nicks (**Fig. 17A** **and** **18A**). Similar results were obtained with BRCA2 depletion (**Fig. 17A****).** An equivalent simultaneous increase in overall modifications for Cas9 D10A nickase-induced nicks was also observed upon RAD51 knockdown (from 6.1% with the FF control to 48.4% in siRAD51-treated cells), without a relative increase in gene correction efficiency (**Fig. 17A** **and** **18A**). A significant increase in the size of deletions resulting from Cas9 D10A nickase-induced single nicks was also observed upon RAD51 knockdown (**Fig. 18B**). Overall, these observations suggest that Cas9 D10A nickase-induced nicks are converted to double stranded breaks during S-phase and are then repaired by more error prone pathways than HR. It is possible that Cas9 D10A nickase-induced lesions might not be sensed until S-phase, when they are converted to DSBs (see, *e.g.,* Mayle 2015; Saleh-Gohari 2005; Neelsen and Lopes 2015). This may be due to the inability of RAD51- and BRCA2-deficient cells to undergo replication fork remodeling to prevent replication fork run-off or undesirable processing at the Cas9 D10A nickase-induced nick (**Fig. 18C****,** left panel) (see, *e.g.,* Neelsen and Lopes 2015; Schlacher 2011; Zellweger 2015). Alternatively, the high degree of locus disruption could be due to the inability of RAD51/BRCA2 deficient cells to complete HR if run-off has occurred (**Fig. 18C****,** right panel). The observed increase in the size of the deletions in the absence of RAD51 could suggest the involvement of A-NHEJ pathways in the repair of Cas9 D10A nickase-induced nicks during S-phase. These data cumulatively provide evidence that both RAD51 and BRCA2 are critical in promoting high fidelity repair of nicks that escape the SSBR pathway.

In summary, Cas9 nickases (D10A and N863A) showed comparable levels of efficacy compared to wild type Cas9. Different DNA ends engage different repair pathways. The use of a wild type Cas9 generates a blunt end, which are preferentially repaired by canonical NHEJ. Use of a Cas9 nickase with two gRNAs generates either 3' or 5' overhangs, which are not suitable substrates to be repaired by canonical NHEJ but can be repaired by alternative pathways.

The 5' protruding end was mostly repaired through a mechanism called gene conversion in which the *HBB* gene is repaired by using the *HBD* locus as a template. Use of nickase is advantageous to promote HDR. In the experiments in which a donor was provided, a significantly higher rate of HDR was observed using the Cas9 D10A nickase as compared to the wild type Cas9. Also, a significantly higher rate of gene correction was observed using the Cas9 D10A nickase as compared to the wild type Cas9 or the Cas9 N863A nickase. The nature of the donor template influences the outcome as HDR was preferentially observed when a single stranded (SS) oligo was used.

### Methods

*gRNA selection and production* - A list of gRNAs directing *S. pyogenes* Cas9 were designed to target the human *hemoglobin beta (HBB)* locus requiring a NGG PAM (protospacer adjacent motif). Potential gRNAs were aligned against the human genome sequence to identify potential off-target sites. Variations in PAM sequence (NGG or NAG) were allowed for gRNAs with spacer lengths of 20 nucleotides. Primers containing gRNA sequences were synthesized (Integrated DNA Technologies (IDT)). gRNAs 8 and 15 were generated by cloning annealed gRNA sequence oligos containing the target sequence into pMLM3636 (Addgene plasmid # 43860) which contains a *S*. *pyogenes* Cas9-TRACR and a customizable U6-driven gRNA scaffold. Plasmid DNA was purified using MaxiPrep Kit (ThermoFisher) and sequences were confirmed by Sanger sequencing.

*Cell lines and cell culture* - HEK293 (ATCC #CRL-1573), K562 (ATCC #CCL-243) and U2-OS (ATCC #HTB-96) cells were maintained in Dulbecco's Modified Eagle Medium (DMEM; Life Technologies) supplemented with 10 % fetal bovine serum (FBS) and 5 % penicillin/streptomycin, and 2mM Glutamax. Cells were kept at 37 °C in a 5% CO₂ incubator.

*Transfections*/*Electroporations* - 2.5 x 10⁵ cells were transfected using the Lonza 4D Nucleofector (AAF-1002B 4D-Nucleofector™ Core unit, AAF-1002X 4D-Nucleofector™ X unit, SE Cell Line 4D-Nucleofector X Kit S V4XC-1032) with 200 ng of gRNA plasmid and 750 ng of Cas9-variant encoding plasmid (pJDS246-WT; Cas9/pAF001-N863A; Cas9/pJDS271-D10ACas9) in the presence or absence of 50 pmol single-stranded deoxynucleotide donor (ssODN) using the DN-100 program. After nucelofection, cells were incubated at room temperature for 10 min. and then resuspended in complete DMEM before transfer to 6-well plates containing pre-warmed media. Media was changed 72 hours after nucleofection, and cells were harvested 5 days post nucleofection.

*siRNA Knockdowns* - To knock down BRCA2 and RAD51, immediately before nucleofection with Cas9-variant encoding plasmid and gRNA(s), cells were treated with 30 pmols of siBRCA2 (siGENOME Human BRCA2 (675) siRNA - SMARTpool; M-003462-01-0005), 15 pmols of both siRAD51 (siGENOME Human RAD51 (5888) siRNA; D-003530-05-0005), or 15 pmols of control firefly luciferase siRNA ("FF") (FF siRNA; CTM-127256). Samples were collected for gDNA extraction and further processing 5 days after nucleofection and knockdown efficiency assessed by Western Blot.

*Genomic DNA Analysis* - Genomic DNA was extracted from cells (Beckman Coulter Agencourt DNAdvance #A48706) according to the manufacturer's directions. The targeted genomic region of *HBB* was amplified by PCR in a 50 µl reaction mixture (10 µL of 5X Phusion HF buffer; 0.5 µM forward primer (F1_HBB, 5'-AGGCCATCACTAAAGGCACC (SEQ ID NO: 16313)); 0.5 µM reverse primer (R_HBB, 5'-TAAGCCAGTGCCAGAAGAGC (SEQ ID NO:16314)); 200 µM dNTP; 1.5 µL DMSO; 0.5 µl of Phusion polymerase; 100 ng of gDNA template) using the following PCR conditions: 30 s at 98°C for initial denaturation, followed by 30 cycles of 10 seconds (s) at 98°C for denaturation, 15s at 64°C for annealing, 30s at 72°C for extension, and 5 minutes at 72°C for the final extension. PCR products were purified using (1.8×) Agencourt AMPure XP beads (Beckman Coulter Agencourt AMPure XP - PCR Purification #A63882) as directed by the manufacturer. Amplified locus fragments were cloned into pCR4-TOPO vectors using the ZeroBlunt TOPO Cloning Kit (Life Technologies Cat. No. #K287540) and transformed in One Shot Top10 chemically competent *E. coli* cells. Cells were plated on carbenicillin LB agar plates and incubated overnight at 37°C. Plasmid DNA from 96 colonies per sample was sequenced at Macrogen Corp. and Genewiz, Inc. using an M13 forward or reverse primer.

*Single cell cloning* - 2.5 x 10⁵ U2OS cells per condition were nucleofected using Lonza 4D Nucleofector (AAF-1002B 4D-Nucleofector™ Core unit, AAF-1002X 4D-Nucleofector™ X unit). The cells were plated in 6 well dishes. For each of the conditions, 0.3 cells/well were plated in 3X 96 well plates. After 3 weeks, the 96 well plates were visually scored using microscopy for single colony formations. More than 80 clones were picked for each condition and analyzed by TOPO cloning the PCR products of the amplified locus. Competent cells were plated onto divided bioassay plates with LB agar containing ampicillin (Molecular Devices Vented QTray, Fischer Scientific Cat. No. # NC0183882) and 6 colonies were picked for each subclone. Genomic DNA was extracted from cells using DNAdvance kits (Beckman Coulter Agencourt DNAdvance - Nucleic Acid Isolation from Mammalian Tissue #A48706). The *HBB* locus of the genomic DNA from individual clones was analyzed as described above. 6 colonies were analyzed for each subclone.

*Illumina Next Generation Sequencing* - Two rounds of PCR were performed on gDNA. The first round of amplification was used to amplify the *HBB* locus. Amplification was performed in a 50 µl reaction volume, (10 µL of 5X HercII buffer; 0.5 µM forward primer (HBB Miseq 1F: 5' CCATCTCATCCCTGCGTGTCTCCGACCACCAGCAGCCTAAGG (SEQ ID NO:16315)); 0.5 µM reverse primer (HBB Miseq 1R: 5' CCTCTCTATGGGCAGTCGGTGATGGCCATCTATTGCTTACATTTGCT (SEQ ID NO:16316)); 50 µM dNTP; 50mM MgCl₂; 0.5 µl of HercII polymerase; 250 ng of gDNA template) using the following conditions: 2 min. at 95°C for initial denaturation, followed by 30 cycles of 20 s at 95°C for denaturation, 20 s at 60°C for annealing, 20 s at 72°C for extension, and 3 min. at 72°C for the final extension. A second round of PCR amplification was performed to incorporate the P7 and P5 Illumina adapters and a unique 8-mer barcode sequence in a 50 µl reaction mixture (10 µL of 5X HercII buffer; 0.5 µM forward primer; 0.5 µM reverse primer; 50uM dNTP; 0.5ul of HercII polymerase; 3ul of round 1 PCR product) using the amplification conditions as the first round of PC R amplification. PCR products were purified using (1.8×) Agencourt AMPure XP beads, as directed by the manufacturer, eluted in 40 µl of H₂0, and size verified by capillary electrophoresis using the Qiagen QIAxcel Advanced System. PCR products were quantified using Picogreen, normalized into one library pool and sequenced on the Illumina MiSeq according to the manufacturer's protocols.

*Quantification of editing events in NGS data* - The CRISPResso software ( v0.9.1)(see, *e.g.,* Pinello *et al.*) was used to identify editing events in high-throughput sequencing data and determine the frequencies of insertion, deletion, gene conversion and gene correction events. To identify insertions and deletions events, CRISPResso default settings were used to obtain the number of reads with either insertions or deletions. To identify gene conversion and gene correction events, CRISPResso was used separately for each sample and potential HDR template (*i.e.*, the orthologous HBD sequence and the ssODN) with a required sequence similarity of 95% (using the hdr_perfect_alignment_threshold parameter).

*Determination of insertion sizes* - To identify insertions from Sanger sequencing data, the Needleman-Wunsch algorithm implementation in the Biopython (pairwise2.globalms) package was used with the following parameters: base match score = 2, base mismatch penalty = -1, gap open penalty = -50, gap extend penalty = -1. Insertion sequences were identified by taking the contiguous bases aligned to gap characters in the reference sequence.

*Overhang analysis* - Overhang sequence was identified as located between the two predicted cleavage sites for guide RNA pairs, under the assumption that cleavage occurs three nucleotides 5' of the start of the NGG PAM. For the gRNA 8/15 pair, the predicted overhang sequence is:
TCATCCACGTTCACCTTGCCCCACAGGGCAGTAACGGCAGACTTCTC (SEQ ID NO:16317).
Insertion sequences were classified according to the absence of matching overhang sequence, the presence of a partial matching sequence (at least one 9-mer in common), or a the presence off a full matching sequence (complete overhang sequence contained within the insertion sequence). Fisher's exact test was used to determine statistical significance of differences in the proportion of sequences with partial or full overhang matches generated with Cas9 D10A nickase or Cas9 N863A nickase. Segments of the overhang sequence overrepresented in insertions were identified by extracting all 4-mer sequences present in each insertion sequence and identifying the positions in the overhang where the 4-mer sequence exactly matched. 4-mers found multiple times within the overhang sequence were excluded from downstream analyses. Statistical significance of the distribution of 4-mer positions resulting from Cas9 D10A nickase- or Cas9 N863A nickase-induced lesions was tested using a permutation test (permTS function from the "perm" R package with default settings).

### Example 5: Assessment of Gene Targeting in Hematopoietic Stem Cells

Transplantation of autologous CD34⁺ hematopoietic stem cells (HSCs, also known as hematopoietic stem/progenitor cells or HSPCs) genetically modified to correct the Sickle Cell Disease (SCD) mutation in the human β-hemoglobin gene (*HBB*) would prevent deformability (sickling) after deoxygenation in the erythrocyte progeny of corrected HSCs which could ameliorate symptoms associated with SCD. Genome editing with the CRISPR/Cas9 platform precisely alters endogenous gene targets by creating an indel at the targeted cut site that can lead to knock down of gene expression at the edited locus. In this Example, genome editing in the human K562 bone marrow erythroleukemia cell line, which serve as a proxy for HSCs and which can be predictive of genome editing in HSCs, were electroporated with Cas9 mRNA and gRNA HBB-8 and gRNA HBB-15 to induce gene editing at the human *HBB* locus.

K562 cells were grown in RPMI media (Life Technologies) containing 10% fetal bovine serum (FBS). *S. pyogenes* Cas9 mRNA and gRNA HBB-15 and gRNA HBB-8 were prepared by *in vitro* transcription using linearized plasmid DNA as templates and the Ambion mMessage mMachine® T7 Ultra Transcription kit (Life Technologies) according to the manufacturer's instructions. In this embodiment, both the Cas9 and gRNA were *in vitro* transcribed using a T7 polymerase. For example, a 5' ARCA cap was added to both RNA species simultaneous to transcription while a polyA tail was added after transcription to the 3' end of the RNA species by an *E. coli* polyA polymerase. Capped and tailed gRNA HBB-8 and gRNA HBB-15 were complexed at room temperature with *S. pyogenes* H-NLS-Cas9 protein at a molar ratio of ∼25:1 (gRNA : Cas9 protein) in a total of 30 µg RNP. Briefly, three million K562 cells were suspended in 100 µL electroporation buffer and transferred to the RNP solution (13µL). In addition, K562 cells were electroporated with *S. pyogenes* Cas9 mRNA and each of the gRNA HBB-8 and gRNA HBB-15. For the mRNA/gRNA electroporation, 10 µg of gRNA HBB-8 (or 10 µg of *HBB* gRNA HBB-15) were mixed with 10 µg of Cas9 mRNA. Four million K562 cells were suspended in 100 µL electroporation buffer and then transferred to the mRNA/gRNA solution (13 µL). K562 cells mixed with either RNP or RNA were electroporated. At 48 hours after electroporation, K562 cells were enumerated by trypan blue exclusion and were determined to have >88% viability in the electroporated cell populations. Genomic DNA was extracted from K562 cells 48 hours after electroporation and *HBB* locus-specific PCR reactions were performed.

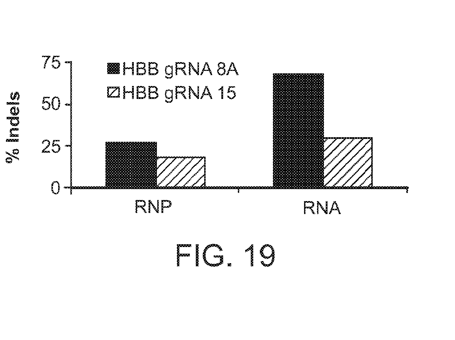

In order to detect indels at the *HBB l*ocus, T7E1 assays were performed on *HBB* locus-specific PCR products that were amplified from genomic DNA samples from electroporated K562 cells and the percentage of indels detected at the *HBB* locus was calculated (**Fig. 19**).

Co-delivery of 10 µg RNP which contains wild-type *S. pyogenes* Cas9 protein with *HBB* gRNA 8 or HBB gRNA 15 resulted in 26.8% and 16.1% indels, respectively, at the *HBB* locus in gDNA from K562 cells (molar ratio protein: gRNA 24:1). Co-delivery of Cas9 mRNA with gRNA HBB-8 or HBB-15 led to 66.9% and 29.5% indels at the *HBB* locus in gDNA from K562 cells (10 µg of each RNA/4 million cells). This example shows that delivery of Cas9 mRNA/gRNA and Cas9 RNPs leads to editing of the *HBB* locus in a relevant bone marrow derived hematopoietic cell line (K562 cells). Clinically, transplantation of autologous HSCs in which the *HBB* locus has been edited to correct the genetic mutation that causes red blood cell sickling could be used to ameliorate symptoms of SCD.

### Example 6: Contribution of Different Pairs of gRNA Molecules for Gene Correction

The CRISPR/Cas9 system was used to target the human *HBB* gene in the region of the sickle cell anemia-causing mutation.

Three different gRNA pairs were evaluated to examine how different pairs of gRNA molecules allocated at different distances can affect the frequency of different DNA repair outcomes in the presence of dual-nicks, in which the nicks are placed on opposite strands and leave either 3' or 5' overhangs of varying lengths. DNA repair outcomes including, e.g., indel mutations resulting from non-homologous end-joining (NHEJ), homology-dependent repair (HDR) using a donor as a template, and HDR using the closely related *HBD* gene as an endogenous template, were characterized. The frequencies of these various repair outcomes under different conditions offer insights into the mechanism of DNA repair and how it is impacted by the nature of the DNA break, the length of the overhangs, and/or the nature of the sequence exposed.

In this study different gRNA molecules for the *HBB* region that surround the nucleotides encoding the amino acid that is most commonly mutated in sickle cell disease were tested in 293T cells with wild type Cas9. The gRNA molecules that induced similar high rates of NHEJ and had PAMs facing in opposite orientations were selected to test as pairs with Cas9 D10A and Cas9 N863A nickases.

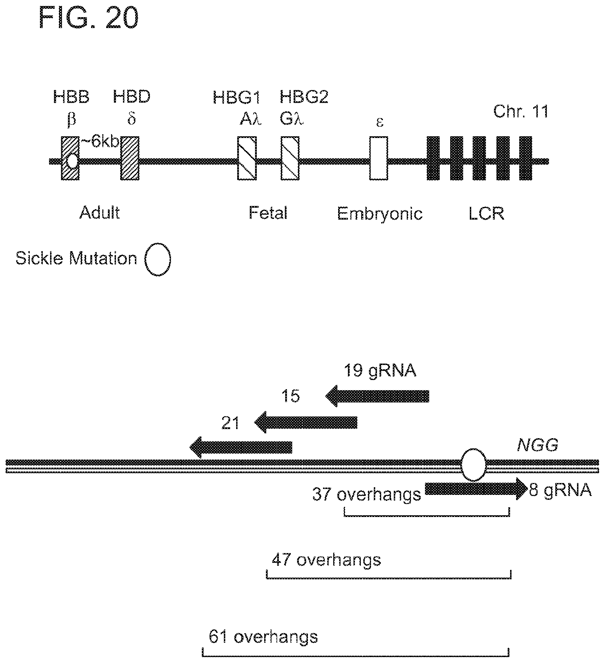

As shown in **Fig. 20**, the gRNA pairs 8/15 ("8gRNA"/"15gRNA" pair), 8/19 ("8gRNA"/ "19gRNA" pair) and 8/21 ("8gRNA"/"21gRNA" pair) were selected. ("8gRNA"/"15gRNA" pair). The targeting domain sequences for "8gRNA" and "15gRNA" are described above. "19gRNA" has the targeting domain sequence of CCUGUGGGGCAAGGUGAACG (SEQ ID NO: 419) and "21gRNA" has the targeting domain sequence of UGAAGUUGGUGGUGAGGCCC (SEQ ID NO: 431). These pairs of gRNA molecules used in combination with the mutant Cas9 D10A generated a 5' overhang of 47 bp, 37 bp and 61 bp, respectively; in combination with the mutant Cas9 N863A generated a 3' overhang of 47 bp, 37 bp and 61 bp, respectively.

In this Example, U2OS cells were electroporated with 200 ng of each gRNA and 750 ng of plasmid that encodes mutant Cas9 (D10A or N863A). Cells were collected 6 days after electroporation and genomic DNA was extracted. PCR amplification of the *HBB* locus was performed and the product was subcloned into a Topo Blunt Vector. For each condition in each experiment, 96 colonies were sequenced with Sanger sequencing. In the experiments assessing HDR efficacy, cells were electroporated with 2.5 µg of single stranded oligo or double stranded oligo in addition to the gRNA and the Cas9-encoding plasmid.

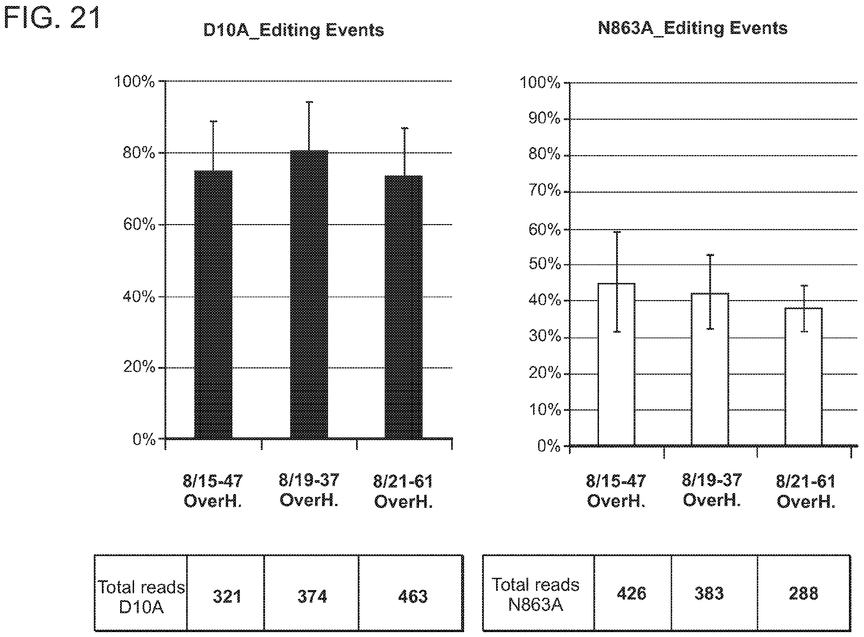

As shown in **Fig. 21**, the total percentages of all editing events detected by Sanger sequencing of the *HBB* locus were similar using the different pairs of gRNA molecules (Cas9 N863A was less active compared to Cas9 D10A). **Fig. 21** indicates that the overall editing frequencies for both Cas9 D10A and Cas9 N863A nickases were independent of overhang length and composition.

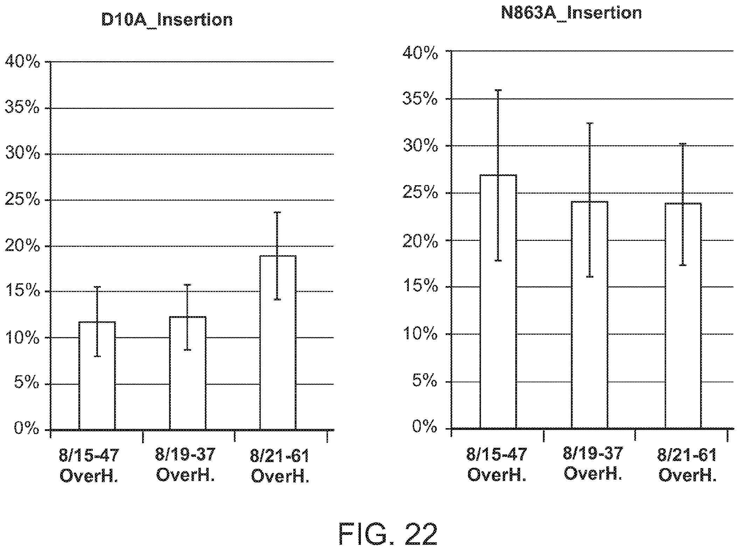

**Fig. 22** shows that for the Cas9 N863A nickase, a majority of the gene editing events were insertions with all three pairs of gRNA molecules (Cas9 N863A had a higher level of insertions compared to Cas9 D10A). **Fig. 22** indicates that the Cas9 N863 nickase yielded a higher frequency of insertions, which was independent of overhang length and composition, while the Cas9 D10A insertions were overall less frequent, they appeared to be sensitive to overhang length and composition.

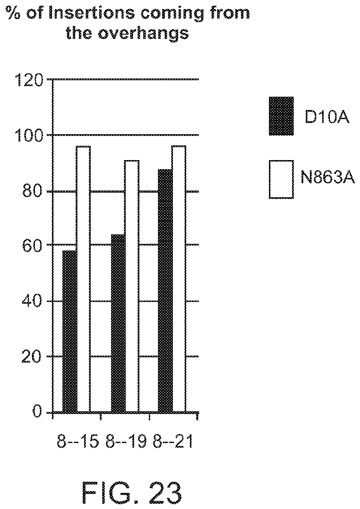

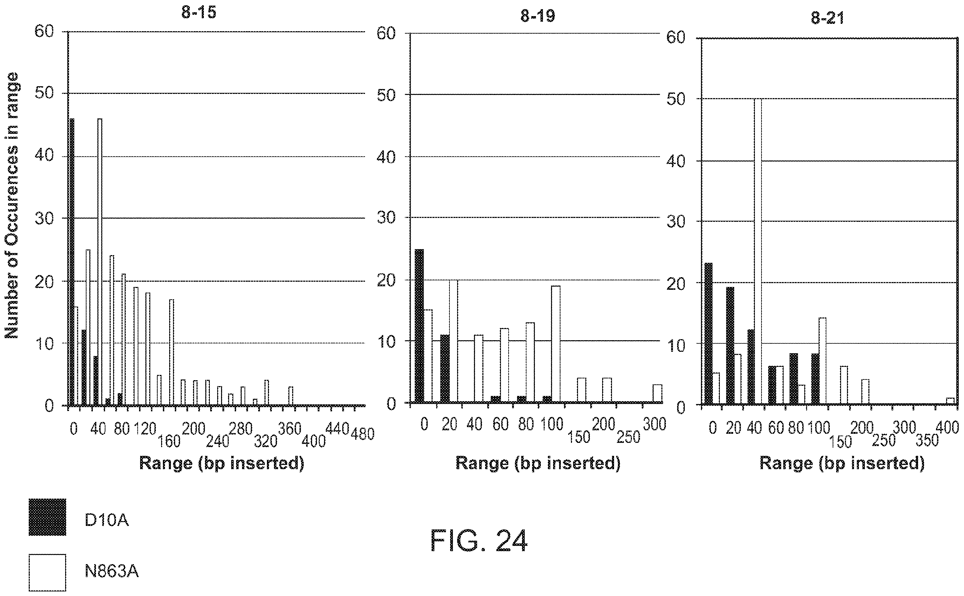

As shown in **Fig. 23**, the Cas9 N863A-induced insertions are mostly duplications of the overhang regions, as opposed to the overhangs coming from the Cas9 D10A, *i.e.,* with specific gRNA pair combinations, the Cas9 N863 nickase had a higher percentage of insertions derived from the overhang sequence than the Cas9 D10A nickase. Moreover, the lengths of the insertions between Cas9 D10A and Cas9 N863A were substantially different with longer insertions being observed using the Cas9 N863A nickase (**Fig. 24**).

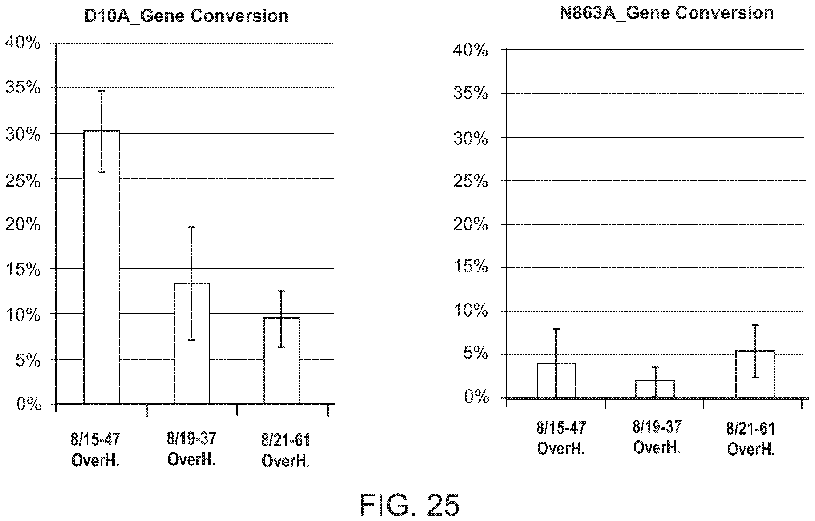

As shown in **Fig. 25**, in the case of the Cas9 D10A nickase, which leaves a 5' protruding end, the lesion was mostly repaired through a mechanism of gene conversion with a significantly higher rate compared to the Cas9 N863A nickase. Not all gRNA pairs tested in this example behaved similarly. The 8/15 gRNA pair was mostly repaired using a gene conversion mechanism, while the 8/19 and 8/21 pairs yielded a significant reduction of gene conversion frequency. Specifically, the 8/21 pair displayed a 3 fold reduction in gene conversion efficiency. These data suggest that either the length of the overhang or the nature of the sequence exposed, or both, could affect the DNA repair machinery. These data indicate that gene conversion was depending on the type of Cas nickase and on the sequence and length of overhangs.

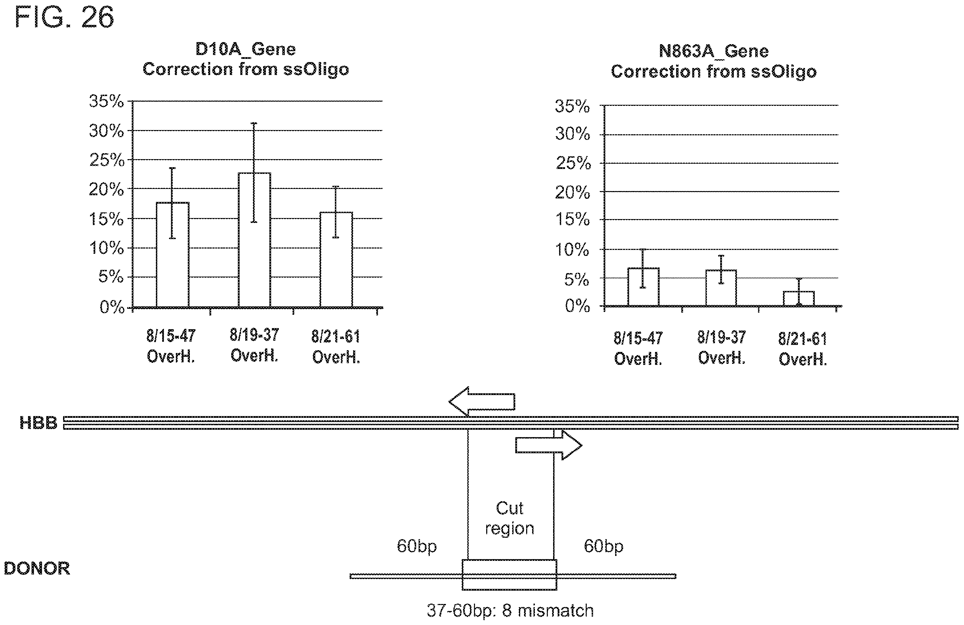

To test the effect that different pairs had on the engagement of HDR, a donor template was provided as a single-strand oligo or as a double stranded (ds) DNA donor. In both cases the length of the donor is approximately 170 bp with 60 bp of homology outside the nicks and with 8 mismatches (**Fig. 26**). As shown in **Fig. 26**, all three pairs resulted in similar HDR frequencies, *i.e.,* the frequency of gene correction from exogenous donor was comparable despite of the length of overhangs. Neither Cas9 D10A nor Cas9 N863A induced gene correction from exogenous donor was dependent on overhang length and composition. **Fig. 26** indicates that gene correction from exogenous donor was more efficient with the Cas9 D10A nuclease compared to the Cas9 N863A nuclease.

### Example 7: Contact between S. pyogenes Cas9 ribonucleoprotein complexed to gRNAs targeting the HBB genetic locus supports gene editing in adult human hematopoietic stem cells

Transplantation of autologous CD34⁺ hematopoietic stem cells (HSCs) collected from patients affected with hemoglobinopathies (*e.g*., sickle cell disease [SCD], β-thalessemia), that have been genetically modified with a lentivirus vector that expresses non-sickling β-hemoglobin gene (*HBB*) has been shown to restore expression of functional adult hemoglobin (HbA) thus preventing the formation of sickle hemoglobin (HbSS), in erythroid cells derived from transduced CD34⁺ cells and ameliorating clinical symptoms in affected patients (Press Release from Bluebird Bio, June 13, 2015, "bluebird bio Reports New Beta-thalassemia major and Severe Sickle Cell Disease Data from HGB-205 study at EHA"). However, delivery of a transgene encoding a non-sickling β-hemoglobin does not correct the causative mutation or prevent expression of the mutant *(e.g.,* sickling) form of *HBB.* Furthermore, lentivirus vector transduction of CD34⁺ cells can lead to the occurrence of multiple transgene integration sites per cell, and the long-term effects of multiple transgene integration sites is currently undetermined.

In contrast, genome editing with the CRISPR/Cas9 platform precisely alters endogenous gene targets by creating an insertion or deletion (indel) at the cut site that can lead to gene disruption at the edited locus. Co-delivery of two gRNAs each targeting regions proximal to the single nucleotide polymorphism (SNP) that encodes HbSS (*e.g.,* GAG→ GTG, which results in a change in the amino acid residue from glutamic acid to valine) co-delivered with a Cas9 D10A nickase supports a low level of homology directed repair (HDR) in human cell lines *(e.g.,* gene conversion using a region of homology in the *HBD* locus as DNA repair template).

In this Example, genome editing in adult human mobilized peripheral blood CD34⁺ HSCs after co-delivery of Cas9 D10A nickase with two gRNAs targeting the *HBB* locus was evaluated. The edited CD34⁺ cells were then differentiated into myeloid and erythroid cells to determine the hematopoietic activity of the HSCs. Gene editing at the *HBB* locus was evaluated by T7E1 analysis and DNA sequencing. Expression of *HBB* protein was also analyzed in erythroid progeny.

Human CD34⁺ HSCs cells from mobilized peripheral blood (AllCells®) were thawed into StemSpan Serum-Free Expansion Medium (SFEM™, StemCell Technologies) containing 300 ng/mL each of human stem cell factor (SCF) and flt-3 ligand (FL), 100 ng/mL thrombopoietin (TPO), and 60 ng/mL of IL-6, and 10 µM PGE2 (Cayman Biochemicals; all other supplements were from PeproTech® unless otherwise indicated). Cells were grown for 3 days in a humidified incubator and 5% CO₂ 20% O₂. On day 3 (morning), media was replaced with fresh Stemspan-SFEM™ supplemented with human SCF, TPO, FL and PGE2. In the afternoon of day 3, 2.5 million CD34⁺ cells per sample were suspended in electroporation buffer.

The gRNA was generated by *in vitro* transcription using a T7 polymerase. A 5' ARCA cap was added to the RNA simultaneous to transcription while a polyA tail was added after transcription to the 3' end of the RNA species by an *E. coli* polyA polymerase.

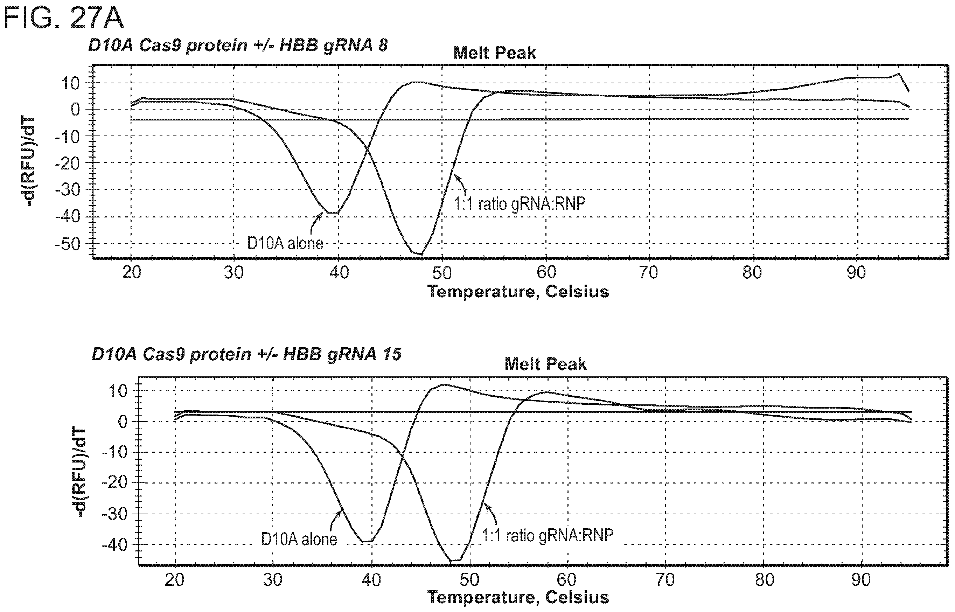

After the gRNAs were *in vitro* transcribed and tailed, the quality and quantity of gRNAs were evaluated with the Bioanalyzer (Nanochip®) to determine RNA concentration and by Differential Scanning Fluorimetry (DSF) assay, a thermal shift assay that quantifies the change in the thermal denaturation temperature of Cas9 protein with and without complexing to gRNA. In DSF assays, the Cas9 protein was mixed with gRNA and allowed to form complexes for 10 minutes. Cas9 protein : gRNA were mixed at a molar ration of 1:1, and the DSF assay performed as a measure of Cas9 stability and as an indirect measure of gRNA quality, since a 1:1 ratio of gRNA:Cas9 should support a thermal shift if the gRNA is of good quality (**Figure 27A**).

For half of the samples, *in vitro* transcribed capped and tailed guide (g)RNAs *HBB-8* and *HBB-15* were added at a 2:1 molar ratio to 12.5µg D10A Cas9 ribonucleoprotein (RNP) (5µg RNP per million cells) to 2.5 million cells. "HBB-8" has the targeting domain sequence of GUAACGGCAGACUUCUCCUC (SEQ ID NO:388), and "HBB-15" has the targeting domain sequence of AAGGUGAACGUGGAUGAAGU (SEQ ID NO:387). D10A protein and gRNAs RNP complexes were transferred to 2.5 million adult CD34⁺ cells in electroporation buffer. The RNP/cell mixture was transferred to an electroporation cartridge, and the cells then electroporated ("Program 2" and "Program 3").

For the second portion of CD34⁺ cell samples, equal amounts (5 µg or 10 µg [2XgRNA] each) of *in vitro* transcribed capped and tailed guide (g)RNAs *HBB-8* and *HBB-15* were added to 10 µg of *in vitro* transcribed Cas9 D10A mRNA. The mRNA : gRNA: cell mixture was transferred to an electroporation cartridge and electroporated using Program 2 (P2).

For all samples, the cells were collected from the cartridge and placed at 37°C for 20 minutes (recovery period). Then, the cells were either transferred to pre-warmed cytokine supplemented Stemspan-SFEM™ media and placed at 30°C for 2 hours (cold shock samples) or placed directly into 37°C. For the cold shocked samples, the cells were transferred to the 37°C incubator after the 2-hour incubation period at 30°C. At 24, 48, and 72 hours after electroporation, the CD34⁺ cells were counted by trypan blue exclusion (cell survival) and divided into 3 portions for the following analyses: a) flow cytometry analysis for assessment of viability by co-staining with 7-Aminoactinomycin-D (7-AAD) and allophycocyanin (APC)-conjugated Annexin-V antibody (eBioscience); b) flow cytometry analysis for maintenance of HSC phenotype (after co-staining with phycoerythrin (PE)-conjugated anti-human CD34 antibody (BD Biosciences) and APC-conjugated CD133 (Miltenyi Biotech; c) hematopoietic colony forming cell (CFC) analysis by plating 800 cells in semi-solid methylcellulose based Methocult medium (StemCell Technologies H4435) that supports differentiation of erythroid and myeloid blood cell colonies from HSCs and serves as a surrogate assay to evaluate HSC multipotency and differentiation potential *ex vivo;* d) genomic DNA analysis for detection of editing at the *HBB* locus. Genomic DNA was extracted from the HSCs at 48 and 72 hours after electroporation and *HBB* locus-specific PCR reactions were performed. The purified PCR products were analyzed for insertions/deletions (indels) in T7E1 assays and by DNA sequencing of individual clones (PCR product was transformed and sub-cloned into TOPO-vector, individual colonies picked, and plasmid DNA containing individual PCR products were sequenced).

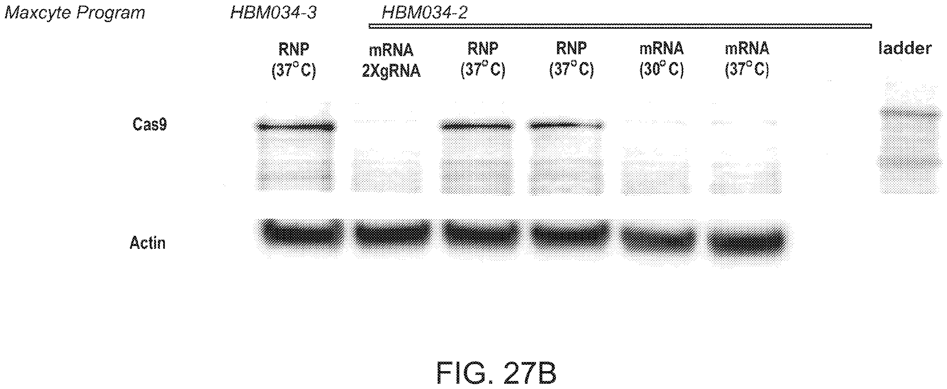

Western blot analysis of cell lysates extracted from the Cas9/gRNA electroporated CD34⁺ cells indicated the presence of Cas9 protein at 72 hours after electroporation of CD34⁺ cells that received Cas9 RNP and gRNA pair. Very low levels of Cas9 protein were detected in the lysates of cells that were electroporated with Cas9 mRNA **(****Figure 27B**).

Electroporated CD34⁺ cells maintained a stem cell phenotype (*e.g*., co-expression of CD34 and CD133) and viability (*e.g.,* 75% AnnexinV⁻7AAD⁻) as determined by flow cytometry analysis **(****Figure 28A**). The absolute number of viable CD34⁺ cells was maintained across most samples over a 72-hour culture period after electroporation **(****Figure 28B**). In addition, gene edited CD34⁺ cells maintained *ex vivo* hematopoietic activity and multipotency as indicated by their ability to give rise to erythroid (*e.g*., CFU-E or CFU-GEMM) and myeloid (*e.g*., CFU-G, -M or-GM) (**Figure 28C**).

Gene editing at the *HBB* locus was then evaluated at 72 hours after electroporation of D10A mRNA or RNP co-delivered with two gRNAs (HBB-8 and HBB-15). Briefly, genomic (g)DNA was isolated from electroporated CD34⁺ cells at 72 hours after electroporation, and PCR amplification of a ∼607bp fragment of the *HBB* locus (which captured both of the individual genomic locations that were targeted by the two gRNAs HBB-8 and HBB-15) was performed. After cleanup of the *HBB* PCR product with AMPURE beads, insertions/deletions (indels) at the targeted genomic location were evaluated by T7E1 assay and by DNA sequencing. For the CD34⁺ cells electroporated with D10A mRNA and HBB targeting gRNA pair, no indels were detected (**Table 10**). In contrast to the negative results obtained after delivery of D10A mRNA, ∼30-60% indels were detected by T7E1 and sequencing analysis of CD34⁺ cells that were electroporated with D10A RNP with the *HBB* targeting gRNA pair (**Table 10,** **Figure 29A-29C**). The cells that were cultured for 2 hours at 30°C (after a 20-minute recovery period at 37°C) exhibited 57% editing as determined by DNA sequencing. In addition, gene conversion (*e.g., HBD* genomic sequence used as a template copy for DNA repair of the disrupted *HBB* locus) was detected in the gDNA from CD34⁺ cells that were 'cold shocked' (30°C incubation) at a frequency of 3% relative to the total gene editing events (**Figure 29C**).

**Table 10. Summary of gene editing results in adult CD34⁺ cells 72 hours after co-delivery of D10A Cas9 and HBB specific gRNA pair. *Alternate electroporation program (P3).**

| **D10A source** | **µg D10A** | **µg HBB-8 gRNA** | **µg HBB-15 gRNA** | **Temperature of 2-hour recovery** | **% indels (T7E1)** | **% indels (sequencing)** |
|---|---|---|---|---|---|---|
| mRNA | 10 | 5 | 5 | 37°C | 0 | ND |
| mRNA | 10 | 5 | 5 | 30°C | 0 | ND |
| mRNA | 10 | 10 | 10 | 37°C | 0 | ND |
| RNP | 12.5 | 3.3 | 3.3 | 37°C | 45 | 30 |
| RNP | 12.5 | 3.3 | 3.3 | 30°C | 39 | 57 |
| RNP* | 12.5 | 3.3 | 3.3 | 37°C | 39 | 39 |

To determine whether targeted disruption of the *HBB* locus induced changes in expression of b-hemoglobin protein, CFU-E colonies were picked, dissociated, fixed, permeabilized and stained with PE-conjugated mouse anti-human β-hemoglobin antibody (Santa Cruz Biotechnology®). The erythroid progeny of *HBB* gene edited cells exhibited a 7-to-10 fold reduction in β-hemoglobin expression compared to the CFU-Es differentiated from untreated control CD34⁺ cells (**Figure 30**). These data show that the progeny of gene edited cells retain erythroid differentiation potential and that gene editing events detected in the parental CD34⁺ cells result in reduced protein expression in erythroid progeny.

### Example 8: Contact between S. pyogenes D10A Cas9 nickase ribonucleoprotein complexed to gRNAs targeting the HBB genetic locus supports gene editing in fresh umbilical cord blood derived human CD34+ hematopoietic stem cells

In this Example, genome editing in freshly collected umbilical cord blood (CB) CD34⁺ HSCs after co-delivery of D10A Cas9 nickase with two gRNAs targeting the *HBB* locus was evaluated. The edited CB CD34⁺ cells were then differentiated into myeloid and erythroid cells to determine the hematopoietic activity of the HSCs. Targeted disruption of the *HBB* locus was evaluated by T7E1 analysis and DNA sequencing.

Human CD34⁺ HSCs cells were isolated from freshly obtained human umbilical cord blood by ficoll gradient density centrifugation followed by MACS® (antibody conjugated immunomagnetic bead sorting) with mouse anti-human CD34⁺ immunomagnetic beads using the human CD34 cell enrichment kit and LS magnetic columns from Miltenyi Biotech. The cells were plated into StemSpan Serum-Free Expansion Medium (SFEM™, StemCell Technologies) containing 100 ng/mL each of human stem cell factor (SCF) and flt-3 ligand (FL), 20 ng/mL each of thrombopoietin (TPO) and IL-6, and 10 µM PGE2 (Cayman Biochemicals; all other supplements were from Peprotech unless otherwise indicated). Cells were grown for 3 days in a humidified incubator and 5% CO₂ 20% O₂. On day 3 (morning), media was replaced with fresh Stemspan-SFEM™ supplemented with human SCF, TPO, FL and PGE2. In the afternoon of day 3, 2.5 million CD34⁺ cells per sample were suspended in electroporation buffer.

The gRNAs were generated by *in vitro* transcription using a T7 polymerase. A 5' ARCA cap was added to the RNA simultaneous to transcription while a polyA tail was added after transcription to the 3' end of the RNA species by an *E. coli* polyA polymerase. After the gRNAs were *in vitro* transcribed and tailed, the quality and quantity of gRNAs were evaluated with the Bioanalyzer (Nanochip) to determine RNA concentration and by DSF assay performed as a measure of D10A Cas9 nickase RNP stability and as an indirect measure of gRNA quality.

*In vitro* transcribed capped and tailed guide gRNAs *HBB-8* and *HBB-15* were added at a 2:1 molar ratio (total gRNA : Cas9 protein) to 125µg D10A nickase ribonucleoprotein (RNP) (5µg RNP per million cells) to each of two samples each containing 2.5 million CB CD34⁺ cells. A third CB CD34⁺ cell aliquot was mixed with 25µg D10A nickase RNP and HBB gRNAs (total gRNA: D10A ratio at 2:1). For each experimental sample, the D10A RNP/cell mixture was transferred to an electroporation cartridge, and the cells then electroporated using Program 2.

For all samples, the cells were collected from the cartridge and placed at 37°C for 20 minutes (recovery period). For the CB CD34⁺ HSCs that were contacted with 125µg D10A nickase RNP, one sample was transferred to pre-warmed cytokine supplemented Stemspan-SFEM™ media and placed at 30°C for 2 hours (cold shock samples) or placed directly into the same media at 37°C. For the cold shocked samples, the cells were transferred to the 37°C incubator after the 2-hour incubation period at 30°C. At 24, 48, and 72 hours after electroporation, the CB CD34⁺ HSCs were counted by trypan blue exclusion (cell survival) and divided into 3 portions for the following analyses: a) flow cytometry analysis for assessment of viability by co-staining with 7-Aminoactinomycin-D (7-AAD) and allophycocyanin (APC)-conjugated Annexin-V antibody (eBioscience); b) flow cytometry analysis for maintenance of HSC phenotype (after co-staining with phycoerythrin (PE)-conjugated anti-human CD34 antibody (BD Biosciences) and APC-conjugated CD133 (Miltenyi Biotech; c) hematopoietic colony forming cell (CFC) analysis by plating 800 CB CD34⁺ HSCs in semi-solid methylcellulose based Methocult medium (StemCell Technologies H4435) that supports differentiation of erythroid and myeloid blood cell colonies from HSCs and serves as a surrogate assay to evaluate HSC multipotency and differentiation potential *ex vivo;* d) genomic DNA analysis for detection of editing at the *HBB* locus. Genomic DNA was extracted from the HSCs at 48 and 72 hours after electroporation and *HBB* locus-specific PCR reactions were performed. The purified PCR products were analyzed for insertions/deletions (indels) in T7E1 assays and by DNA sequencing of individual clones (PCR product was transformed and subcloned into TOPO-vector, individual colonies picked, and plasmid DNA containing individual PCR products were sequenced).

Electroporated CB CD34⁺ cells maintained a stem cell phenotype (*e.g*., co-expression of CD34 and CD133, ∼90% CD34⁺CD133⁺) and viability (*e.g*., 91% AnnexinV⁻7AAD⁻) as determined by flow cytometry analysis (**Figure 31A**). The absolute number of viable CD34⁺ cells was maintained across most samples over a 72-hour culture period after electroporation. In addition, gene edited CD34⁺ cells maintained *ex vivo* hematopoietic activity and multipotency as indicated by their ability to give rise to erythroid *(e.g*., CFU-E or CFU-GEMM) and myeloid (*e.g*., CFU-G, -M, or -GM) (**Figure 31B**).

Gene editing at the *HBB* locus was then evaluated at 72 hours after electroporation of D10A nickase RNP co-delivered with 2 gRNAs (HBB-8 and HBB-15). Briefly, gDNA was isolated from electroporated CD34⁺ cells at 72 hours after electroporation, and PCR amplification of a ∼607bp fragment of the *HBB* locus (which captures both of the individual genomic locations that were targeted by gRNAs HBB-8 and HBB-15) was performed. After cleanup of the *HBB* PCR product with AMPURE beads, insertions/deletions (indels) at the targeted genomic location were evaluated by T7E1 assay and by DNA sequencing. For the CD34⁺ cells electroporated with 5 µg per million cells of D10A nickase RNP and HBB-specific gRNA pair, ∼20% indels were detected by T7E1 analysis (**Table 11**). In contrast to adult CD34⁺ cells, a 2-hour incubation at 30°C did not alter the level of gene editing as determined by either T7E1 analysis or DNA sequence analysis. In addition, doubling the D10A nickase RNP/gRNA concentration to 10 µg RNP per million cells nearly doubled the gene editing at the *HBB* locus to 57%, as determined by DNA sequencing analysis (**Table 11**, **Figures 32A-32C**). Stratification of DNA repair events through DNA sequencing analysis revealed that ∼50-70% of the sequence reads contained small insertions, ∼20-40% contained large deletions, and -8% showed evidence of *HBB*/*HBD* gene conversion events in the targeted *HBB* genomic location (**Figure 32C**).

**Table 11. Summary of gene editing results in CB CD34⁺ cells 72 hours after co-delivery of D10A nickase RNP and HBB-specific gRNA pair.**

| **Total µg D10A RNP** | **µg D10A RNP/1E6 cells** | **µg HBB-8 gRNA** | **µg HBB-15 gRNA** | **Temperature of 2-hour recovery** | **Electroporation Program** | **%indels (T7E1)** | **%indels (sequencing)** |
|---|---|---|---|---|---|---|---|
| 12.5 | 5 | 3.3 | 3.3 | 37°C | 2 | 20 | 23 |
| 12.5 | 5 | 3.3 | 3.3 | 30°C | 2 | 27 | 20 |
| 25 | 10 | 6.6 | 6.6 | 37°C | 2 | 36 | 51 |

In contrast to adult CD34⁺ cells, CB CD34⁺ cells are fetal in origin and therefore the progeny of CB CD34⁺ cells express fetal hemoglobin (HbF) which contains γ-hemoglobin instead of β-hemoglobin. Given the lack of β-hemoglobin by CB eyrthroblasts, disruption of the *HBB* locus in this model system will not impact expression of hemoglobin protein. CB CD34⁺ cells are used as a model system for evaluation of gene editing in HSCs, since these umbilical cord blood derived CD34⁺ cells are more readily available for research use and reconstitute immune-deficient mouse xenografts more efficiently compared to adult CD34⁺ cells.

To determine whether gene edited cells retained their erythroid differentiation potential, the edited CD34⁺ cells were induced to differentiate into erythroblasts. Briefly, CD34⁺ cells were co-cultured with human plasma, holotransferrin, insulin, hydrocortisone, and cytokines (erythropoietin, SCF, IL3), for 20 days in which the latter 4 growth factors were added at different stages of differentiation to direct erythroid specification program. The cells were then evaluated by flow cytometry for the acquisition of erythroid phenotypic characteristics including: co-expression of the transferrin receptor (CD71) and Glycophorin A (CD235); expression of HbF, and enucleation (as indicated by the absence dsDNA detected by the dsDNA dye DRAQ5) and loss of CD45 expression. By day 18 of differentiation, the erythroblast progeny of edited CD34⁺ cells possessed this red blood cell phenotype (**Figures 33A-33C**). These data, along with the CFC data shown in **Figures 31A-31B** show that the gene editing does not negatively impact the differentiation potential of CD34⁺ cells.

In summary, the data in this Example indicate: 1) electroporation of fresh CB CD34⁺ HSCs with D10A nickase and paired capped/tailed gRNAs does not impact cell viability, proliferation potential, or multipotency; and 2) contact between CB CD34⁺ HSCs and 10 µg D10A RNP (per million cells) supports >50% gene editing with HDR events (8% gene conversion events of total).

### Example 9: Contact between S. pyogenes wild-type Cas9 RNP or D10A nickase RNP complexed to gRNAs targeting the HBB genetic locus supports up to 60% gene editing in human cord blood hematopoietic stem cells

In this Example, umbilical cord blood (CB) CD34⁺ HSCs were contacted with *S*. *pyogenes* wild-type Cas9 RNP, N863A nickase RNP, or D10A nickase RNP complexed with 2 gRNAs targeting the *HBB* locus. The percentage of gene editing and type of editing event (*e.g.,* HDR (*e.g.,* gene conversion) or NHEJ) were evaluated to determine the optimal Cas9 activity (*e.g.,* type of cut, *e.g.,* double-strand break from wild-type Cas9 or off-set nicks on opposite DNA strands by nickases) for gene editing in HSCs that would favor HDR (*e.g.,* gene conversion) over NHEJ (*e.g.,* ends of the DNA left exposed after the cut, *e.g.,* blunt ends left by wild-type Cas9 cut, 5' overhang left by D10A nickase cut or 3' overhang left by N863A nickase cut). Other optimizations included: 1) removal of endotoxin from Cas9 protein preparation to reduce toxicity of Cas9 protein; 2) use of 10 µg RNP per million CD34⁺ cells to increase gene editing (shown to double gene editing in fresh CB CD34⁺ cells compared to 5 µg RNP, as indicated in **Example 8**); 3) testing of human CD34⁺ cells that were isolated from cord blood (CB), cryopreserved, and confirm that gene editing was not impacted by cryopreservation (compared to freshly isolated HSCs, described in **Example 8**); and 4) evaluate Cas9 RNP levels in CD34⁺ cells over time to understand Cas9 RNP stability in HSCs.

Human CD34⁺ HSCs cells were isolated from freshly obtained human umbilical cord blood by ficoll gradient density centrifugation followed by MACS sorting with mouse anti-human CD34⁺ immunomagnetic beads. CD34₊ cells were cryopreserved, thawed at a later date, and plated into StemSpan Serum-Free Expansion Medium (SFEM™, StemCell Technologies) containing 100 ng/mL each of human stem cell factor (SCF) and flt-3 ligand (FL), 20 ng/mL each of thrombopoietin (TPO) and IL-6, and 10 µM PGE2 (Cayman Biochemicals; all other supplements were from PeproTech® unless otherwise indicated). Cells were grown for 3 days in a humidified incubator and 5% CO₂ 20% O₂. On day 3 (morning), media was replaced with fresh Stemspan-SFEM™ supplemented with human SCF, TPO, FL and PGE2. In the afternoon of day 3, 2.2 million CD34⁺ cells per sample were suspended in electroporation buffer.

The gRNAs were generated by *in vitro* transcription using a T7 polymerase. A 5' ARCA cap was added to the RNA simultaneous to transcription while a polyA tail was added after transcription to the 3' end of the RNA species by an *E. coli* polyA polymerase. After the gRNAs were *in vitro* transcribed and tailed, the quality and quantity of gRNAs were evaluated with the Bioanalyzer (Nanochip) to determine RNA concentration and by DSF assay performed as a measure of D10A Cas9 nickase RNP stability and as an indirect measure of gRNA quality.

*In vitro* transcribed capped and tailed guide gRNAs *HBB-8* and *HBB-15* were added at a 2:1 molar ratio (total gRNA : Cas9 protein) to 10µg RNP per million cells. The RNPs tested include the following: wild-type (WT) Cas9, endotoxin-free WT Cas9, N863A nickase, D10A nickase. For each experimental sample, the D10A RNP/cell mixture was transferred to the electroporation cartridge, and the cells then electroporated using Program 2 (P2).

For all samples, the cells were collected from the cartridge and placed at 37°C for 20 minutes (recovery period). For the CB CD34⁺ cells that were contacted with 10µg D10A nickase RNP (per million cells), one sample was transferred to pre-warmed cytokine supplemented Stemspan-SFEM™ media and placed at 30°C for 2 hours (cold shock recovery) or placed directly into the same media at 37°C. For the cold shocked samples, the cells were transferred to the 37°C incubator after the 2-hour incubation period at 30°C. At 24, 48, and 72 hours after electroporation, the CB CD34⁺ cells were counted by trypan blue exclusion (cell survival) and divided into 3 portions for the following analyses: a) flow cytometry analysis for assessment of viability by co-staining with 7-Aminoactinomycin-D (7-AAD) and allophycocyanin (APC)-conjugated Annexin-V antibody (ebioscience); b) flow cytometry analysis for maintenance of HSC phenotype (after co-staining with phycoerythrin (PE)-conjugated anti-human CD34 antibody (BD Biosciences) and APC-conjugated CD133 (Miltenyi Biotech; c) hematopoietic colony forming cell (CFC) analysis by plating 800 cells in semi-solid methylcellulose based Methocult medium (StemCell Technologies H4435) that supports differentiation of erythroid and myeloid blood cell colonies from HSCs and serves as a surrogate assay to evaluate HSC multipotency and differentiation potential *ex vivo;* d) genomic DNA analysis for detection of editing at the *HBB* locus; and e) Western blot analysis of protein to evaluate the stability of Cas9 RNP in CD34⁺ HSCs. gDNA was extracted from the HSCs at 48 and 72 hours after electroporation and *HBB* locus-specific PCR reactions were performed. The purified PCR products were analyzed for insertions/deletions (indels) in T7E1 assays and by DNA sequencing of individual clones (PCR product was transformed and subcloned into TOPO-vector, individual colonies picked, and plasmid DNA containing individual PCR products were sequenced).

Electroporated CB CD34⁺ cells maintained a stem cell phenotype (*e.g*., co-expression of CD34 and CD133, >90% CD34⁺CD133⁺) and as determined by flow cytometry analysis. The absolute number of viable CD34⁺ cells was maintained across most samples over a 72-hour culture period after electroporation (**Figure 34A**). Gene edited CD34⁺ cells maintained *ex vivo* hematopoietic activity and multipotency as indicated by their ability to give rise to erythroid *(e.g.,* CFU-E, or CFU-GEMM) and myeloid (*e.g.,* CFU-G, -M, or -GM) (**Figure 34B**).

Gene editing at the *HBB* locus was then evaluated at 72 hours after electroporation of WT Cas9, N863A, or D10A nickases co-delivered with 2 gRNAs (HBB-8 and HBB-15). Briefly, gDNA was isolated from electroporated CD34⁺ cells at 72 hours after electroporation, and PCR amplification of a ∼607bp fragment of the *HBB* locus (which captured both of the individual genomic locations that were targeted by gRNAs HBB-8 and HBB-15) was performed. After cleanup of the *HBB* PCR product with AMPURE beads, insertions/deletions (indels) at the targeted genomic location were evaluated by T7E1 assay and by DNA sequencing. For the CD34⁺ cells electroporated with WT Cas9 and endotoxin-free WT Cas9 the percentages of indels detected by T7E1 analysis at 72 hours was 59% and 51%, respectively **(****Figure 35A**), which correlated with the indels detected by DNA sequencing (**Table 12**). CD34⁺ cells electroporation with N863A nickase and HBB-specific gRNA pair, had only 1% indels detected by T7E1 analysis. CD34⁺ HSCs electroporated with D10A nickase with and without cold shock supported gene editing at percentages of 39% and 48% indels detected by T7E1 analysis, respectively. In order to confirm that this low level of editing observed in CD34⁺ HSCs contacted with N863A nickase, was not due to the lack of N863A RNP contacting the cells, western blot analysis was performed **(****Figure 35B**).

Cas9 protein was present in all electroporated samples (*e.g*., cells that received WT Cas9, D10A nickase, and N863A nickase). For these samples, Cas9 protein was detected at 24 and 48 hours after electroporation, suggesting that the lack of N863A activity in the CD34⁺ HSCs was not due to the lack of protein.

Gene editing in CD34⁺ HSCs that contacted WT Cas9, endotoxin-free Cas9, and D10A nickase was 54-60%, based on DNA sequencing analysis (**Table 12,** **Figure 36A**). Stratification of DNA repair events through DNA sequencing analysis revealed that >90% of the gene editing events were deletions in CD34⁺ HSCs that contacted WT Cas9 and endotoxin-free WT Cas9 (insertions or combination of insertion and deletion comprised the remaining 3-6% of editing events) **(****Figure 36A**). In contrast, gDNA from CD34⁺ HSCs that contacted D10A nickases had 3% HDR (*e.g.,* gene conversion), up to 75% insertions, and up to 22% deletions (**Figure 36B**).

In summary, the data in this Example indicate: 1) endotoxin removal does not negatively impact Cas9 functionality or CD34⁺ HSC cell viability, proliferation potential, or multipotency; 2) use of 10 µg D10A RNP supports 60% gene editing in CD34⁺ HSCs with HDR events (*e.g.,* gene conversion); and 3) after contacting CD34⁺ HSCs, WT Cas9 and nickase RNPs are detected for up to 48 hours, but is not detectable thereafter.

**Table 12. Summary of gene editing results in CB CD34⁺ cells 72 hours after co-delivery of wild-type Cas9, N863A nickase, D10A nickase RNP and HBB-specific gRNA pair.**

| **Cas9** | **Total µg RNP/1E6 cells** | **Temperature of 2-hour recovery** | **Electroporation Program** | **% indels (T7E1)** | **% indels (sequencing)** |
|---|---|---|---|---|---|
| WT | 10 | 37°C | 2 | 59 | 56 |
| Endo-Free WT | 10 | 37°C | 2 | 51 | 60 |
| N863A | 10 | 37°C | 2 | 1 | ND |
| D10A | 10 | 37°C | 2 | 39 | 60 |
| D10A | 10 | 30°C | 2 | 48 | 54 |

### Example 10: Gene Conversion Modulation

The CRISPR/Cas9 system was used to target the human *HBB* gene in the region of the sickle cell anemia-causing mutation.

The nature of the targeted break affects the frequency of different DNA repair outcomes. Blunt double-strand breaks, single-strand nicks, and dual-nicks in which the nicks are placed on opposite strands and leave either 3' or 5' overhangs of varying lengths, were introduced by utilizing the wild type Cas9 nuclease, as well as two different Cas9 nickases. Several different DNA repair outcomes including, e.g., indel mutations resulting from non-homologous end-joining (NHEJ) and homology-dependent repair (HDR) using the closely related *HBD* gene as an endogenous template, were characterized. The frequency of these various repair outcomes under different conditions offers insight into the mechanisms of DNA repair and how it is impacted by the nature of the DNA break. Here, we examined how different repair pathways affect the frequency of gene conversion (HDR from the closely related HBD gene as an endogenous template). Specifically, we evaluated the effect of down-regulation of the alternative NHEJ (alt-NHEJ) and HDR pathways on the gene conversion rate in the presence of dual-nicks.

In this study, different gRNAs targeting the *HBB* region that surround the nucleotides encoding the amino acid most commonly mutated in sickle cell disease were tested in 293T cells with wild type Cas9. The gRNAs that induced similar high rates of NHEJ and had PAMs facing in opposite orientations were selected to test as pairs with Cas9 D10A (which would generate a 5' overhang) and Cas9 N863A nickases (which would generate a 3' overhang).

As a first example of enzymes that modulate gene conversion frequency, we selected the polymerase Pol theta, which is a central player in the alt-NHEJ pathway. In this example, U2OS cells were electroporated with 200 ng of each gRNA (8 and 15), 750 ng of plasmid that encodes wild type Cas9 or mutant Cas9 (D10A or N863A) and 30 pmol of scrambled siRNA as a negative control or 30 pmol of siRNA against Pol Theta. Cells were collected 6 days after electroporation and genomic DNA was extracted. PCR amplification of the *HBB* locus was performed and subcloned into a Topo Blunt Vector. For each condition in each experiment 96 colonies were sequenced with Sanger sequencing.

As shown in **Fig. 37**, down regulation of Pol theta in the context of the D10A Cas9 does not affect the gene editing profile. In contrast, Pol theta down-regulation in the context of the N863A Cas9 nuclease leads to a strong reduction of the insertion frequency and an increase in the gene conversion rate. These data suggest that the 3' protruding ends generated by the N863A Cas9 nuclease are substrates for processing by Pol Theta, resulting in a high accumulation of insertions. Upon down-regulation of Pol Theta, the 3' protruding DNA ends are available for engaging the gene conversion pathway (**Fig. 37**).

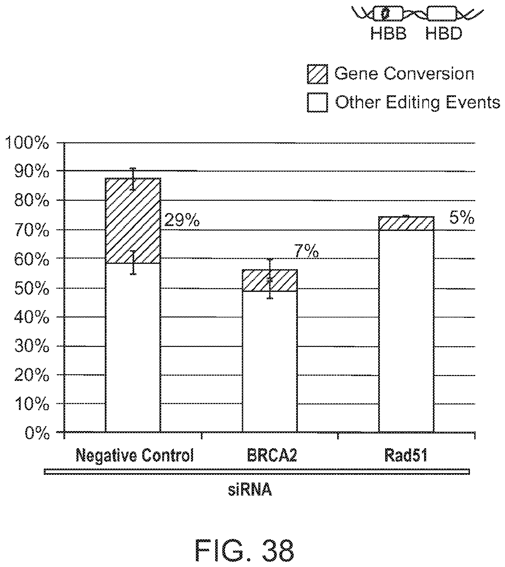

Next, we dissected the genetic requirements of the gene conversion pathway in the context of different Cas9 nucleases (**Fig. 38**), using BRCA2 and RAD51 - central players of canonical HR - as an example. U2OS cell were electroporated with 200 ng of each gRNA (8 and 15), 750 ng of plasmid that encodes wild type Cas9 or mutant Cas9 (D10A or N863A) and 30 pmol of scrambled siRNA as a negative control or 30 pmol of siRNA against BRCA2 or RAD51. As shown in **Fig. 38**, the majority of the gene conversion events are mediated by BRCA2 and RAD51. These data are consistent with the notion that gene conversion using the HBD gene as a donor in cis is an event mediated by canonical HR.

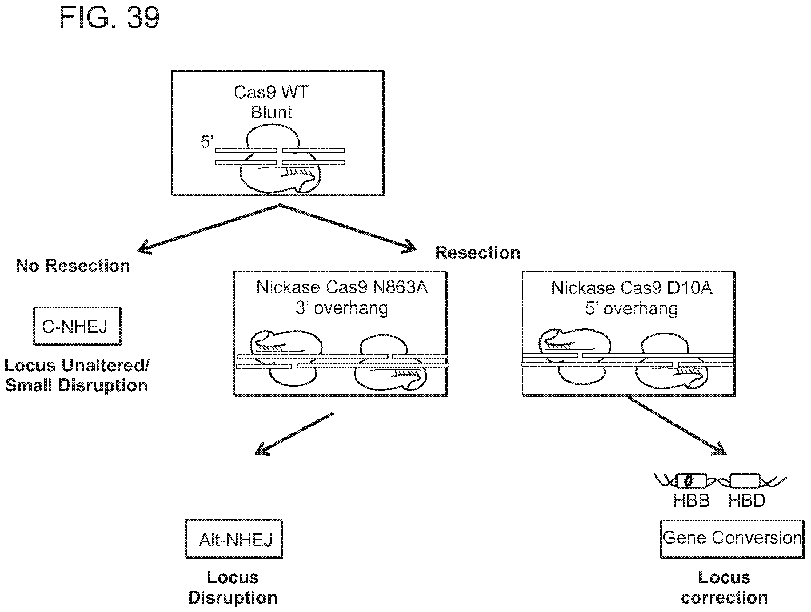

In summary, different DNA ends engage different repair pathways. **Fig. 39** depicts a model of the genetic requirements of the gene conversion pathway in the context of different Cas9 nucleases (wild type, D10A or N863A). Use of a Cas9 nickase with two gRNAs generates either 3' or 5' overhangs, which are most likely not suitable substrates to be repaired by canonical NHEJ, but can be repaired by alternative pathways. The 3' end is predominantly a substrate for repair by the Alt NHEJ. The key player in the pathway is Pol Theta, which refills the ends, thereby generating insertions deriving from the overhangs.

The 5' protruding end is mostly repaired through gene conversion, in which the *HBB* gene is repaired using the homologous *HBD* locus as a template. This pathway is dependent on BRCA2 and RAD51, which suggests the action of the canonical HR pathway.

In conclusion, these data support a therapeutic approach in which correction of the sickle-cell mutation is efficiently mediated through HDR from the endogenous *HBD* gene, and it elucidates strategies of how to promote or suppress this event.

### Example 11: CRISPR/Cas9 RNP supports gene editing of T lymphocytes derived from a Sickle Cell Disease Patient Ex Vivo

To determine whether Cas9 RNP mediated gene editing could correct the sickle cell disease (SCD) mutation, peripheral blood mononuclear cells (MNCs) from a SCD patient were obtained (Conversant Bio). To expand the T lymphocyte fraction from bulk MNCs, MNCs were thawed into T cell culture media (X-VIVO 15 supplemented with human AB serum, N-acetylcysteine, Glutamax (L-glutamine), and human cytokines (IL-2, IL-7, IL-15)). The T lymphocyte fraction was activated with CD3/CD28 immunomagnetic beads. By day 3 of MNC culture in T cell media, the cell population was 72% CD3⁺. After 7 days, a population comprising >98% CD3⁺T lymphocytes was obtained, most of which were also CD4⁺ (**Fig. 40A**). Genomic DNA (gDNA) was extracted from the CD3⁺T lymphocytes and sequenced to confirm the presence of the SCD mutation.

After confirmation of the sickle mutation in the CD3⁺T lymphocytes, gRNA was designed to target the SCD mutation (HBB-8-sickle gRNA targeting sequence: GUAACGGCAGACUUCUCCAC (SEQ ID NO:16318), underline indicates SCD mutation). The gRNA was *in vitro* transcribed from a DNA PCR product template. The HBB-8-sickle was modified to contain a 5' ARCA cap and a 3' polyA tail. Another gRNA HBB-15 (SEQ ID NO:387) was also *in vitro* transcribed and was modified to contain a 5' ARCA cap and a 3' polyA tail. The gRNAs were complexed to *S. pyogenes* Cas9 D10A nickase protein for RNP production.

To evaluate Cas RNP editing in SCD patient cells, 2 x 10⁶ cells were electroporated with Cas9 D10A nickase RNP, in which the nickase was complexed to 2 different gRNA pairs to target the sequence specific to the *HBB* gDNA of SCD patients (HBB-8-sickle and HBB-15). Dual D10A nickase RNP (10µg per 1 x 10⁶ cells) was co-electroporated with a single strand oligonucleotide donor (SSODN, 250 pmoles per 1 x 10⁶ cells). After electroporation, cells were plated onto T cell media with or without CD3/CD28 immunomagnetic beads to determine whether restimulation of T cells after electroporation would improve survival and viability of the gene-edited cells. Re-plating the edited cells into culture with CD3/CD28 beads for cell reactivation improved the total viability of the electroporated cells in comparison to cells plated in T cell media without CD3/CD28 beads (% viability determined by flow cytometry analysis after staining with apoptosis stains 7-AAD and Annexin V; **Fig. 40B**). Gene editing was evaluated by DNA sequencing. The gRNA combination HBB-8-sickle and HBB-15 (each complexed to Cas9 D10A nickase) supported 48% total editing, as detected by T7E1 endonuclease assay analysis of the *HBB* PCR product (**Fig. 40C**). Ten percent higher editing was detected in gDNA from the T lymphocytes that were reactivated with CD3/CD28 beads after electroporation, compared to cells cultured in T cell medial alone. These data show that Cas9 RNP targeting the SCD mutation supports gene editing in SCD patient cells.

### References

Anders et al. Nature 513(7519):569-573 (2014)
Bae et al. Bioinformatics 30(10):1473-1475 (2014)
Caldecott Nat. Rev. Genet. 9(8):619-631 (2008)
Cong et al. Science 399(6121):819-823 (2013)
Chylinski et al. RNA Biol. 10(5):726-737 (2013)
Deveau et al. J. Bacteriol. 190(4): 1390-1400 (2008)
Esvelt et al. Nature 472(7344): 499-503 (2011)
Friedland et al. Genome Biol. 16:257 (2015)
Fu et al. Nat. Biotechnol. 32:279-284 (2014)
Haft et al. PLoS Computational Biology 1(6): e60 (2005)
Heigwer et al. Nat. Methods 11(2): 122-3 (2014)
Horvath et al. Science 327(5962): 167-170 (2010)
Hsu et al. Nat. Biotechnol. 31(9): 827-32 (2013)
Jinek et al. Science 337(6096):816-821 (2012)
Jinek et al. Science 343(6176):1247997 (2014)
Kleinstiver et al. Nat. Biotechnol. 33(12):1293-8 (2015)
Lee et al. Nano Lett. 12(12):6322-6327 (2012)
Li Cell. Res. 18(1):85-98 (2008)
Makarova et al. Nature Review Microbiology 9:467-477 (2011)
Mali et al. Science 399(6121): 823-826 (2013)
Marteijn et al. Nat. Rev. Mol. Cell. Biol. 15(7):465-481 (2014)
Mayle et al. Science 349: 742-47 (2015)
Neelsen and Lopes Nat. Rev. Mol. Cell. Biol. 16: 207-20 (2015)
Nishimasu et al. Cell 156(5):935-949 (2014)
Ran et al. Cell 154(6): 1380-1389 (2013)
Saleh-Gohari et al. Mol. Cell. Biol. 25: 7158-69 (2005)
Schlacher et al. Cancer Cell 22: 106-16 (2012)
Sternberg et al. Nature 507(7490):62-67 (2014)
Wang et al. Cell 153(4):910-918 (2013)
Xiao A. et al.Bioinformatics 30 (8):1180-1182 (2014)
Zellweger et al. J. Cell. Biol. 205: 563-79 (2015)
Zhou et al., Nucleic Acids Res. 42(3):e19 (2014)

## Claims

1. An *in vitro* or *ex vivo* method of modifying an endogenous target gene in a cell, the method comprising:
contacting the cell with a first gRNA molecule, a first enzymatically active Cas9 (eaCas9) molecule, a second gRNA molecule, and a second eaCas9 molecule;
wherein the first gRNA molecule and the first eaCas9 molecule associate with the target gene and generate a first single strand break on a first strand of the target gene;
wherein the second gRNA molecule and the second eaCas9 molecule associate with the target gene and generate a second single strand break on a second strand of the target gene, thereby forming a double strand break having a first overhang and a second overhang; and
wherein the first overhang and the second overhang in the target gene are repaired using an endogenous homologous region,
wherein the cell is not contacted with an exogenous nucleic acid homologous to the target gene, thereby modifying the endogenous target gene in the cell, wherein the target gene is an *HBB* gene; and
wherein the cell is a population of cells, and wherein the first overhang and the second overhang in the target gene are repaired by (a) gene conversion in about 12% to about 45% of the cells in the population of cells; or (b) non-homologous end joining (NHEJ) in less than 40% of the cells in the population of cells. , wherein the method does not encompass a process for modifying the germ line genetic identity of human beings.

2. The method of claim 1, wherein, after repair of the first overhang and the second overhang, the target gene comprises the sequence of the endogenous homologous region.

3. The method of any one of claims 1-2, wherein the first overhang and the second overhang are repaired by homology directed repair (HDR); optionally wherein the HDR is gene conversion.

4. The method of any one of claims 1-3, wherein the first overhang is a 5' overhang, and the second overhang is a 5' overhang; optionally wherein the first overhang and the second overhang undergo processing, exposing a first 3' overhang and a second 3' overhang.

5. The method of any one of claims 1-4, wherein the method is used to correct a mutation in the endogenous target gene, and wherein the mutation is located (a) between the first single strand break and the second single strand break; and/or (b) within fewer than 50 nucleotides of the first single strand break, or within fewer than 50 nucleotides of the second single strand break.

6. The method of any one of claims 1-5, wherein the endogenous target gene has at least 80% sequence homology with the endogenous homologous region.

7. The method of any one of claims 1-6, wherein the method is used to correct a mutation in the endogenous target gene, and wherein the endogenous homologous region does not comprise the mutation.

8. The method of any one of claims 1-7, wherein the endogenous homologous region comprises at least one of: a control region which is homologous to a control region of the target gene; a coding region which is homologous to a coding region of the target gene; a non-coding region which is homologous to a non-coding region of the target gene; an intron which is homologous to an intron of the target gene; or an exon which is homologous to an exon of the target gene.

9. The method of any one of claims 1-8, wherein the first eaCas9 molecule is a first nickase molecule and the second eaCas9 molecule is a second nickase molecule; and/or wherein the first eaCas9 molecule and the second eaCas9 molecule are the same species of eaCas9 molecule; optionally wherein (i) the eaCas9 molecule (a) comprises HNH-like domain cleavage activity but has no N-terminal RuvC-like domain cleavage activity; or (b) is an HNH-like domain nickase; and/or (ii) the eaCas9 molecule comprises a mutation at an amino acid position corresponding to amino acid position D10 of *Streptococcus pyogenes* Cas9.

10. The method of any one of claims 1-9, wherein (a) the first gRNA molecule is a gRNA molecule comprising SEQ ID NO:387, and wherein the second gRNA molecule is a gRNA molecule comprising SEQ ID NO:16318; or (b) the first gRNA molecule is a gRNA molecule comprising any one of SEQ ID NOs: 387-485, 6803-6871, or 16010-16256, and wherein the second gRNA molecule is a gRNA molecule comprising any one of SEQ ID NOs: 387-485, 6803-6871, or 16010-16256; and/or (c) the cell is taken from a patient having sickle cell disease or beta thalassemia; and/or (d) the endogenous homologous region comprises a region of an endogenous *HDB* gene.

11. The method of any one of claims 1-10, wherein the NHEJ results in a deletion in the target gene or wherein the NHEJ results in an insertion of the target gene.

12. The method of any one of claims 1-11, wherein the cell is selected from the group consisting of a blood cell, a neuronal cell, an immune cell, a muscle cell, a stem cell, a progenitor cell, and a diseased cell; and/or wherein the cell is a mammalian cell; and/or a human cell.

13. A composition, comprising.
a first non-naturally occurring gRNA molecule;
a first non-naturally occurring enzymatically active Cas9 (eaCas9) molecule
a second non-naturally occurring gRNA molecule; and
a second non-naturally occurring eaCas9 molecule;
wherein the first gRNA molecule and the first eaCas9 molecule are designed to associate with a target gene and generate a first single strand break on a first strand of the target gene;
wherein the second gRNA molecule and the second eaCas9 molecule are designed to associate with the target gene and generate a second single strand break on a second strand of the target gene, thereby forming a double strand break in the target gene having a first overhang and a second overhang; and
wherein the first overhang and the second overhang in the target gene can be repaired by gene conversion using an endogenous homologous region, wherein the target gene is the *HBB* gene; optionally wherein the first non-naturally occurring eaCas9 molecule is an HNH-like domain nickase, and wherein the second non-naturally occurring eaCas9 molecule is an HNH-like domain nickase.

14. The composition of claim 13, for use as a medicament; or for use in the treatment of sickle cell disease or beta thalassemia.

15. An *in vitro* or *ex vivo* method of modifying a target region of a target gene in a mammalian cell, the method comprising:
generating, within the cell, a first single strand break on a first strand of the target gene and second single strand break on a second strand of the target gene, thereby forming a double strand break in the target gene having a first 5' overhang and a second 5' overhang;
wherein the target region of the target gene is located
(a) between the first single strand break and the second single strand break,
(b) within fewer than 50 nucleotides of the first single strand break, and/or
(c) within fewer than 50 nucleotides of the second single strand break;
wherein the double strand break is repaired by gene conversion,
thereby modifying the target region of the target gene in the mammalian cell, wherein the target gene is an *HBB* gene; optionally wherein (a) the step of generating the first single strand break and the second single strand break comprises contacting the cell with at least one eaCas9 molecule, a first gRNA molecule, and a second gRNA molecule, wherein the first gRNA molecule and the at least one eaCas9 molecule associate with the target gene and generate the first single strand break, and wherein the second gRNA molecule and the at least one eaCas9 molecule associate with the target gene and generate the second single strand break; optionally wherein the at least one eaCas9 molecule is at least one eaCas9 nickase molecule; optionally wherein the at least one eaCas9 nickase molecule is at least one HNH-type nickase molecule. , wherein the method does not encompass a process for modifying the germ line genetic identity of human beings.

16. The method of claim 15, wherein the double strand break is repaired by gene conversion using an endogenous homologous region and wherein the endogenous homologous region is a region of an *HBD* gene.

17. The method of claim 16, wherein (a) the first gRNA molecule comprises a targeting sequence comprising SEQ ID NO: 387 and the second gRNA molecule comprises a targeting sequence comprising SEQ ID NO: 16318; or (b) the first gRNA molecule is a gRNA molecule comprising any one of SEQ ID NOs: 387-485, 6803-6871, or 16010-16256, and wherein the second gRNA molecule is a gRNA molecule comprising any one of SEQ ID NOs: 387-485, 6803-6871, or 16010-16256.

## Patentansprüche

1. In vitro oder ex vivo Verfahren zur Modifizierung eines endogenen Zielgens in einer Zelle, wobei das Verfahren umfasst:
Inkontaktbringen der Zelle mit einem erste gRNA-Molekül, einem ersten enzymatisch aktiven Cas9 (eaCas9)-Molekül, einem zweiten gRNA-Molekül, und einem zweiten eaCas9-Molekül;
wobei das erste gRNA-Molekül und das erste eaCas9-Molekül mit dem Zielgen assoziieren und einen ersten Einzelstrangbruch an einem ersten Strang des Zielgens erzeugen;
wobei das zweite gRNA-Molekül und das zweite eaCas9-Molekül mit dem Zielgen assoziieren und einen zweiten Einzelstrangbruch an einem zweiten Strang des Zielgens erzeugen, wodurch ein Doppelstrangbruch mit einem ersten Überhang und einem zweiten Überhang gebildet wird; und wobei der erste Überhang und der zweite Überhang in dem Zielgen unter Verwendung eines endogenen homologen Bereichs repariert wird,
wobei die Zelle nicht mit einer exogenen Nukleinsäure homolog zu dem Zielgen in Kontakt gebracht wird, wodurch das endogene Zielgen in der Zelle modifiziert wird, wobei das Zielgen ein HBB-Gen ist; und
wobei die Zelle eine Zell-Population ist, und wobei der erste Überhang und der zweite Überhang in dem Zielgen repariert werden durch (a) Genkonversion in etwa 12% bis etwa 45% der Zellen in der Zell-Population; oder durch (b) nicht-homologe Endverbindung (NHEJ) in weniger als 40% der Zellen in der Zell-Population, wobei das Verfahren kein Verfahren zur Modifizierung der genetischen Identität der Keimbahnlinie von Menschen umfasst.

2. Verfahren nach Anspruch 1, wobei nach Reparatur des ersten Überhangs und des zweiten Überhangs, das Zielgen die Sequenz des endogenen homologen Bereichs umfasst.

3. Verfahren nach einem der Ansprüche 1-2, wobei der erste Überhang und der zweite Überhang mittels Homologie gerichteter Reparatur (HDR) repariert werden; wahlweise wobei das HDR Genkonversion ist.

4. Verfahren nach einem der Ansprüche 1-3, wobei der erste Überhang ein 5'-Überhang ist, und der zweite Überhang ein 5'-Überhang ist; wahlweise wobei der erste Überhang und der zweite Überhang prozessiert werden und einen ersten 3'-Überhang und einen zweiten 3'-Überhang aufweisen.

5. Verfahren nach einem der Ansprüche 1-4, wobei das Verfahren dazu verwendet wird, eine Mutation in dem endogenen Zielgen zu korrigieren, und wobei die Mutation angeordnet ist (a) zwischen dem ersten Einzelstrangbruch und dem zweiten Einzelstrangbruch; und/oder (b) innerhalb von weniger als 50 Nukleotiden vom ersten Einzelstrangbruch, oder innerhalb von weniger als 50 Nukleotiden vom zweiten Einzelstrangbruch.

6. Verfahren nach einem der Ansprüche 1-5, wobei das endogene Zielgen mindestens 80% Sequenz-Homologie mit dem endogenen homologen Bereich aufweist.

7. Verfahren nach einem der Ansprüche 1-6, wobei das Verfahren dazu verwendet wird, eine Mutation in dem endogenen Zielgen zu korrigieren, und wobei der endogene homologe Bereich die Mutation nicht enthält.

8. Verfahren nach einem der Ansprüche 1-7, wobei der endogene homologe Bereich mindestens eines umfasst von: einem Kontroll-Bereich, der homolog ist zu einem Kontroll-Bereich des Zielgens; einem kodierenden Bereich, der homolog ist zu einem kodierenden Bereich des Zielgens; einen nicht-kodierenden Bereich, der homolog ist zu einem nicht-kodierenden Bereich des Zielgens; einem Intron, das homolog ist zu einem Intron des Zielgens; oder einem Exon, das homolog ist zu einem Exon des Zielgens.

9. Verfahren nach einem der Ansprüche 1-8, wobei das erste eaCas9-Molekül ein erstes Nickase-Molekül ist und das zweite eaCas9-Molekül ein zweites Nickase-Molekül ist; und/oder wobei das erste eaCas9-Molekül und das zweite eaCas9-Molekül die gleiche Spezies eines eaCas9-Moleküls sind; wahlweise wobei (i) das eaCas9-Molekül (a) eine HNH-ähnliche Domäne Spaltungsaktivität aufweist, jedoch keine N-terminale RuvC-ähnliche Domäne Spaltungsaktivität; oder (b) eine HNH-ähnliche Domäne Nickase ist; und/oder wobei (ii) das eaCas9-Molekül eine Mutation am einer Aminosäureposition umfasst entsprechend der Aminosäureposition D10 von Streptococcus pyogenes Cas9.

10. Verfahren nach einem der Ansprüche 1-9, wobei (a) das erste gRNA-Molekül ein gRNA-Molekül mit der SEQ ID NO:387 ist, und wobei das zweite gRNA-Molekül ein gRNA-Molekül mit der SEQ ID NO:16318 ist; oder wobei (b) das erste gRNA-Molekül ein gRNA-Molekül mit einer von SEQ ID NOs: 387-485, 6803-6871 oder 16010-16256 ist, und wobei das zweite gRNA-Molekül ein gRNA-Molekül mit einer SEQ ID NOs: 387-485, 6803-6871 oder 16010-16256 ist; und/oder wobei (c) die Zelle von einem Patienten mit einer SichelzellenErkrankung oder beta-Thalassämie ist; und/oder wobei (d) der endogene homologe Bereich einen Bereich eines endogen HDB-Gens umfasst.

11. Verfahren nach einem der Ansprüche 1-10, wobei das NHEJ zu einer Entfernung in dem Zielgen führt oder wobei das NHEJ zu einer Insertion das Zielgen führt.

12. Verfahren nach einem der Ansprüche 1-11, wobei die Zelle ausgewählt ist aus der Gruppe bestehend aus einer Blutzelle, einer neuronalen Zelle, einer Immun-Zelle, einer Muskel-Zelle, einer Stamm-Zelle, einer Vorläufer-Zelle, und einer erkrankten Zelle; und/oder wobei die Zelle eine Säugerzelle ist; und/oder ein menschliche Zelle.

13. Zusammensetzung, welche umfasst:
ein erstes, nicht natürlich vorkommendes gRNA-Molekül;
ein erstes, nicht natürlich vorkommendes enzymatisch aktives Cas9 (eaCas9)-Molekül
ein zweites, nicht natürlich vorkommendes gRNA-Molekül; und
ein zweites, nicht natürlich vorkommendes eaCas9-Molekül;
wobei das erste gRNA-Molekül und das erste eaCas9-Molekül ausgestaltet sind, mit einem Zielgen zu assoziieren und einen ersten Einzelstrangbruch an einem ersten Strang des Zielgens zu erzeugen;
wobei das zweite gRNA-Molekül und das zweite eaCas9-Molekül ausgestaltet sind, mit dem Zielgen zu assoziieren und einen zweiten Einzelstrangbruch an einem zweiten Strang des Zielgens zu erzeugen, wodurch ein Doppelstrangbruch in dem Zielgen mit einem ersten Überhang und einem zweiten Überhang erzeugt wird; und
wobei der erste Überhang und der zweite Überhang in dem Zielgen repariert werden kann durch Genkonversion unter Verwendung eines endogenen homologen Bereichs, wobei das Zielgen das HBB-Gen ist; wahlweise wobei das erste nicht natürlich vorkommende eaCas9-Molekül eine HNH-ähnliche Domäne Nickase ist, und wobei das zweite nicht natürlich vorkommende eaCas9-Molekül eine HNH-ähnliche Domäne Nickase ist.

14. Zusammensetzung nach Anspruch 13, zur Verwendung als ein Medikament, oder zur Verwendung bei der Behandlung einer Sichelzellenerkrankung oder beta-Thalassämie.

15. In vitro oder ex vivo Verfahren zur Modifizierung eines Zielbereichs eines Zielgens in einer Säugerzelle, wobei das Verfahren umfasst:
Erzeugen, in der Zelle, eines ersten Einzelstrangbruchs an einem ersten Strang des Zielgens und eines zweiten Einzelstrangbruchs an einem zweiten Strang des Zielgens, wodurch ein Doppelstrangbruch in dem Zielgen mit einem ersten 5'-Überhang und einem zweiten 5'-Überhang gebildet wird;
wobei der Zielbereich des Zielgens angeordnet ist,
(a) zwischen dem ersten Einzelstrangbruch und dem zweiten Einzelstrangbruch,
(b) innerhalb weniger als 50 Nucleotiden von dem ersten Einzelstrangbruch, und/oder
(c) innerhalb weniger als 50 Nucleotiden von dem zweiten Einzelstrangbruch;
wobei der Doppelstrangbruch repariert wird durch Genkonversion, wodurch der Zielbereich des Zielgens in der Säugerzelle modifiziert wird, wobei das Zielgen ein HBB-Gen ist; wahlweise wobei (a) der Schritt der Erzeugung des ersten Einzelstrangbruchs und des zweiten Einzelstrangbruchs umfasst, Inkontaktbringen der Zelle mit mindestens einem eaCas9-Molekül, einem ersten gRNA-Molekül, und einem zweiten gRNA-Molekül, wobei das erste gRNA-Molekül und das mindestens eine eaCas9-Molekül mit dem Zielgen assoziieren und den ersten Einzelstrangbruch erzeugen, und wobei das zweite gRNA-Molekül und das mindestens eine eaCas9-Molekül mit dem Zielgen assoziieren und den zweiten Einzelstrangbruch erzeugen; wahlweise wobei das mindestens eine eaCas9-Molekül mindestens ein eaCas9 Nickase-Molekül ist; wahlweise wobei das mindestens eine eaCas9 Nickase-Molekül mindestens ein HNH-Typ Nickase-Molekül ist, wobei das Verfahren kein Verfahren zur Modifizierung der genetischen Identität der Keimbahnlinie von Menschen umfasst.

16. Verfahren nach Anspruch 15, wobei der Doppelstrangbruch repariert wird durch Genkonversion unter Verwendung eines endogenen homologen Bereichs und wobei der endogene homologe Bereich ein Bereich eines HBD-Gens ist.

17. Verfahren nach Anspruch 16, wobei (a) das erste gRNA-Molekül eine Zielsequenz mit der SEQ ID NO: 387 umfasst und das zweite gRNA-Molekül eine Zielsequenz mit der SEQ ID NO: 16318 umfasst; oder wobei (b) das erste gRNA-Molekül ein gRNA-Molekül mit einer SEQ ID NOs: 387-485, 6803-6871 oder 16010-16256 ist, und wobei das zweite gRNA-Molekül ein gRNA-Molekül mit einer SEQ ID NOs: 387-485, 6803-6871, oder 16010-16256 ist.

## Revendications

1. Procédé *in vitro* ou ex *vivo* de modification d'un gène cible endogène dans une cellule, le procédé comprenant :
mettre en contact la cellule avec une première molécule d'ARNg, une première molécule Cas9 enzymatiquement active (eaCas9), une seconde molécule d'ARNg et une seconde molécule eaCas9 ;
dans lequel la première molécule d'ARNg et la première molécule eaCas9 s'associent au gène cible et génèrent une première cassure simple brin sur un premier brin du gène cible ;
dans lequel la seconde molécule d'ARNg et la seconde molécule eaCas9 s'associent au gène cible et génèrent une seconde cassure simple brin sur un second brin du gène cible, formant ainsi une cassure double brin ayant un premier dépassement et un deuxième dépassement ; et
dans lequel le premier dépassement et le second dépassement dans le gène cible sont réparés à l'aide d'une région homologue endogène,
dans lequel la cellule n'est pas mise en contact avec un acide nucléique exogène homologue au gène cible, modifiant ainsi le gène cible endogène dans la cellule, dans lequel le gène cible est un gène *HBB* ; et
dans lequel la cellule est une population de cellules, et dans lequel le premier dépassement et le second dépassement dans le gène cible sont réparés par (a) conversion génique dans environ 12 % à environ 45 % des cellules dans la population de cellules ; ou (b) jonction d'extrémité non homologue (NHEJ) dans moins de 40 % des cellules dans la population de cellules, dans lequel le procédé ne comprend pas un procédé de modificiation de l'identité génétique de la lignée germinale des êtres humains.

2. Procédé selon la revendication 1, dans lequel, après réparation du premier dépassement et du second dépassement, le gène cible comprend la séquence de la région homologue endogène.

3. Procédé selon l'une quelconque des revendications 1 à 2, dans lequel le premier dépassement et le second dépassement sont réparés par réparation dirigée par homologie (HDR) ; facultativement dans lequel la HDR est une conversion génique.

4. Procédé selon l'une quelconque des revendications 1 à 3, dans lequel le premier dépassement est un dépassement en 5' et le second dépassement est un dépassement en 5' ; facultativement dans lequel le premier dépassement et le second dépassement subissent un traitement, exposant un premier dépassement en 3' et un second dépassement en 3'.

5. Procédé selon l'une quelconque des revendications 1 à 4, dans lequel le procédé est utilisé pour corriger une mutation dans le gène cible endogène, et dans lequel la mutation est située (a) entre la première cassure simple brin et la seconde cassure simple brin ; et/ou (b) à moins de 50 nucléotides de la première cassure simple brin, ou à moins de 50 nucléotides de la seconde cassure simple brin.

6. Procédé selon l'une quelconque des revendications 1 à 5, dans lequel le gène cible endogène a au moins 80 % d'homologie de séquence avec la région homologue endogène.

7. Procédé selon l'une quelconque des revendications 1 à 6, dans lequel le procédé est utilisé pour corriger une mutation dans le gène cible endogène, et dans lequel la région homologue endogène ne comprend pas la mutation.

8. Procédé selon l'une quelconque des revendications 1 à 7, dans lequel la région homologue endogène comprend au moins l'une parmi : une région de contrôle qui est homologue à une région de contrôle du gène cible ; une région codante qui est homologue à une région codante du gène cible ; une région non codante qui est homologue à une région non codante du gène cible ; un intron qui est homologue à un intron du gène cible ; ou un exon qui est homologue à un exon du gène cible.

9. Procédé selon l'une quelconque des revendications 1 à 8, dans lequel la première molécule eaCas9 est une première molécule de nickase et la seconde molécule eaCas9 est une seconde molécule de nickase ; et/ou dans lequel la première molécule eaCas9 et la seconde molécule eaCas9 sont la même espèce de molécule eaCas9 ; facultativement dans lequel (i) la molécule eaCas9 (a) comprend une activité de clivage de domaine de type HNH mais n'a pas d'activité de clivage de domaine de type RuvC N-terminal ; ou (b) est une nickase de domaine de type HNH ; et/ou (ii) la molécule eaCas9 comprend une mutation à une position d'acide aminé correspondant à la position d'acide aminé D10 de Cas9 de *Streptococcus pyogenes.*

10. Procédé selon l'une quelconque des revendications 1 à 9, dans lequel (a) la première molécule d'ARNg est une molécule d'ARNg comprenant SEQ ID NO : 387, et dans lequel la seconde molécule d'ARNg est une molécule d'ARNg comprenant SEQ ID NO : 16318 ; ou (b) la première molécule d'ARNg est une molécule d'ARNg comprenant l'une quelconque des SEQ ID NO : 387-485, 6803-6871 ou 16010-16256, et dans lequel la seconde molécule d'ARNg est une molécule d'ARNg comprenant l'une quelconque des SEQ ID NO : 387-485, 6803-6871 ou 16010-16256 ; et/ou (c) la cellule est prélevée d'un patient souffrant de drépanocytose ou de bêta-thalassémie ; et/ou (d) la région homologue endogène comprend une région d'un gène *HDB* endogène.

11. Procédé selon l'une quelconque des revendications 1 à 10, dans lequel le NHEJ entraîne une délétion dans le gène cible ou dans lequel le NHEJ entraîne une insertion du gène cible.

12. Procédé selon l'une quelconque des revendications 1 à 11, dans lequel la cellule est choisie dans le groupe consistant en une cellule sanguine, une cellule neuronale, une cellule immunitaire, une cellule musculaire, une cellule souche, une cellule progénitrice et une cellule malade ; et/ou dans lequel la cellule est une cellule de mammifère ; et/ou une cellule humaine.

13. Composition, comprenant :
une première molécule d'ARNg non naturelle ;
une première molécule Cas9 enzymatiquement active (eaCas9) non naturelle ;
une seconde molécule d'ARNg non naturelle ; et
une seconde molécule eaCas9 non naturelle ;
dans laquelle la première molécule d'ARNg et la première molécule eaCas9 sont conçues pour s'associer à un gène cible et générer une première cassure simple brin sur un premier brin du gène cible ;
dans laquelle la seconde molécule d'ARNg et la seconde molécule eaCas9 sont conçues pour s'associer au gène cible et générer une seconde cassure simple brin sur un second brin du gène cible, formant ainsi une cassure double brin dans le gène cible ayant un premier dépassement et un second dépassement ; et
dans laquelle le premier dépassement et le second dépassement dans le gène cible peuvent être réparés par conversion génique à l'aide d'une région homologue endogène, dans laquelle le gène cible est le gène *HBB ;* facultativement dans laquelle la première molécule eaCas9 non naturelle est une nickase de domaine de type HNH, et dans laquelle la seconde molécule eaCas9 non naturelle est une nickase de domaine de type HNH.

14. Composition selon la revendication 13, pour une utilisation en tant que médicament ; ou pour une utilisation dans le traitement de la drépanocytose ou de la bêta-thalassémie.

15. Procédé *in vitro* ou *ex vivo* de modification d'une région cible d'un gène cible dans une cellule de mammifère, le procédé comprenant :
générer, à l'intérieur de la cellule, une première cassure simple brin sur un premier brin du gène cible et une seconde cassure simple brin sur un second brin du gène cible, formant ainsi une cassure double brin dans le gène cible ayant un premier dépassement en 5' et un second dépassement en 5' ;
dans lequel la région cible du gène cible est située
(a) entre la première cassure simple brin et la seconde cassure simple brin,
(b) à moins de 50 nucléotides de la première cassure simple brin, ou
(c) à moins de 50 nucléotides de la seconde cassure simple brin ;
dans lequel la cassure double brin est réparée par conversion génique,
modifiant ainsi la région cible du gène cible dans la cellule de mammifère,
dans lequel le gène cible est un gène *HBB* ; facultativement dans lequel (a) l'étape de génération de la première cassure simple brin et de la seconde cassure simple brin comprend mettre en contact la cellule avec au moins une molécule eaCas9, une première molécule d'ARNg et une seconde molécule d'ARNg, dans lequel la première molécule d'ARNg et la ou les molécules eaCas9 s'associent au gène cible et génèrent la première cassure simple brin, et dans lequel la seconde molécule d'ARNg et la ou les molécules eaCas9 s'associent au gène cible et génèrent la seconde cassure simple brin ; facultativement dans lequel la au moins une molécule eaCas9 est au moins une molécule de nickase eaCas9 ; facultativement dans lequel la au moins une molécule de nickase eaCas9 est une molécule de nickase de type HNH, dans lequel le procédé ne comprend pas un procédé pour modifier l'identité génétique de la lignée germinale des êtres humains.

16. Procédé selon la revendication 15, dans lequel la cassure double brin est réparée par conversion génique à l'aide d'une région homologue endogène et dans lequel la région homologue endogène est une région d'un gène *HBD.*

17. Procédé selon la revendication 16, dans lequel (a) la première molécule d'ARNg comprend une séquence de ciblage comprenant SEQ ID NO : 387 et la seconde molécule d'ARNg comprend une séquence de ciblage comprenant SEQ ID NO : 16318 ; ou (b) la première molécule d'ARNg comprend une séquence de ciblage comprenant l'une quelconque des SEQ ID NO : 387-485, 6803-6871 ou 16010-16256, et dans lequel la seconde molécule d'ARNg est une molécule d'ARNg comprenant l'une quelconque des SEQ ID NO : 387-485, 6803-6871 ou 16010-16256.
